# EUROPEAN PATENT APPLICATION

(11) **EP 4 821 351 A1**
(43) Date of publication of application: **14.10.2026**
(21) Application number: 24923129.1
(22) Date of filing: 17.04.2024
(51) Int. Cl.: A01K 67/027, C07K 16/46, C07K 16/00, C12N 15/13, C12N 15/85, C12N 5/18, C12N 5/10

(54) **GENETICALLY MODIFIED MOUSE FOR PREPARING ANTIBODY AND PREPARATION METHOD THEREFOR**

(30) Priority: 07.02.2024 WO PCT/CN2024/076606
(71) Applicant: Cyagen Biosciences (Suzhou) Inc., Suzhou, Jiangsu 215400 (CN)
(72) Inventor: HAN, Lanqing, Suzhou, Jiangsu 215400 (CN); SU, Cui, Suzhou, Jiangsu 215400 (CN); MOU, Xing, Suzhou, Jiangsu 215400 (CN); WANG, Kang, Suzhou, Jiangsu 215400 (CN); LI, Xiaoliang, Suzhou, Jiangsu 215400 (CN); ZHANG, Liang, Suzhou, Jiangsu 215400 (CN); CHEN, Xiangyun, Suzhou, Jiangsu 215400 (CN); ZHOU, Shun, Suzhou, Jiangsu 215400 (CN); HUO, Kugeng, Suzhou, Jiangsu 215400 (CN)
(74) Representative: Sun, Yiming
(86) International application number: PCT/CN2024/088168
(87) International publication number: WO 2025/166904

(57) **Abstract**

Disclosed in the present invention is genetically modified mice in which immunoglobulin loci are modified to insert gene segments of a human immunoglobulin variable region. The mice can be bred normally and produce human-mouse chimeric antibodies including a human variable region and a mouse constant region. The present invention also provides a method for preparing the genetically modified mice and use of the mice.

## Description

### TECHNICAL FIELD

The present invention relates to genetically modified mice, cells, embryos, and tissues. In particular, the present invention relates to mice in which an immunoglobulin heavy chain variable region locus, a Kappa light chain variable region locus, and a Lambda light chain locus are all humanized and a method for preparing the mice. The present invention also relates to a genome of the modified mice, a cell and tissue including the genome, and a method for preparing a monoclonal antibody by using the mice, and use. The present invention also relates to mice having a modified genome.

### BACKGROUND

Humanized mice exhibit a fully functional humoral immune system that is essentially indistinguishable from that of wild type mice. They show normal cell populations at all stages of B cell development. They exhibit normal lymphoid organ morphology. Antibody sequences of the mice exhibit normal V(D)J rearrangements and normal somatic hypermutation frequencies. The antibody populations in these mice reflect the isotype distribution caused by normal isotype switching (such as normal isotype cis-switching). Immunization of mice results in a potent humoral immune response that generates a large and diverse antibody lineage with human immunoglobulin variable regions suitable for use as therapeutic candidates.

The precise replacement of mouse immunoglobulin variable sequences with human immunoglobulin variable sequences enables the formation of genetically modified or transgenic mice. However, because of the divergent evolution of immunoglobulin loci between mice and humans, even through sequential recombineering of very large human immunoglobulin sequence segments to precisely replace endogenous mouse immunoglobulin sequences at heavy and light chain loci with corresponding human immunoglobulin sequences, certain problems may arise. For example, intergenic sequences interspersed within immunoglobulin loci are not identical between mice and humans, and in some cases may not be functionally equivalent. Differences between mice and humans in their immunoglobulin loci may still lead to abnormalities in humanized mice, particularly when certain portions of endogenous mouse immunoglobulin heavy chain loci are humanized or otherwise manipulated. Some modifications at mouse immunoglobulin heavy chain loci are deleterious, e.g., a loss of the ability of the modified mice to mate and produce offspring. It has been found that the decrease or disappearance of fertility in male mice is associated with impairment of an Adam6 gene.

An ADAM6 protein is a member of an ADAM protein family, where ADAM is an acronym for ADisintegrin And Metalloprotease. The ADAM protein family is large and diverse and has diverse functions including cell adhesion. Some members of the ADAM family are involved in spermatogenesis and fertilization. For example, ADAM2 encodes a subunit of a protein fertilin that is involved in sperm-egg interactions. ADAM3 or cyritestin appears to be required for binding of sperms to a zona pellucida. The absence of ADAM2 or ADAM3 will result in infertility. It has been hypothesized that ADAM2, ADAM3 and ADAM6 form a complex on the surfaces of mouse sperm cells. A human ADAM6 gene is located between human VH genes VH1-2 and VH6-1. In mice, there are two ADAM6 genes, ADAM6a and ADAM6b, present in an intergenic region between mouse VH and DH gene segments, and in mice, the direction of transcription of ADAM6a and ADAM6b genes is opposite to that of surrounding immunoglobulin gene segments.

CN105861548B discloses ADAM6 mice, wherein one or more human immunoglobulin gene sequences are inserted at an immunoglobulin heavy chain locus in a germline of the mice, wherein the insertion disrupts the function of an endogenous ADAM6 gene; then, a nucleic acid sequence encoding a mouse ADAM6a protein and a nucleic acid sequence encoding a mouse ADAM6b protein are inserted into the germline of the mice, wherein the mouse ADAM6a protein and the mouse ADAM6b protein are expressed by the nucleic acid sequences, wherein the mouse ADAM6a protein and the mouse ADAM6b protein are expressed from the nucleic acid sequences and improve or restore fertility when expressed in male mice. Disruption of

the endogenous ADAM6 gene is undesirable and may have an impact on the long-term reproductive ability of the mice.

In view of this, the present invention provides a heavy chain locus modified mouse genome that maintains the endogenous ADAM6 gene and its function and can produce human-mouse chimeric antibodies while maintaining fertility.

### SUMMARY

In one aspect, the present invention provides a method for preparing genetically modified mice, including:
(i) obtaining heavy chain locus modified mice, including
   (ia) inserting a first partial segment of a human immunoglobulin heavy chain variable region locus between an mIgHJ region and an mIgHC region of an immunoglobulin heavy chain locus of a first mouse, wherein the first partial segment includes a first partial hIgHV contiguous segment, a whole hIgHD segment and a whole hIgHJ segment, the first partial segment does not include a segment between downstream of an hIgHV1-2 gene and upstream of an hIgHV6-1 gene, and first recombination sites are included upstream of the first partial segment and downstream of the mIgHJ region;
   (ib) inserting a second partial segment of the human immunoglobulin heavy chain variable region locus between an mIgHJ region and an mIgHC region of an immunoglobulin heavy chain locus of a second mouse, wherein the second partial segment is located upstream of the first partial segment, the second partial segment includes a second partial hIgHV contiguous segment, and second recombination sites are included between downstream of the second partial hIgHV contiguous segment and upstream of the mIgHC region;
   (ic) hybridizing the first mouse with the second mouse, screening a third mouse, and inserting the second partial segment of the human immunoglobulin heavy chain variable region locus and the first partial segment downstream of the second partial segment between an mIgHJ region and an mIgHC region of an immunoglobulin heavy chain locus of the third mouse, wherein third recombination sites are present between the second partial segment and the first partial segment; and
   (id) respectively knocking out an mIgHV5-1 gene and a whole mIgHV segment upstream of the mIgHV5-1 gene, a contiguous segment between an mIgHD1-1 gene and an mIgHJ4 gene, and mIgHD3-1, mIgHD5-1, and mIgHD1-3 genes of the immunoglobulin heavy chain locus of the third mouse to obtain the heavy chain locus genetically modified mice;
(ii) obtaining Kappa light chain locus modified mice, wherein immunoglobulin Kappa light chain loci of the Kappa light chain locus modified mice sequentially include a whole hIgKV segment, a whole hIgKJ segment and an mIgKC segment, and a whole mIgKV segment and a whole mIgKJ segment are knocked out;
(iii) obtaining Lambda light chain locus modified mice, wherein immunoglobulin Lambda light chain loci of the Lambda light chain locus modified mice sequentially include a whole hIgLV segment and a whole hIgLJ-C segment, and a whole mIgLV segment, an mIgLJ segment, and a whole mIgLC segment are knocked out; and
(iv) mating the modified mice obtained in the steps (i), (ii) and (iii), screening triple positive mice, mating the obtained male and female triple positive mice with each other, and screening homozygous mice, wherein preferably, the modified mice obtained in the steps (i) and (ii) are mated and screening is performed to obtain first double positive homozygous mice, and the modified mice obtained in the steps (i) and (iii) are mated and screening is performed to obtain second double positive homozygous mice, and the first double positive homozygous mice and the second double positive homozygous mice are mated with each other and screening is performed to obtain triple positive homozygous mice.

In the present invention, the first partial hIgHV contiguous segment of the first partial segment of the human immunoglobulin heavy chain variable region locus may start from a gene (including a functional gene, a pseudogene or an ORF) of any one hIgHV segment upstream of the hIgHV1-2 gene, and end at the hIgHV1-2 gene.

In an embodiment, the gene of any one hIgHV segment upstream of the hIgHV1-2 gene may be any one functional gene, pseudogene or ORF between an hIgHV (III)-82 gene and the hIgHV1-2 gene. In one embodiment, the gene of any one hIgHV segment upstream of the hIgHV1-2 gene is a functional gene, such as hIgHV3-74, hIgHV3-73, hIgHV3-72, hIgHV2-70, hIgHV1-69D, hIgHV1-69-2, hIgHV2-70D, hIgHV1-69, hIgHV3-66, hIgHV3-64, hIgHV3-62, hIgHV4-61, hIgHV4-59, hIgHV1-58, hIgHV3-53, hIgHV5-51, hIgHV3-49, hIgHV3-48, hIgHV1-46, hIgHV1-45, hIgHV3-43, hIgHV4-39, hIgHV3-43D, hIgHV4-38-2, hIgHV3-35, hIgHV4-34, hIgHV3-33, hIgHV4-31, hIgHV3-30-5, hIgHV4-30-4, hIgHV3-30-3, hIgHV4-30-2, hIgHV4-30-1, hIgHV3-30, hIgHV4-28, hIgHV2-26, hIgHV1-24, hIgHV3-23D, hIgHV3-23, hIgHV3-21, hIgHV3-20, hIgHV1-18, hIgHV3-15, hIgHV3-13, hIgHV3-11, hIgHV5-10-1, hIgHV3-9, hIgHV3-64D, hIgHV3-8, hIgHV3-7, hIgHV2-5, hIgHV7-4-1, hIgHV4-4 or hIgHV1-3.

In one embodiment, the first partial hIgHV contiguous segment of the first partial segment starts from hIgHV3-74, hIgHV3-73, hIgHV3-72, hIgHV2-70, hIgHV1-69D, hIgHV1-69-2, hIgHV2-70D, hIgHV1-69, hIgHV3-66, hIgHV3-64, hIgHV3-62, hIgHV4-61, hIgHV4-59, hIgHV1-58, hIgHV3-53, hIgHV5-51, hIgHV3-49, hIgHV3-48, hIgHV1-46, hIgHV1-45, hIgHV3-43, hIgHV4-39, hIgHV3-43D, hIgHV4-38-2, hIgHV3-35, hIgHV4-34, hIgHV3-33, hIgHV4-31, hIgHV3-30-5, hIgHV4-30-4, hIgHV3-30-3, hIgHV4-30-2, hIgHV4-30-1, hIgHV3-30, hIgHV4-28, hIgHV2-26, hIgHV1-24, hIgHV3-23D, hIgHV3-23, hIgHV3-21, hIgHV3-20, hIgHV1-18, hIgHV3-15, hIgHV3-13, hIgHV3-11, hIgHV5-10-1, hIgHV3-9, hIgHV3-64D, hIgHV3-8, hIgHV3-7, hIgHV2-5, hIgHV7-4-1, hIgHV4-4 or hIgHV1-3, and ends at the hIgHV1-2 gene, both containing the described gene as an end. In an embodiment, the first partial hIgHV contiguous segment of the first partial segment is a contiguous segment between an hIgHV4-28 gene and the hIgHV1-2 gene.

In the present invention, the second partial segment of the heavy chain variable region locus is located upstream of the first partial segment of the heavy chain variable region locus, and the second partial hIgHV contiguous segment may start from any one gene (including a functional gene, a pseudogene or an ORF) of the hIgHV segment and end at a gene (including a functional gene, a pseudogene or an ORF) of any one hIgHV segment upstream of the start gene of the first partial hIgHV contiguous segment. In some embodiments, the second partial segment and the first partial segment together constitute all functional genes upstream of hIgHV1-2 and a contiguous segment between the functional genes, i.e., a contiguous segment between the hIgHV3-74 gene and the hIgHV1-2 gene. For example, in some embodiments, the second partial hIgHV contiguous segment includes or is a contiguous segment between the hIgHV3-74 gene and the hIgHV3-30 gene, and the first partial hIgHV contiguous stretch includes or is a contiguous segment between the hIgHV4-28 gene and the hIgHV1-2 gene.

In some embodiments, immunoglobulin heavy chain loci of the obtained genetically modified mice sequentially include: (i) an Adam6a gene of the mice; (ii) an Adam6b gene of the mice; (iii) a contiguous segment between an hIgHV3-74 gene and an hIgHV3-30 gene; (iv) a contiguous segment between an hIgHV4-28 gene and the hIgHV1-2 gene; (v) a contiguous segment between the hIgHV6-1 gene and an hIgHJ6 gene; and (vi) an mIgHC region.

In some embodiments, in the step (ia), the first partial hIgHV contiguous segment, the whole hIgHD segment and the whole hIgHJ segment of the first partial segment are inserted between the mIgHJ region and the mIgHC region by at least two steps including:
(ia1) inserting a first contiguous segment included between the hIgHV6-1 gene and an hIgHJ6 gene between the mIgHJ region and the mIgHC region; and
(ia2) inserting a second contiguous segment included between an hIgHV4-28 gene and the hIgHV1-2 gene upstream of the first contiguous segment.

In some embodiments, the step (ia1) specifically includes:
(ia11) inserting a contiguous segment included between an hIgHD6-25 gene and the hIgHJ6 gene between the mIgHJ region and the mIgHC region;
(ia12) inserting a contiguous segment included between an hIgHD6-13 gene and an hIgHD5-24 gene upstream of the inserted segment in the step (ia11);
(ia13) inserting a contiguous segment included between an hIgHD1-1 gene and an hIgHD5-12 gene upstream of the inserted segment in the step (ia12); and
(ia14) inserting a contiguous segment included between the hIgHV6-1 gene and the hIgHD1-1 gene, and loxP and lox2272-PB5' sites upstream of the inserted segment in the step (ia13).

In some embodiments, the step (ia2) specifically includes:
(ia21) recombining a BAC vector including the second contiguous segment between the hIgHV4-28 gene and the hIgHV1-2 gene, and an loxP site and an lox2272 site which are located at both ends of the second contiguous segment and in the same direction as that in the step (ia14), and a Cre recombinase with a genome obtained in the step (ia1), wherein PB3' is included between the lox2272 site and the second contiguous segment; and
(ia22) contacting a recombinant genome obtained in the step (ia21) with a PiggyBac transposase, and screening a genome including the first partial segment and the loxP site located upstream of the first partial segment.

In some embodiments, in the step (ib), the second partial hIgHV contiguous segment of the second partial segment is inserted upstream of the first partial segment by at least the following steps including:
(ib1) inserting a contiguous segment included between the hIgHV3-74 gene and an hIgHV3-72 gene between the mIgHJ region and the mIgHC region;
(ib2) inserting a contiguous segment included between an hIgHV2-70 gene and an hIgHV1-69D gene, and PB3'-lox5171 and loxP sites downstream of the inserted segment in the step (ib1);
(ib3) recombining a BAC vector including a contiguous segment between an hIgHV1-69-2 gene and the hIgHV3-30 gene, and loxP and lox5171 which are located at both ends and in the same direction as that in the step (ib2), and a Cre recombinase with a genome obtained in the step (ib2), wherein PB5' is included between the lox5171 site and the contiguous segment described in this step; and
(ib4) contacting a recombinant genome obtained in the step (ib3) with a PiggyBac transposase, and screening a genome including the second partial segment and the loxP site located downstream of the second partial segment.

In some embodiments, the step (ic) includes:
(ic1) screening Cre-positive mice including the first partial segment and the second partial segment; and
(ic2) mating the mice obtained in the step (ic1) with wild type mice, and screening mice that do not carry Cre but include the first partial segment and the second partial segment, wherein loxP sites are present between the second partial segment and the first partial segment.

In some embodiments, the step (id) includes:
(id1) mating the third mouse with a wild type mouse, and screening positive mice; and
(id2) mating the male and female positive mice obtained in the step (id1) with each other, and screening homozygous mice.

In some embodiments, the step (ii) includes:
(iia) inserting a first partial segment of a human immunoglobulin Kappa light chain variable region locus between an mIgKJ region and an mIgKC region of an immunoglobulin Kappa light chain locus of a fourth mouse, wherein the first partial segment includes a first partial hIgKV contiguous segment and a whole hIgKJ segment, and fourth recombination sites are included upstream of the first partial segment and downstream of the mIgKJ region;
(iib) inserting a second partial segment of the human immunoglobulin Kappa light chain variable region locus between an mIgKJ region and an mIgKC region of an immunoglobulin Kappa light chain locus of a fifth mouse, wherein the second partial segment is located upstream of the first partial segment, the second partial segment includes a second partial hIgKV contiguous segment, the first partial hIgKV contiguous segment and the second partial hIgKV contiguous segment together constitute the whole hIgKV segment, and fifth recombination sites are included between downstream of the second partial hIgKV contiguous segment and upstream of the mIgKC region;
(iic) hybridizing the fourth mouse with the fifth mouse, screening a sixth mouse, and inserting the second partial segment of the human immunoglobulin Kappa light chain variable region locus and the first partial segment downstream of the second partial segment between an mIgKJ region and an mIgKC region of an immunoglobulin Kappa light chain locus of the sixth mouse, wherein sixth recombination sites are present between the second partial segment and the first partial segment; and
(iid) knocking out a whole mIgKV segment and a whole mIgKJ segment of the immunoglobulin Kappa light chain locus of the sixth mouse to obtain the Kappa light chain locus modified mice.

In the present invention, the first partial hIgKV contiguous segment of the first partial segment of the human immunoglobulin Kappa light chain variable region locus can start from a gene (including a functional gene, a pseudogene or an ORF) of any one hIgKV segment upstream of an hIgKV4-1 gene and end at the hIgKV4-1 gene.

In one embodiment, the gene of any one hIgKV segment upstream of the hIgKV4-1 gene may be any one functional gene, pseudogene or ORF between an hIgKV3D-7 gene and the hIgKV4-1 gene. In one embodiment, the gene of any one hIgKV segment upstream of the hIgKV4-1 gene is a functional gene or an ORF, such as hIgKV3D-7, hIgKV1D-8, hIgKV1D-43, hIgKV1D-42, hIgKV3D-11, hIgKV1D-12, hIgKV1D-13, hIgKV3D-15, hIgKV1D-16, hIgKV1D-17, hIgKV6D-41, hIgKV3D-20, hIgKV6D-21, hIgKV2D-24, hIgKV2D-26, hIgKV2D-28, hIgKV2D-29, hIgKV2D-30, hIgKV1D-33, hIgKV1D-37, hIgKV1D-39, hIgKV2D-40, hIgKV2-40, hIgKV1-39, hIgKV1-37, hIgKV1-33, hIgKV2-30, hIgKV2-29, hIgKV2-28, hIgKV2-27, hIgKV2-24, hIgKV6-21, hIgKV3-20, hIgKV1-17, hIgKV1-16, hIgKV3-15, hIgKV1-13, hIgKV1-12, hIgKV3-11, hIgKV1-9, hIgKV1-8, hIgKV3-7, hIgKV1-6, hIgKV1-5 or hIgKV5-2.

In one embodiment, the first partial hIgKV contiguous segment of the first partial segment starts from hIgKV3D-7, hIgKV1D-8, hIgKV1D-43, hIgKV1D-42, hIgKV3D-11, hIgKV1D-12, hIgKV1D-13, hIgKV3D-15, hIgKV1D-16, hIgKV1D-17, hIgKV6D-41, hIgKV3D-20, hIgKV6D-21, hIgKV2D-24, hIgKV2D-26, hIgKV2D-28, hIgKV2D-29, hIgKV2D-30, hIgKV1D-33, hIgKV1D-37, hIgKV1D-39, hIgKV2D-40, hIgKV2-40, hIgKV1-39, hIgKV1-37, hIgKV1-33, hIgKV2-30, hIgKV2-29, hIgKV2-28, hIgKV2-27, hIgKV2-24, hIgKV6-21, hIgKV3-20, hIgKV1-17, hIgKV1-16, hIgKV3-15, hIgKV1-13, hIgKV1-12, hIgKV3-11, hIgKV1-9, hIgKV1-8, hIgKV3-7, hIgKV1-6, hIgKV1-5 or hIgKV5-2, and ends at the hIgKV4-1 gene, both containing the described gene as an end. In one embodiment, the first partial hIgKV contiguous segment of the first partial segment is a contiguous segment between an hIgKV1-37 gene and the hIgKV4-1 gene.

In the present invention, the second partial segment of the Kappa light chain variable region locus is located upstream of the first partial segment of the Kappa light chain variable region locus, and the second partial hIgKV contiguous segment may start from any one gene (including a functional gene, a pseudogene or an ORF) of the hIgKV segment and end at a gene (including a functional gene, a pseudogene or an ORF) of any one hIgKV segment upstream of the start gene of the first partial hIgKV contiguous segment. In some embodiments, the second partial segment and the first partial segment together constitute all functional genes upstream of hIgKV4-1 and a contiguous segment between the functional genes, i.e., a contiguous segment between the hIgKV4-1 gene and the hIgKV3D-7 gene. For example, in some embodiments, the second partial hIgKV contiguous segment includes or is a contiguous segment between the hIgKV3D-7 gene and the hIgKV2D-40 gene, and the first partial hIgKV contiguous segment includes or is a contiguous segment between the hIgKV1-37 gene and the hIgKV4-1 gene.

In some embodiments, wherein in the step (iia), the first partial hIgKV contiguous segment and the whole hIgKJ segment of the first partial segment are inserted between the mIgKJ region and the mIgKC region by at least two steps including:
(iia1) inserting a first contiguous segment included between an hIgKV1-5 gene and an hIgKJ5 gene between the mIgKJ region and the mIgKC region; and
(iia2) inserting a second contiguous segment included between an hIgKV1-37 and an hIgKV1-6 gene upstream of the first contiguous segment.

In some embodiments, wherein the step (iia1) specifically includes:
(iia11) inserting a contiguous segment included between an hIgKJ1 gene and the hIgKJ5 gene between the mIgKJ region and the mIgKC region;
(iia12) inserting a contiguous segment included between an hIgKV4-1 gene and an hIgKV5-2 gene upstream of the inserted segment in the step (iia11);
(iia13) inserting genes in a contiguous segment included between upstream of hIgKV5-2 and downstream of hIgHKV2-4 upstream of the inserted segment in the step (iia12); and
(iia14) inserting a contiguous segment included between an hIgHKV2-4 gene and an hIgKV1-5 gene, and loxP and lox2272-PB5' sites upstream of the inserted segment in the step (iia13).

In some embodiments, wherein the step (iia2) specifically includes:
(iia21) recombining a BAC vector including the second contiguous segment between the hIgKV1-6 gene and the hIgKV1-37 gene, and an loxP site and an lox2272 site which are located at both ends of the second contiguous segment and in the same direction as that in the step (iia14), and a Cre recombinase with a genome obtained in step (iia1), wherein PB3' is included between the lox2272 site and the second contiguous segment; and
(iia22) contacting a recombinant genome obtained in the step (iia21) with a PiggyBac transposase, and screening a genome including the first partial segment and the loxP site located upstream of the first partial segment.

In some embodiments, wherein in the step (iib), the second partial hIgKV contiguous segment of the second partial segment is inserted upstream of the first partial segment by at least the following steps including:
(iib1) inserting a contiguous segment included between the hIgKV2D-40 gene and an hIgKV2D-38 gene between the mIgKJ region and the mIgKC region;
(iib2) inserting a contiguous segment included between an hIgKV1D-37 and an hIgKV1D-35 gene, and PB3'-lox5171 and loxP sites downstream of the inserted segment in the step (iib1);
(iib3) recombining a BAC vector including a contiguous segment between an hIgKV3D-34 gene and the hIgKV3D-7 gene, and loxP and lox5171 which are located at both ends and in the same direction as that in the step (iib2), and a Cre recombinase with a genome obtained in the step (iib2), wherein PB5' is included between the lox5171 site and the contiguous segment described in this step; and
(iib4) contacting a recombinant genome obtained in the step (iib3) with a PiggyBac transposase, and screening a genome including the second partial segment and the loxP site located downstream of the second partial segment.

In some embodiments, wherein the step (iic) includes:
(iic1) screening Cre-positive mice including the first partial segment and the second partial segment; and
(iic2) mating the mice obtained in the step (iic1) with wild type mice, and screening mice that do not carry Cre but include the first partial segment and the second partial segment, wherein loxP sites are present between the second partial segment and the first partial segment.

In some embodiments, wherein the step (iid) includes:
(iid1) mating the sixth mouse with a wild type mouse, and screening positive mice; and
(iid2) mating the male and female positive mice obtained in the step (iid1) with each other, and screening homozygous mice.

In some embodiments, wherein the step (iii) includes:
(iiia) inserting a first partial segment of a human immunoglobulin Lambda light chain locus between mIgLC1 and mIgL Enhancer 3-1 of an immunoglobulin Lambda light chain locus of a seventh mouse, wherein the first partial segment includes a first partial hIgLV contiguous segment, a whole hIgLJ segment, and a whole hIgLC segment, and seventh recombination sites are included upstream of the first partial segment and downstream of mIgLC1;
(iiib) inserting a second partial segment of the human immunoglobulin Lambda light chain locus between mIgLC1 and mIgL Enhancer 3-1 of an immunoglobulin Lambda light chain locus of an eighth mouse, wherein the second partial segment is located upstream of the first partial segment on the human immunoglobulin Lambda light chain locus, the second partial segment includes a second partial hIgLV contiguous segment, the first partial hIgLV contiguous segment and the second partial hIgLV contiguous segment together constitute the whole hIgLV segment, and eighth recombination sites are included between downstream of the second partial hIgLV contiguous segment and upstream of mIgL Enhancer 3-1;
(iiic) hybridizing the seventh mouse with the eighth mouse, screening a ninth mouse, and inserting the second partial segment of the human immunoglobulin Lambda light chain locus and the first partial segment downstream of the second partial segment between mIgLC1 and mIgL Enhancer 3-1 of an immunoglobulin Lambda light chain locus of the ninth mouse, wherein ninth recombination sites are present between the second partial segment and the first partial segment; and
(iiid) knocking out a whole mIgLV segment, a whole mIgLJ segment, and a whole mIgLC segment of the immunoglobulin Lambda light chain locus of the ninth mouse to obtain the Lambda light chain locus modified mice.

In the present invention, the first partial hIgLV contiguous segment of the first partial segment of the human immunoglobulin Lambda light chain locus may start from a gene (including a functional gene, a pseudogene or an ORF) of any one hIgLV segment upstream of an hIgLV3-1 gene and end at an hIgLVI-70 gene.

In one embodiment, the gene of any one hIgLV segment upstream of the hIgLV3-1 gene may be any one functional gene, pseudogene or ORF between the hIgLV3-1 gene and the hIgLVI-70 gene. In one embodiment, the gene of any one hIgLV segment upstream of the hIgLV3-1 gene is a functional gene, a pseudogene or an ORF, such as hIgLV3-2, hIgLV4-3, hIgLV3-4, hIgLV2-5, hIgLV3-6, hIgLV3-7, hIgLV2-8, hIgLV3-9, hIgLV3-10, hIgLV2-11, hIgLVI-11-1, hIgLV3-12, hIgLV3-13, hIgLV2-14, hIgLV3-15, hIgLV3-16, hIgLV3-17, hIgLV2-18, hIgLV3-19, hIgLVI-20, hIgLV3-21, hIgLV3-22, hIgLVVI-22-1, hIgLV2-23, hIgLV3-24, hIgLVIII-24-1, hIgLV3-25, hIgLVVI-25-1, hIgLV3-26, hIgLV3-27, hIgLV3-28, hIgLV3-29, hIgLV3-30, hIgLV3-31, hIgLV3-32, hIgLV2-33, hIgLV2-34, hIgLV7-35, hIgLV1-36, hIgLV5-37, hIgLVI-38, hIgLV5-39, hIgLV1-40, hIgLV1-41, hIgLVVII-41-1, hIgLVI-42, hIgLV7-43, hIgLV1-44, hIgLV5-45, hIgLV7-46, hIgLV1-47, hIgLV5-48, hIgLV9-49, hIgLV1-50, hIgLV1-51, hIgLV5-52, hIgLVVPREB1, hIgLVIV-53, hIgLV10-54, hIgLV11-55, hIgLVIV-55-1, hIgLVI-55, hIgLV6-57, hIgLVTGFb1, hIgLVV-58, hIgLVIV-59, hIgLVIII-59-1, hIgLV4-60, hIgLVIII-60-1, hIgLV8-61, hIgLV1-62, hIgLVI-63, hIgLVIV-64, hIgLVIV-65, hIgLVV-66, hIgLVIV-66-1, hIgLV10-67, hIgLVI-68, hIgLV4-69 or hIgLVI-70.

In one embodiment, the first partial hIgLV contiguous segment of the first partial segment starts from hIgLV3-2, hIgLV4-3, hIgLV3-4, hIgLV2-5, hIgLV3-6, hIgLV3-7, hIgLV2-8, hIgLV3-9, hIgLV3-10, hIgLV2-11, hIgLVI-11-1, hIgLV3-12, hIgLV3-13, hIgLV2-14, hIgLV3-15, hIgLV3-16, hIgLV3-17, hIgLV2-18, hIgLV3-19, hIgLVI-20, hIgLV3-21, hIgLV3-22, hIgLVVI-22-1, hIgLV2-23, hIgLV3-24, hIgLVIII-24-1, hIgLV3-25, hIgLVVI-25-1, hIgLV3-26, hIgLV3-27, hIgLV3-28, hIgLV3-29, hIgLV3-30, hIgLV3-31, hIgLV3-32, hIgLV2-33, hIgLV2-34, hIgLV7-35, hIgLV1-36, hIgLV5-37, hIgLVI-38, hIgLV5-39, hIgLV1-40, hIgLV1-41, hIgLVVII-41-1, hIgLVI-42, hIgLV7-43, hIgLV1-44, hIgLV5-45, hIgLV7-46, hIgLV1-47, hIgLV5-48, hIgLV9-49, hIgLV1-50, hIgLV1-51, hIgLV5-52, hIgLVVPREB1, hIgLVIV-53, hIgLV10-54, hIgLV11-55, hIgLVIV-55-1, hIgLVI-55, hIgLV6-57, hIgLVTGFb1, hIgLVV-58, hIgLVIV-59, hIgLVIII-59-1, hIgLV4-60, hIgLVIII-60-1, hIgLV8-61, hIgLV1-62, hIgLVI-63, hIgLVIV-64, hIgLVIV-65, hIgLVV-66, hIgLVIV-66-1, hIgLV10-67, hIgLVI-68, hIgLV4-69 or hIgLVI-70, and ends the hIgLV3-1 gene, both containing the described gene as an end. In one embodiment, the first partial hIgLV contiguous segment of the first partial segment is a contiguous segment between the hIgLV2-34 gene and the hIgLV3-1 gene.

In the present invention, the second partial segment of the Lambda light chain locus is located upstream of the first partial segment of the Lambda light chain locus, and the second partial hIgLV contiguous segment may start from any one gene (including a functional gene, a pseudogene or an ORF) of the hIgLV segment and end at a gene (including a functional gene, a pseudogene or an ORF) of any one hIgLV segment upstream of the start gene of the first partial hIgLV contiguous segment. In some embodiments, the second partial segment and the first partial segment together constitute all functional genes upstream of hIgLV3-1 and a contiguous segment between the functional genes, i.e., a contiguous segment between the hIgLV3-1 gene and the hIgLVI-70 gene. For example, in some embodiments, the second partial hIgLV contiguous segment includes or is a contiguous segment between the hIgLV7-35 gene and the hIgLVI-70 gene, and the first partial hIgLV contiguous segment includes or is a contiguous segment between the hIgLV2-34 gene and the hIgLV3-1 gene.

In some embodiments, the first partial hIgLV contiguous segment includes a contiguous segment between the hIgLV3-1 gene and the hIgLV2-34 gene; and the second partial hIgLV contiguous segment includes a contiguous segment between the hIgLVI-70 gene and the hIgLV7-35 gene.

In some embodiments, the seventh recombination sites, the eighth recombination sites, and the ninth recombination sites are loxP sites.

In some embodiments, step (iiia) includes:
(iiia1) introducing loxP and lox5171-PB5' between mIgLC1 and mIgL Enhancer 3-1;
(iiia2) recombining a BAC vector including the first partial segment of the human immunoglobulin Lambda light chain locus, and an loxP site and an lox5171 site which are located at both ends of the first partial segment and in the same direction as that in the step (iiia1), and a Cre recombinase with a genome obtained in the step (iiia1), wherein PB3' is included between the lox5171 site and the first partial segment; and
(iiia3) contacting a recombinant genome obtained in the step (iiia2) with a PiggyBac transposase, and screening a genome including the first partial segment and the loxP site located upstream of the first partial segment.

In some embodiments, the step (iiib) includes:
(iiib1) inserting a contiguous segment included between an hIgLV4-69 gene and an hIgLV10-67 gene between the mIgLC1 and the mIgL Enhancer 3-1;
(iiib2) inserting a contiguous segment included between an hIGLVIV-66-1 and an hIGLVIV-65 gene, and PB3'-lox2272 and loxP sites downstream of the inserted segment in the step (iiib1);
(iiib3) recombining a BAC vector including a contiguous segment between an hIGLV7-35 and an hIGLVI-63 gene, and loxP and lox2272 which are located at both ends and in the same direction as that in the step (iiib2), and a Cre recombinase with a genome obtained in the step (iiib2), wherein PB5' is included between the lox2272 site and the contiguous segment described in this step; and
(iiib4) contacting a recombinant genome obtained in the step (iiib3) with a PiggyBac transposase, and screening a genome including the second partial segment and the loxP site located downstream of the second partial segment.

In some embodiments, the step (iiic) includes:
(iiic1) screening Cre-positive mice including the first partial segment and the second partial segment; and
(iiic2) mating the mice obtained in the step (iiic1) with wild type mice, and screening mice that do not carry Cre but include the first partial segment and the second partial segment, wherein loxP sites are present between the second partial segment and the first partial segment.

In some embodiments, the step (iiid) includes:
(iiid1) mating the ninth mouse with a wild type mouse, and screening positive mice; and
(iiid2) mating the male and female positive mice obtained in the step (iiid1) with each other, and screening homozygous mice.

In another aspect, the present invention provides a genetically modified mouse genome, wherein
(a) immunoglobulin heavy chain loci of genetically-modified mice sequentially include: (i) an Adam6a gene of the mice; (ii) an Adam6b gene of the mice; (iii) a second partial segment of a human immunoglobulin heavy chain variable region locus, including a second partial hIgHV contiguous segment; (iv) a first partial segment of the human immunoglobulin heavy chain variable region locus, located downstream of the second partial segment and including a first partial hIgHV contiguous segment, a whole hIgHD segment and a whole hIgHJ segment, the first partial segment not including a segment between downstream of an hIgHV1-2 gene and upstream of an hIgHV6-1 gene; and (v) an mIgHC region;
(b) immunoglobulin Kappa light chain loci of the genetically modified mice sequentially include: (i) a second partial segment of a human immunoglobulin Kappa light chain variable region locus, including a second partial hIgKV contiguous segment; (ii) a first partial segment of the human immunoglobulin Kappa light chain variable region locus, located downstream of the second partial segment and including a first partial hIgKV contiguous segment and a wholel hIgHJ segment, the first partial segment and the second partial segment together constituting a whole hIgKV segment; and (iii) an mIgKC region; and
(c) immunoglobulin Lambda light chain loci of the genetically modified mice sequentially include: (i) a second partial segment of a human immunoglobulin Lambda light chain locus, including a second partial hIgLV contiguous segment; and (ii) a first partial segment of the human immunoglobulin Lambda light chain locus, located downstream of the second partial segment and including a first partial hIgLV contiguous segment and a whole hIgHJ-C segment, the first partial segment and the second partial segment together constituting a whole hIgLV segment.

In some embodiments, the first partial hIgHV contiguous segment of the first partial segment may start from a gene (including a functional gene, a pseudogene or an ORF) of any one hIgHV segment upstream of the hIgHV1-2 gene and end at the hIgHV1-2 gene.

In an embodiment, the gene of any one hIgHV segment upstream of the hIgHV1-2 gene may be any one functional gene, pseudogene or ORF between an hIgHV (III)-82 gene and the hIgHV1-2 gene. In one embodiment, the functional gene of any one hIgHV segment upstream of the hIgHV1-2 gene is, for example, hIgHV3-74, hIgHV3-73, hIgHV3-72, hIgHV2-70, hIgHV1-69D, hIgHV1-69-2, hIgHV2-70D, hIgHV1-69, hIgHV3-66, hIgHV3-64, hIgHV3-62, hIgHV4-61, hIgHV4-59, hIgHV1-58, hIgHV3-53, hIgHV5-51, hIgHV3-49, hIgHV3-48, hIgHV1-46, hIgHV1-45, hIgHV3-43, hIgHV4-39, hIgHV3-43D, hIgHV4-38-2, hIgHV3-35, hIgHV4-34, hIgHV3-33, hIgHV4-31, hIgHV3-30-5, hIgHV4-30-4, hIgHV3-30-3, hIgHV4-30-2, hIgHV4-30-1, hIgHV3-30, hIgHV4-28, hIgHV2-26, hIgHV1-24, hIgHV3-23D, hIgHV3-23, hIgHV3-21, hIgHV3-20, hIgHV1-18, hIgHV3-15, hIgHV3-13, hIgHV3-11, hIgHV5-10-1, hIgHV3-9, hIgHV3-64D, hIgHV3-8, hIgHV3-7, hIgHV2-5, hIgHV7-4-1, hIgHV4-4 or hIgHV1-3.

In one embodiment, the first partial hIgHV contiguous segment of the first partial segment starts from hIgHV3-74, hIgHV3-73, hIgHV3-72, hIgHV2-70, hIgHV1-69D, hIgHV1-69-2, hIgHV2-70D, hIgHV1-69, hIgHV3-66, hIgHV3-64, hIgHV3-62, hIgHV4-61, hIgHV4-59, hIgHV1-58, hIgHV3-53, hIgHV5-51, hIgHV3-49, hIgHV3-48, hIgHV1-46, hIgHV1-45, hIgHV3-43, hIgHV4-39, hIgHV3-43D, hIgHV4-38-2, hIgHV3-35, hIgHV4-34, hIgHV3-33, hIgHV4-31, hIgHV3-30-5, hIgHV4-30-4, hIgHV3-30-3, hIgHV4-30-2, hIgHV4-30-1, hIgHV3-30, hIgHV4-28, hIgHV2-26, hIgHV1-24, hIgHV3-23D, hIgHV3-23, hIgHV3-21, hIgHV3-20, hIgHV1-18, hIgHV3-15, hIgHV3-13, hIgHV3-11, hIgHV5-10-1, hIgHV3-9, hIgHV3-64D, hIgHV3-8, hIgHV3-7, hIgHV2-5, hIgHV7-4-1, hIgHV4-4 or hIgHV1-3, and ends at the hIgHV1-2 gene, both containing the described gene as an end. In an embodiment, the first partial hIgHV contiguous segment of the first partial segment is a contiguous segment between an hIgHV4-28 gene and the hIgHV1-2 gene.

In the present invention, the second partial segment is located upstream of the first partial segment, and the second partial hIgHV contiguous segment may start from any one gene (including a functional gene, a pseudogene or an ORF) of the hIgHV segment and end at a gene (including a functional gene, a pseudogene or an ORF) of any one hIgHV segment upstream of the start gene of the first partial hIgHV contiguous segment. In some embodiments, the second partial segment and the first partial segment together constitute all functional genes upstream of hIgHV1-2 and a contiguous segment between the functional genes, i.e., a contiguous segment between the hIgHV3-74 gene and the hIgHV1-2 gene. For example, in some embodiments, the second partial hIgHV contiguous segment includes or is a contiguous segment between the hIgHV3-74 gene and the hIgHV3-30 gene, and the first partial hIgHV contiguous stretch includes or is a contiguous segment between the hIgHV4-28 gene and the hIgHV1-2 gene.

In the present invention, the first partial hIgKV contiguous segment of the first partial segment of the human immunoglobulin Kappa light chain variable region locus can start from a gene (including a functional gene, a pseudogene or an ORF) of any one hIgKV segment upstream of an hIgKV4-1 gene and end at the hIgKV4-1 gene.

In one embodiment, the gene of any one hIgKV segment upstream of the hIgKV4-1 gene may be any one functional gene, pseudogene or ORF between an hIgKV3D-7 gene and the hIgKV4-1 gene. In one embodiment, the gene of any one hIgKV segment upstream of the hIgKV4-1 gene is a functional gene or an ORF, such as hIgKV3D-7, hIgKV1D-8, hIgKV1D-43, hIgKV1D-42, hIgKV3D-11, hIgKV1D-12, hIgKV1D-13, hIgKV3D-15, hIgKV1D-16, hIgKV1D-17, hIgKV6D-41, hIgKV3D-20, hIgKV6D-21, hIgKV2D-24, hIgKV2D-26, hIgKV2D-28, hIgKV2D-29, hIgKV2D-30, hIgKV1D-33, hIgKV1D-37, hIgKV1D-39, hIgKV2D-40, hIgKV2-40, hIgKV1-39, hIgKV1-37, hIgKV1-33, hIgKV2-30, hIgKV2-29, hIgKV2-28, hIgKV2-27, hIgKV2-24, hIgKV6-21, hIgKV3-20, hIgKV1-17, hIgKV1-16, hIgKV3-15, hIgKV1-13, hIgKV1-12, hIgKV3-11, hIgKV1-9, hIgKV1-8, hIgKV3-7, hIgKV1-6, hIgKV1-5 or hIgKV5-2.

In one embodiment, the first partial hIgKV contiguous segment of the first partial segment starts from hIgKV3D-7, hIgKV1D-8, hIgKV1D-43, hIgKV1D-42, hIgKV3D-11, hIgKV1D-12, hIgKV1D-13, hIgKV3D-15, hIgKV1D-16, hIgKV1D-17, hIgKV6D-41, hIgKV3D-20, hIgKV6D-21, hIgKV2D-24, hIgKV2D-26, hIgKV2D-28, hIgKV2D-29, hIgKV2D-30, hIgKV1D-33, hIgKV1D-37, hIgKV1D-39, hIgKV2D-40, hIgKV2-40, hIgKV1-39, hIgKV1-37, hIgKV1-33, hIgKV2-30, hIgKV2-29, hIgKV2-28, hIgKV2-27, hIgKV2-24, hIgKV6-21, hIgKV3-20, hIgKV1-17, hIgKV1-16, hIgKV3-15, hIgKV1-13, hIgKV1-12, hIgKV3-11, hIgKV1-9, hIgKV1-8, hIgKV3-7, hIgKV1-6, hIgKV1-5 or hIgKV5-2, and ends at the hIgKV4-1 gene, both containing the described gene as an end. In one embodiment, the first partial hIgKV contiguous segment of the first partial segment is a contiguous segment between an hIgKV1-37 gene and the hIgKV4-1 gene.

In the present invention, the second partial segment of the Kappa light chain variable region locus is located upstream of the first partial segment of the Kappa light chain variable region locus, and the second partial hIgKV contiguous segment may start from any one gene (including a functional gene, a pseudogene or an ORF) of the hIgKV segment and end at a gene (including a functional gene, a pseudogene or an ORF) of any one hIgKV segment upstream of the start gene of the first partial hIgKV contiguous segment. In some embodiments, the second partial segment and the first partial segment together constitute all functional genes upstream of hIgKV4-1 and a contiguous segment between the functional genes, i.e., a contiguous segment between the hIgKV4-1 gene and the hIgKV3D-7 gene. For example, in some embodiments, the second partial hIgKV contiguous segment includes or is a contiguous segment between the hIgKV3D-7 gene and the hIgKV2D-40 gene, and the first partial hIgKV contiguous segment includes or is a contiguous segment between the hIgKV1-37 gene and the hIgKV4-1 gene.

In the present invention, the first partial hIgLV contiguous segment of the first partial segment of the human immunoglobulin Lambda light chain locus may start from a gene (including a functional gene, a pseudogene or an ORF) of any one hIgLV segment upstream of an hIgLV3-1 gene and end at an hIgLVI-70 gene.

In one embodiment, the gene of any one hIgLV segment upstream of the hIgLV3-1 gene may be any one functional gene, pseudogene or ORF between the hIgLV3-1 gene and the hIgLVI-70 gene. In one embodiment, the gene of any one hIgLV segment upstream of the hIgLV3-1 gene is a functional gene, a pseudogene or an ORF, such as hIgLV3-2, hIgLV4-3, hIgLV3-4, hIgLV2-5, hIgLV3-6, hIgLV3-7, hIgLV2-8, hIgLV3-9, hIgLV3-10, hIgLV2-11, hIgLVI-11-1, hIgLV3-12, hIgLV3-13, hIgLV2-14, hIgLV3-15, hIgLV3-16, hIgLV3-17, hIgLV2-18, hIgLV3-19, hIgLVI-20, hIgLV3-21, hIgLV3-22, hIgLVVI-22-1, hIgLV2-23, hIgLV3-24, hIgLVIII-24-1, hIgLV3-25, hIgLVVI-25-1, hIgLV3-26, hIgLV3-27, hIgLV3-28, hIgLV3-29, hIgLV3-30, hIgLV3-31, hIgLV3-32, hIgLV2-33, hIgLV2-34, hIgLV7-35, hIgLV1-36, hIgLV5-37, hIgLVI-38, hIgLV5-39, hIgLV1-40, hIgLV1-41, hIgLVVII-41-1, hIgLVI-42, hIgLV7-43, hIgLV1-44, hIgLV5-45, hIgLV7-46, hIgLV1-47, hIgLV5-48, hIgLV9-49, hIgLV1-50, hIgLV1-51, hIgLV5-52, hIgLVVPREB1, hIgLVIV-53, hIgLV10-54, hIgLV11-55, hIgLVIV-55-1, hIgLVI-55, hIgLV6-57, hIgLVTGFb1, hIgLVV-58, hIgLVIV-59, hIgLVIII-59-1, hIgLV4-60, hIgLVIII-60-1, hIgLV8-61, hIgLV1-62, hIgLVI-63, hIgLVIV-64, hIgLVIV-65, hIgLVV-66, hIgLVIV-66-1, hIgLV10-67, hIgLVI-68, hIgLV4-69 or hIgLVI-70.

In one embodiment, the first partial hIgLV contiguous segment of the first partial segment starts from hIgLV3-2, hIgLV4-3, hIgLV3-4, hIgLV2-5, hIgLV3-6, hIgLV3-7, hIgLV2-8, hIgLV3-9, hIgLV3-10, hIgLV2-11, hIgLVI-11-1, hIgLV3-12, hIgLV3-13, hIgLV2-14, hIgLV3-15, hIgLV3-16, hIgLV3-17, hIgLV2-18, hIgLV3-19, hIgLVI-20, hIgLV3-21, hIgLV3-22, hIgLVVI-22-1, hIgLV2-23, hIgLV3-24, hIgLVIII-24-1, hIgLV3-25, hIgLVVI-25-1, hIgLV3-26, hIgLV3-27, hIgLV3-28, hIgLV3-29, hIgLV3-30, hIgLV3-31, hIgLV3-32, hIgLV2-33, hIgLV2-34, hIgLV7-35, hIgLV1-36, hIgLV5-37, hIgLVI-38, hIgLV5-39, hIgLV1-40, hIgLV1-41, hIgLVVII-41-1, hIgLVI-42, hIgLV7-43, hIgLV1-44, hIgLV5-45, hIgLV7-46, hIgLV1-47, hIgLV5-48, hIgLV9-49, hIgLV1-50, hIgLV1-51, hIgLV5-52, hIgLVVPREB1, hIgLVIV-53, hIgLV10-54, hIgLV11-55, hIgLVIV-55-1, hIgLVI-55, hIgLV6-57, hIgLVTGFb1, hIgLVV-58, hIgLVIV-59, hIgLVIII-59-1, hIgLV4-60, hIgLVIII-60-1, hIgLV8-61, hIgLV1-62, hIgLVI-63, hIgLVIV-64, hIgLVIV-65, hIgLVV-66, hIgLVIV-66-1, hIgLV10-67, hIgLVI-68, hIgLV4-69 or hIgLVI-70, and ends the hIgLV3-1 gene, both containing the described gene as an end. In one embodiment, the first partial hIgLV contiguous segment of the first partial segment is a contiguous segment between the hIgLV2-34 gene and the hIgLV3-1 gene.

In the present invention, the second partial segment of the Lambda light chain locus is located upstream of the first partial segment of the Lambda light chain locus, and the second partial hIgLV contiguous segment may start from any one gene (including a functional gene, a pseudogene or an ORF) of the hIgLV segment and end at a gene (including a functional gene, a pseudogene or an ORF) of any one hIgLV segment upstream of the start gene of the first partial hIgLV contiguous segment. In some embodiments, the second partial segment and the first partial segment together constitute all functional genes upstream of hIgLV3-1 and a contiguous segment between the functional genes, i.e., a contiguous segment between the hIgLV3-1 gene and the hIgLVI-70 gene. For example, in some embodiments, the second partial hIgLV contiguous segment includes or is a contiguous segment between the hIgLV7-35 gene and the hIgLVI-70 gene, and the first partial hIgLV contiguous segment includes or is a contiguous segment between the hIgLV2-34 gene and the hIgLV3-1 gene.

In some embodiments, the first partial segment and the second partial segment of a heavy chain, a Kappa light chain, and a Lambda light chain are not rearranged.

In some embodiments, immunoglobulin heavy chain loci of the obtained genetically modified mice sequentially include: (i) an Adam6a gene of the mice; (ii) an Adam6b gene of the mice; (iii) a contiguous segment between an hIgHV3-74 gene and an hIgHV3-30 gene; (iv) a contiguous segment between the hIgHV4-28 gene and the hIgHV1-2 gene; (v) a contiguous segment between the hIgHV6-1 gene and an hIgHJ6 gene; and (vi) an mIgHC region; immunoglobulin Kappa light chain loci of the obtained genetically modified mice sequentially include: (i) a contiguous segment between the hIgKV3D-7 gene and the hIgKV4-1 gene; (ii) a contiguous segment between an hIgKJ1 gene and an hIgKJ5 gene; and (iii) an mIgKC region; and immunoglobulin Lambda light chain loci of the obtained genetically modified mice sequentially include the whole hIgLV segment and a whole hIgLJ-C segment.

In some embodiments, the immunoglobulin heavy chain loci of the obtained genetically modified mice do not include mIgHV5-1 and a whole mIgHV segment upstream of the mIgHV5-1 gene, a contiguous segment between an mIgHD1-1 gene and an mIgHJ4 gene, and mIgHD3-1, mIgHD5-1, and mIgHD1-3 genes; and the immunoglobulin Kappa light chain loci of the obtained genetically modified mice do not include a contiguous segment between an mIgKV2-137 gene and an mIgKVJ5 gene.

In some embodiments, the fertility of the obtained genetically modified mice is not reduced compared with non-genetically modified wild type mice.

In some embodiments, the obtained genetically modified mice can produce a human-mouse chimeric antibody including a human heavy chain variable region and a mouse heavy chain constant region and a human Kappa light chain variable region and a mouse Kappa light chain constant region, or including a human heavy chain variable region and a mouse heavy chain constant region and a human Lambda light chain variable region and a human Lambda light chain constant region.

In another aspect, the present invention provides a cell, tissue, organ or mouse including the mouse genome described above.

In some embodiments, the present invention provides a cell including the mouse genome described above, wherein the cell is an embryonic cell, a B cell, or a hybridoma cell.

In some embodiments, the present invention provides a tissue including the mouse genome described above, wherein the tissue is a white pulp of a spleen or lymphoid nodules thereof.

In some embodiments, the present invention provides an organ including the mouse genome described above, wherein the organ is a spleen.

In some embodiments, the present invention provides a mouse including the mouse genome described above.

In another aspect, the present invention provides a method for preparing a monoclonal antibody, including
(a) immunizing mice having any one of the genomes described in the present invention with an antigen;
(b) isolating cells producing and comprising a monoclonal antibody against the antigen from the mice; and
(c) culturing the cells to obtain the monoclonal antibody.

In some embodiments, the cells in the step (c) are splenocytes, B cells, or hybridoma cells.

In some embodiments, the monoclonal antibody has a human heavy chain variable region, a human Kappa light chain variable region, a mouse heavy chain constant region, and a mouse Kappa light chain constant region, or the monoclonal antibody has a human heavy chain variable region, a human Lambda light chain variable region, a mouse heavy chain constant region, and a human Lambda light chain constant region, and does not have a mouse heavy chain variable region, a mouse Kappa light chain variable region, and a mouse Lambda light chain constant region.

In another aspect, the present invention provides use of any one of the cells, tissues, organs or mice of the present invention in the preparation of a monoclonal antibody.

In some embodiments, the monoclonal antibody has a human heavy chain variable region, a human Kappa light chain variable region, a mouse heavy chain constant region, and a mouse Kappa light chain constant region, or the monoclonal antibody has a human heavy chain variable region, a human Lambda light chain variable region, a mouse heavy chain constant region, and a human Lambda light chain constant region, and does not have a mouse heavy chain variable region, a mouse Kappa light chain variable region, and a mouse Lambda light chain constant region.

The present invention provides a heavy chain locus modified mouse genome that maintains the endogenous ADAM6 gene and its function and can produce human-mouse chimeric antibodies while maintaining fertility. The human-mouse chimeric antibody (i) has a human heavy chain variable region, and a human Kappa light chain variable region, and does not have a mouse heavy chain variable region, and a mouse Kappa light chain variable region; (ii) has a human heavy chain variable region, and a human Lambda light chain variable region, and does not have a mouse heavy chain variable region, and a mouse Lambda light chain variable region; (iii) has a human heavy chain variable region, a human Kappa light chain variable region, a mouse heavy chain constant region and a mouse Kappa light chain constant region; or (iii) has a human heavy chain variable region, a human Lambda light chain variable region, a mouse heavy chain constant region and a human Lambda light chain constant region.

### BRIEF DESCRIPTION OF THE DRAWINGS

FIGS. 1, 3, 5, 7, 9, 11, 13, 15, 17, 19, 21, 23, 25, 27, 29, 31, 33, 34, 35, 50, 52, 54, 56, 58, 60, 62, 64, 66, 68, 70, 72, 74, 76, 78, 80, 82, 90, 92, 94, 96, 98, 100, 102, 104, 106, 108, 110, 112, 114, and 116 show the schematic diagrams of genomic changes involved in each modification step, respectively.
FIGS. 2, 4, 6, 8, 10, 12, 14, 16, 18, 20, 22, 24, 26, 28, 30, 32, 34, 36, 37, 38, 39, 51, 53, 55, 57, 59, 61, 63, 65, 67, 69, 71, 73, 75, 77, 79, 81, 83, 84, 85, 86, 91, 93, 95, 97, 99, 101, 103, 105, 107, 109, 111, 113, 115, 117, 118, 119 and 120 show the results of PCR identification in each modification step, respectively.
FIGS. 40 to 42 show a distribution graph of a gene usage frequency at a reads level based on the obtained gene usage frequency data.
FIG. 43 shows a distribution graph of a CDR3 amino acid length at the Reads level based on the obtained CDR3 length frequency data.
FIG. 44 shows the results of Weblogo profiling of a CDR3 amino acid sequence of each sample at the reads level.
FIG. 45 shows that genetically modified mice and wild type mice have similar immune performance.
FIG. 46 shows representative test cases of positive hybridoma cells obtained by screening.
FIG. 47 shows that dozens of positive hybridoma cells were screened, 5 of which had a very strong binding ability to antigen-positive cells.
FIG. 48 shows that all four genetically modified homozygous mice exhibited a high immune response capacity.
FIG. 49 shows that antibody sequences obtained from genetically modified mice are all human antibody sequences, and the sequence diversity is rich.
FIGS. 87 and 88 show distribution graphs of a usage frequency of genes in an IgGKV region and genes in an IgGKJ region at the reads level based on the obtained gene usage frequency data.
FIG. 89 shows a distribution graph of a CDR3 amino acid length at the Reads level based on the obtained CDR3 length frequency data.
FIG. 121 shows a distribution graph of a usage frequency of genes in an IgGLV region at the reads level based on the obtained gene usage frequency data.
FIG. 122 shows Weblogo profiling of a CDR3 amino acid sequence of each sample at the reads level.
FIGS. 123 to 126 are graphs of PCR identification of heavy-chain and Kappa light-chain double gene heterozygous mice.
FIGS. 127 to 130 are graphs of PCR identification of heavy-chain and Kappa light-chain double gene homozygous mice.
FIGS. 131 to 134 are graphs of PCR identification of heavy-chain and Lambda light-chain double gene heterozygous mice.
FIGS. 135 to 138 are graphs of PCR identification of heavy-chain and Lambda light-chain double gene homozygous mice.
FIGS. 139 to 144 are graphs of PCR identification of heavy-chain homozygous, Kappa light-chain heterozygous, and Lambda chain heterozygous mice.
FIGS. 145 to 150 are graphs of PCR identification of heavy-chain, Kappa light-chain, and Lambda light-chain three gene homozygous mice.
FIG. 151 shows the results of serum titer detection, where HKL-10, HKL-47, and HKL-49 are mice capable of generating fully human antibodies; WT-C57 is wild-type mice; and NC is serum of unimmunized mice in which antibodies are not generated as a negative control.
FIG. 152 shows the results of sequencing identification of clones randomly picked from a titer plate for determining the number of library transformants, showing a VH-V Kappa fully human antibody sequence.
FIG. 153 shows the results of sequencing identification of clones randomly picked from a titer plate for determining the number of library transformants, showing a VH-V Lambda fully human antibody sequence.

### DETAILED DESCRIPTION

### Definitions

"hIgHV" in the present invention refers to a V region of a human immunoglobulin heavy chain variable region locus, and refers to an entire V region of the human immunoglobulin heavy chain variable region locus when used independently, and when "hIgHV" is suffixed with a specific gene number, e.g., "hIgHV3-30" denotes a 3-30 gene located in the V region of the human immunoglobulin heavy chain variable region locus . Similarly, the present invention also uses "hIgHD" and "hIgHJ" to respectively refer to a D region and a J region of the human immunoglobulin heavy chain variable region locus.

"hIgKV" in the present invention refers to a V region of a human immunoglobulin Kappa light chain variable region locus, and refers to an entire V region of the human immunoglobulin Kappa light chain variable region locus when used independently, and when "hIgKV" is suffixed with a specific gene number, e.g., "hIgKV1-37" denotes a 1-37 gene located in the V region of the human immunoglobulin Kappa light chain variable region locus. Similarly, the present invention also uses "hIgKJ" to refer to a J region of the human immunoglobulin Kappa light chain variable region locus.

"hIgLV" in the present invention refers to a V region of a human immunoglobulin Lambda light chain variable region locus, and refers to an entire V region of the human immunoglobulin Lambda light chain variable region locus when used independently, and when "hIgLV" is suffixed with a specific gene number, e.g., "hIgLV3-1" denotes a 3-1 gene located in the V region of the human immunoglobulin Lambda light chain variable region locus. Similarly, the present invention also uses "hIgLJ" to refer to a J region of the human immunoglobulin Lambda light chain locus; and "hIgLC" refers to a C region of the human immunoglobulin Lambda light chain locus. In addition, "hIgLJ-C" is also used herein to refer to "hIgLJ" and "hIgLC" due to the alternating arrangement of the J region and the C region (e.g., J1-C1-J2-C2-J3-C3...) in the human immunoglobulin Lambda light chain locus.

"mIgHV" in the present invention refers to a V region of a mouse immunoglobulin heavy chain variable region locus, and refers to an entire V region of the mouse immunoglobulin heavy chain variable region locus when used independently, and when "mIgHV" is suffixed with a specific gene number, e.g., "mIgHV5-1" denotes a 5-1 gene located in the V region of the mouse immunoglobulin heavy chain variable region locus. Similarly, the present invention also uses "mIgHD" and "mIgHJ" to respectively refer to a D region and a J region of the mouse immunoglobulin heavy chain variable region locus.

"mIgKV" in the present invention refers to a V region of a mouse immunoglobulin Kappa light chain variable region locus, and refers to an entire V region of the mouse immunoglobulin Kappa light chain variable region locus when used independently, and when "mIgKV" is suffixed with a specific gene number, e.g., "mIgKV2-137" denotes a 2-137 gene located in the V region of the mouse immunoglobulin Kappa light chain variable region locus. Similarly, the present invention also uses "mIgKJ" to refer to a J region of the mouse immunoglobulin Kappa light chain variable region locus.

"mIgLV" in the present invention refers to a V region of a mouse immunoglobulin Lambda light chain variable region locus, and refers to an entire V region of the mouse immunoglobulin Lambda light chain variable region locus when used independently, and when "mIgLV" is suffixed with a specific gene number, e.g., "mIgLV1" denotes a gene 1 located in the V region of the mouse immunoglobulin Lambda light chain variable region locus. Similarly, the present invention also uses "mIgLJ" to refer to a J region of a mouse immunoglobulin Lambda light chain locus; and "mIgLC" refers to a C region of the mouse immunoglobulin Lambda light chain locus. mIgL enhancer 3-1 is present downstream of mouse mIgLC1. In the present invention, human immunoglobulin Lambda light chain variable and constant regions are inserted between downstream of mouse mIgLC1 and upstream of mIgL Enhancer 3-1 (i.e., mlgLEλ3-1).

A "contiguous segment" refers to an uninterrupted nucleotide fragment between two specified endpoint genes, which includes a functional gene, a pseudogene, an ORF, and other nucleotide sequences (e.g., a spacer sequence) located between the two endpoint genes. The term "contiguous segment between an A gene and a B gene" is meant to include the A gene, the B gene and a contiguous gene segment between the A gene and the B gene. The term "upstream of the A gene" or "downstream of the A gene" does not include the A gene itself.

### Examples

### Example 1. Preparation of heavy chain locus modified mice

### I. Mouse construction based on an ES cell line I

1. Insertion of about 20 kb of a human gene sequence (including IGHJ1-6, IGHD7-27, IGHD1-26, IGHD6-25 and all intergenic sequences) between a J region and a C region of mice

A constructed vector was electroporated into wild-type ES cells. The vector was Neo-resistant. The cells were subjected to G418 drug screening. Related ES clones were selected for culture, amplified and identified by PCR typing.

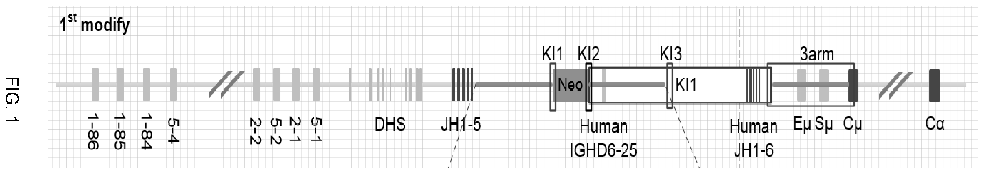

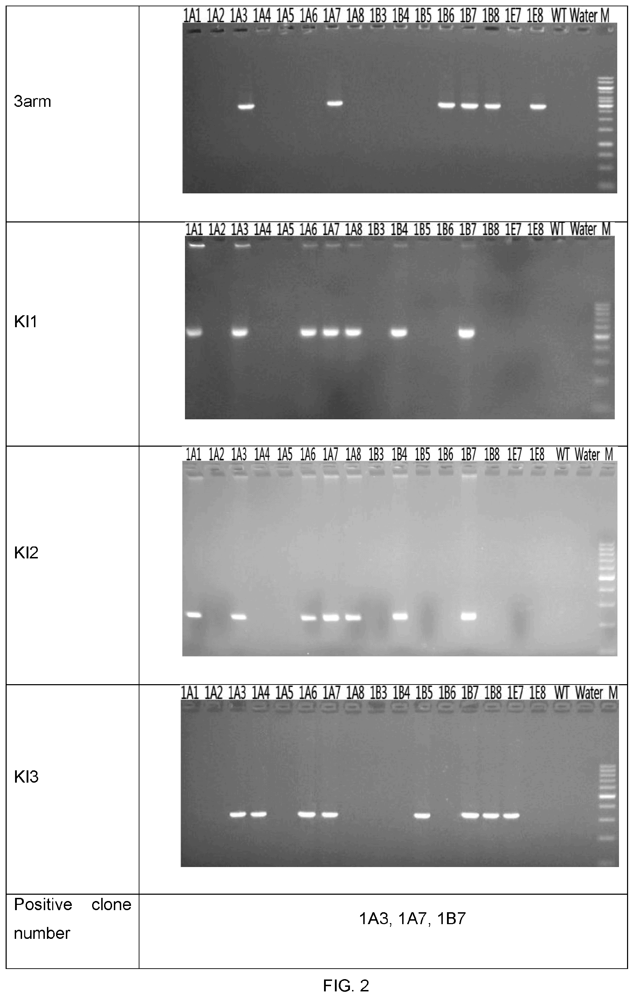

The insertion of the sequence into the corresponding position was identified by four pairs of primers (the sequences are shown in Table 1 below, 5'→ 3', similarly hereinafter, and primer identification positions are simultaneously labeled in a figure (FIG. 1)). There were three clones with positive bands simultaneously amplified by the four pairs of primers (FIG. 2): 1A3, 1A7, and 1B7, and the three clones were used as positive clones in the first step.

**Table 1.**

| Aspect | Identification region | Product size | Primer name | Primer sequence | SEQ ID NO. |
|---|---|---|---|---|---|
| ES1-V1 | 3'arm | 2868 | H1-F1 | gcccatttgaaggagaggtcg | 1 |
| | | | H1-R1 | CATCTCAGCTCAGAACA GTCCAGTG | 2 |
| | KI1 | 538 | H1-F2 | acaggcagggaacagaatgtg | 3 |
| | | | H1-R2 | TGGGCTATGAACTAATGA CCC | 4 |
| | KI2 | 229 | H1-F3 | gcatcgcattgtctgagtaggt | 5 |
| | | | H1-R3 | GATGGAGCTGAGGTCTG TGC | 6 |
| | KI3 | 322 | H1-F4 | tctcaccagccacattcaag | 7 |
| | | | H1-R4 | GACACCATTTCACCTCTG TTGA | 8 |

2. Insertion of about 20 kb of a human D genome sequence (including IGHD5-24, IGHD4-23, IGHD3-22, IGHD2-21, IGHD1-20, IGHD6-19, IGHD5-18, IGHD4-17, IGHD3-16, IGHD2-15, IGHD1-14, IGHD6-13 and all intergenic sequences) in front of a genome of the cells obtained in the first step
A constructed vector was electroporated into the positive cell clone 1A7 in the first step: the vector carries Puro resistance and a homology arm on one end is placed on the human sequence with the positive clones in the first step, so the Neo resistance will be deleted by homologous recombination in this step. Cells were subjected to Puromycin drug screening, and related ES clones were selected for culture, amplified and identified by PCR typing.

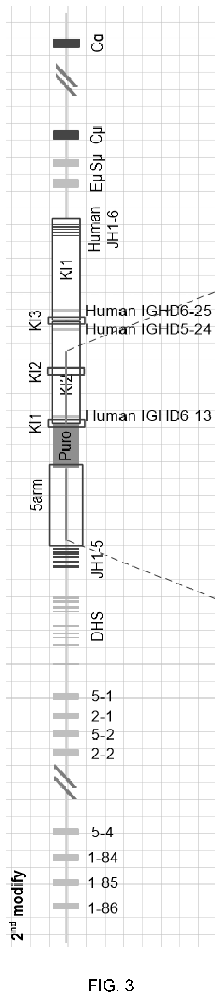

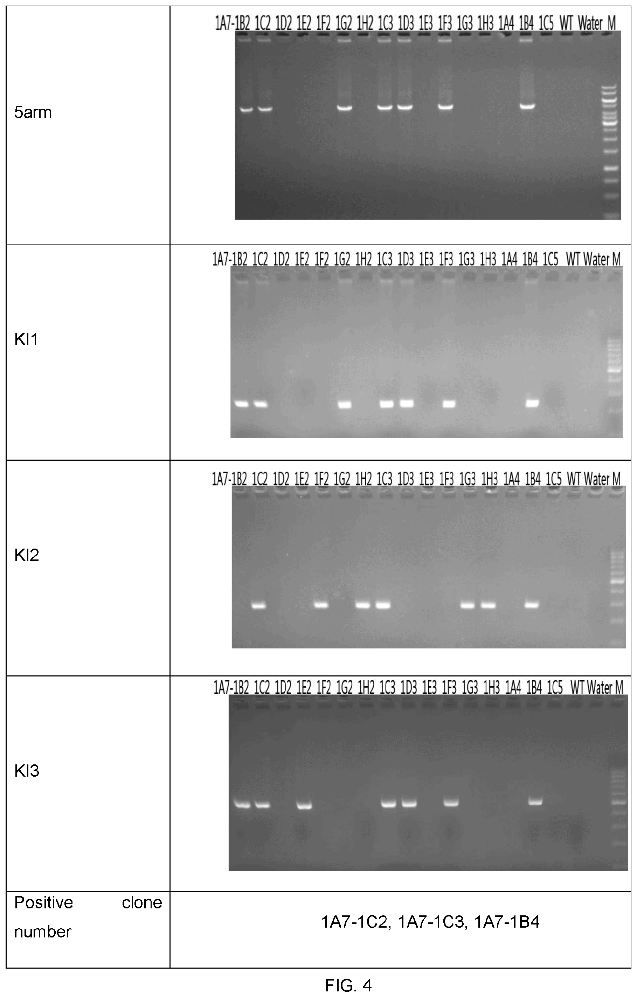

The insertion of the sequence into the corresponding position was identified by four pairs of primers (the sequences are shown in Table 2 below, and primer identification positions are simultaneously labeled in a figure (FIG. 3)). There were three clones with positive bands simultaneously amplified by the four pairs of primers (FIG. 4): 1A7-1C2, 1A7-1C3, and 1A7-1B4, and the three clones were used as positive clones in the second step.

**Table 2.**

| Aspect | Identification region | Product size | Primer name | Primer sequence | SEQ ID NO. |
|---|---|---|---|---|---|
| ES1-V1V2 | 5'arm | 5032 | H2-F1 | gaatggacaaccaatgactggc | 9 |
| | | | H2-R1 | CGTGCTACTTCCATTTGT CACG | 10 |
| | KI1 | 232 | H2-F2 | gccttctagttgccagccatc | 11 |
| | | | H2-R2 | CCAGCCTACAGCCATAG AGCC | 12 |
| | KI2 | 282 | H2-F3 | ccagagccaactccaggagc | 13 |
| | | | H2-R3 | GCCACCAGCCAGTAGTT CTTCC | 14 |
| | KI3 | 510 | H2-F4 | cagagccagtgcagacagaggc | 15 |
| | | | H2-R4 | CTGTCCCTCTGTCTGGT GTCTGG | 16 |

3. Insertion of about 20 kb of a human D genome sequence (including IGHD5-12, IGHD4-11, IGHD3-10, IGHD3-9, IGHD2-8, IGHD1-7, IGHD6-6, IGHD5-5, IGHD4-4, IGHD3-3, IGHD2-2, IGHD1-1 and all intergenic sequences) in front of a genome of the cells obtained in the second step
A constructed vector was electroporated into the positive cell clone 1A7-1C3 in the second step: the vector carries Neo resistance and a homology arm on one end is placed on the human sequence with the positive clones in the second step, so the Puro resistance will be deleted by homologous recombination in this step; cells were subjected to G418 drug screening and related ES clones were selected for culture, amplified and identified by PCR typing.

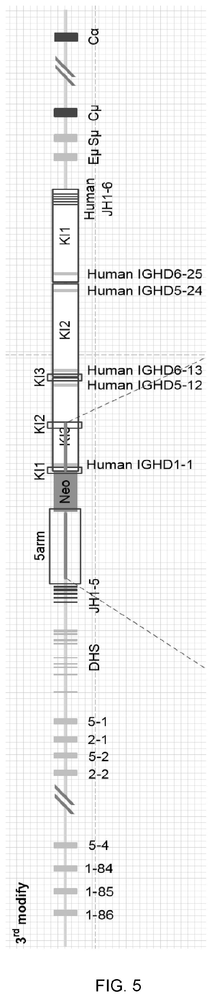

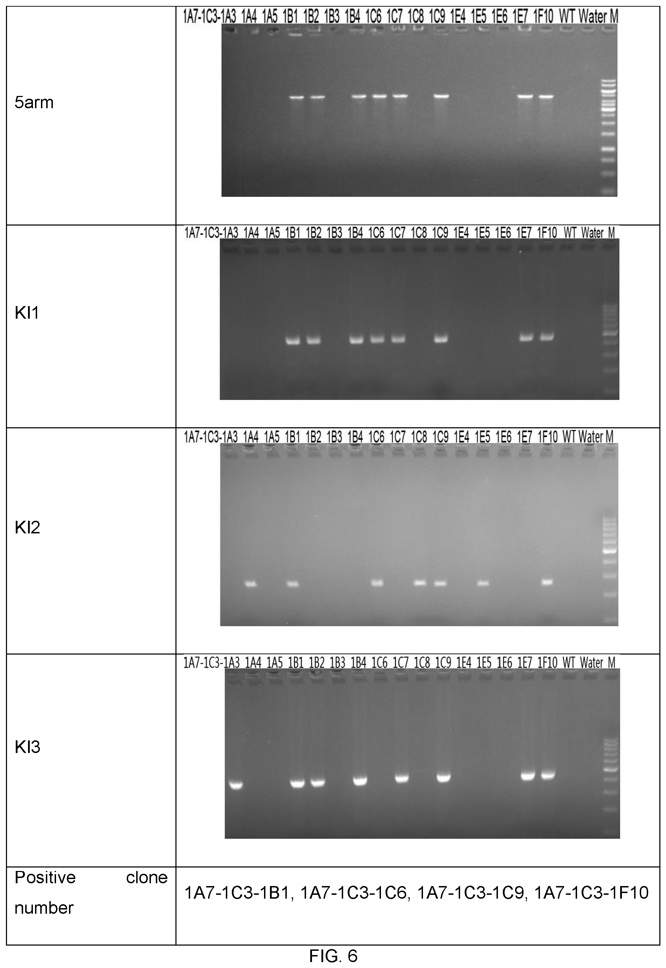

The insertion of the sequence into the corresponding position was identified by four pairs of primers (the sequences are shown in Table 3 below, and primer identification positions are simultaneously labeled in a figure (FIG. 5)). There were four clones with positive bands simultaneously amplified by the four pairs of primers (FIG. 6): 1A7-1C3-1B1, 1A7-1C3-1C6, 1A7-1C3-1C9, and 1A7-1C3-1F10, and the four clones were used as positive clones in the third step.

**Table 3.**

| Aspect | Identification region | Product size | Primer name | Primer sequence | SEQ ID NO. |
|---|---|---|---|---|---|
| ES1-V1V2V 3 | 5'arm | 4831 | H2-F1 | gaatggacaaccaatgactggc | 9 |
| | | | H1-R2 | TGGGCTATGAACTAATG ACCC | 4 |
| | KI1 | 453 | H1-F3 | gcatcgcattgtctgagtaggt | 5 |
| | | | H3-R2 | AGCGGAACACCCACAG AGAC | 17 |
| | KI2 | 256 | H3-F3 | atctgaggccgcacctgacac | 18 |
| | | | H3-R3 | CGGAACACCCACAGAG ACCGAG | 19 |
| | KI3 | 422 | H3-F4 | ggcatttctcactgtcacttctgg | 20 |
| | | | H3-R4 | CTCCTTCATGGCTCTTA GCTCC | 21 |

4. Insertion of about 20 kb of a human V genome sequence (containing all spacer sequences between IGHV6-1 and upstream of IGHD1-1) in front of a genome of the cells obtained in the third step while introducing two lox sites loxp-lox2272
A constructed vector was electroporated into the positive cell clone 1A7-1C3-1B1 in the third step: the vector carries Puro resistance and a homology arm on one end is placed on the human sequence with the positive clones in the second step, so the Puro resistance will be deleted by homologous recombination in this step, and both ends of Puro resistance carry loxP and lox2272-PB5' (5' ITR, 5' inverted terminal repeat) elements, respectively; and cells were subjected to Puromycin drug screening and related ES clones were selected for culture, amplified and identified by PCR typing.

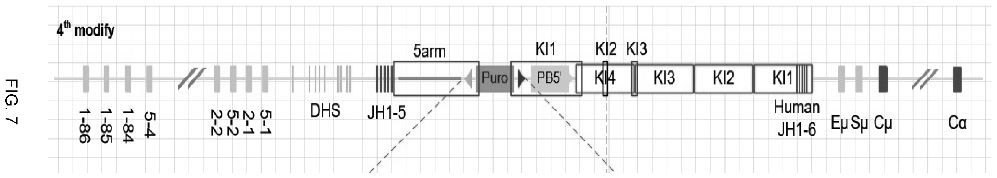

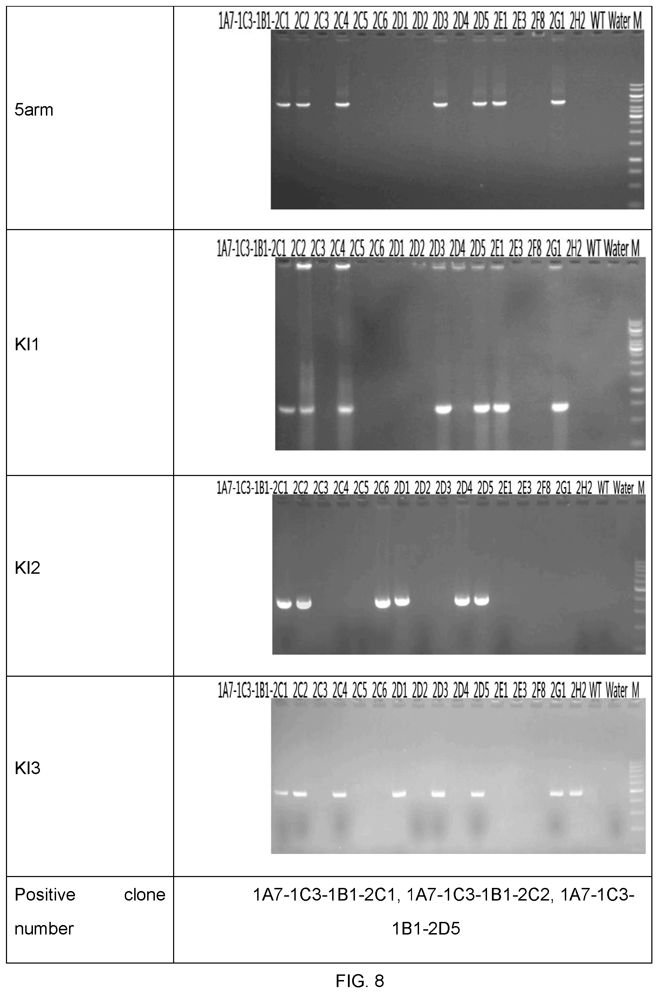

The insertion of the sequence into the corresponding position was identified by four pairs of primers (the sequences are shown in Table 4 below, and primer identification positions are simultaneously labeled in a figure (FIG. 7)). There were three clones with positive bands simultaneously amplified by the four pairs of primers (FIG. 8): 1A7-1C3-1B1-2C1, 1A7-1C3-1B1-2C2, and 1A7-1C3-1B1-2D5, and the three clones were used as positive clones in the fourth step.

**Table 4**

| Aspect | Identification region | Product size | Primer name | Primer sequence | SEQ ID NO. |
|---|---|---|---|---|---|
| ES1-V1V2V3 V4 | 5'arm | 5066 | H2-F1 | gaatggacaaccaatgactggc | 9 |
| | | | H2-R1 | CGTGCTACTTCCATTT GTCACG | 10 |
| | KI1 | 724 | H2-F2 | gccttctagttgccagccatc | 11 |
| | | | H4-R2 | AGGTCAGCATTCTGGT CCAGG | 22 |
| | KI2 | 402 | H4-F3 | cctgagagaatgatgtgctgaga cc | 23 |
| | | | H4-R3 | GGAATGAGGGTTGGG TGTCCAA | 24 |
| | KI3 | 473 | H4-F4 | ggactcagcagtaaccctcagg | 25 |
| | | | H3-R2 | AGCGGAACACCCACA GAGAC | 17 |

5. Insertion of about 400 kb of a human V genome sequence (including IGHV1-2, IGHV1-3, IGHV4-4, IGHV7-4-1, IGHV2-5, IGHV3-7, IGHV3-64D, IGHV5-10-1, IGHV3-11, IGHV3-13, IGHV3-15, IGHV3-16, IGHV1-18, IGHV3-20, IGHV3-21, IGHV3-23, IGHV1-24, IGHV2-26, IGHV4-28 and all intergenic sequences) in front of a genome of the cells obtained in the fourth step
Constructed fused BAC and Cre were electroporated into the positive cell clone 1A7-1C3-1B1-2D5 in the fourth step: BAC has Neo resistance (both ends of a Neo antibody have inverted terminal repeats), and both ends of a human sequence on the BAC carry loxP and lox2272 elements which are in the same direction as those on the positive clones in the fourth step, and PB3' (3' ITR, 3' inverted terminal repeat) is present between the lox2272 element and a human genome sequence, and sequences between loxP and lox2272 on the BAC and the positive cell clones in the fourth step were replaced under the action of a Cre recombinase, and Puro resistance was replaced by the human genome sequence and Neo resistance on the BAC; and cells were subjected to G418 drug screening and related ES clones were selected for culture, amplified and identified by PCR typing.

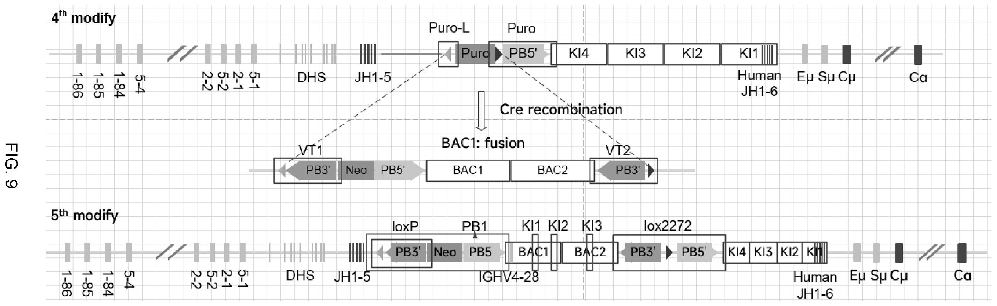

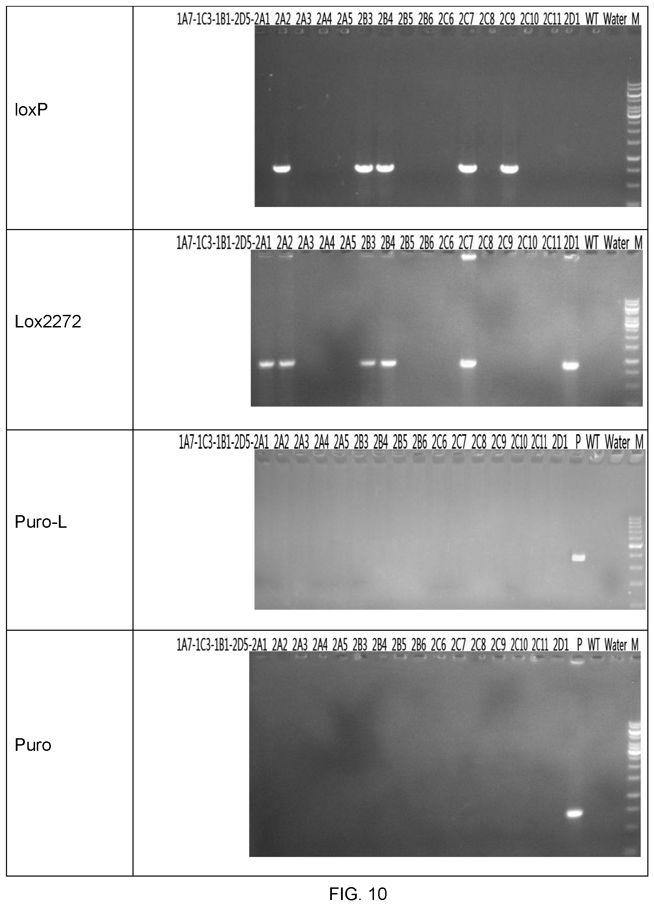

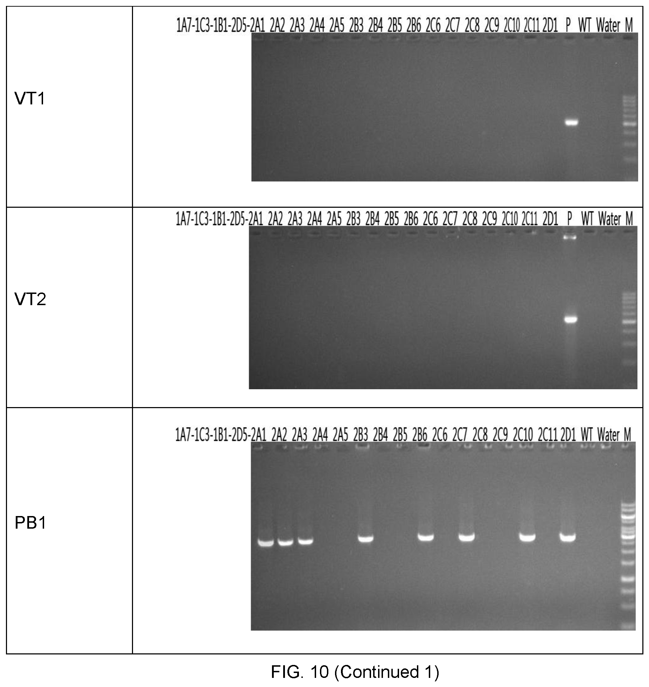

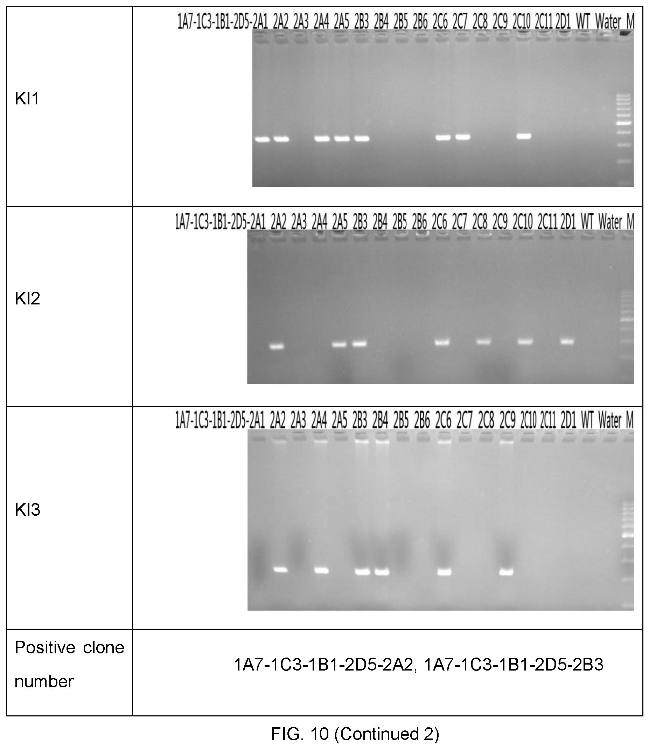

Replacement of the sequences was identified by 11 pairs of primers (the sequences are shown in Table 5 below, and primer identification positions are simultaneously labeled in a figure (FIG. 9)). There were two clones with positive bands simultaneously amplified by the 11 pairs of primers (FIG. 10): 1A7-1C3-1B1-2D5-2A2 and 1A7-1C3-1B1-2D5-2B3, and the two clones were used as positive clones in the fifth step.

**Table 5**

| Aspect | Identification region | Product size | Primer name | Primer sequence | SEQ ID NO. |
|---|---|---|---|---|---|
| ES1-FV+BA C | loxP | 807 | H1-F2 | acaggcagggaacagaatgtg | 3 |
| | | | H1-R2 | TGGGCTATGAACTAATG ACCC | 4 |
| | Iox2272 | 889 | H4-F4 | ggactcagcagtaaccctcagg | 25 |
| | | | H4-R2 | AGGTCAGCATTCTGGTC CAGG | 22 |
| | Puro-L | 360 | H5-F3 | gatgatgggatagggactttgg | 26 |
| | | | H2-R1 | CGTGCTACTTCCATTTG TCACG | 10 |
| | Puro | 724 | H2-F2 | gccttctagttgccagccatc | 11 |
| | | | H4-R2 | AGGTCAGCATTCTGGTC CAGG | 22 |
| | VT1 | 508 | H5-F5 | gtgataataagcggatgaatgg | 27 |
| | | | H1-R2 | TGGGCTATGAACTAATG ACCC | 4 |
| | VT2 | 574 | H4-F4 | ggactcagcagtaaccctcagg | 25 |
| | | | H5-R6 | GTGGATTGCTGCTGTGT CCTG | 28 |
| | PB1 | 2930 | H1-F2 | acaggcagggaacagaatgtg | 3 |
| | | | H5-R7 | CTCTTGTTCTGTAATTCC TCCTTCC | 29 |
| | PB2=lox2272 | 889 | H4-F4 | ggactcagcagtaaccctcagg | 25 |
| | | | H4-R2 | AGGTCAGCATTCTGGTC CAGG | 22 |
| | KI1 | 361 | H5-F9 | caagatggagtctgcctgcttc | 30 |
| | | | H5-R9 | AGACGTGGTGAACCGA GCTGT | 31 |
| | KI2 | 289 | H5-F10 | ccgtgtattactttgagggacac | 32 |
| | | | H5-R10 | CCTCATAGTGTCCTGAG CGC | 33 |
| | KI3 | 224 | H5-F11 | ccttacccacactatctcttgtgtcc | 34 |
| | | | H5-R11 | CAGGCTCCCAGGTGCT CTAATTC | 35 |

6. Electroporation of pBase (a PiggyBac transposase) on the cell clones obtained in the fifth step
pBase (the PiggyBac transposase) was electroporated into the positive cell clone 1A7-1C3-1B1-2D5-2B3 in the fifth step: under the action of the PiggyBac transposase, two inverted terminal repeats will be cut from a genome, a Neo sequence and lox2272 will be lost, and the positive cell clone will have only the inserted human gene sequence and one loxP element; and ES clones were selected for culture, amplified and identified by PCR typing.

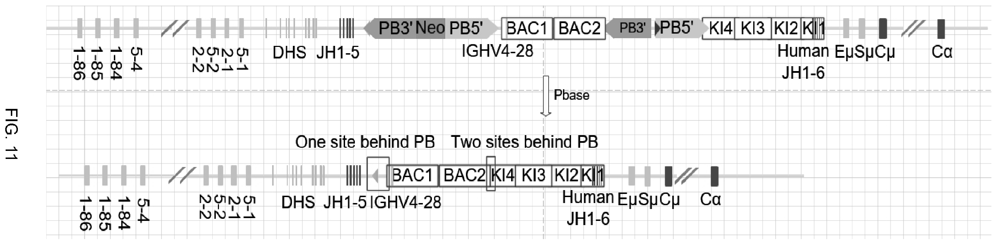

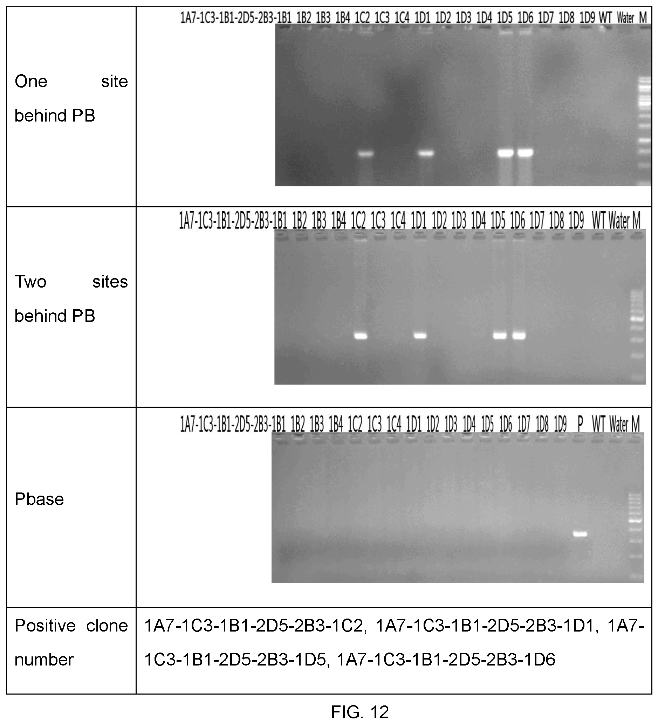

Deletion of sequences was identified by two pairs of primers (the sequences are shown in Table 6 below, and primer identification positions are simultaneously labeled in a figure (FIG. 11)). There were four clones with positive bands simultaneously amplified by the pairs of primers (FIG. 12): 1A7-1C3-1B1-2D5-2B3-1C2, 1A7-1C3-1B1-2D5-2B3-1D1, 1A7-1C3-1B1-2D5-2B3-1D5, and 1A7-1C3-1B1-2D5-2B3-1D6, and the four clones were used as positive clones in the sixth step.

**Table 6.**

| Aspect | Identification region | Product size | Primer name | Primer sequence | SEQ ID NO. |
|---|---|---|---|---|---|
| ES1-FV+BA C-PB | One site behind PB | 735 | H1-F2 | acaggcagggaacagaatgtg | 3 |
| | | | H5-R7 | CTCTTGTTCTGTAATTC CTCCTTCC | 29 |
| | Two sites behind PB | 307 | H4-F4 | ggactcagcagtaaccctcagg | 25 |
| | | | H4-R2 | AGGTCAGCATTCTGGT CCAGG | 22 |
| | pbase | 352 | pbase-F1 | CTGGACGAGCAGAAC GTGATCG | 36 |
| | | | pbase-R1 | CGAAGAAGGCGTAGAT CTCGTCCTC | 37 |

7. F0 mice obtained from ES1
The positive cell clones obtained in the sixth step were injected into a blastocyst, and the blastocyst was then transplanted into surrogate female mice, and mice were born by gestation of about 20 days; and paws of young mice of 5-7 d were cut, DNA was extracted, and genotypes of the mice were confirmed by PCR typing identification.

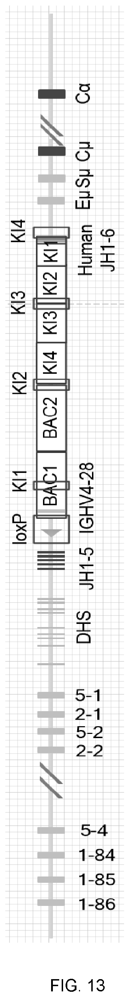

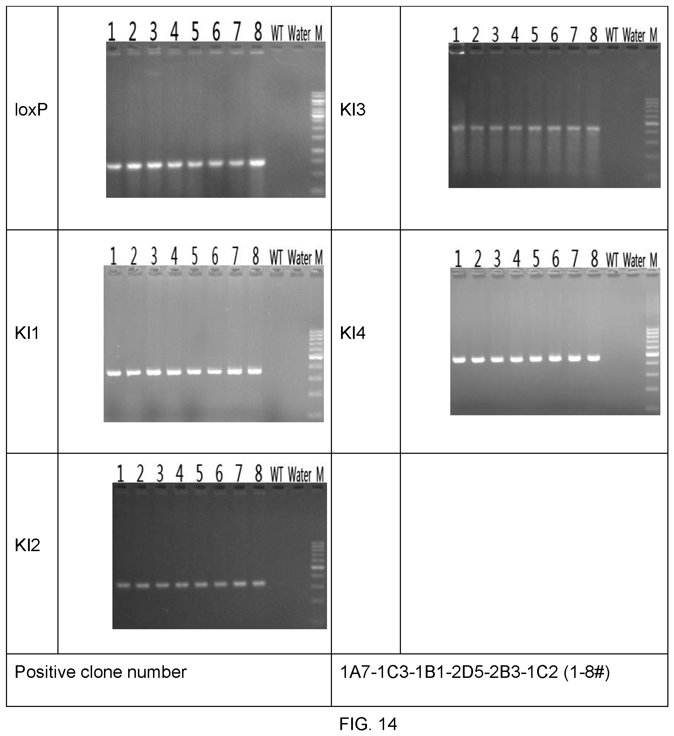

A total of 8 mice were born after injection of the clone 1A7-1C3-1B1-2D5-2B3-1C2, and all the 8 mice were co-identified as positive mice by 5 pairs of primers (Table 7) (FIGS. 13 and 14).

**Table 7.**

| Aspect | Identification region | Product size | Primer name | Primer sequence | SEQ ID NO. |
|---|---|---|---|---|---|
| ES1-F0 | loxP | 735 | H1-F2 | acaggcagggaacagaatgtg | 3 |
| | | | H5-R7 | CTCTTGTTCTGTAATTCC TCCTTCC | 29 |
| | KI1 | 361 | H5-F9 | caagatggagtctgcctgcttc | 30 |
| | | | H5-R9 | AGACGTGGTGAACCGAG CTGT | 31 |
| | KI2 | 307 | H4-F4 | ggactcagcagtaaccctcagg | 25 |
| | | | H4-R2 | AGGTCAGCATTCTGGTC CAGG | 22 |
| | KI3 | 432 | H3-F4 | ggcatttctcactgtcacttctgg | 20 |
| | | | H6-R4 | CCATATCTTGCTCCTTCA TGGC | 38 |
| | KI4 | 460 | H1-F1 | gcccatttgaaggagaggtcg | 1 |
| | | | H6-R5 | GCAGGCTCCACCAGACC TCTC | 39 |

8. F1 mice obtained from ES1
The F0 mice that were identified as the positive mice were mated with CMV-Cre mice to obtain F1 generation mice. Genotypes of the mice were confirmed by PCR typing identification of F1 generation mouse tail genomic DNA.

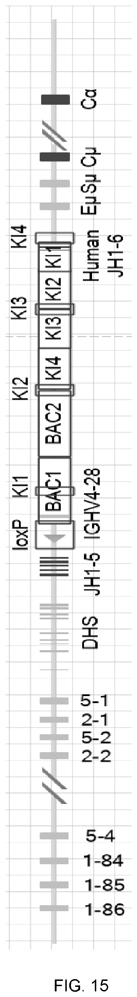

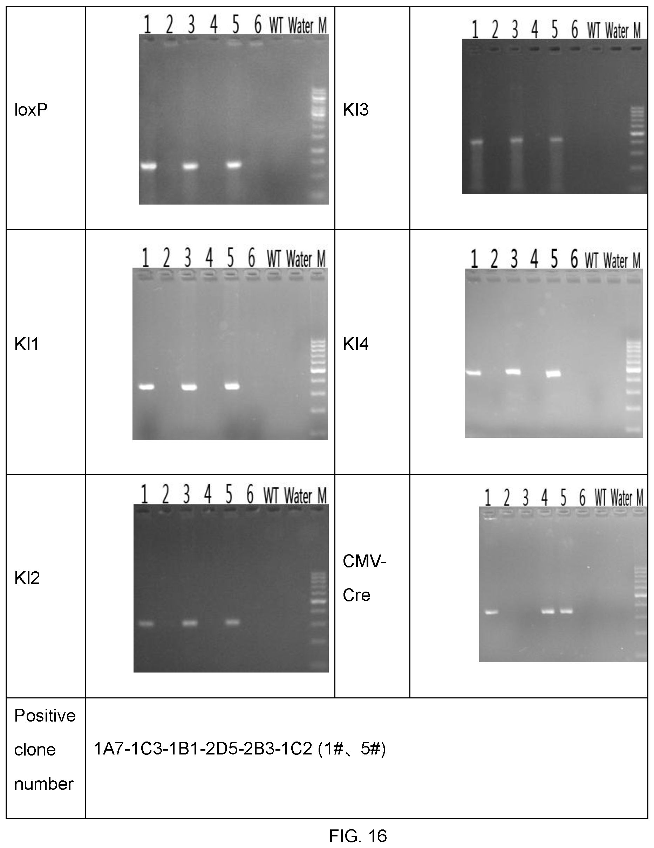

A total of 6 F1 mice were born, ES1 was co-identified with 5 pairs of primers (Table 8), CMV-Cre was identified with 1 pair of primers, and a total of 2 mice (1# and 5#) were double gene positive mice (FIGS. 15 and 16).

**Table 8.**

| Aspect | Identification region | Product size | Primer name | Primer sequence | SEQ ID NO. |
|---|---|---|---|---|---|
| ES1-F1 | loxP | 735 | H1-F2 | acaggcagggaacagaatgtg | 3 |
| | | | H5-R7 | CTCTTGTTCTGTAATTC CTCCTTCC | 29 |
| | KI1 | 361 | H5-F9 | caagatggagtctgcctgcttc | 30 |
| | | | H5-R9 | AGACGTGGTGAACCGA GCTGT | 31 |
| | KI2 | 307 | H4-F4 | ggactcagcagtaaccctcagg | 25 |
| | | | H4-R2 | AGGTCAGCATTCTGGTC CAGG | 22 |
| | KI3 | 432 | H3-F4 | ggcatttctcactgtcacttctgg | 20 |
| | | | H6-R4 | CCATATCTTGCTCCTTC ATGGC | 38 |
| | KI4 | 460 | H1-F1 | gcccatttgaaggagaggtcg | 1 |
| | | | H6-R5 | GCAGGCTCCACCAGAC CTCTC | 39 |
| | CMV-Cre | 349 | CMV-MF2 | GTAGGCGTGTACGGTG GGAGGT | 40 |
| | | | CMV-MR | TCCAGGTATGCTCAGAA AACGCC | 41 |

### II. Mouse construction based on an ES cell line II

1. Insertion of about 20 kb of a human gene sequence (including IGHV3-74, IGHV3-73, IGHV3-72 and all intergenic sequences) between a J region and a C region of mice

A constructed vector was electroporated into wild-type ES cells. The vector was Neo-resistant. The cells were subjected to G418 drug screening. Related ES clones were selected for culture, amplified and identified by PCR typing.

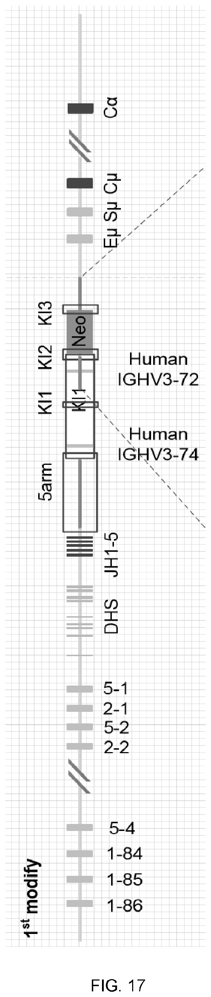

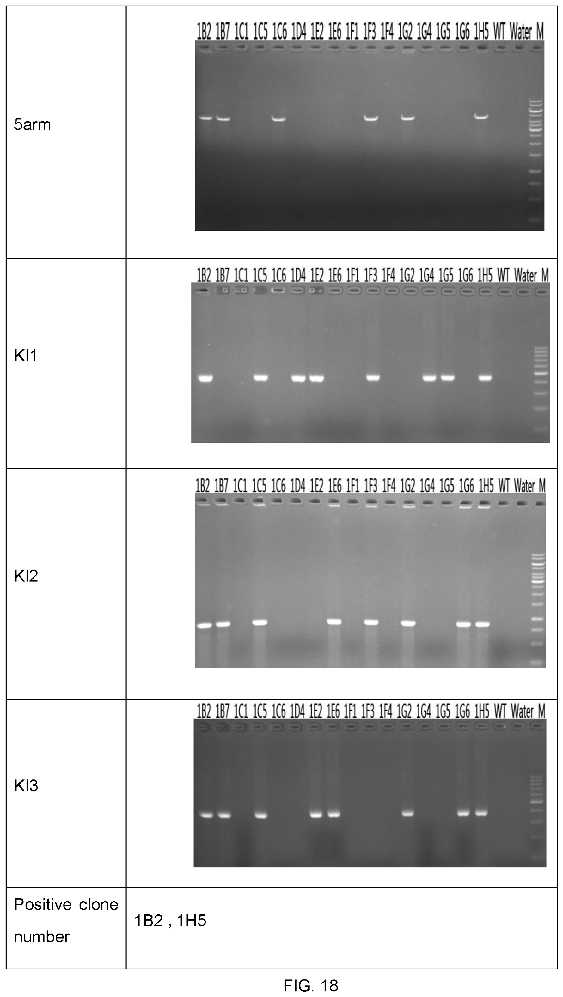

The insertion of the sequence into the corresponding position was identified by four pairs of primers (the sequences are shown in Table 9 below, and primer identification positions are simultaneously labeled in a figure (FIG. 17)). There were two clones with positive bands simultaneously amplified by the four pairs of primers (FIG. 18): 1B2 and 1H5, and the two clones were used as positive clones in the first step.

**Table 9.**

| Aspect | Identification region | Product size | Primer name | Primer sequence | SEQ ID NO. |
|---|---|---|---|---|---|
| ES2-V1 | 5'arm | 4870 | H2-F1 | gaatggacaaccaatgactggc | 9 |
| | | | H1-1R | GACTCAGCAAACGCTT CTCTGG | 42 |
| | KI1 | 420 | H1-2F | tctcaactccatcgtgacggtg | 43 |
| | | | H1-2R | GCACCACCTTCTTGTC AGTTCAGC | 44 |
| | KI2 | 895 | H1-3F | gacaggtcccaggagcaggtg | 45 |
| | | | H1-3R | AATGGAAAGTCCCTATT GGCGTTAC | 46 |
| | KI3 | 370 | H1-4F | tgcatcgcattgtctgagtagg | 47 |
| | | | H1-4R | TTGAGACCGAGGCTAG ATGCC | 48 |

2. Insertion of about 20 kb of a human V genome sequence (including IGHV2-70, IGHV1-69D and all intergenic sequences) downstream of the inserted genome of the cells obtained in the first step while introducing two lox sites loxp-lox5171
A constructed vector was electroporated into the positive cell clone 1H5 in the first step: the vector carries Puro resistance and a homology arm on one end is placed on the human sequence with the positive clones in the first step, so Neo resistance will be deleted by homologous recombination in this step, and both ends of Puro resistance carry PB3' (3' ITR, 3' inverted terminal repeat)-lox5171 and loxP elements, respectively; cells were subjected to Puromycin drug screening and related ES clones were selected for culture, amplified and identified by PCR typing.

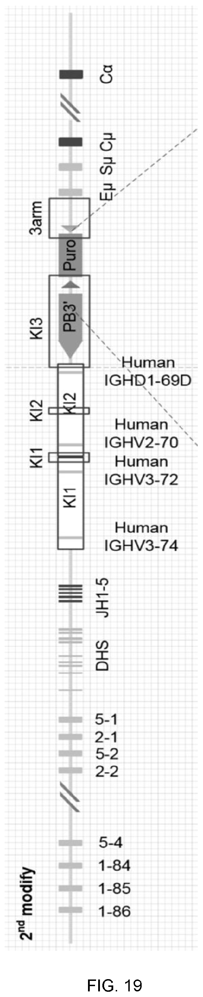

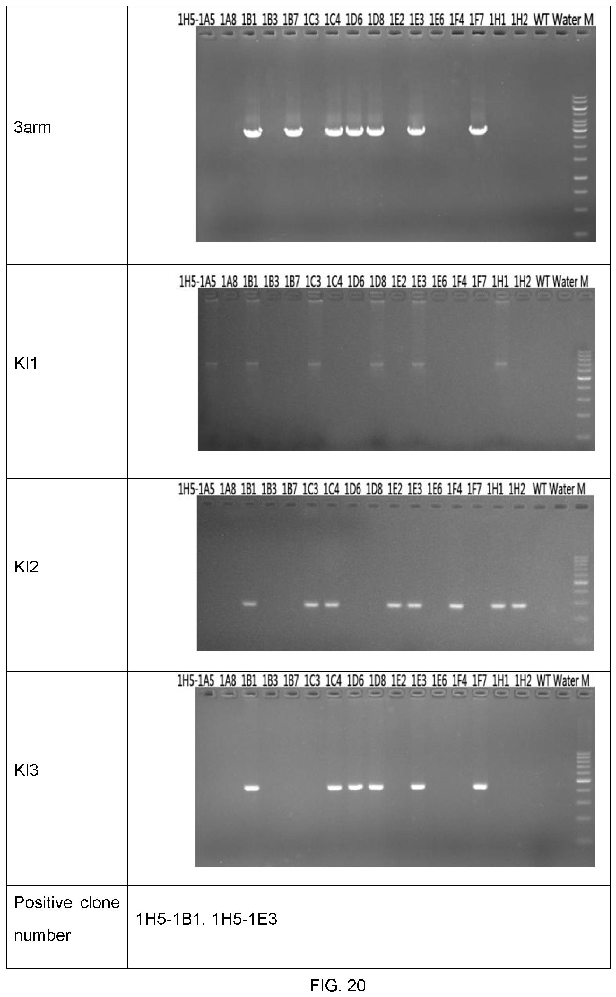

The insertion of the sequence into the corresponding position was identified by four pairs of primers (the sequences are shown in Table 10 below, and primer identification positions are simultaneously labeled in a figure (FIG. 19)). There were two clones with positive bands simultaneously amplified by the four pairs of primers (FIG. 20): 1H5-1B1 and 1H5-1E3, and the two clones were used as positive clones in the second step.

**Table 10.**

| Aspect | Identification region | Product size | Primer name | Primer sequence | SEQ ID NO. |
|---|---|---|---|---|---|
| ES2-V1V2 | 3'arm | 3032 | H2-1F | cataccgacgatctgcgacc | 49 |
| | | | H1-R1 | CATCTCAGCTCAGAACA GTCCAGTG | 2 |
| | KI1 | 690 | H2-2F | ctggagccttatgaggttgcac | 50 |
| | | | H2-2R | GGCCTGATCCAAAGAGA GGC | 51 |
| | KI2 | 277 | H2-3F | cagggaggaatctgggaccatc | 52 |
| | | | H2-3R | AATGCTGGTTACTTGTGC TTGAATT | 53 |
| | KI3 | 436 | H2-4F | agaaggtgggagcatgatgagtc | 54 |
| | | | H2-4R | GCCAGAGGCCACTTGTG TAGC | 55 |

3. Insertion of 400 kb of a human V genome sequence (including IGHV1-69-2, IGHV2-70D, IGHV1-69, IGHV3-66, IGHV3-64, IGHV4-61, IGHV4-59, IGHV1-58, IGHV3-53, IGHV8-5-51, IGHV5-51, IGHV3-49, IGHV3-48, IGHV1-46, IGHV1-45, IGHV3-43, IGHV4-39, IGHV3-38, IGHV3-35, IGHV4-34, IGHV3-33, IGHV4-31, IGHV3-30 and all intergenic sequences) downstream of a genome of the cells obtained in the second step
Constructed fused BAC and Cre were electroporated into the positive cell clone 1H5-1B1 in the second step: BAC has Neo resistance (both ends of Neo resistance have inverted terminal repeats), and both ends of a human sequence on the BAC carry loxP and lox5171 elements which are in the same direction as those on the positive clones in the second step, and PB5' (5' ITR, 5' inverted terminal repeat) is present between the lox5171 element and a human genome sequence, and sequences between loxP and lox5171 on the BAC and the positive cell clones in the second step were replaced under the action of a Cre recombinase, and Puro resistance was replaced by the human genome sequence and Neo resistance on the BAC; and cells were subjected to G418 drug screening and related ES clones were selected for culture, amplified and identified by PCR typing.

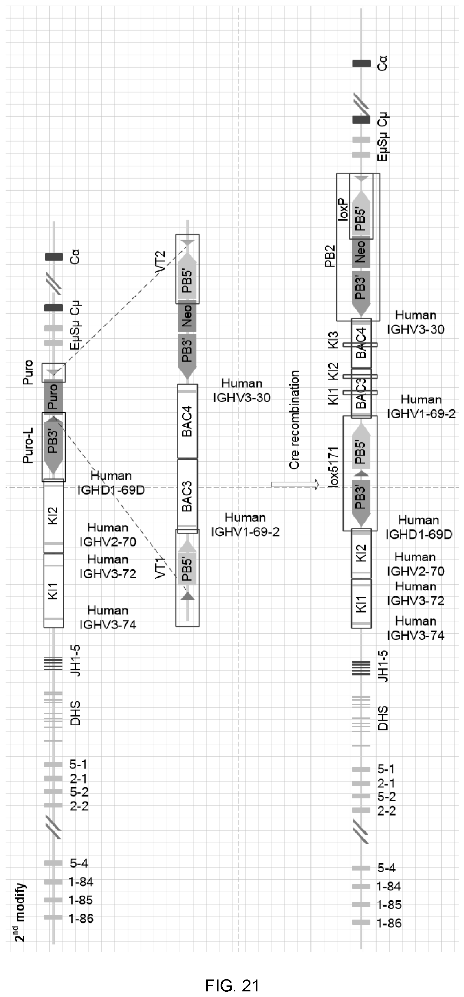

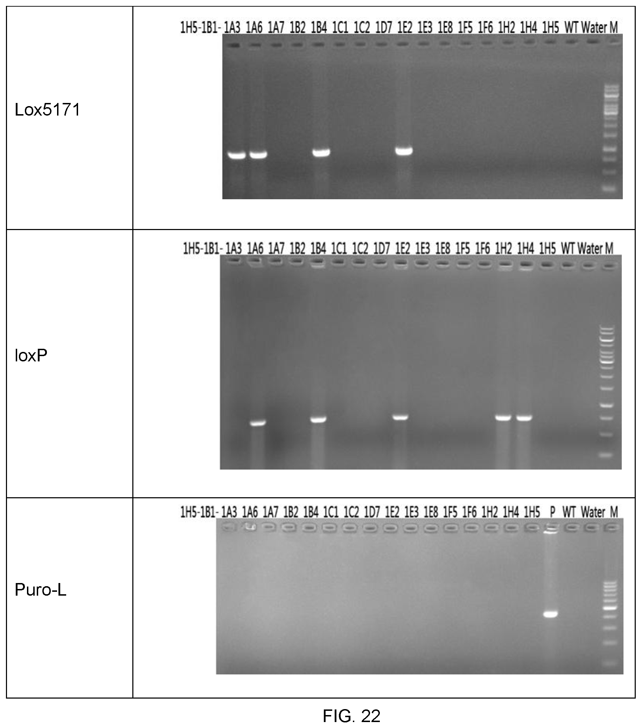

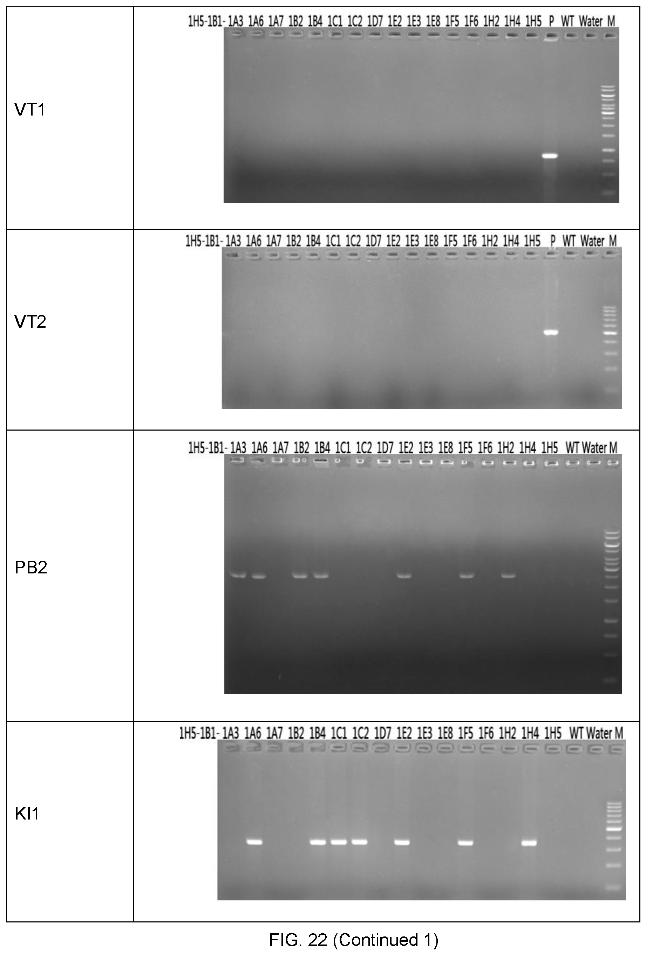

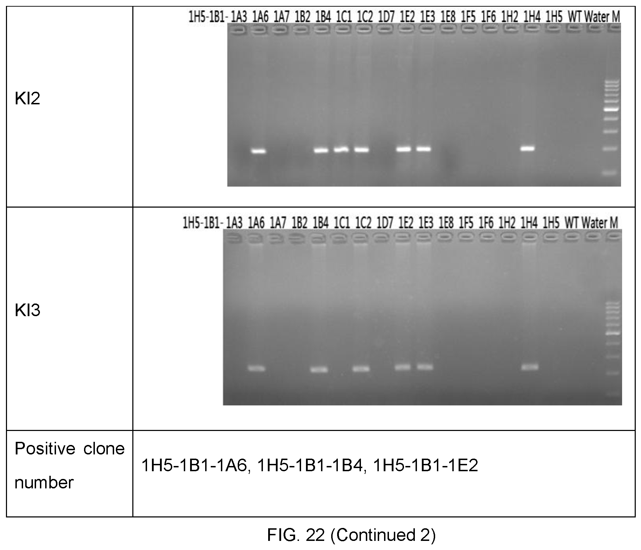

Replacement of the sequences was identified by 11 pairs of primers (the sequences are shown in Table 11 below, and primer identification positions are simultaneously labeled in a figure (FIG. 21)). There were three clones with positive bands simultaneously amplified by the 11 pairs of primers (FIG. 22): 1H5-1B1-1A6, 1H5-1B1-1B4, and 1H5-1B1-1E2, and the three clones were used as positive clones in the third step.

**Table 11.**

| Aspect | Identification region | Product size | Primer name | Primer sequence | SEQ ID NO. |
|---|---|---|---|---|---|
| ES2-FV+BA C | lox5171 | 991 | H3-1F | agaaggtgggagcatgatgagt c | 56 |
| | | | H3-1R | TGGTGTCTCTTTGCGG GCTT | 57 |
| | loxP | 717 | H1-4F | tgcatcgcattgtctgagtagg | 47 |
| | | | H1-4R | TTGAGACCGAGGCTAG ATGCC | 48 |
| | Puro-L | 436 | H2-4F | agaaggtgggagcatgatgagt c | 54 |
| | | | H2-4R | GCCAGAGGCCACTTG TGTAGC | 55 |
| | Puro=loxP | 391 | H1-4F | tgcatcgcattgtctgagtagg | 47 |
| | | | H1-4R | TTGAGACCGAGGCTAG ATGCC | 48 |
| | VT1 | 896 | H3-5F | tggttgctacgcctgaataagtg | 58 |
| | | | H3-1R | TGGTGTCTCTTTGCGG GCTT | 57 |
| | VT2 | 526 | H1-4F | tgcatcgcattgtctgagtagg | 47 |
| | | | H3-6R | ATGCAAGTGTGTCGCT GTCG | 59 |
| | PB1=lox5171 | 991 | H3-1F | agaaggtgggagcatgatgagt c | 56 |
| | | | H3-1R | TGGTGTCTCTTTGCGG GCTT | 57 |
| | PB2 | 2567 | H3-8F | ggtgtgtcaagttgtcctctccat | 60 |
| | | | H1-4R | TTGAGACCGAGGCTAG ATGCC | 48 |
| | KI1 | 347 | H3-9F | catggtggtgcttgaggattgc | 61 |
| | | | H3-9R | GAGAGCATTGATGAAC ACTGATGG | 62 |
| | KI2 | 204 | H3-10F | gggcctgggtagggaagtgtt | 63 |
| | | | H3-10R | CCACTGGCAGCAGTG AGATGAG | 64 |
| | KI3 | 227 | H3-11F | tgcaccaggaactcaaggaag atc | 65 |
| | | | H3-11R | CATCCAGCCTGCTTGA ACTCC | 66 |

4. Electroporation of pBase (a PiggyBac transposase) on the cell clones obtained in the third step
pBase (the PiggyBac transposase) was electroporated into the positive cell clone 1H5-1B1-1A6 in the third step: under the action of the PiggyBac transposase, two inverted terminal repeats will be cut from a genome, a Neo sequence and lox5171 will be lost, and the positive cell clone will have only the inserted human gene sequence and one loxP element; and ES clones were selected for culture, amplified and identified by PCR typing.

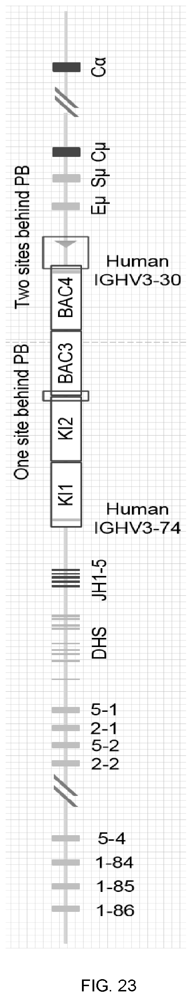

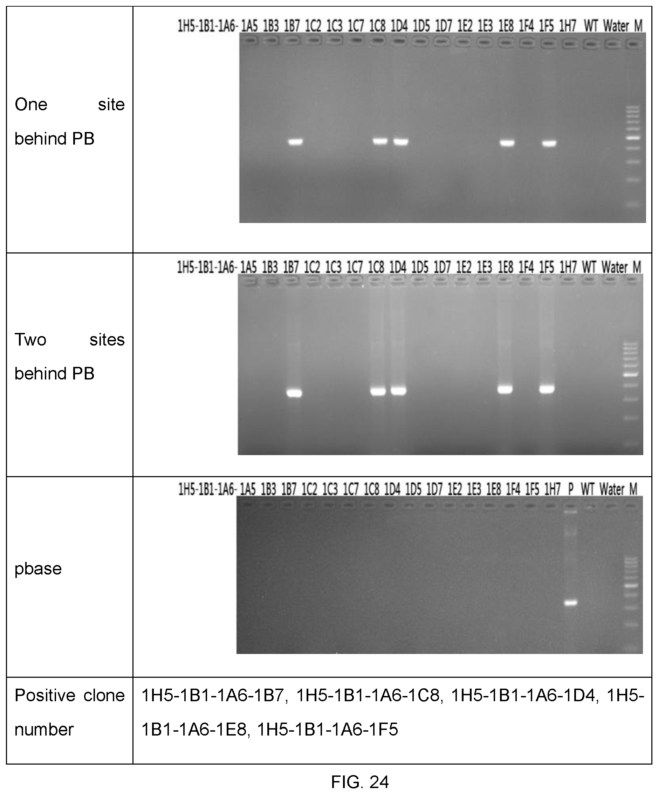

Deletion of sequences was identified by three pairs of primers (the sequences are shown in Table 12 below, and primer identification positions are simultaneously labeled in a figure (FIG. 23)). There were five clones with positive bands simultaneously amplified by the three pairs of primers (FIG. 24): 1H5-1B1-1A6-1B7, 1H5-1B1-1A6-1C8, 1H5-1B1-1A6-1D4, 1H5-1B1-1A6-1E8, and 1H5-1B1-1A6-1F5, and the five clones were used as positive clones in the third step.

**Table 12.**

| Aspect | Identification region | Product size | Primer name | Primer sequence | SEQ ID NO. |
|---|---|---|---|---|---|
| ES2-FV+BA C-PB | One site behind PB | 409 | H3-1F | agaaggtgggagcatgatgag tc | 56 |
| | | | H3-1R | TGGTGTCTCTTTGCG GGCTT | 57 |
| | Two sites behind PB | 372 | H3-8F | ggtgtgtcaagttgtcctctccat | 60 |
| | | | H1-4R | TTGAGACCGAGGCTA GATGCC | 48 |
| | pbase | 352 | pbase-F1 | CTGGACGAGCAGAAC GTGATCG | 36 |
| | | | pbase-R1 | CGAAGAAGGCGTAGA TCTCGTCCTC | 37 |

5. F0 mice obtained from ES2
The positive cell clones obtained in the fourth step were injected into a blastocyst, and the blastocyst was then transplanted into surrogate female mice, and mice were born by gestation of about 20 days; and paws of young mice of 5-7 d were cut, DNA was extracted, and genotypes of the mice were confirmed by PCR typing identification.

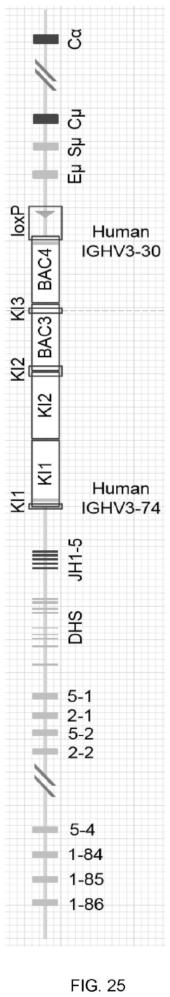

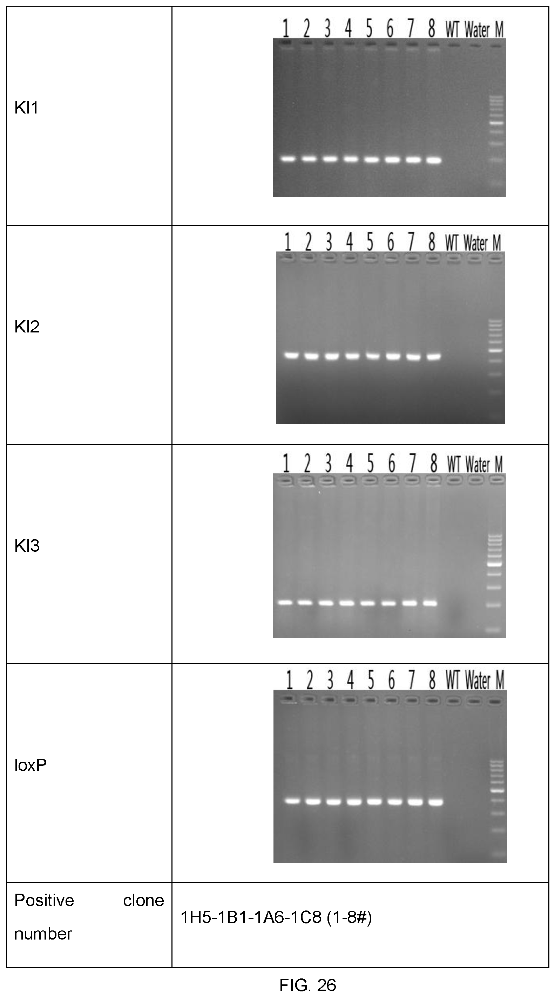

A total of 8 mice were born after injection of the clone 1H5-1B1-1A6-1C8, and all the 8 mice were co-identified as positive mice by 4 pairs of primers (Table 13) (FIGS. 25 and 26).

**Table 13.**

| Aspect | Identification region | Product size | Primer name | Primer sequence | SEQ ID NO. |
|---|---|---|---|---|---|
| ES2-F0 | KI1 | 190 | H4-1F | gtgtgatggtgttggtggagtcc | 67 |
| | | | H1-1R | GACTCAGCAAACGCTT CTCTGG | 42 |
| | KI2 | 409 | H2-4F | agaaggtgggagcatgatgagtc | 54 |
| | | | H3-1R | TGGTGTCTCTTTGCGG GCTT | 57 |
| | KI3 | 204 | H3-10F | gggcctgggtagggaagtgtt | 63 |
| | | | H3-10R | CCACTGGCAGCAGTGA GATGAG | 64 |
| | loxP | 372 | H3-8F | ggtgtgtcaagttgtcctctccat | 60 |
| | | | H1-4R | TTGAGACCGAGGCTAG ATGCC | 48 |

6. F1 mice obtained from ES2
The F0 mice that were identified as the positive mice were mated with wild type mice to obtain F1 generation mice. Genotypes of the mice were confirmed by PCR typing identification of F1 generation mouse tail genomic DNA.

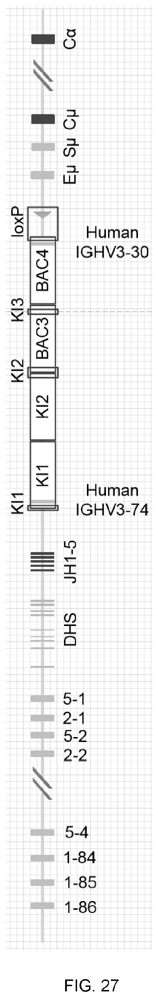

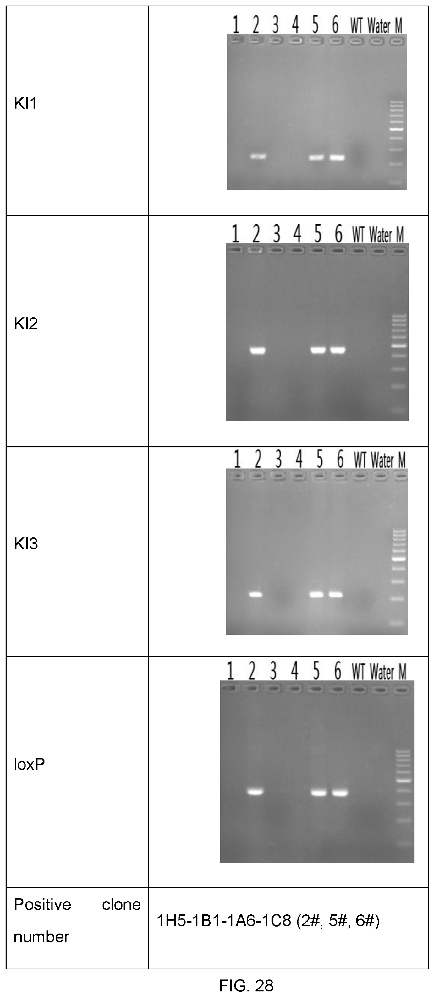

A total of 6 F1 mice were born, ES2 was co-identified with 4 pairs of primers (Table 14), and a total of 3 mice (2#, 5#, and 6#) were double gene positive mice (FIGS. 27 and 28).

**Table 14.**

| Aspect | Identification region | Product size | Primer name | Primer sequence | SEQ ID NO. |
|---|---|---|---|---|---|
| ES2-F1 | KI1 | 190 | H4-1F | gtgtgatggtgttggtggagtcc | 67 |
| | | | H1-1R | GACTCAGCAAACGCTT CTCTGG | 42 |
| | KI2 | 409 | H2-4F | agaaggtgggagcatgatgagt c | 54 |
| | | | H3-1R | TGGTGTCTCTTTGCGG GCTT | 57 |
| | KI3 | 204 | H3-10F | gggcctgggtagggaagtgtt | 63 |
| | | | H3-10R | CCACTGGCAGCAGTG AGATGAG | 64 |
| | loxP | 372 | H3-8F | ggtgtgtcaagttgtcctctccat | 60 |
| | | | H1-4R | TTGAGACCGAGGCTA GATGCC | 48 |

Double gene positive F1 mice of ES1 were hybridized with the F1 positive mice obtained from ES2 to obtain Cre-positive, and ES1-positive and ES2-positive F2 generation mice; the obtained F2 positive mice were mated with wild type mice to obtain a blastocyst of 3.5 d, ES cell lines were established, and different ES cell lines were tested by PCR, and cells that do not carry Cre but have undergone ectopia encompassing the human genomic sequences of ES1 and ES2 and having a loxP sequence between IGHV3-30 and IGHV4-28 were screened.

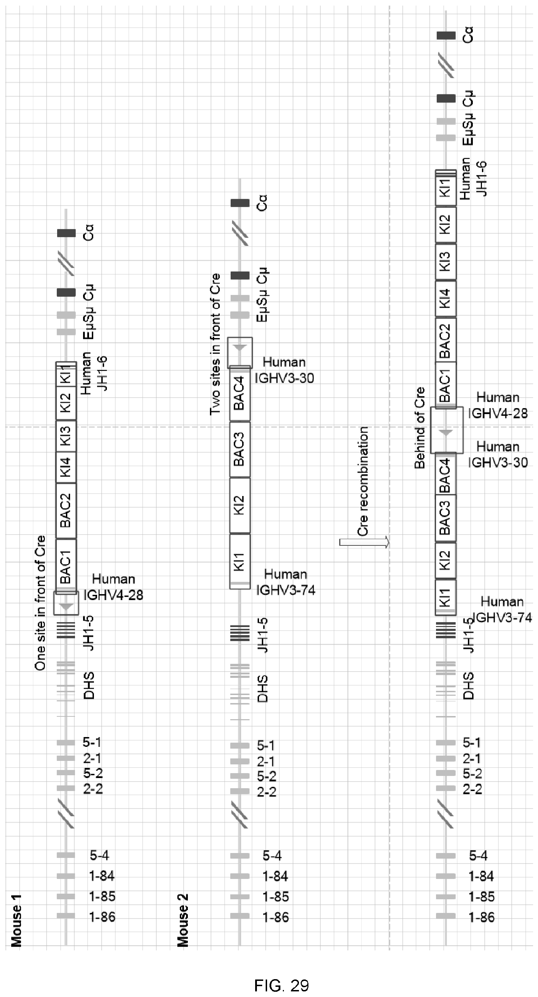

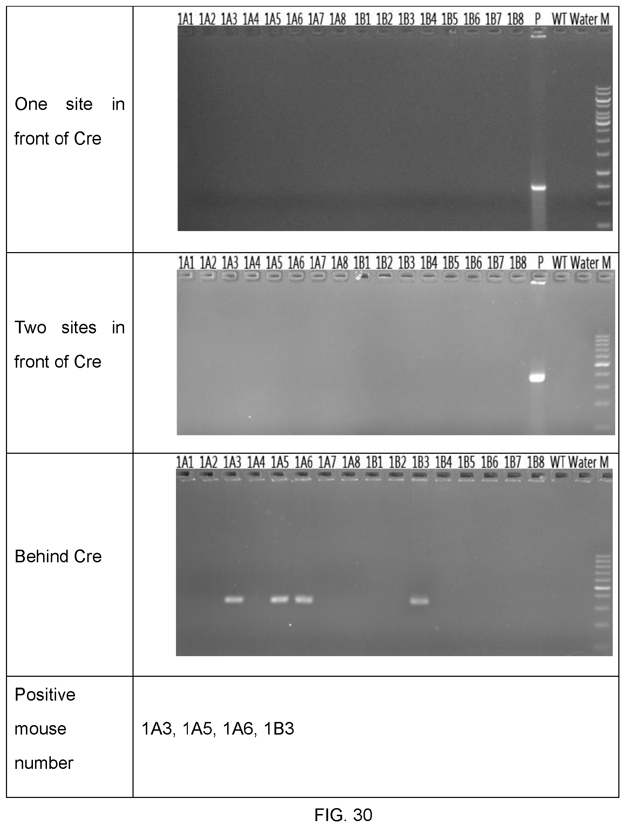

The gene was identified as ectopic by three pairs of primers (Table 15), and four clones were identified as ectopic (FIGS. 29 and 30): 1A3, 1A5, 1A6, and 1B3, and the four clones were used as an ES cell line III.

**Table 15.**

| Aspect | Identification region | Product size | Primer name | Primer sequence | SEQ ID NO. |
|---|---|---|---|---|---|
| ES1+Cre F1 Generation X ES2 F1 Generation | One site in front of Cre | 735 | H1-F2 | acaggcagggaacag aatgtg | 3 |
| | | | H5-R7 | CTCTTGTTCTGT AATTCCTCCTTC C | 29 |
| | Two sites in front of Cre | 372 | H3-8F | ggtgtgtcaagttgtcct ctccat | 60 |
| | | | H1-4R | TTGAGACCGAG GCTAGATGCC | 48 |
| | Behind Cre | 320 | Cre-F | gagcaggttcaaggct caacc | 68 |
| | | | Cre-R | GAGAGGAATCC ATAACTTCGTATA GC | 69 |

### III. Gene knockout (KO)

### 1. Performing first homologous recombination KO (a V region) on an ES cell line III

A vector with Neo resistance (both ends of Neo have recombinase sites) was constructed with a 5-terminal homology arm sequence including 10 kb of a continuous sequence upstream of mIgHV1-86 and a 3-terminal homology arm sequence including 8 kb of a continuous sequence downstream of an mlgHV5-2 gene. The constructed vector was electroporated into the ES cell line III. The cells were subjected to G418 drug screening, and related ES clones were selected for culture, amplified and identified by PCR typing.

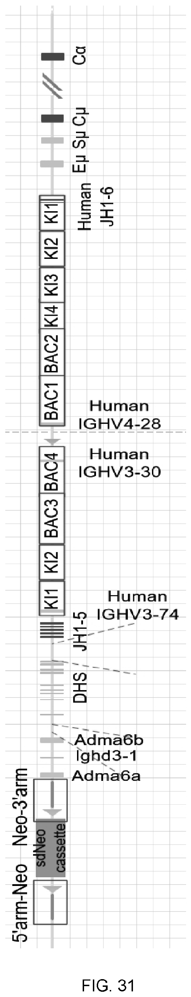

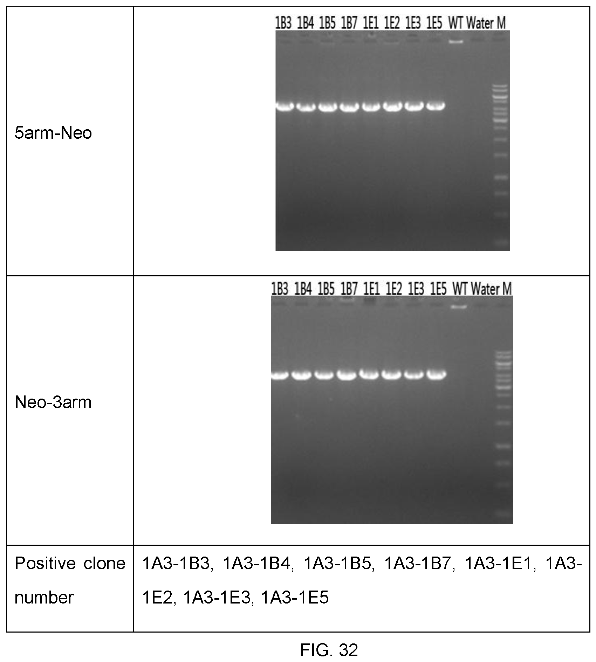

Genes (mIgHV1-86 to mIgHV5-2) in the V region were identified to have been recombined by Neo resistance by two pairs of primers (the sequences are shown in Table 16 below, and primer identification positions are simultaneously labeled in a figure (FIG. 31)), representing that mouse IgH chain V genes had undergone recombinant deletion. There were eight clones with positive bands simultaneously amplified by the two pairs of primers (FIG. 32): 1A3-1B3, 1A3-1B4, 1A3-1B5, 1A3-1B7, 1A3-1E1, 1A3-1E2, 1A3-1E3, and 1A3-1E5, and the eight clones were used as positive clones in the first step.

**Table 16. First-step KO on ES cells**

| Step | Aspect | Identification site | Size | Primer name | Primer sequence | SEQ ID NO. |
|---|---|---|---|---|---|---|
| V region KO1 | ES cells | 5arm-Neo | 4346 | H-KO1-F1 | acaaatatccacat agaagcaa | 70 |
| | | | | H-KO1-R1 | tcaggacatagcg ttggctaccc | 71 |
| | | Neo-3arm | 4067 | H-KO1-F2 | agtgaccactgtg ggcacagggga | 72 |
| | | | | H-KO1-R2 | ggtgagaattctttg ttcag | 73 |

### 2. Performing second homologous recombination KO (knockout of all gene sequences between IgHJ4 and IgHD1-1) on the cells obtained in the first step

A vector with Puro resistance (both ends of Puro have recombinase sites) was constructed with a 5-terminal homology arm sequence including 8 kb of a continuous sequence upstream of mIgHD1-1 and a 3-terminal homology arm sequence including 5 kb of a continuous sequence downstream of an mIgHJ4 gene. The constructed vector was electroporated into the positive cell clone 1A3-1B3 in the first step. The cells were subjected to Puromycin drug screening, and related ES clones were selected for culture, amplified and identified by PCR typing.

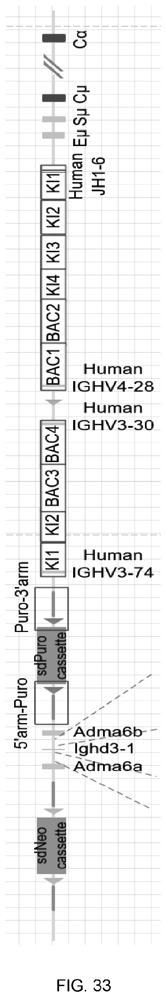

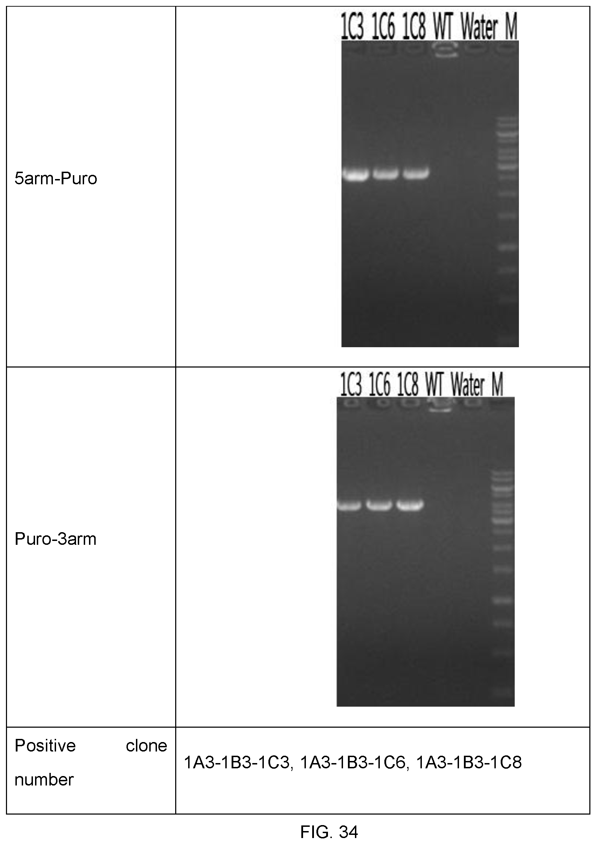

Genes between mIgHJ4 and mIgHD1-1 were identified to have been recombined by Puro resistance by two pairs of primers (the sequences are shown in Table 17 below, and primer identification positions are simultaneously labeled in a figure (FIG. 33)), representing that the genes between mIgHJ4 and mIgHD1-1 of a mouse IgH chain had undergone recombinant deletion. There were three clones with positive bands simultaneously amplified by the two pairs of primers (FIG. 34): 1A3-1B3-1C3, 1A3-1B3-1C6, and 1A3-1B3-1C8, and the three clones were used as positive clones in the second step.

**Table 17. Second-step KO on ES cell-1A3-1B3**

| Step | Aspect | Identification site | Size | Primer name | Primer sequence | SEQ ID NO. |
|---|---|---|---|---|---|---|
| D and J region KO2 | ES cells | 5arm-Puro | 2776 | H-KO2-F3 | aggtgcctggg gaatcactcag | 74 |
| | | | | H-KO2-R3 | tgtgccttctagtt gccagcc | 75 |
| | | Puro-3arm | 4016 | H-KO-F4 | gtggaatgtgtg cgaggccag | 76 |
| | | | | H-KO2-R4 | tatgatgttagct atgaacttgt | 77 |

### 3. Performing third homologous recombination KO (knockout of mIgHD3-1, mIgHD5-1 and mIgHD1-3 genes) on the cells obtained in the second step

A vector with Hygro resistance (both ends of Hygro have recombinase sites) was constructed with a 5-terminal homology arm sequence including 8 kb of a continuous sequence upstream of mIgHD1-3 gene and a 3-terminal homology arm sequence including 5 kb of a continuous sequence downstream of an mIgHD3-1 gene. The constructed vector was electroporated into the positive clone 1A3-1B3-1C6 in the second step. The cells were subjected to Hygromycin B drug screening, and related ES clones were selected for culture, amplified and identified by PCR typing.

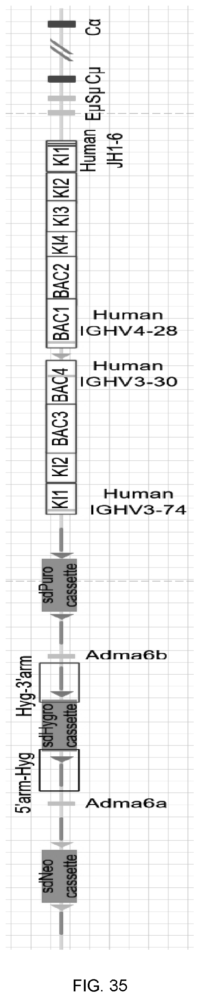

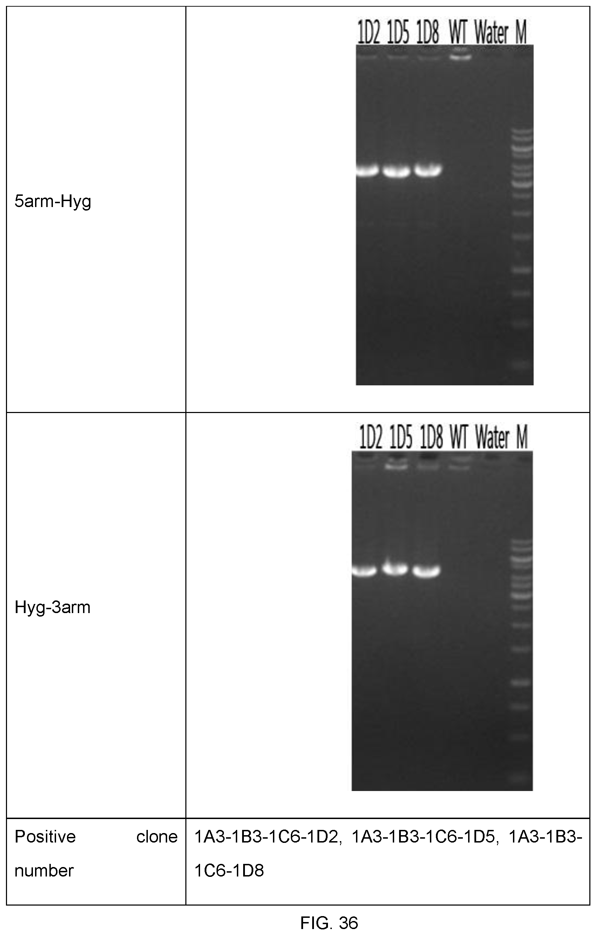

The mIgHD3-1, mIgHD5-1, and mIgHD1-3 genes were identified to have been recombined by Hygro resistance by two pairs of primers (the sequences are shown in Table 18 below, and primer identification positions are simultaneously labeled in a figure (FIG. 35)), representing that the mIgHD3-1, mIgHD5-1, and mIgHD1-3 genes of a mouse IgH chain had undergone recombinant deletion. There were three clones with positive bands simultaneously amplified by the two pairs of primers (FIG. 36): 1A3-1B3-1C6-1D2, 1A3-1B3-1C6-1D5, and 1A3-1B3-1C6-1D8, and the three clones were used as positive clones in the third step.

**Table 18. Third-step KO on ES cell-1A3-1B3-1C6**

| Step | Aspect | Identification site | Size | Primer name | Primer sequence | SEQ ID NO. |
|---|---|---|---|---|---|---|
| mIgHD3-1, mIgHD5-1 and mIgHD1-3 region KO3 | ES cells | 5arm-Hyg | 3777 | H-KO3-F5 | gtctgagccc tgtgttgaca gt | 78 |
| | | | | H-KO3-R5 | accctggaa ggtgccactc | 79 |
| | | Hyg-3arm | 4313 | H-KO3-F6 | gacttctcttg gtcccagtcc a | 80 |
| | | | | H-KO3-R6 | cacaactgg cagttatcat gta | 81 |

### 4. Injection of the positive cell clones obtained in the third step to obtain positive F0 mice

The positive cell clones obtained in the third step were injected into a blastocyst, and the blastocyst was then transplanted into surrogate female mice, and mice were born by gestation of about 20 days; and paws of young mice of 5-7 d were cut, DNA was extracted, and genotypes of the mice were confirmed by PCR typing identification.

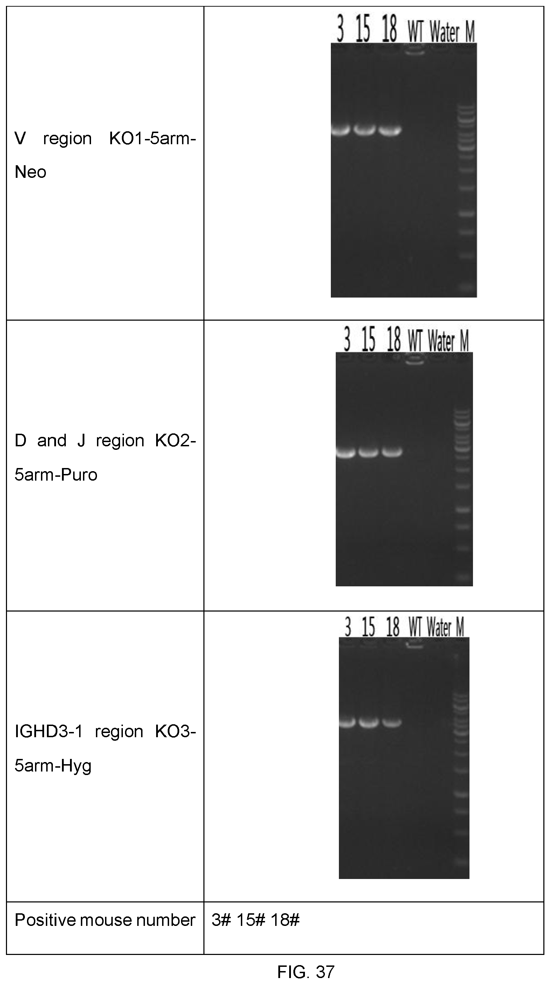

A total of 18 mice were born after injection of the clone 1A3-1B3-1C6-1D8, and three mice were co-identified as positive mice by three pairs of primers (Table 19): 3# 15# 18# (FIG. 37).

**Table 19. Injection of ES cell-1A3-1B3-1C6-1D8 after KO into F0 mice**

| Aspect | Identification region | Identifi cation site | Size | Primer name | Primer sequence | SEQ ID NO. |
|---|---|---|---|---|---|---|
| F0 mice | V region KO1 | 5arm-Neo | 4346 | H-KO1-F1 | acaaatatccacataga agcaa | 70 |
| | | | | H-KO1-R1 | tcaggacatagcgttgg ctaccc | 71 |
| | D and J region KO2 | 5arm-Puro | 2776 | H-KO2-F3 | aggtgcctggggaatca ctcag | 74 |
| | | | | H-KO2-R3 | tgtgccttctagttgccag cc | 75 |
| | IGHD3-1 region KO3 | 5arm-Hyg | 3777 | H-KO3-F5 | gtctgagccctgtgttga cagt | 78 |
| | | | | H-KO3-R5 | accctggaaggtgcca ctc | 79 |

### 5. Obtaining positive F1 mice

The F0 mice that were identified as the positive mice were mated with wild type mice to obtain F1 generation mice. Genotypes of the mice were confirmed by PCR typing identification of F1 generation mouse tail genomic DNA.

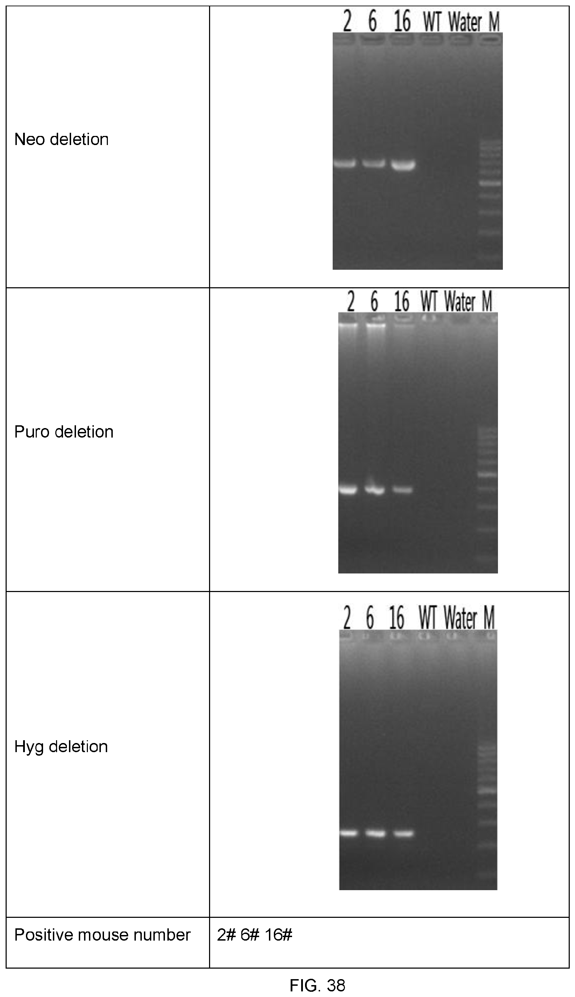

A total of 16 F1 mice were born, and co-identified by 3 pairs of primers (Table 20), and a total of 3 mice (2# 6# 16#) were double gene positive mice (FIG. 38).

**Table 20. Mating and breeding of F0-3# mice with WT to obtain F1 mice**

| Aspect | Identification region | Identification site | Size | Primer name | Primer sequence | SEQ ID NO. |
|---|---|---|---|---|---|---|
| F1 mice | V region KO1 | Neo deletion | 653 | H-KO1-F | gcctctcggcctgtcaagca g | 82 |
| | | | | H-KO1-R | cagactggtgccaggaaag ca | 83 |
| | D and J region KO2 | Puro deletion | 385 | H-KO2-F | agtgctcacacagctctgcc c | 84 |
| | | | | H-KO2-R | ggtgtctgttacttttgcagaa | 85 |
| | IGHD3-1 region KO3 | Hyg deletion | 259 | H-KO3-F | agtgctcacacagctctgcc c | 86 |
| | | | | H-KO3-R | ggtgtctgttacttttgcagaa | 87 |

### 6. Obtaining homozygous mice

The female and male F1 mice that were identified as the positive mice were mated with each other to obtain homozygous mice. Genotypes of the mice were confirmed by PCR typing identification of F2 generation mouse tail genomic DNA.

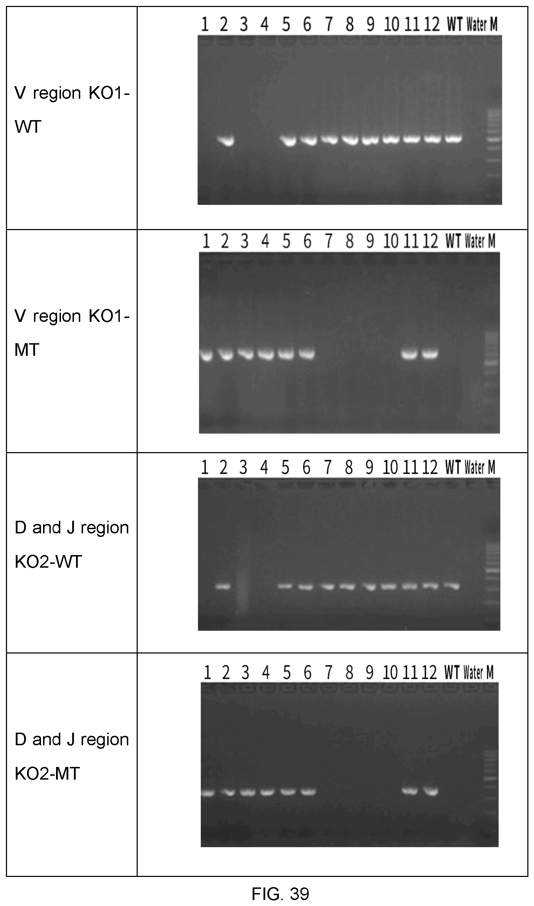

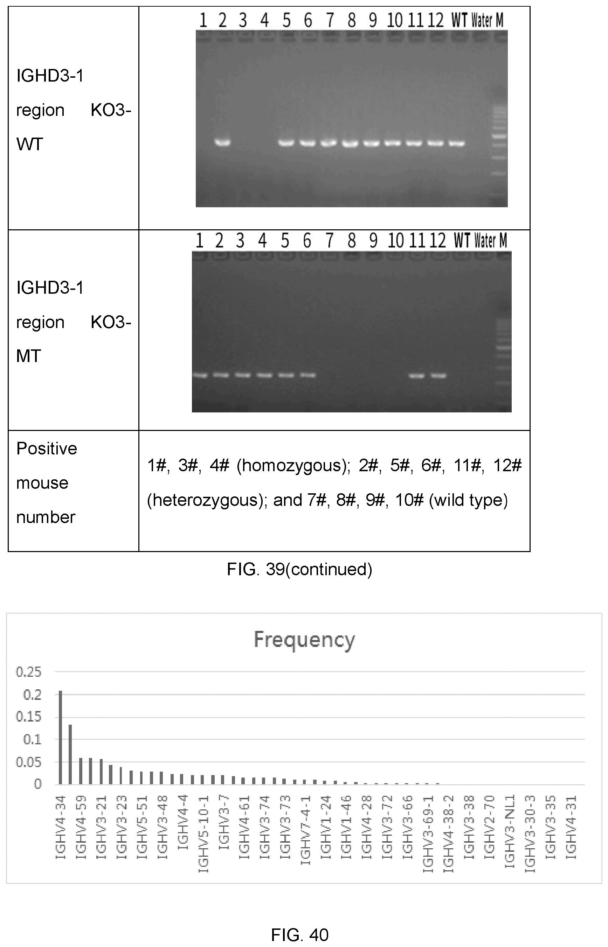

A total of 12 F2 mice were born, and co-identified by 6 pairs of primers (Table 21) to determine that the genotype of mice 1# , 3# and 4# was homozygous; the genotype of mice 2#, 5#, 6#, 11#, and 12# was heterozygous; and the genotype of mice 7#, 8#, 9# and 10# was wild-type (FIG. 39).

**Table 21. Mating and breeding of F1-2# ♂ with F1-16# ♀**

| Aspect | Identification region | Identification site | Size | Primer name | Primer sequence | SEQ ID NO. |
|---|---|---|---|---|---|---|
| F2 mice | V region KO1 | MT | 653 | H-KO1-F | gcctctcggc ctgtcaagca g | 82 |
| | | | | H-KO1-R | cagactggtg ccaggaaag ca | 83 |
| | | WT | 524 | H-KO1-WT-F | ggatgctgcc acattcccac ct | 88 |
| | | | | H-KO1-WT-R | AAGCCAT GTGAGG CAGGATC CT | 89 |
| | | | | | | |
| | D and J region KO2 | MT | 385 | H-KO2-F | agtgctcaca cagctctgcc c | 84 |
| | | | | H-KO2-R | ggtgtctgtta cttttgcagaa | 85 |
| | | WT | 342 | H-KO2-WT-F | cttgtcagtga ggaagtccc cc | 90 |
| | | | | H-KO2-WT-R | TAGCACT GTGGTAG CTACTAC C | 91 |
| | | | | | | |
| | IGHD3-1 region KO3 | MT | 259 | H-KO3-F | agtgctcaca cagctctgcc c | 86 |
| | | | | H-KO3-R | ggtgtctgtta cttttgcagaa | 87 |
| | | WT | 407 | H-KO3-WT-F | tgggcacag ctcgggctac cac | 92 |
| | | | | H-KO3-WT-R | ggtgtctgtta cttttgcagaa | 93 |

### IV. Breeding performance testing

The obtained homozygous mice were then mated, and the breeding performance of the mice was counted.

A total of 25 breeding pairs were mated, and the number of mice born after 1 month of mating was counted. There were a total of 25 breeding pairs, 20 breeding pairs gave birth to mice after 1 month of mating, and the effective breeding pairs reached 80%, while the mice had an average birth of 5.35, which was consistent with the number of births of wild type mice. The data are shown in Table 22.

**Table 22.**

| Breeding pair | Number of births | Breeding pair | Number of births | Breeding pair | Number of births |
|---|---|---|---|---|---|
| Breeding pair 1 | 5 | Breeding pair 11 | 5 | Breeding pair 21 | 7 |
| Breeding pair 2 | 6 | Breeding pair 12 | 6 | Breeding pair 22 | 7 |
| Breeding pair 3 | 4 | Breeding pair 13 | 4 | Breeding pair 23 | 0 |
| Breeding pair 4 | 6 | Breeding pair 14 | 0 | Breeding pair 24 | 5 |
| Breeding pair 5 | 6 | Breeding pair 15 | 0 | Breeding pair 25 | 5 |
| Breeding pair 6 | 5 | Breeding pair 16 | 7 | | |
| Breeding pair 7 | 2 | Breeding pair 17 | 0 | | |
| Breeding pair 8 | 5 | Breeding pair 18 | 4 | | |
| Breeding pair 9 | 0 | Breeding pair 19 | 5 | | |
| Breeding pair 10 | 7 | Breeding pair 20 | 6 | | |

### V. H-chain immune repertoire sequencing detection

The obtained homozygous mice were euthanized, spleens of the mice were dissected, after RNA was extracted and totalRNA extracted from a sample was detected to be qualified, library construction was performed, followed by immune repertoire sequencing (Immuno-Seq uses B/T lymphocytes as a research objective to specifically amplify a variable region (a V region) that determines the diversity of a B cell receptor (BCR) or a T cell receptor (TCR) by 5' RACE or a multiplex PCR technology, so as to comprehensively evaluate the diversity of an immune system combined with a high-throughput sequencing technology); and sequences obtained by sequencing were subjected to quality control by using quality control software, and the sequencing background was filtered, then the sequences were aligned with V, D, and J genes of an IMGT immune cell receptor library, the corresponding gene fragments were searched to find accurate V, D and J gene fragments and sequence loci, and information such as V, D and J gene frequencies, clone frequency distribution, and the number of polypeptide sequences were statistically analyzed.

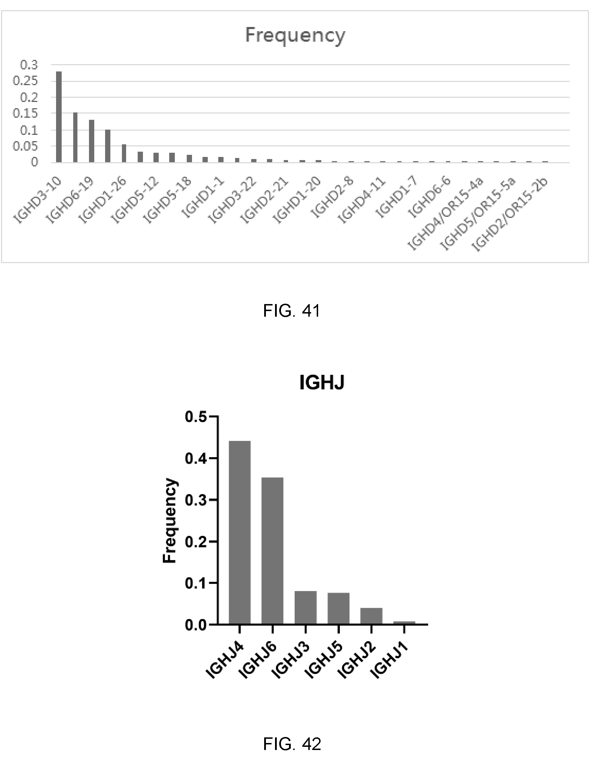

FIGS. 40 to 42 show a distribution graph of a gene usage frequency at a reads level based on the obtained gene usage frequency data. The results of FIG. 40 indicate that in unimmunized homozygous mice, the usage frequency of IGHV43-34 was the highest at the reads level, followed by IGHV6-1, and the usage frequency of IGHV4-59, IGHV4-39, IGHV3-21, IGHV2-26, IGHV3-23, IGHV2-5, IGHV5-51, IGHV1-18, IGHV3-48, IGHV3-15, IGHV4-4, IGHV3-43, IGHV5-10-1, IGHV3-30, IGHV3-7, IGHV1-3, IGHV4-61, IGHV3-20, IGHV3-74, IGHV1-69, IGHV3-73, IGHV1-2, IGHV7-4-1, IGHV3-64D, IGHV1-24, IGHV3-53, IGHV1-46, IGHV3-11, IGHV4-28, IGHV3-13, IGHV3-72, IGHV1-58, IGHV3-66, IGHV3-64, IGHV3-69-1, IGHV3-49, IGHV4-38-2, IGHV3-43D, IGHV3-38, IGHV3-33, IGHV2-70, IGHV3-71, IGHV3-NL1, IGHV3-22, IGHV3-30-3, IGHV3-38-3, IGHV3-35, IGHV3-52, IGHV4-31 and IGHV4/OR15-8 sequentially decreased. The results of FIG. 41 indicate that in unimmunized homozygous mice, the usage frequency of IGHD3-10 was the highest at the reads level, followed by IGHD6-13, and the usage frequency of IGHD6-19, IGHD3-9, IGHD1-26, IGHD7-27, IGHD5-12, IGHD4-17, IGHD5-18, IGHD2-2, IGHD1-1, IGHD2-15, IGHD3-22, IGHD3-16, IGHD2-21, IGHD4-23, IGHD1-20, IGHD1-14, IGHD2-8, IGHD3-3, IGHD4-11, IGHD5-24, IGHD1-7, IGHD3/OR15-3a, IGHD6-6, IGHD6-25, IGHD4/OR15-4a, IGHD2/OR15-2a, IGHD5/OR15-5a, IGHD1/OR15-la, IGHD2/OR15-2b and IGHD4/OR15-4b sequentially decreased. The results of FIG. 42 indicate that in unimmunized homozygous mice, the usage frequency of IGHJ4 was the highest at the reads level, followed by IGHJ6, and the usage frequency of IGHJ3, IGHJ5, IGHJ2, and IGHJ1 sequentially decreased.

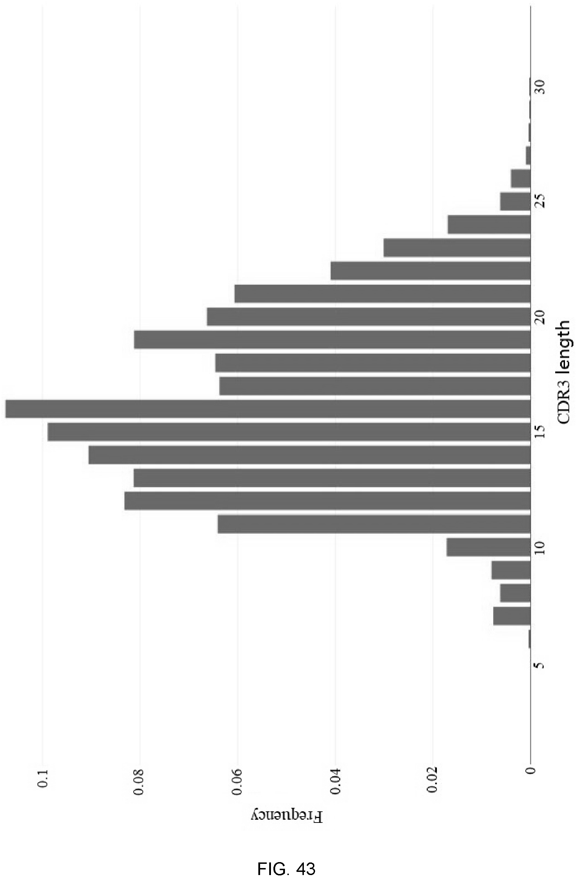

FIG. 43 shows a distribution graph of a CDR3 amino acid length at the Reads level based on the obtained CDR3 length frequency data. The results indicate that the CDR3 amino acid length distribution approaches the normal distribution.

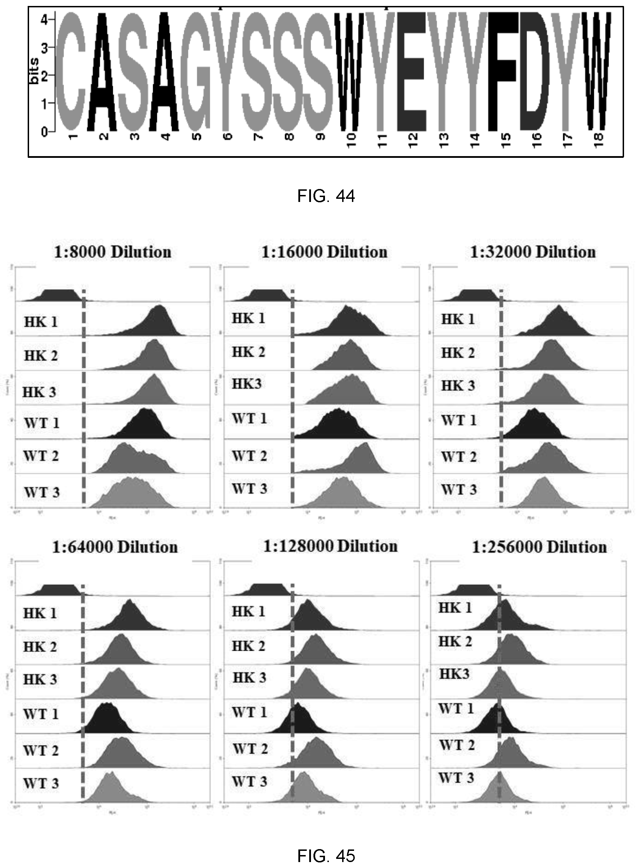

The statistical analysis of CDR3 amino acid sequence characteristics is an important step in immunopanel analysis, by refining to each clone, different CDR3 sequences evolved from a sequence of each germline antibody are subjected to quantitative sequence abundance analysis, and finally, it is convenient to select sequences for further antibody expression. Weblogo profiling of a CDR3 amino acid sequence of each sample at the reads level is shown in FIG. 44. The results of FIG. 44 show an amino acid sequence with the highest CDR3 abundance in unimmunized homozygous mice.

### VI. Antibody preparation

Three 6- to 8-week-old C57BL/6N female mice and three 6- to 8-week-old HK homozygous female mice (homozygous mice of which a heavy chain variable region was humanized according to I to III above and a Kappa light chain variable region was also humanized) were immunized with a B7-H3-CHO-K1 stable cell line. An immunization schedule includes three routine immunizations plus one booster immunization. The three routine immunizations were performed every two weeks, and the mice were injected intraperitoneally with 2×10⁷ B7-H3-CHO-K1 cells each time. Three days after the third routine immunization, peripheral blood of the mice was collected by orbital blood collection, serum was collected, and a serum antibody titer of the mice was detected by flow cytometry. After the titer was qualified, the booster immunization was performed. The booster immunization was performed 10 days after the third routine immunization by intraperitoneal injection of 2×10⁷ B7-H3-CHO-K1 cells. Three days after the booster immunization, spleens of the mice were taken for hybridoma fusion to prepare hybridoma cells. After growth of the hybridoma cells, positive hybridoma cells were identified: a supernatant was taken, and whether the supernatant contained a B7-H3 antibody was detected by flow cytometry. Experimental results showed that the immune performance of the HK homozygous mice was not inferior to that of wild type C57BL/6N mice, and the serum antibody titers of the HK homozygous mice and the wild-type C57BL/6N mice were comparable after three immunizations, with the highest titers being greater than 1:256000; and hybridoma cells were then prepared by fusing HK mouse spleen cells with myeloma SP20 cells, and more than 70 B7-H3 antibody-positive hybridoma cells were obtained by multiple screening, where antibodies secreted by 5 hybridoma cells has a very strong binding ability to antigen-positive cells.

After the booster immunization, sera from mice were collected for antibody titer detection, and a serum dilution gradient was set as: 1:8000, 1:16000, 1:32000, 1:64000, 1:128000, and 1:256000. The detection results of FIG. 45 showed that the HK mice and the wild type mice had similar immune performance, and the serum titers of both mice were greater than 1:256000.

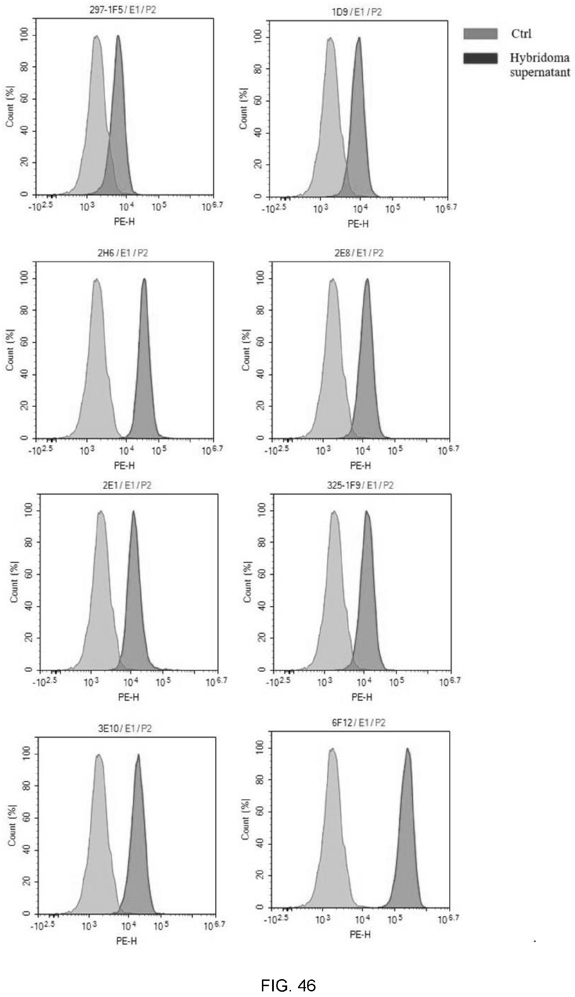

After the hybridoma cells were fused, the successfully fused cell culture supernatant was selected for co-incubation with antigen-positive cells to identify positive hybridoma cells capable of secreting antibodies. FIG. 46 shows representative test cases of positive hybridoma cells obtained by screening.

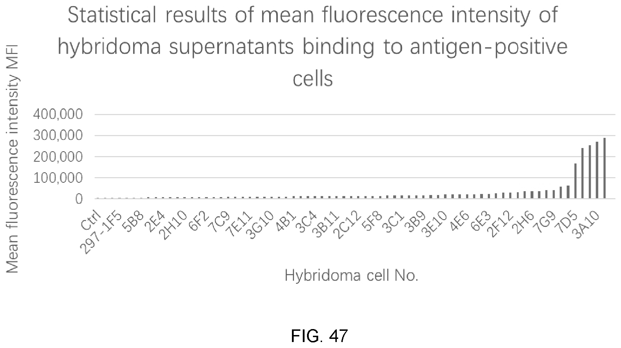

The mean fluorescence intensity of positive hybridoma cell supernatant binding to antigen-positive cells was counted and the results in FIG. 47 showed that dozens of positive hybridoma cells were screened, 5 of which had a very strong binding ability to antigen-positive cells: 5H9, 3A10, 6F12, 325-1G10, and 7D5.

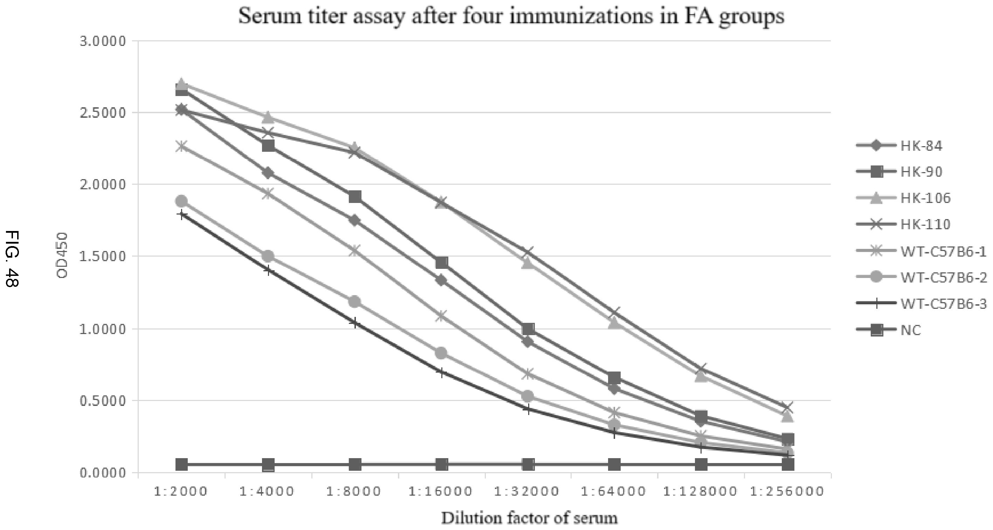

In order to generate a humoral immune response against PD-L1 in HK homozygotes (mouse homozygotes in which both the heavy chain variable region and the Kappa light chain variable region were humanized), the first immunization was performed by subcutaneous injection with a Complete Freund's Adjuvant (CFA) uniformly mixed with 0.05 mg of PD-L1 His (Kactus Biosystems, PDL-HM110) protein, followed by subcutaneous injection with an Incomplete Freund's Adjuvant (IFA) uniformly mixed with 0.025 mg of PD-L1 His protein, immunization was performed once every other 2 weeks, and a total of four immunizations were performed, so that antigen-specific antibodies were generated in the HK homozygous mice. Sera from the HK homozygote mice after the third and fourth immunizations were taken for serum titer monitoring. 2 µg/ml of antigen was coated overnight with CBS (a carbonate buffered solution), and after washing with PBST (phosphate buffered saline), blocking was performed with 2% BSA at 37°C for 2 h. After washing with PBST, blocking was performed with serum dilutions (fold dilutions starting from 1:2000, 8 gradients) at 37°C for 2 h. After washing with PBST, a horseradish peroxidase-labeled goat anti-mouse secondary antibody (diluted in 1:1 W in PBS) was added, and incubation was performed at 37°C for 1 h. After washing with PBST, a TMB substrate solution (Beyotime, P0209-500ml) was added for color development at 37°C for 5-10 min, and the reaction was stopped by addition of a stop solution, and a serum titer was detected by measuring the optical density at 450 nm. As shown in FIG. 48, all four HK homozygous mice showed higher immune response ability, reaching 256K or more (HK-84, HK-90, HK-106, and HK-110 were HK homozygous mice; WT-C57B6 was wild type mice; and NC was unimmunized mouse serum as a negative control).

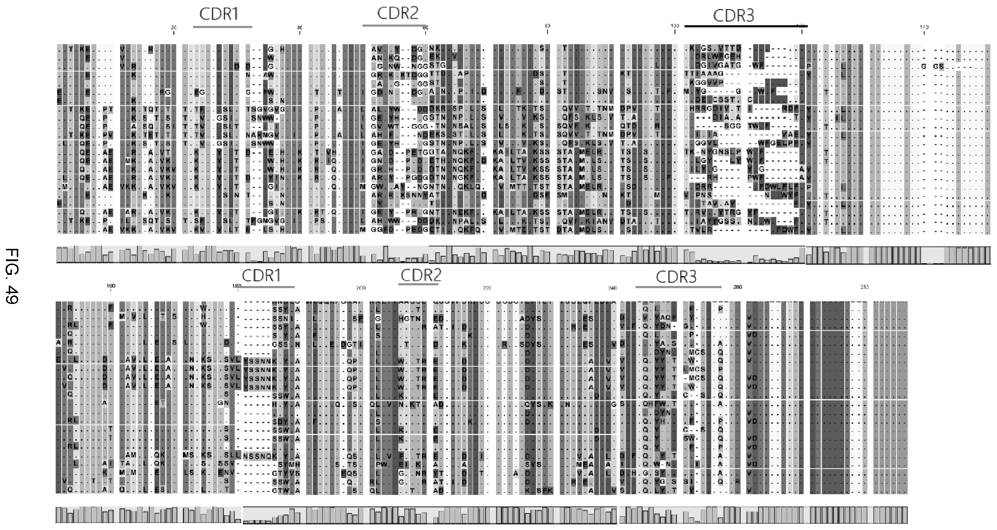

Spleens of mice with the highest serum titer after four rounds of immunizations were collected and ground to collect cells. Total RNA of spleen cells was extracted with an RNA extraction kit (Foregene, RE-03011). cDNA was synthesized and antibody VH and VL sequences were amplified by nested PCR, a vector pComb3XSS (EDITGENE, 63890) and a target fragment were separately recovered by Sfil digestion, a ligation product was electrotransformed into TG1 competent cells, a PD-L1 scFv antibody library was constructed and the library size was determined, with a size of 1.36×10⁹ CFM. 48 clones were randomly picked from a titer plate for determining the number of library transformants for sequencing identification and the results (FIG. 49) indicated that the antibody sequences were all human antibody sequences and the sequence diversity was rich.

### Example 2. Preparation of Kappa light chain locus modified mice

### I. ES cell line I

1. Insertion of about 20 kb of a human gene sequence (including IGKJ1-5 and all intergenic sequences) between a J region and a C region of mice.

A constructed vector was electroporated into wild-type ES cells. The vector was Neo-resistant. The cells were subjected to G418 drug screening. Related ES clones were selected for culture, amplified and identified by PCR typing.

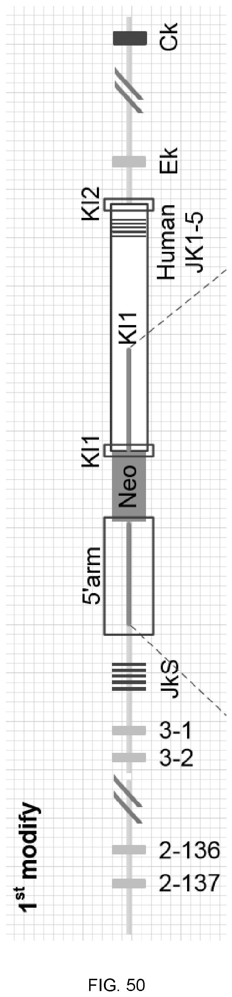

The cell clones were amplified by three pairs of primers (the sequences are shown in Table 23 below, and primer identification positions are simultaneously labeled in a figure (FIG. 50)) to identify the insertion of the sequence into the corresponding position.

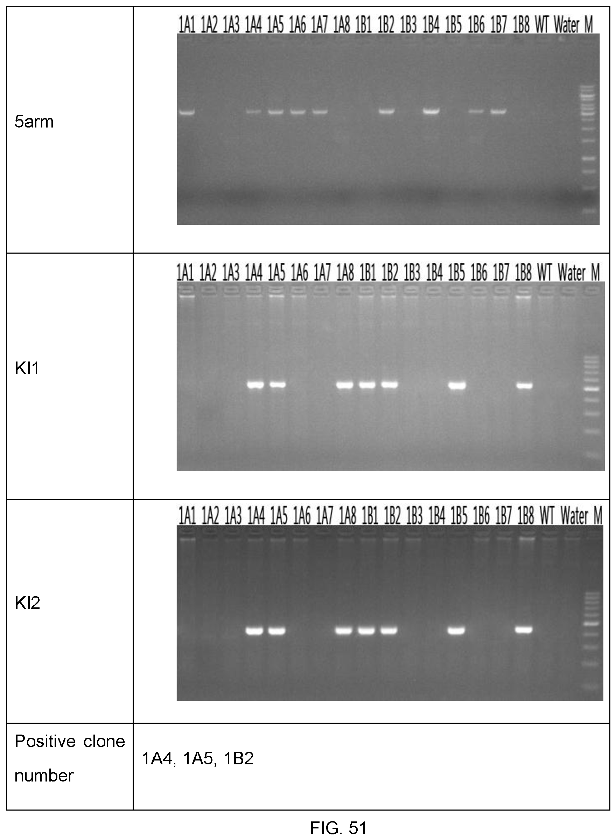

There were three clones with positive bands simultaneously amplified by the three pairs of primers (FIG. 51): 1A4, 1A5 and 1B2, and the three clones were used as positive clones in the first step.

**Table 23**

| Aspect | Identification region | Product size | Primer name | Primer sequence | SEQ ID NO. |
|---|---|---|---|---|---|
| ES1-V1 | 5'arm | 3000 | K1-F1 | ccactaactgctgagccacctc | 94 |
| | | | K1-R1 | GGAGACTTGGAAATCCC CGTGAGT | 95 |
| | KI1 | 520 | K1-F2 | ctagttgccagccatctgttgt | 96 |
| | | | K1-R2 | AGGCTGGTCTTGAACTC CTGAG | 97 |
| | KI2 | 423 | K1-F3 | tcgattgagtggctttggtggc | 98 |
| | | | K1-R3 | TCTCACATTAGTGTAGTC TGTCA | 99 |

2. Insertion of about 20 kb of a human V-region genome sequence (including IGKV4-1, IGKV5-2 and all intergenic sequences) in front of a genome of the cells obtained in the first step.

A constructed vector was electroporated into the positive cell clone 1A5 in the first step: the vector carries Puro resistance and a homology arm on one end is placed on the human sequence with the positive clones in the first step, so Neo resistance will be deleted by homologous recombination in this step; and cells were subjected to Puromycin drug screening and related ES clones were selected for culture, amplified and identified by PCR typing.

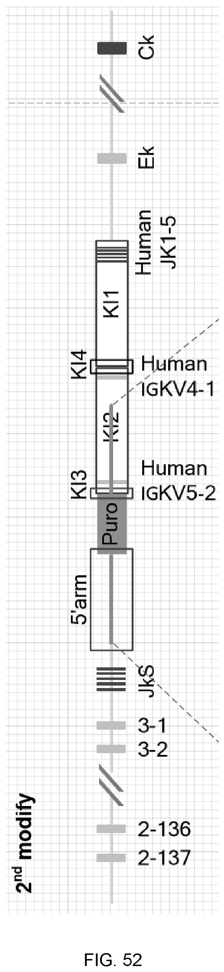

The insertion of the sequence into the corresponding position was identified by three pairs of primers (the sequences are shown in Table 24 below, and primer identification positions are simultaneously labeled in a figure (FIG. 52)).

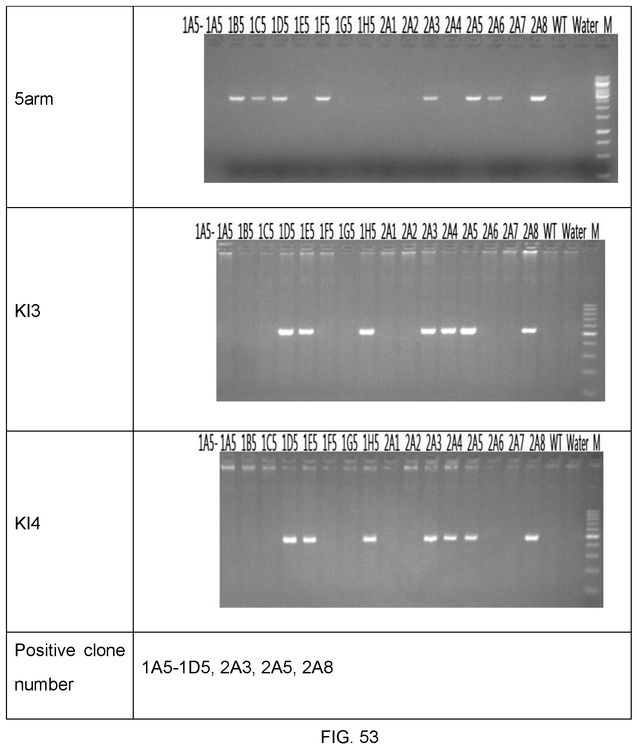

There were four clones with positive bands simultaneously amplified by the three pairs of primers (FIG. 53): 1A5-1D5, 1A5-2A3, 1A5-2A5, and 1A5-2A8, and the four clones were used as positive clones in the second step.

**Table 24**

| Aspect | Identification region | Product size | Primer name | Primer sequence | SEQ ID NO. |
|---|---|---|---|---|---|
| ES1-V1V2 | 5'arm | 3000 | K1-F4 | ctctgtcagagaagcccaagc gc | 100 |
| | | | K1-R4 | GGCGAACGCGGCGG CGAGGGTGC | 101 |
| | KI3 | 520 | K1-F5 | ccctggaaggtgccactccca ct | 102 |
| | | | K1-R5 | TCAGAACTCCTCTCT GTTTAGCA | 103 |
| | KI4 | 480 | K1-F6 | tctcgcactgcactccagcctg | 104 |
| | | | K1-R6 | TAATCCACCCGCCTT GGCCTCC | 105 |

3. Insertion of about 20 kb of a human V-region genome sequence (including IGKV7-3 and adjacent sequences) in front of a genome of the cell obtained in the second step.

A constructed vector was electroporated into the positive cell clone 1A5-2A3 in the second step: the vector carries Neo resistance and a homology arm on one end is placed on the human sequence with the positive clones in the second step, so the Puro resistance will be deleted by homologous recombination in this step; cells were subjected to G418 drug screening and related ES clones were selected for culture, amplified and identified by PCR typing.

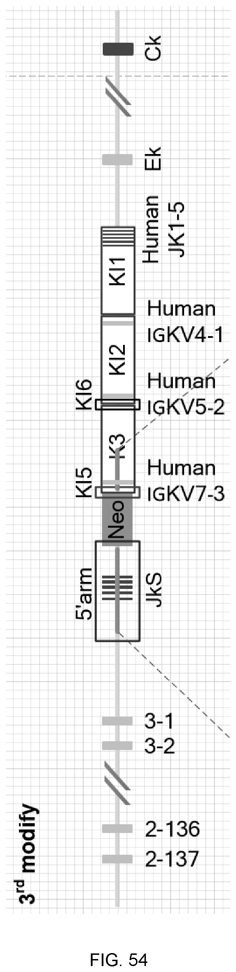

The insertion of the sequence into the corresponding position was identified by three pairs of primers (the sequences are shown in Table 25 below, and primer identification positions are simultaneously labeled in a figure (FIG. 54)).

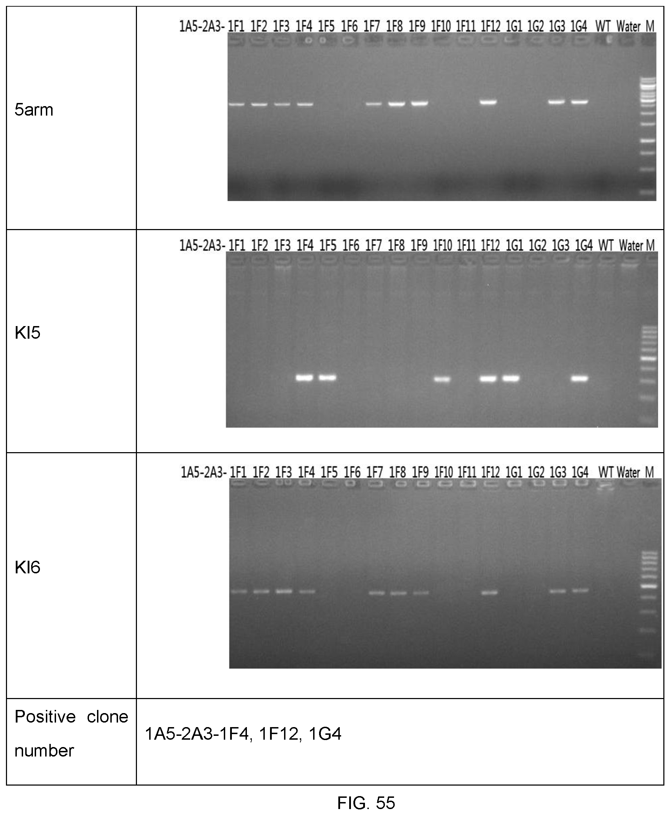

There were three clones with positive bands simultaneously amplified by the three pairs of primers (FIG. 55): 1A5-2A3-1F4, 1A5-2A3-1F12, and 1A5-2A3-1G4, and the three clones were used as positive clones in the third step.

**Table 25**

| Aspect | Identification region | Product size | Primer name | Primer sequence | SEQ ID NO. |
|---|---|---|---|---|---|
| ES1-V1V2V3 | 5'arm | 3000 | K1-F7 | agcgagtgccactaactgctga gc | 106 |
| | | | K1-R7 | GGAAATCCCCGTGAGT CAAACCGC | 107 |
| | KI5 | 310 | K1-F8 | ccttgaccctggaaggtgccact | 108 |
| | | | K1-R8 | TTCACTATCAGAAATG GGTAGATCC | 109 |
| | KI6 | 450 | K1-F9 | ttttgtgactgatctaccttggactt | 110 |
| | | | K1-R9 | CCTTGGACTTGTAATG CACATTAC | 111 |

4. Insertion of about 20 kb of a human V-region genome sequence (including IGKV2-4, IGKV1-5 and all intergenic sequences) in front of a genome of the cell obtained in the third step while introducing two lox sites loxp-lox2272.

A constructed vector was electroporated into the positive cell clone 1A5-2A3-1G4 in the third step: the vector carries Puro resistance and a homology arm on one end is placed on the human sequence with the positive clones in the second step, so the Neo resistance will be deleted by homologous recombination in this step, and both ends of a Puro antibody carry loxP and lox2272-PB5' (5' ITR, 5' inverted terminal repeat) elements, respectively; and cells were subjected to Puromycin drug screening and related ES clones were selected for culture, amplified and identified by PCR typing.

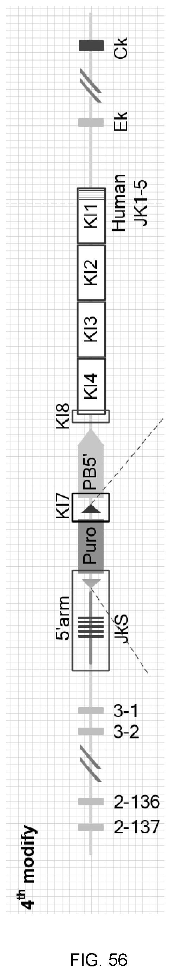

The insertion of the sequence into the corresponding position was identified by three pairs of primers (the sequences are shown in Table 26 below, and primer identification positions are simultaneously labeled in a figure (FIG. 56)).

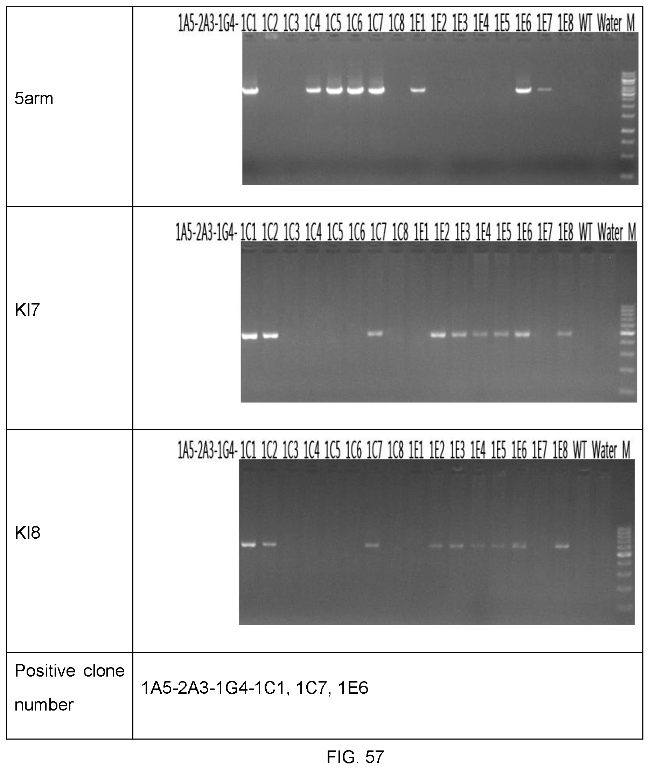

There were three clones with positive bands simultaneously amplified by the three pairs of primers (FIG. 57): 1A5-2A3-1G4-1C1, 1A5-2A3-1G4-1C7, and 1A5-2A3-1G4-1E6, and the three clones were used as positive clones in the fourth step.

**Table 26**

| Aspect | Identification region | Product size | Primer name | Primer sequence | SEQ ID NO. |
|---|---|---|---|---|---|
| ES1-V1V2V3V4 | 5'arm | 3600 | K1-F10 | aaggctgtggcaggattgga ag | 112 |
| | | | K1-R10 | GCCCAGAAAGCGAA GGAGCAA | 113 |
| | KI7 | 500 | K1-F11 | tgagctctagagctcgctgat ca | 114 |
| | | | K1-R11 | TCTCAGTCGCCGCT TGGAGCTCCC | 115 |
| | KI8 | 600 | K1-F12 | cacttaccgcattgacaagc acg | 116 |
| | | | K1-R12 | TAAGGCTTCAGGGC AGGAGGTTGT | 117 |

5. Insertion of about 350 kb of a human V genome sequence (including IGKV1-6, IGKV3-7, IGKV1-8, IGKV1-9, IGKV2-10, IGKV3-11, IGKV1-12, IGKV1-13, IGKV2-14, IGKV3-15, IGKV1-16, IGKV1-17, IGKV2-18, IGKV2-19, IGKV3-20, IGKV6-21, IGKV1-22, IGKV2-23, IGKV2-24, IGKV3-25, IGKV2-26, IGKV1-27, IGKV2-28, IGKV2-29, IGKV2-30, IGKV3-31, IGKV1-32, IGKV1-33, IGKV3-34, IGKV1-35, IGKV2-36, IGKV1-37 and all intergenic sequences) between loxP and lox2272 of a genome of the cells obtained in the fourth step

Constructed fused BAC and Cre were electroporated into the positive cell clone 1A5-2A3-1G4-1C1 in the fourth step: BAC has Neo resistance (both ends of a Neo antibody have inverted terminal repeats), and both ends of a human sequence on the BAC carry loxP and lox2272 elements which are in the same direction as those on the positive clones in the fourth step, and PB3' (3' ITR, 3' inverted terminal repeat) is present between the lox2272 element and a human genome sequence, and sequences between loxP and lox2272 on the BAC and the positive cell clones in the fourth step were replaced under the action of a Cre recombinase, and Puro resistance was replaced by the human genome sequence and Neo resistance on the BAC; and cells were subjected to G418 drug screening and related ES clones were selected for culture, amplified and identified by PCR typing.

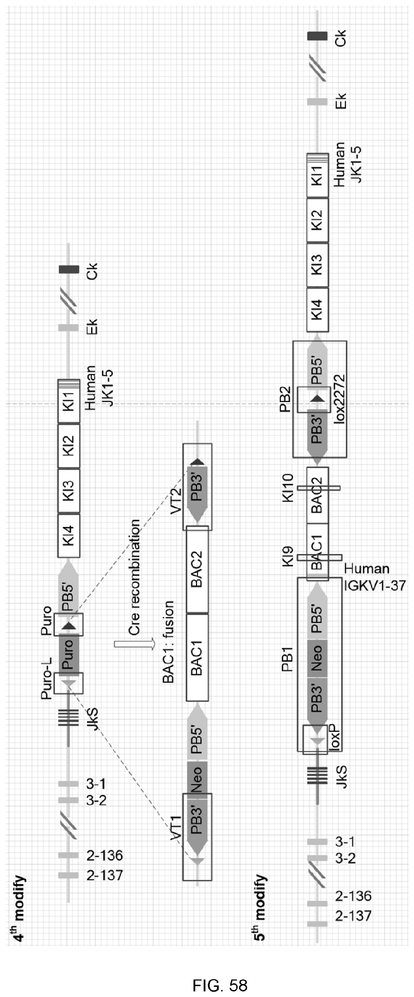

Replacement of the sequences was identified by 10 pairs of primers (the sequences are shown in Table 27 below, and primer identification positions are simultaneously labeled in a figure (FIG. 58)), where 2 pairs of the primers, primers for VT1 and VT2, were used to identify random insertions and the cells needed to be identified as negative before they were confirmed to be correctly cloned.

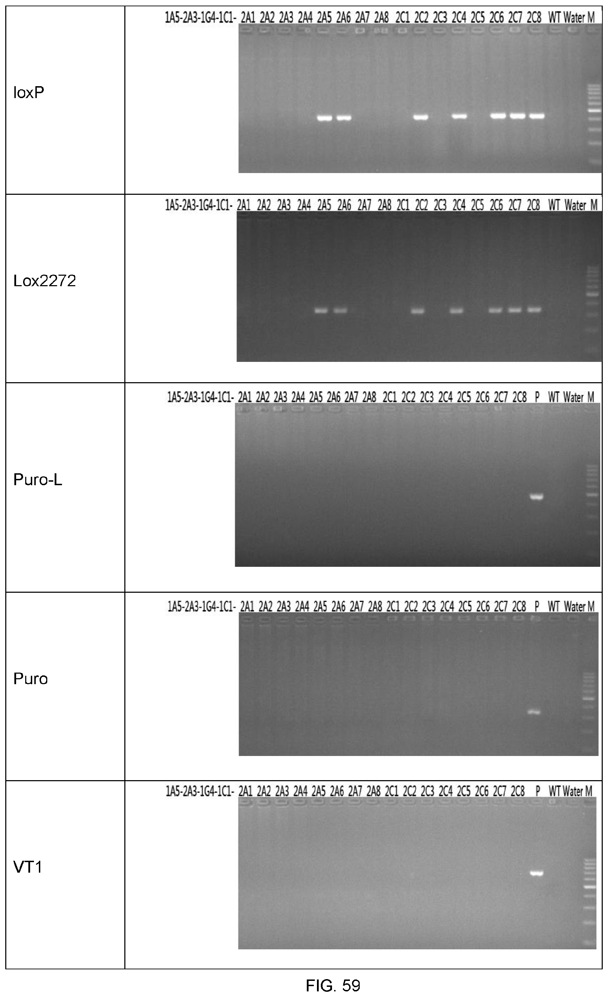

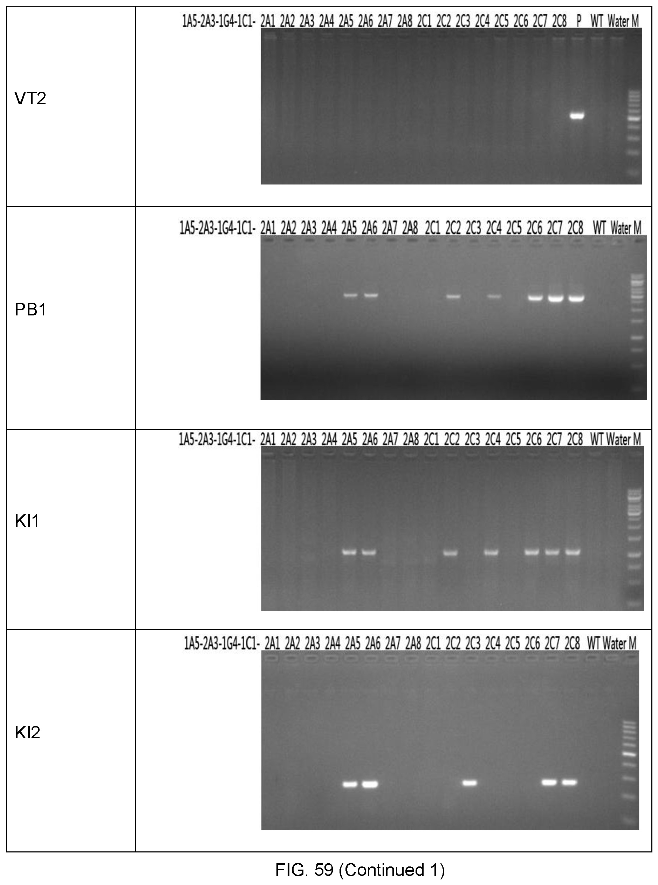

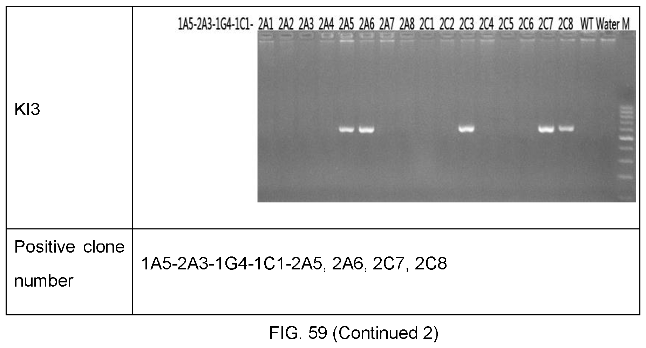

There were four clones with positive bands simultaneously amplified by the remaining eight pairs of primers (FIG. 59): 1A5-2A3-1G4-1C1-2A5, 1A5-2A3-1G4-1C1-2A6, 1A5-2A3-1G4-1C1-2C7, and 1A5-2A3-1G4-1C1-2C8, and the four clones were used as positive clones in the fifth step.

**Table 27**

| Aspect | Identification region | Product size | Primer name | Primer sequence | SEQ ID NO. |
|---|---|---|---|---|---|
| ES1-FV+BAC | IoxP | 420 | K1-F13 | gggtttgaactatatgttttaat | 118 |
| | | | K1-R13 | CGTTGGGCGGTCAG CCAGGC | 119 |
| | Iox2272 | 340 | K1-F14 | tgacgcatgtgttttatcggtc | 120 |
| | | | K1-R14 | GAGTCAAAATGACGC ATGATTA | 121 |
| | Puro-L | 470 | K1-F15 | gtgggatagcagagttgagtg ag | 122 |
| | | | K1-R15 | CAGCGGGGCTGCTA AAGCGCATGC | 123 |
| | Puro | 350 | K1-F16 | cttgaccctggaaggtgccact cc | 124 |
| | | | K1-R16 | TATAACGACCGCGTG AGTCAAA | 125 |
| | VT1 | 688 | K1-F17 | acggttgagtaataaatggatg c | 126 |
| | | | K1-R17 | ACGTAGATGTACTGC CAAGTAG | 127 |
| | VT2 | 510 | K1-F18 | ttgcttgatctaatactagtaag | 128 |
| | | | K1-R18 | GCTGTGTCCTGCTTA TCCACAAC | 129 |
| | PB1 | 2900 | K1-F15 | gtgggatagcagagttgagtg ag | 122 |
| | | | K1-R19 | GCTGCCACAATATGA TTATAT | 130 |
| | PB2 | 1100 | K1-F19 | acaaaggcataacaccattac c | 131 |
| | | | K1-R12 | TAAGGCTTCAGGGCA GGAGGTTGT | 117 |
| | KI9 | 270 | K1-F20 | ggatccagtggggatgttgtga t | 132 |
| | | | K1-R20 | CTCAGCCTCCACCCT GCTGAT | 133 |
| | KI10 | 610 | K1-F21 | ctgtgtaaagccctgttgcaa | 134 |
| | | | K1-R21 | CACACCTCACCAAGC TCAGCTT | 135 |

6. Electroporation of pBase (a PiggyBac transposase) on the cell clones obtained in the fifth step.

pBase (the PiggyBac transposase) was electroporated into the positive cell clone 1A5-2A3-1G4-1C1-2C7 in the fifth step: under the action of the PiggyBac transposase, two inverted terminal repeats will be cut from a genome, a Neo sequence and lox2272 will be lost, and the positive cell clone will have only the inserted human gene sequence and one loxP element; and ES clones were selected for culture, amplified and identified by PCR typing.

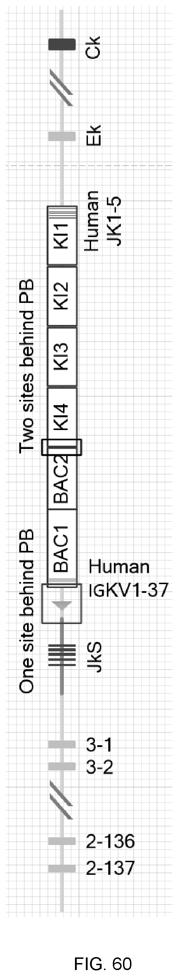

Deletions of sequences were identified by three pairs of primers (the sequences are shown in Table 28 below, and primer identification positions are simultaneously labeled in a figure (FIG. 60)).

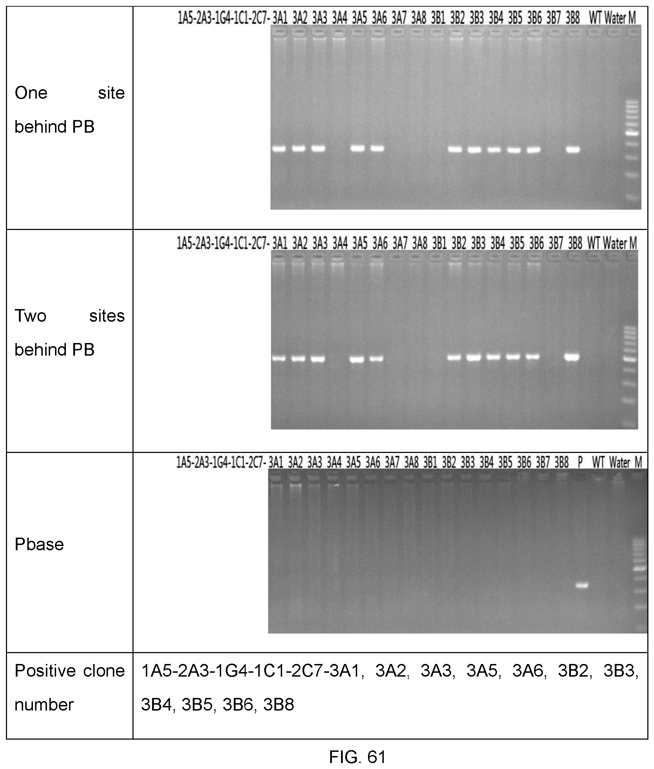

There were 11 clones with positive bands simultaneously amplified by the three pairs of primers (FIG. 61): 1A5-2A3-1G4-1C1-2C7-3A1, 1A5-2A3-1G4-1C1-2C7-3A2, 1A5-2A3-1G4-1C1-2C7-3A3, 1A5-2A3-1G4-1C1-2C7-3A5, 1A5-2A3-1G4-1C1-2C7-3A6, 1A5-2A3-1G4-1C1-2C7-3B2, 1A5-2A3-1G4-1C1-2C7-3B3, 1A5-2A3-1G4-1C1-2C7-3B4, 1A5-2A3-1G4-1C1-2C7-3B5, 1A5-2A3-1G4-1C1-2C7-3B6 and 1A5-2A3-1G4-1C1-2C7-3B8, and the 11 clones were used as positive clones in the sixth step.

**Table 28**

| Aspect | Identification region | Product size | Primer name | Primer sequence | SEQ ID NO. |
|---|---|---|---|---|---|
| ES1-FV+BAC-PB | One site behind PB | 350 | K1-F22 | gtatgactgcttgccatgtagat | 136 |
| | | | K1-R22 | CATGCTGCTGAGGAA TGGGAATGG | 137 |
| | Two sites behind PB | 500 | K1-F23 | ggtgcacctcatttagtctctgg | 138 |
| | | | K1-R23 | CAGAGAATGTGTTTC TACTGGGG | 139 |
| | pbase | 352 | pbase-F | CTGGACGAGCAGAA CGTGATCG | 36 |
| | | | pbase-R | CGAAGAAGGCGTAGA TCTCGTCCTC | 37 |

### 7. F0 mice obtained from ES1

The positive cell clones obtained in the sixth step were injected into a blastocyst, and the blastocyst was then transplanted into surrogate female mice, and F0 mice were born by gestation of about 20 days; and paws of young mice of 5-7 d were cut, DNA was extracted, and genotypes of the mice were confirmed by PCR typing identification.

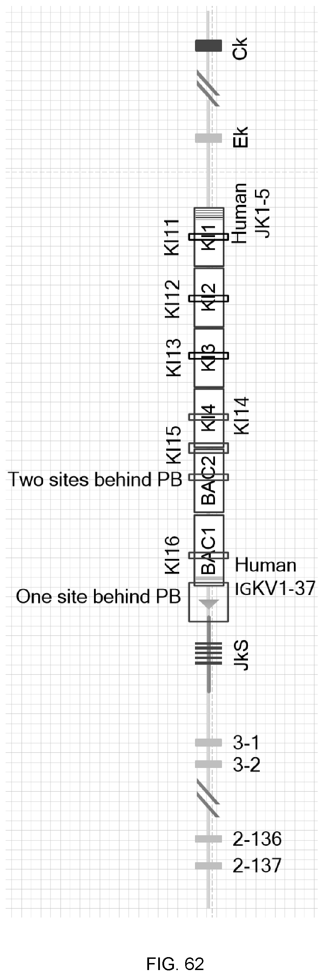

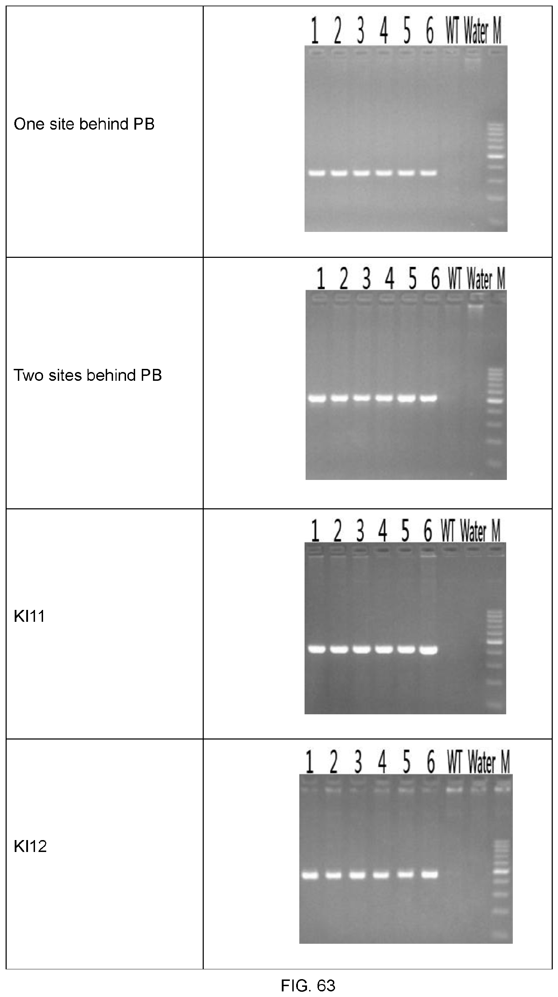

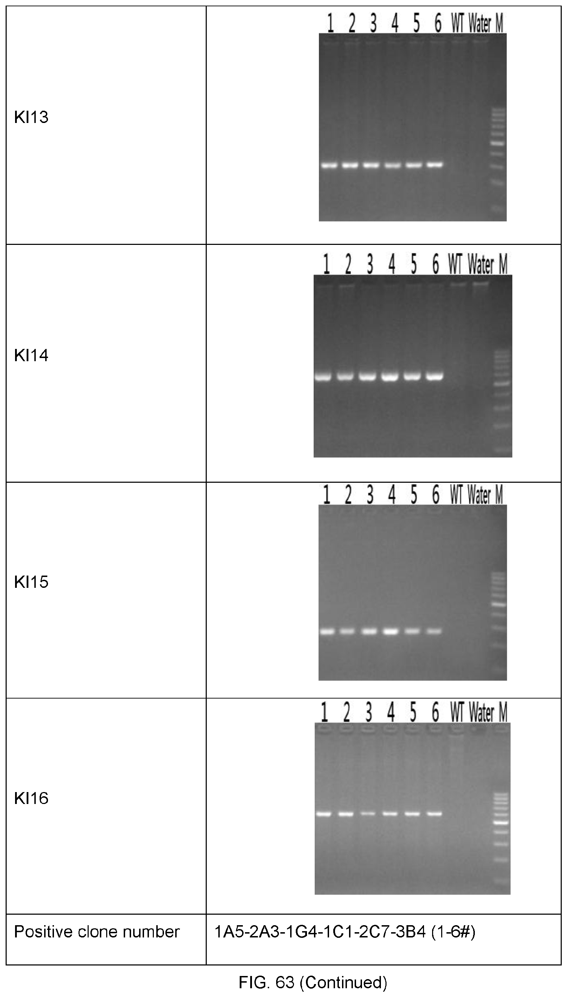

A total of 6 mice were born after injection of the clone 1A5-2A3-1G4-1C1-2C7-3B4 and all the 6 mice were co-identified as positive mice by 8 pairs of primers (Table 29). A schematic diagram of a genome of the F0 mice is shown in FIG. 62, and the identification results are shown in FIG. 63.

**Table 29**

| Aspect | Identification region | Product size | Primer name | Primer sequence | SEQ ID NO. |
|---|---|---|---|---|---|
| ES1-F0 | One site behind PB | 350 | K1-F22 | gtatgactgcttgccatgtagat | 136 |
| | | | K1-R22 | CATGCTGCTGAGGAATG GGAATGG | 137 |
| | Two sites behind PB | 500 | K1-F23 | ggtgcacctcatttagtctctgg | 138 |
| | | | K1-R23 | CAGAGAATGTGTTTCTA CTGGGG | 139 |
| | KI11 | 423 | K1-F24 | tgagatccctcactgtggctc | 142 |
| | | | K1-R24 | ACTGGCCATCAGACCCA AATT | 143 |
| | KI12 | 480 | K1-F25 | cacaatagctgttcataacag | 144 |
| | | | K1-R25 | TCAGTAAAATGGACTTT CACTC | 145 |
| | KI13 | 310 | K1-F26 | tgtggaagctaatgatactgca | 146 |
| | | | K1-R26 | AAGTAGAAAACTTCTGA GAGGT | 147 |
| | KI14 | 600 | K1-F27 | acaggaattccttcaacactct | 148 |
| | | | K1-R27 | ACCCTCTCTCACTGATC ACTGT | 149 |
| | KI15 | 270 | K1-F28 | ccagtagaaacacattctct | 150 |
| | | | K1-R28 | AGAAGCCCCAGGAGCT GAGCGG | 151 |
| | KI16 | 610 | K1-F29 | atctcacagcatcaggcaaagga | 152 |
| | | | K1-R29 | GATTACTCTGAAAGTATG TTAA | 153 |

### 8. F1 mice obtained from ES1

The F0 mice that were identified as the positive mice were mated and bred with wild type mice to obtain F1 generation mice.

Genotypes of the mice were confirmed by PCR typing identification of F1 generation mouse tail genomic DNA.

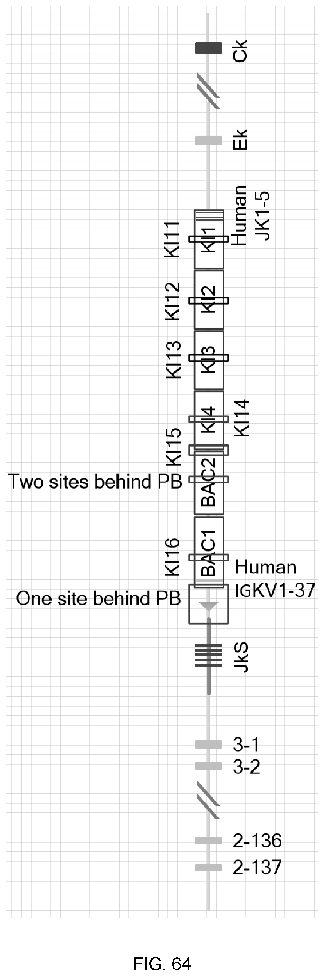

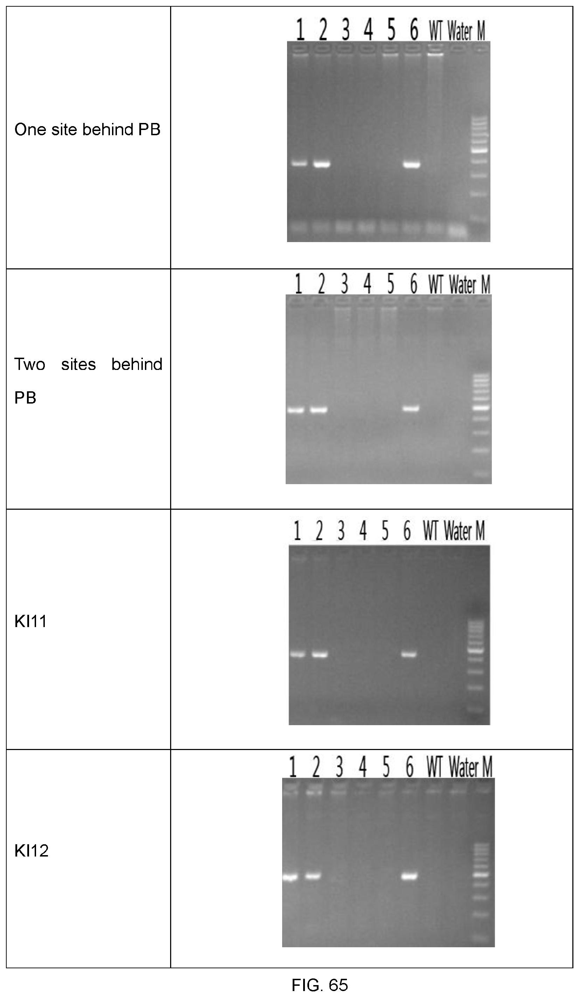

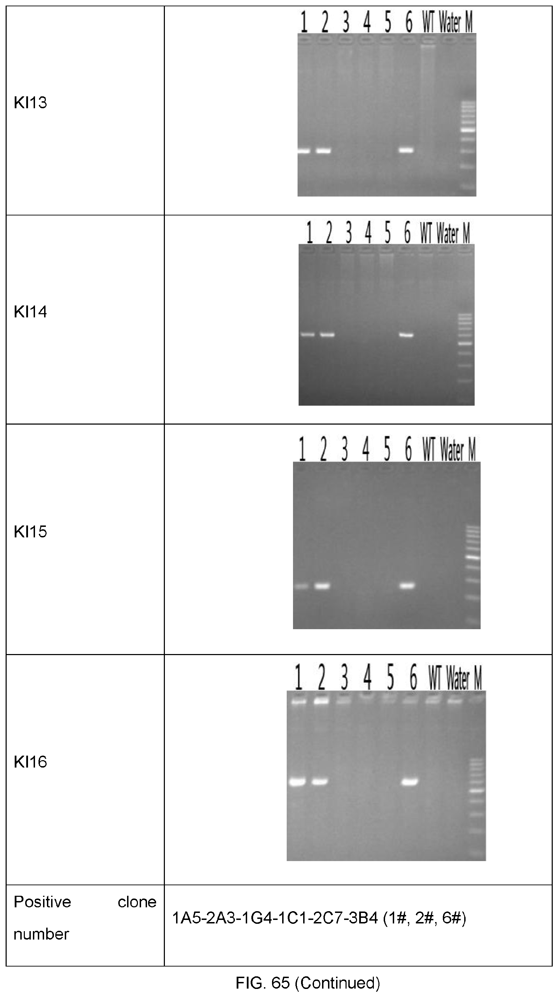

A total of 6 F1 mice were born, ES1 was co-identified with 8 pairs of primers (Table 30), and a total of 3 mice (1#, 2#, and 6#) were positive F1 mice. A schematic diagram of a genome of the F1 mice is shown in FIG. 64, and the identification results are shown in FIG. 65.

**Table 30**

| Aspect | Identification region | Product size | Primer name | Primer sequence | SEQ ID NO. |
|---|---|---|---|---|---|
| ES1-F1 | One site behind PB | 350 | K1-F22 | gtatgactgcttgccatgtagat | 136 |
| | | | K1-R22 | CATGCTGCTGAGGAATG GGAATGG | 137 |
| | Two sites behind PB | 500 | K1-F23 | ggtgcacctcatttagtctctgg | 138 |
| | | | K1-R23 | CAGAGAATGTGTTTCTA CTGGGG | 139 |
| | KI11 | 423 | K1-F24 | tgagatccctcactgtggctc | 142 |
| | | | K1-R24 | ACTGGCCATCAGACCCA AATT | 143 |
| | KI12 | 480 | K1-F25 | cacaatagctgttcataacag | 144 |
| | | | K1-R25 | TCAGTAAAATGGACTTT CACTC | 145 |
| | KI13 | 310 | K1-F26 | tgtggaagctaatgatactgca | 146 |
| | | | K1-R26 | AAGTAGAAAACTTCTGA GAGGT | 147 |
| | KI14 | 600 | K1-F27 | acaggaattccttcaacactct | 148 |
| | | | K1-R27 | ACCCTCTCTCACTGATC ACTGT | 149 |
| | KI15 | 270 | K1-F28 | ccagtagaaacacattctct | 150 |
| | | | K1-R28 | AGAAGCCCCAGGAGCT GAGCGG | 151 |
| | KI16 | 610 | K1-F29 | atctcacagcatcaggcaaagga | 152 |
| | | | K1-R29 | GATTACTCTGAAAGTAT GTTAA | 153 |

### II. ES cell line II

Insertion of about 20 kb of a human gene sequence (including IGKV2D-40, IGKV1D-39, IGKV2D-38 and all intergenic sequences) between a J region and a C region of mice.

A constructed vector was electroporated into wild-type ES cells. The vector was Neo-resistant. The cells were subjected to G418 drug screening. Related ES clones were selected for culture, amplified and identified by PCR typing.

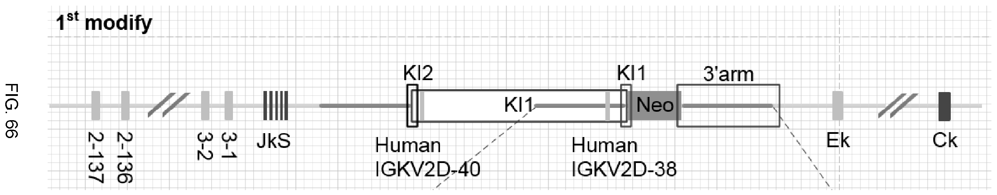

The insertion of the sequence into the corresponding position was identified by three pairs of primers (the sequences are shown in Table 31 below, and primer identification positions are simultaneously labeled in a figure (FIG. 66)).

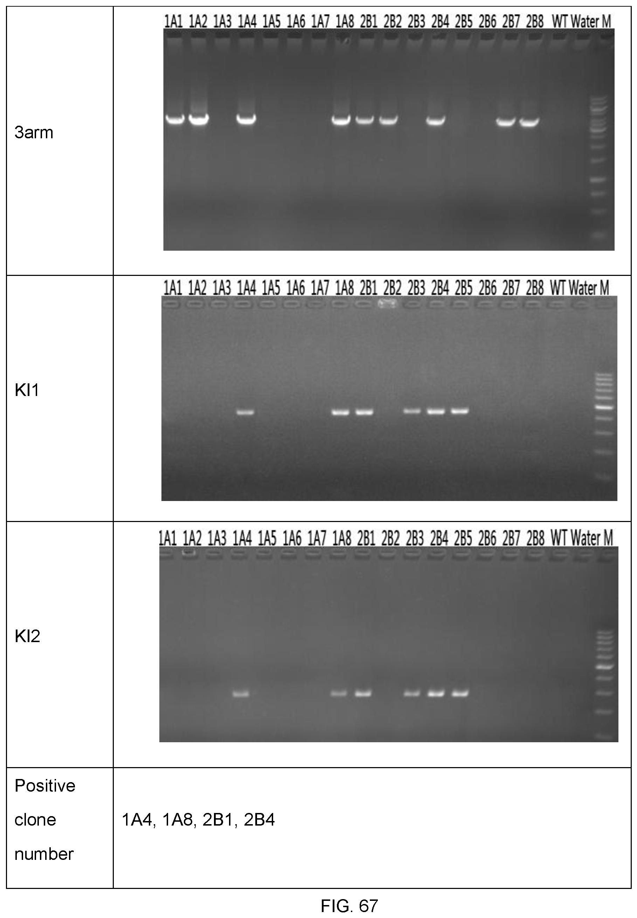

There were four clones with positive bands simultaneously amplified by the three pairs of primers (FIG. 67): 1A4, 1A8, 2B1, and 2B4, and the four clones were used as positive clones in the first step.

**Table 31**

| Aspect | Identification region | Product size | Primer name | Primer sequence | SEQ ID NO. |
|---|---|---|---|---|---|
| ES2-V1 | 3'arm | 3200 | K2-F1 | gctctatggcttctgaggcgga | 154 |
| | | | K2-R1 | ATCCTGGATCCCCTACT TTAT | 155 |
| | KI1 | 450 | K2-F2 | tgtgtacctggttggaccttga | 156 |
| | | | K2-R2 | GGGAACATACGTCATTA TTGACG | 157 |
| | KI2 | 290 | K2-F3 | tgactgcttgccatgtagatacc | 158 |
| | | | K2-R3 | AAGGTAGTGGGATTAC AGGTGTGA | 159 |

2. About 20 kb of a human V genome sequence (including IGKV1D-37, IGKV2D-36, IGKV1D-35 and all intergenic sequences) while introducing two lox sites loxp-lox5171 to obtain positive clones.

A constructed vector was electroporated into the positive cell clone 1A4 in the first step: the vector carries Puro resistance and a homology arm on one end is placed on the human sequence with the positive clones in the first step, so Neo resistance will be deleted by homologous recombination in this step, and both ends of Puro resistance carry PB3' (3' ITR, 3' inverted terminal repeat)-lox5171 and loxP elements, respectively; and cells were subjected to Puromycin drug screening and related ES clones were selected for culture, amplified and identified by PCR typing.

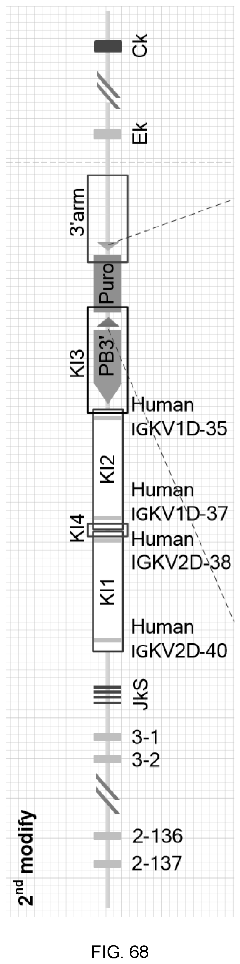

The insertion of the sequence into the corresponding position was identified by three pairs of primers (the sequences are shown in Table 32 below, and primer identification positions are simultaneously labeled in a figure (FIG. 68)).

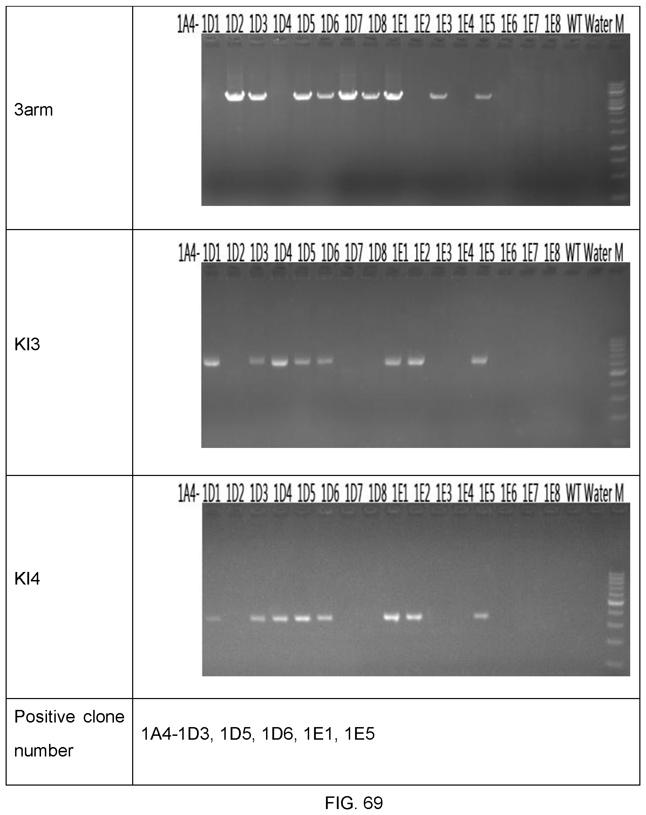

There were five clones twith positive bands simultaneously amplified by the three pairs of primers (FIG. 69): 1A4-1D3, 1A4-1D5, 1A4-1D6, 1A4-1E1, and 1A4-1E5, and the five clones were used as positive clones in the second step.

**Table 32**

| Aspect | Identification region | Product size | Primer name | Primer sequence | SEQ ID NO. |
|---|---|---|---|---|---|
| ES2-V1V2 | 3'arm | 4400 | K2-F4 | tccataccgacgatctgcgacc | 160 |
| | | | K2-R4 | ATACAGCATGTGACATT AGATT | 161 |
| | KI3 | 650 | K2-F5 | ggcaaagtaggttcatgatgctg | 162 |
| | | | K2-R5 | CGTGCTACTTCCATTT GTCACGT | 163 |
| | KI4 | 350 | K2-F6 | cctcttctgctgctgtcagactc | 164 |
| | | | K2-R6 | TTGATGTCTCATGTCT CACTGAAA | 165 |

3. Insertion of about 350 kb of a human V genome sequence (including IGKV3D-34, IGKV1D-33, IGKV1D-32, IGKV3D-31, IGKV2D-30, IGKV2D-29, IGKV2D-28, IGKV1D-27, IGKV2D-26, IGKV3D-25, IGKV2D-24, IGKV2D-23, IGKV1D-22, IGKV6D-21, IGKV3D-20, IGKV2D-19, IGKV2D-18, IGKV6D-41, IGKV1D-17, IGKV1D-16, IGKV3D-15, IGKV2D-14, IGKV1D-13, IGKV1D-12, IGKV3D-11, IGKV2D-10, IGKV1D-42, IGKV1D-43, IGKV1D-8, IGKV3D-7 and all intergenic sequences) between loxp and lox5171 of a genome of the cells obtained in the second step

Constructed fused BAC and Cre were electroporated into the positive cell clone 1A4-1E5 in the second step: BAC has Neo resistance (both ends of Neo resistance have inverted terminal repeats), and both ends of a human sequence on the BAC carry loxP and lox5171 elements which are in the same direction as those on the positive clones in the second step, and PB5' (5' ITR, 5' inverted terminal repeat) is present between the lox5171 element and a human genome sequence, and sequences between loxP and lox5171 on the BAC and the positive cell clones in the second step were replaced under the action of a Cre recombinase, and Puro resistance was replaced by the human genome sequence and Neo resistance on the BAC; and cells were subjected to G418 drug screening and related ES clones were selected for culture, amplified and identified by PCR typing.

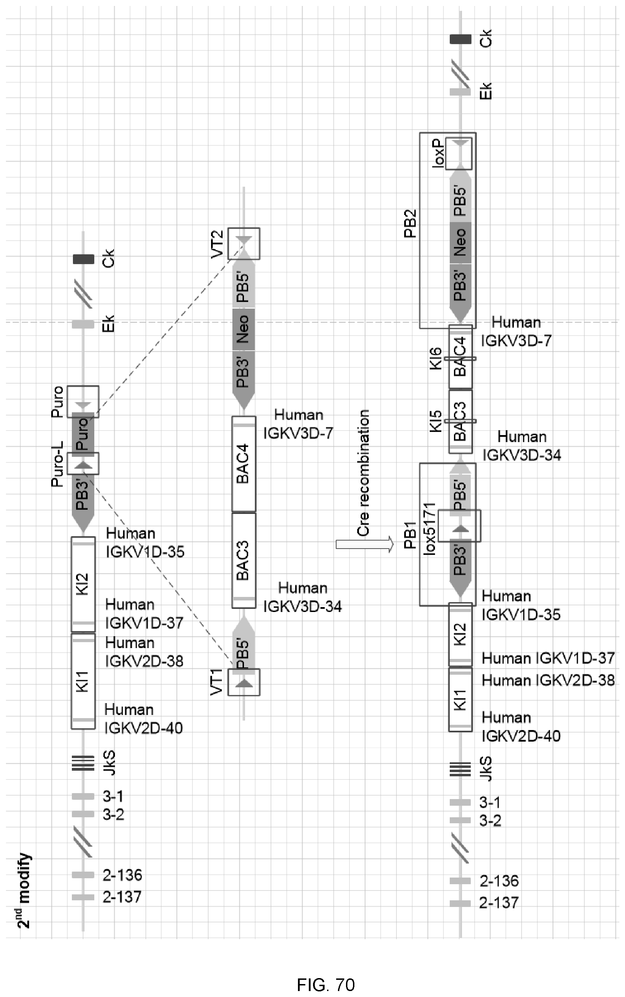

Replacement of the sequences was identified by 10 pairs of primers (the sequences are shown in Table 33 below, and primer identification positions are simultaneously labeled in a figure (FIG. 70)), where 2 pairs of the primers, primers for VT1 and VT2, were used to identify random insertions and the cells needed to be identified as negative before they were confirmed to be correctly cloned.

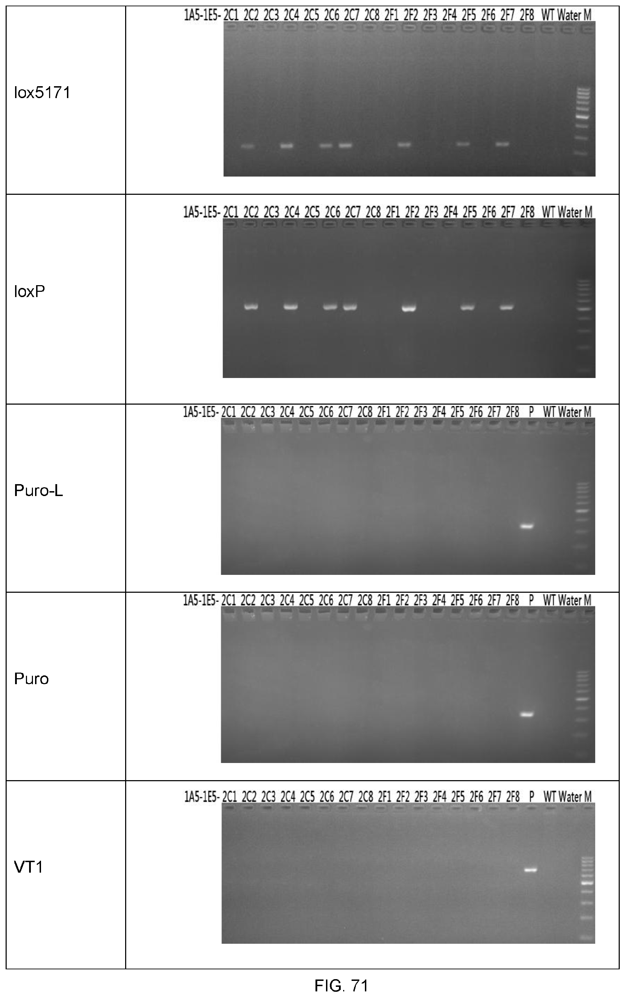

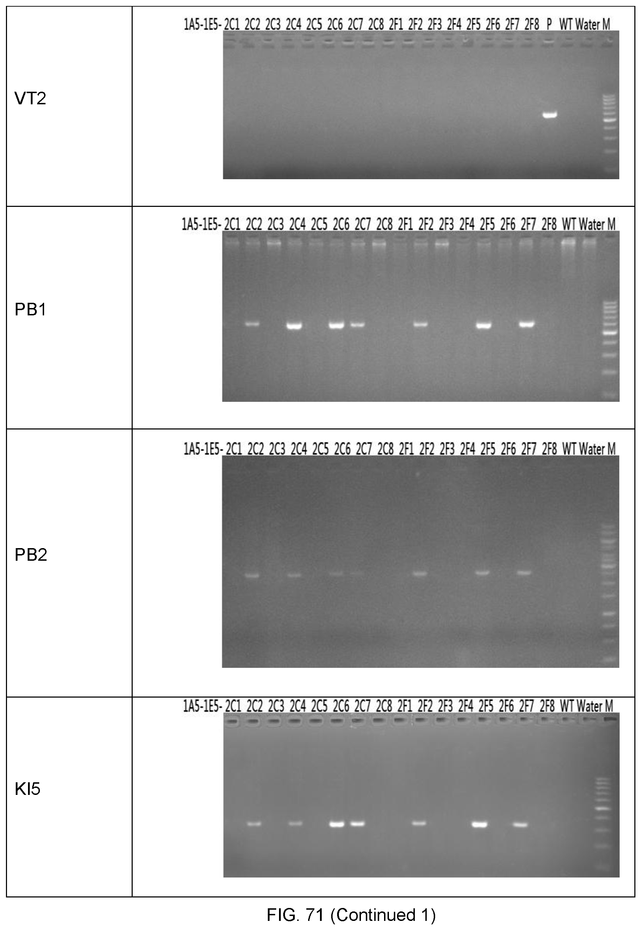

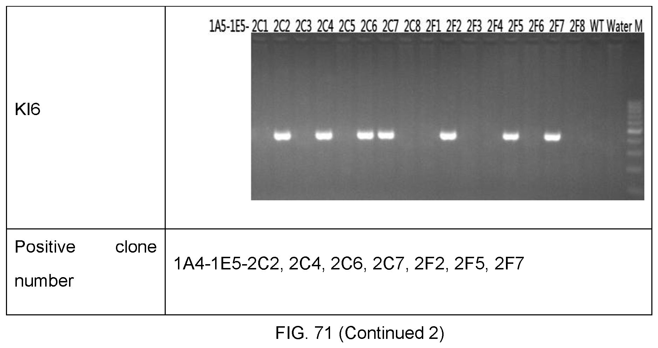

There were 7 clones with positive bands simultaneously amplified by the remaining 8 pairs of primers (FIG. 71): 1A4-1E5-2C2, 1A4-1E5-2C4, 1A4-1E5-2C6, 1A4-1E5-2C7, 1A4-1E5-2F2, 1A4-1E5-2F5, and 1A4-1E5-2F7, and the 7 clones were used as positive clones in the third step.

**Table 33**

| Aspect | Identificati on region | Produ ct size | Primer name | Primer sequence | SEQ ID NO. |
|---|---|---|---|---|---|
| ES2-FV+BA C | lox5171 | 250 | K2-F7 | taataataaattcaacaaac | 166 |
| | | | K2-R7 | GTCAAAATGACGCATGATT AT | 167 |
| | loxP | 507 | K2-F8 | agataatcatgcgtcattttgac | 168 |
| | | | K2-R8 | CTCACATTAGTGTAGTCTG TCA | 169 |
| | Puro-L | 320 | K2-F9 | ttatcggtctgtatatcgagg | 170 |
| | | | K2-R9 | ACCGGTGGATGTGGAATG TGT | 171 |
| | Puro | 690 | K1-F5 | ccctggaaggtgccactcccact | 102 |
| | | | K2-R10 | TTAGAGGAGTTTAGTGAGA G | 172 |
| | VT1 | 400 | K2-F11 | ggcagttattggtgcccttaaa | 173 |
| | | | K2-R7 | GTCAAAATGACGCATGATT AT | 167 |
| | VT2 | 560 | K2-F8 | agataatcatgcgtcattttgac | 168 |
| | | | K2-R12 | TTGTCGGTCTGATTATCGG TCT | 174 |
| | PB1 | 600 | K2-F13 | gccagatgtggttaaccctag | 175 |
| | | | K2-R13 | GCCTCCGTTAACCCTAGAA AGA | 176 |
| | PB2 | 2600 | K2-F14 | aaaggctccccacctggctag | 177 |
| | | | K2-R14 | ATCTCCAATAAGTCACCCT TT | 178 |
| | KI5 | 330 | K2-F15 | ggatgtcatactagtgaagtc | 179 |
| | | | K2-R15 | ATCCAGAAACTGCTCCACC CA | 180 |
| | KI6 | 410 | K2-F16 | aggtatcccatacttgtggatta | 181 |
| | | | K2-R16 | GGAAGCCTCCTGGACCAT CACAG | 182 |

4. Electroporation of pBase (a PiggyBac transposase) on the cell clones obtained in the third step.

pBase (the PiggyBac transposase) was electroporated into the positive cell clone 1A4-1E5-2F5 in the third step: under the action of the PiggyBac transposase, two inverted terminal repeats will be cut from a genome, a Neo sequence and lox5171 will be lost, and the positive cell clone will have only the inserted human gene sequence and one loxP element; and ES clones were selected for culture, amplified and identified by PCR typing.

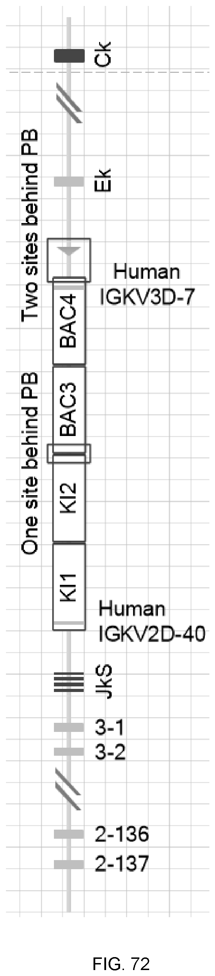

Deletions of sequences were identified by three pairs of primers (the sequences are shown in Table 34 below, and primer identification positions are simultaneously labeled in a figure (FIG. 72)).

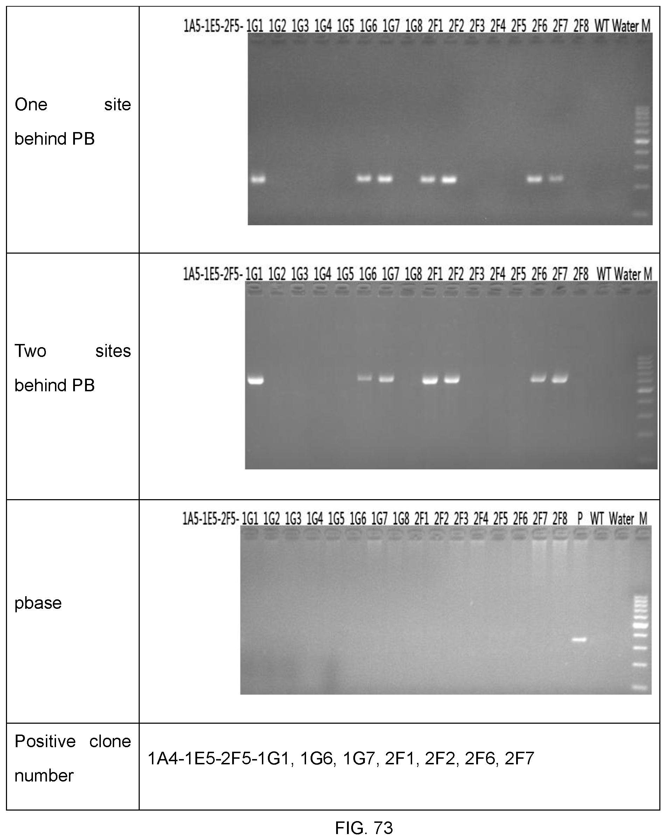

There were seven clones with positive bands simultaneously amplified by the three pairs of primers (FIG. 73): 1A4-1E5-2F5-1G1, 1A4-1E5-2F5-1G6, 1A4-1E5-2F5-1G7, 1A4-1E5-2F5-2F1, 1A4-1E5-2F5-2F2, 1A4-1E5-2F5-2F6, and 1A4-1E5-2F5-2F7, and the seven clones were used as positive clones in the third step.

**Table 34**

| Aspect | Identificati on region | Produ ct size | Primer name | Primer sequence | SEQ ID NO. |
|---|---|---|---|---|---|
| ES2-FV+BA C-PB | One site behind PB | 240 | K2-F17 | ggcaaagtaggttcatgatgct | 183 |
| | | | K2-R17 | CCACATCTGGCAAGACAGTG A | 184 |
| | Two sites behind PB | 650 | K2-F18 | gaagtagacttcaagcaaaa | 185 |
| | | | K2-R18 | TATGCCCTTTCTAATTCCCT | 186 |
| | pbase | 352 | Pbase -F | CTGGACGAGCAGAACGTGAT CG | 36 |
| | | | Pbase -R | CGAAGAAGGCGTAGATCTCG TCCTC | 37 |

### 5. F0 mice obtained from ES2

The positive cell clones obtained in the fourth step were injected into a blastocyst, and the blastocyst was then transplanted into surrogate female mice, and mice were born by gestation of about 20 days; and paws of young mice of 5-7 d were cut, DNA was extracted, and genotypes of the mice were confirmed by PCR typing identification.

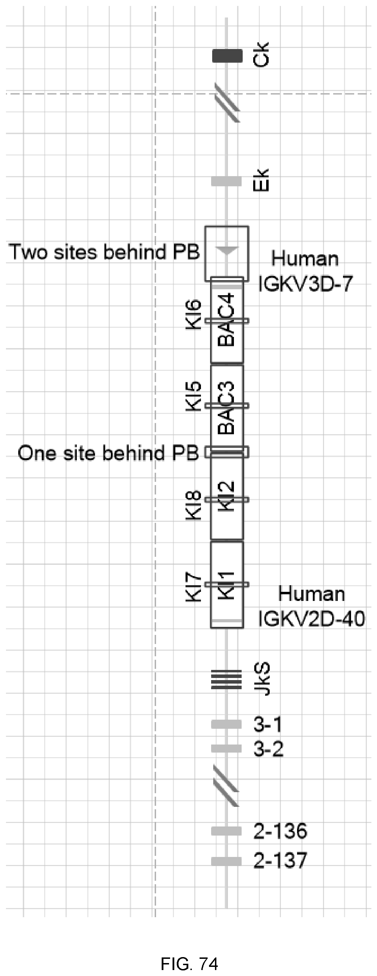

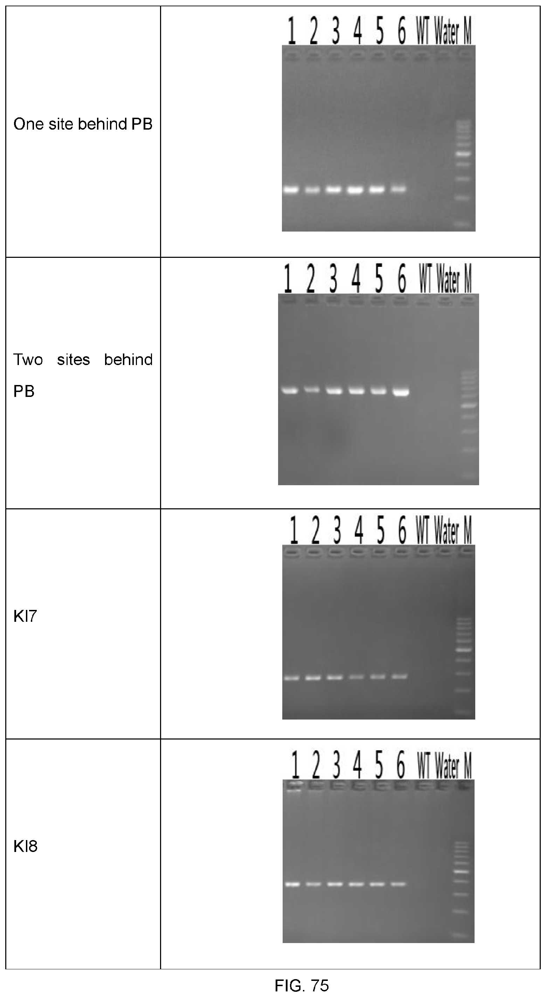

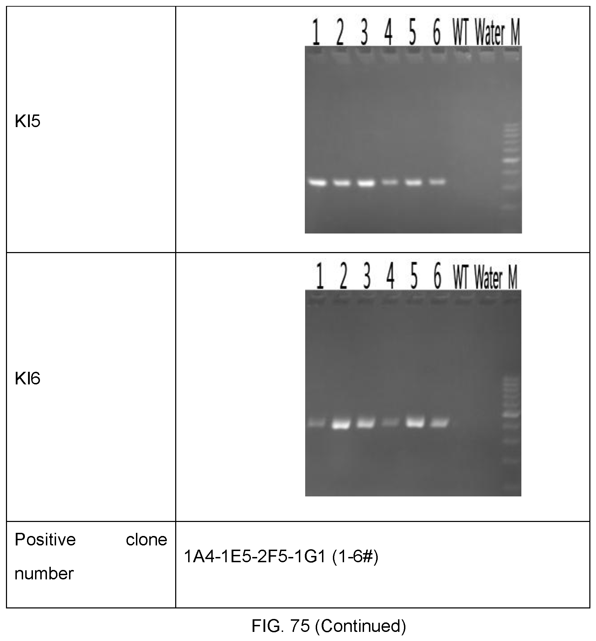

A total of 6 mice were born after injection of the clone 1A4-1E5-2F5-1G1 and all the 6 mice were co-identified as positive mice by 6 pairs of primers (Table 35). A schematic diagram of a genome of the F0 mice is shown in FIG. 74, and the identification results are shown in FIG. 75.

**Table 35**

| Aspect | Identification region | Product size | Primer name | Primer sequence | SEQ ID NO. |
|---|---|---|---|---|---|
| ES2-F0 | One site behind PB | 240 | K2-F17 | ggcaaagtaggttcatgatgct | 183 |
| | | | K2-R17 | CCACATCTGGCAAGAC AGTGA | 184 |
| | Two sites behind PB | 650 | K2-F18 | gaagtagacttcaagcaaaa | 185 |
| | | | K2-R18 | TATGCCCTTTCTAATTCC CT | 186 |
| | KI7 | 290 | K2-F19 | acatctacagaactctccatcc | 187 |
| | | | K2-R19 | GAAAAGACTACTTAGGG TCCCT | 188 |
| | KI8 | 350 | K2-F20 | tgtatggaaatttgcttgataaga | 189 |
| | | | K2-R20 | ATTATCCAGGGTTCAGT TGTCT | 190 |
| | KI5 | 330 | K2-F15 | ggatgtcatactagtgaagtc | 179 |
| | | | K2-R15 | ATCCAGAAACTGCTCCA CCCA | 180 |
| | KI6 | 410 | K2-F16 | aggtatcccatacttgtggatta | 181 |
| | | | K2-R16 | GGAAGCCTCCTGGACC ATCACAG | 182 |

### 6. F1 mice obtained from ES2

The F0 mice that were identified as the positive mice were mated with CMV-Cre mice to obtain F1 generation mice.

Genotypes of the mice were confirmed by PCR typing identification of F1 generation mouse tail genomic DNA.

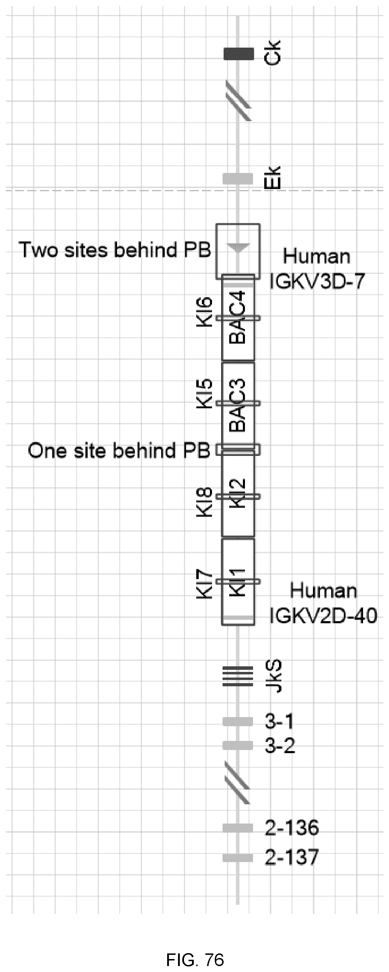

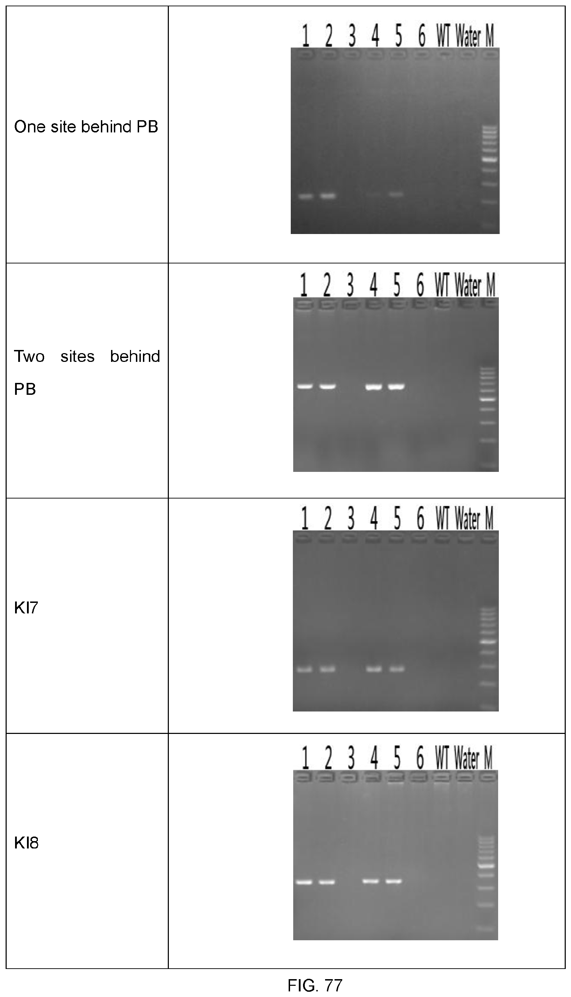

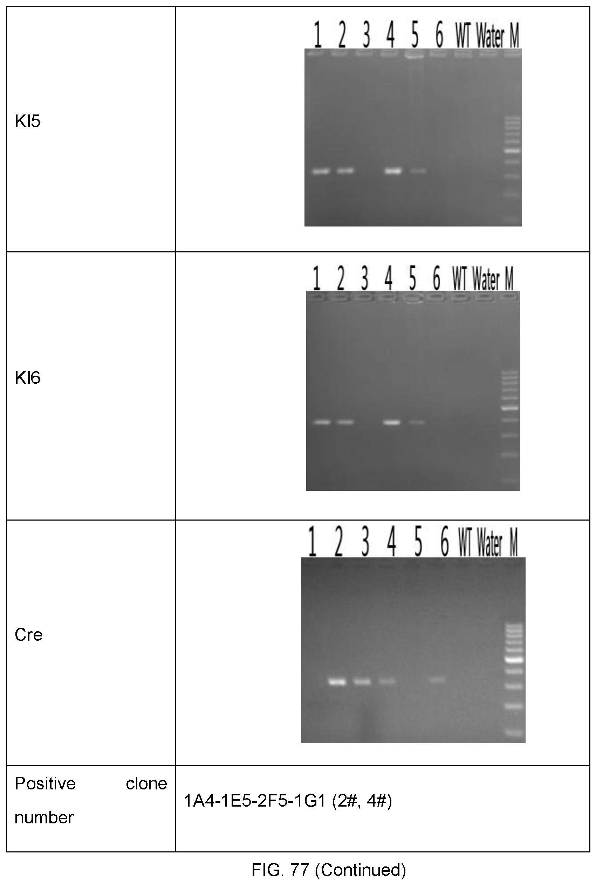

A total of 6 F1 mice were born, ES2 was co-identified with 6 pairs of primers (Table 36), CMV-Cre was identified with 1 pair of primers, and a total of 2 mice (2# and 4#) were identified as double gene positive mice. A schematic diagram of a genome of the F1 mice is shown in FIG. 76, and the identification results are shown in FIG. 77.

**Table 36**

| Aspect | Identification region | Product size | Primer name | Primer sequence | SEQ ID NO. |
|---|---|---|---|---|---|
| ES2-F1 | One site behind PB | 240 | K2-F17 | ggcaaagtaggttcatgatgct | 183 |
| | | | K2-R17 | CCACATCTGGCAAGAC AGTGA | 184 |
| | Two sites behind PB | 650 | K2-F18 | gaagtagacttcaagcaaaa | 185 |
| | | | K2-R18 | TATGCCCTTTCTAATTC CCT | 186 |
| | KI7 | 290 | K2-F19 | acatctacagaactctccatcc | 187 |
| | | | K2-R19 | GAAAAGACTACTTAGG GTCCCT | 188 |
| | KI8 | 350 | K2-F20 | tgtatggaaatttgcttgataaga | 189 |
| | | | K2-R20 | ATTATCCAGGGTTCAG TTGTCT | 190 |
| | KI5 | 330 | K2-F15 | ggatgtcatactagtgaagtc | 179 |
| | | | K2-R15 | ATCCAGAAACTGCTCC ACCCA | 180 |
| | KI6 | 410 | K2-F16 | aggtatcccatacttgtggatta | 181 |
| | | | K2-R16 | GGAAGCCTCCTGGAC CATCACAG | 182 |
| | Cre | 349 | CMV-MF2 | GTAGGCGTGTACGGTG GGAGGT | 40 |
| | | | CMV-MR | TCCAGGTATGCTCAGA AAACGCC | 41 |

### III. ES1 and ES2 mouse hybridization

1. Positive F1 mice of ES1 were hybridized with double gene F1 positive mice obtained from ES2 to obtain Cre-positive, and ES1-positive and ES2-positive F2 generation mice.

The gene was identified as ectopic by three pairs of primers (Table 37), and Cre-positive mice was identified by 1 pair of Cre primers.

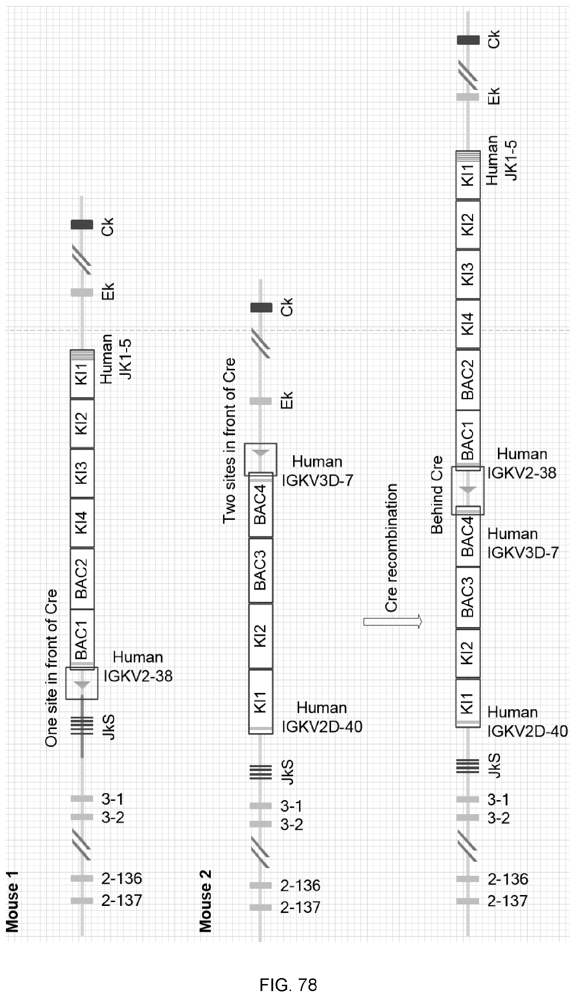

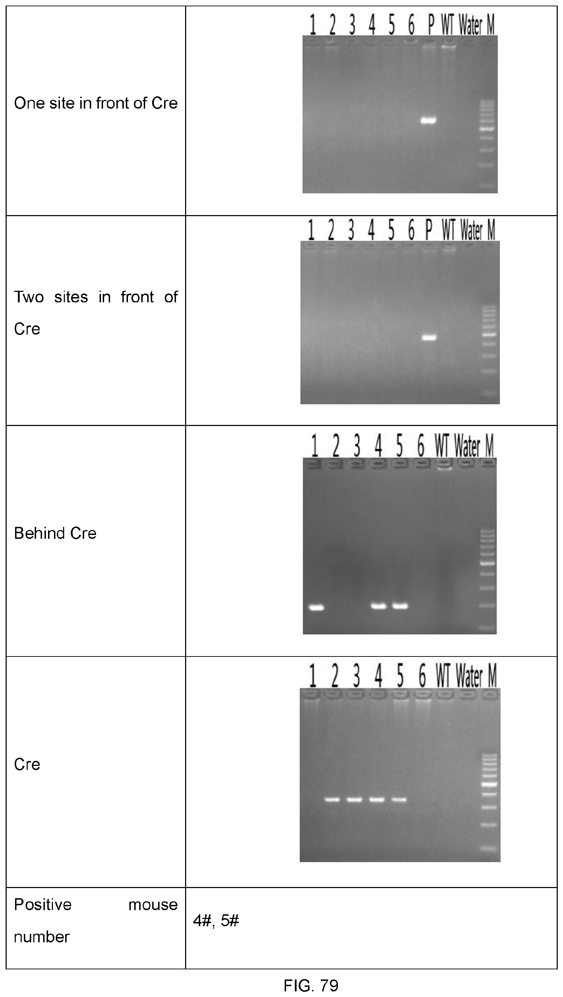

Two mice were identified as ectopic and carrying Cre: 4# and 5#. FIGS. 78 and 79 show a schematic diagram of genomic hybridization and identification results, respectively.

**Table 37**

| Aspect | Identification region | Product size | Primer name | Primer sequence | SEQ ID NO. |
|---|---|---|---|---|---|
| ES1 F1 generation X ES2+Cre F1 generation | One site in front of Cre | 600 | K3-F1 | gactgcttgccatgtag atacc | 193 |
| | | | K3-R1 | ACAACTTCTATA GGCTGCCACA | 194 |
| | Two sites in front of Cre | 480 | K3-F2 | ggaagcaggagtgaa tatattaat | 195 |
| | | | K3-R2 | ATGCTACCGCG GGGGTTCACAT | 196 |
| | Behind Cre | 200 | K3-F3 | tcagacaccccctacc ctgcc | 197 |
| | | | K3-R3 | CATGCTGCTGA GGAATGGGAAT GG | 198 |
| | Cre | 349 | CMV-MF2 | GTAGGCGTGTA CGGTGGGAGGT | 40 |
| | | | CMV-MR | TCCAGGTATGCT CAGAAAACGCC | 41 |

2. The obtained F2 positive mice were mated with wild type mice to obtain a blastocyst of 3.5 d, ES cell lines were established, and different ES cell lines were tested by PCR, and cells that do not carry Cre but have undergone ectopia encompassing the human genomic sequences of ES1 and ES2 and having a loxP sequence between IGKV1-37 and IGKV3D-7 were screened.

The gene was identified as ectopic by three pairs of primers (Table 38), and Cre-positive mice was identified by 1 pair of Cre primers.

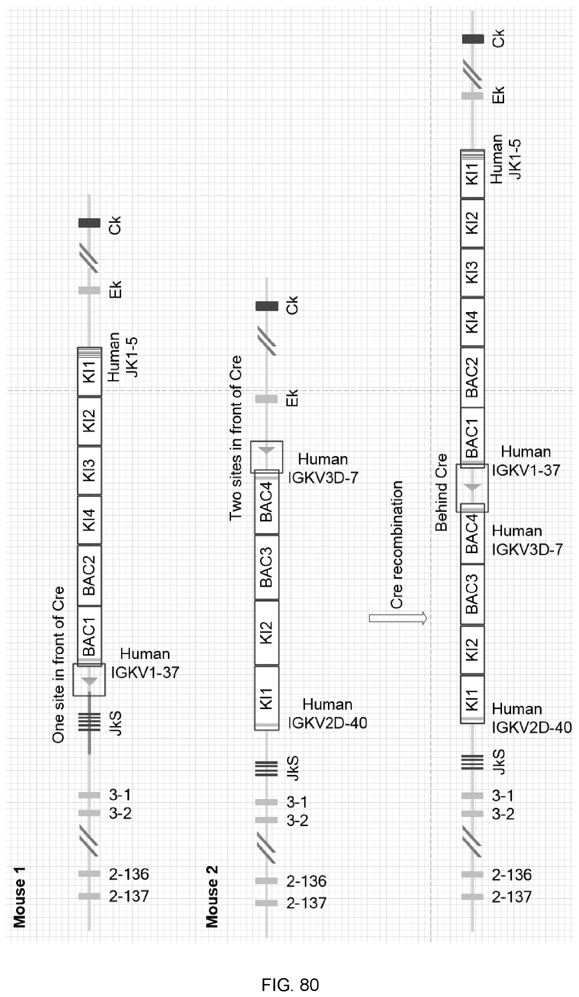

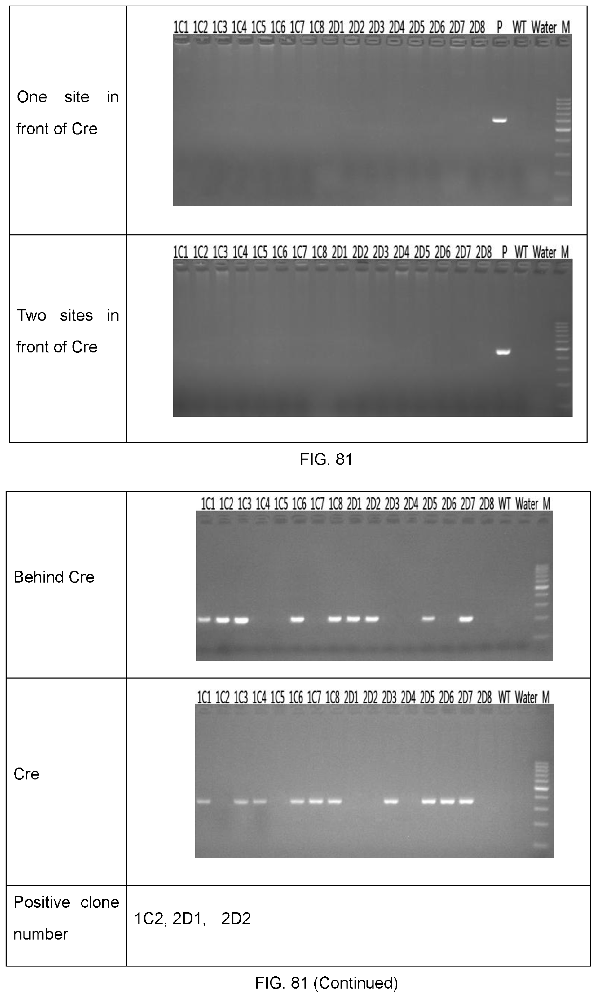

Three clones were identified as ectopic and not carrying Cre: 1C2, 2D1, and 2D2, and the three clones were used as an ES cell line III. FIGS. 80 and 81 show a schematic diagram of genomic hybridization and identification results, respectively.

**Table 38**

| Step | Aspect | Identification site | Size | Primer name | Primer sequence | SEQ ID NO. |
|---|---|---|---|---|---|---|
| Establishing a cell line from F2 | ES cells | One site in front of Cre | 600 | K3-F1 | gactgcttgccatgtagata cc | 193 |
| | | | | K3-R1 | ACAACTTCTATAGG CTGCCACA | 194 |
| | | Two sites in front of Cre | 480 | K3-F2 | ggaagcaggagtgaatat attaat | 195 |
| | | | | K3-R2 | ATGCTACCGCGGG GGTTCACAT | 196 |
| | | Behind Cre | 200 | K3-F3 | tcagacacccccctaccctg cc | 197 |
| | | | | K3-R3 | CATGCTGCTGAGG AATGGGAATGG | 198 |
| | | Cre | 349 | CMV-MF2 | GTAGGCGTGTACG GTGGGAGGT | 40 |
| | | | | CMV-MR | TCCAGGTATGCTCA GAAAACGCC | 41 |

3. Performing second homologous recombination KO (knockout of all gene sequences between Igkj5 and Igkv2-137) on the ES cell line III.

A constructed vector was electroporated into the positive cell clone 2D1 in the first step. The vector was Neo-resistant. The cells were subjected to G418 drug screening. Related ES clones were selected for culture, amplified and identified by PCR typing.

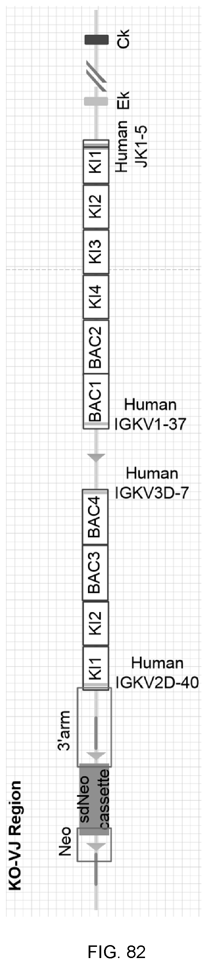

Genes between Igkj5 and Igkv2-137 were identified to have been recombined by Neo resistance by two pairs of primers (the sequences are shown in Table 39 below, and primer identification positions are simultaneously labeled in a figure (FIG. 82)), representing that the genes between Igkj5 and Igkv2-137 of a mouse IgK chain had undergone recombinant deletion.

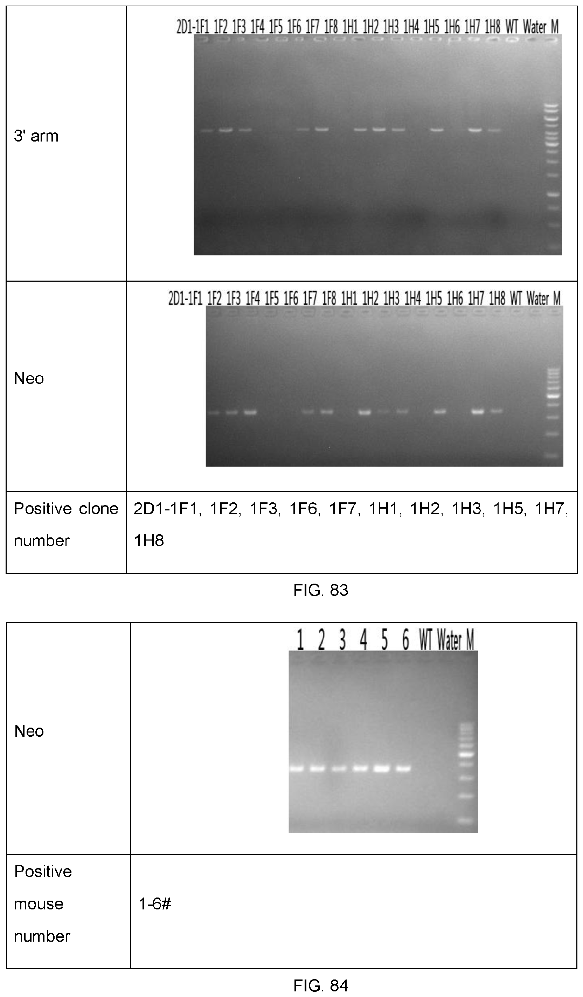

There were 11 clones with positive bands simultaneously amplified by the two pairs of primers (FIG. 83): 2D1-1F1, 2D1-1F2, 2D1-1F3, 2D1-1F6, 2D1-1F7, 2D1-1H1, 2D1-1H2, 2D1-1H3, 2D1-1H5, 2D1-1H7, and 2D1-1H8, and the 11 clones were used as positive clones in the second step.

**Table 39**

| Step | Aspect | Identification site | Size | Primer name | Primer sequence | SEQ ID NO. |
|---|---|---|---|---|---|---|
| V region KO | ES cells | 3'arm | 4300 | K3-F4 | ccagggcctggagcagccag | 199 |
| | | | | K3-R4 | GCAACTGAGGCAAAT CAAACTA | 200 |
| | | Neo | 340 | K3-F5 | ctgtcttgactactcaagact | 201 |
| | | | | K3-R5 | CATTGTCTGAGTAGG TGTCATT | 202 |

4. Injection of the positive cell clones obtained in the third step to obtain positive F0 mice

The positive cell clones obtained in the third step were injected into a blastocyst, and the blastocyst was then transplanted into surrogate female mice, and mice were born by gestation of about 20 days; and paws of young mice of 5-7 d were cut, DNA was extracted, and genotypes of the mice were confirmed by PCR typing identification.

A total of 6 mice were born after injection of the clone 2D1-1F1 and all the 6 mice were identified as positive mice by 1 pair of primers (Table 40). The identification results are shown in FIG. 84.

**Table 40**

| Aspect | Identification region | Identification site | Size | Primer name | Primer sequence | SEQ ID NO. |
|---|---|---|---|---|---|---|
| F0 mice | V region KO | Neo | 340 | K3-F5 | ctgtcttgactactcaaga ct | 201 |
| | | | | K3-R5 | CATTGTCTGAGT AGGTGTCATT | 202 |

### 5. Obtaining positive F1 mice

The F0 mice that were identified as the positive mice were mated with wild type mice to obtain F1 generation mice.

Genotypes of the mice were confirmed by PCR typing identification of F1 generation mouse tail genomic DNA.

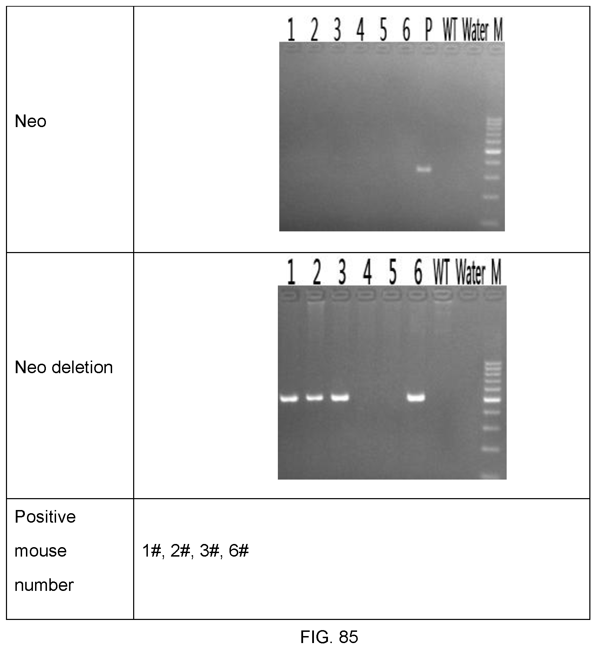

A total of 6 F1 mice were born, and co-identified by 2 pairs of primers (Table 41), and a total of 4 mice (1#, 2#, 3#, and 6#) were positive mice. The identification results are shown in FIG. 85.

**Table 41**

| Aspect | Identification region | Identification site | Size | Primer name | Primer sequence | SEQ ID NO. |
|---|---|---|---|---|---|---|
| F1 mice | V region KO | Neo | 340 | K3-F5 | ctgtcttgactactcaagac t | 201 |
| | | | | K3-R5 | CATTGTCTGAGTA GGTGTCATT | 202 |
| | | Neo deletion | 500 | K3-F6 | tgctgacccagcctttcac agtg | 203 |
| | | | | K3-R6 | TGCCCCTAATCTC ACTAGCTT | 204 |

### 6. Obtaining homozygous mice

The female and male F1 mice that were identified as the positive mice were mated with each other to obtain homozygous mice.

Genotypes of the mice were confirmed by PCR typing identification of F2 generation mouse tail genomic DNA.

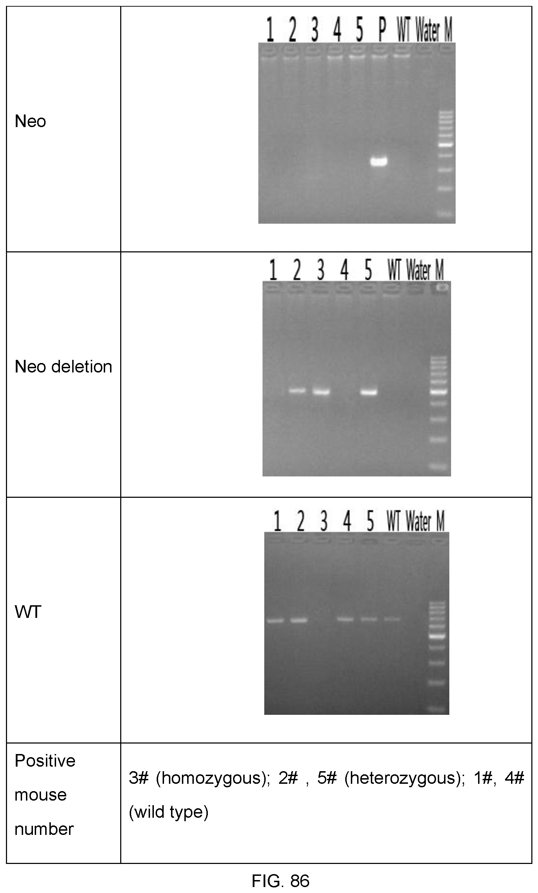

A total of 5 F2 mice were born, and co-identified with 3 pairs of primers (Table 42) to determine that the genotype of a mouse 3# was homozygous; the genotype of mice 2# and 5# was heterozygous; and the genotype of mice 1# and 4# was wild-type. The identification results are shown in FIG. 86.

**Table 42**

| Aspect | Identification region | Identification site | Size | Primer name | Primer sequence | SEQ ID NO. |
|---|---|---|---|---|---|---|
| F2 mice | V region KO | Neo | 340 | K3-F5 | ctgtcttgactactcaag act | 201 |
| | | | | K3-R5 | CATTGTCTGAGT AGGTGTCATT | 202 |
| | | Neo deletion | 500 | K3-F6 | tgctgacccagcctttca cagtg | 203 |
| | | | | K3-R6 | TGCCCCTAATCT CACTAGCTT | 204 |
| | | WT | 690 | K3-F7 | gacagacagctcaagg ggtt | 205 |
| | | | | K3-R7 | CCTAACCAGTTA AGGTCATGTCA | 206 |

### Breeding performance testing

The obtained homozygous mice continued to be bred in a manner of mating one male mouse with one female mouse, and the breeding performance of the mice was counted: a total of 24 breeding pairs were mated, and the number of mice born 1 month after each breeding pair was mated was counted. The results are shown in Table 43.

**Table 43**

| Breeding pair | Number of births | Breeding pair | Number of births |
|---|---|---|---|
| Breeding pair 1 | 0 | Breeding pair 13 | 0 |
| Breeding pair 2 | 6 | Breeding pair 14 | 3 |
| Breeding pair 3 | 5 | Breeding pair 15 | 7 |
| Breeding pair 4 | 5 | Breeding pair 16 | 5 |
| Breeding pair 5 | 6 | Breeding pair 17 | 0 |
| Breeding pair 6 | 4 | Breeding pair 18 | 5 |
| Breeding pair 7 | 3 | Breeding pair 19 | 6 |
| Breeding pair 8 | 7 | Breeding pair 20 | 5 |
| Breeding pair 9 | 5 | Breeding pair 21 | 4 |
| Breeding pair 10 | 5 | Breeding pair 22 | 3 |
| Breeding pair 11 | 5 | Breeding pair 23 | 6 |
| Breeding pair 12 | 4 | Breeding pair 24 | 6 |

There were a total of 24 breeding pairs, 20 breeding pairs gave birth to mice after 1 month of mating, the effective breeding pairs reached 87.5%, while the mice had an average birth of 5, wich was consistent with the number of births of wild type mice, confirming that the breeding performance of mice of this genotype was normal.

### V. Kappa chain (K chain) immune repertoire sequencing detection

The obtained homozygous mice were euthanized, spleens of the mice were dissected, after RNA was extracted and totalRNA extracted from a sample was detected to be qualified, library construction was performed, followed by immune repertoire sequencing (Immuno-Seq uses B/T lymphocytes as a research objective to specifically amplify a variable region (a V region) that determines the diversity of a B cell receptor (BCR) or a T cell receptor (TCR) by 5' RACE or a multiplex PCR technology, so as to comprehensively evaluate the diversity of an immune system combined with a high-throughput sequencing technology); and sequences obtained by sequencing were subjected to quality control by using quality control software, and the sequencing background was filtered, then the sequences were aligned with V and J genes of an IMGT immune cell receptor library, the corresponding gene fragments were searched to find accurate V and J gene fragments and sequence loci, and information such as V and J gene frequencies, clone frequency distribution, and the number of polypeptide sequences were statistically analyzed.

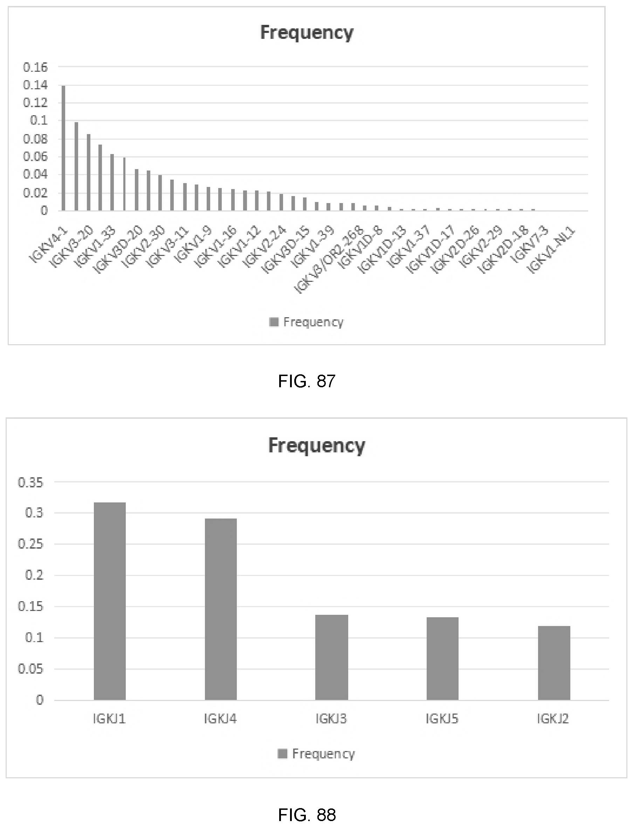

Based on the obtained gene usage frequency data, a distribution graph of a gene usage frequency at a reads level was plotted. The results are shown in FIGS. 87 and 88. Based on the sequencing results, it was confirmed that IGKV4-1 was used at the highest frequency at the reads level in spleen RNA of unimmunized mice, followed by IGKV1-33 and IGKV3D-20, and the usage frequencies sequentially decreased as shown in the figure.

Based on the sequencing results, it was confirmed that IGKJ1 was used at the highest frequency at the reads level in spleen RNA of unimmunized mice, and the usage frequency of IGKJ4, IGJK3, IGKJ5, and IGKJ2 sequentially decreased.

The statistical analysis of CDR3 amino acid sequence characteristics is an important step in immunopanel analysis, by refining to each clone, different CDR3 sequences evolved from a sequence of each germline antibody are subjected to quantitative sequence abundance analysis, and finally, it is convenient to select sequences for further antibody expression.

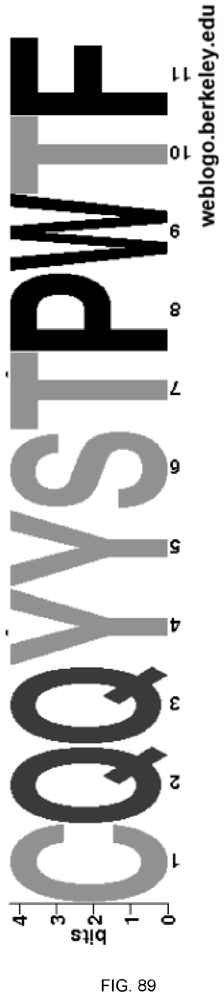

Weblogo profiling of a CDR3 amino acid sequence of each sample at the reads level is shown in FIG. 89. The results showed an amino acid sequence with the highest CDR3 abundance in unimmunized homozygous mice.

### Example 3. Modification of Lambda light chain modified mice

### I. ES cell line I

### 1. Insertion of two lox sites loxp-lox5171 into an Iglc1 gene and downstream of Igl Enhancer to obtain positive clones.

A constructed vector was electroporated into wild-type ES cells: the vector carries Neo with loxP and lox5171-PB5' (5' ITR, 5' inverted terminal repeat) elements at both ends of Neo resistance; and the cells were subjected to G418 drug screening and related ES clones were selected for culture, amplified and identified by PCR typing.

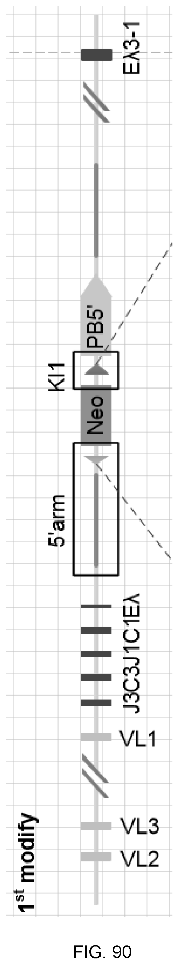

The cell clones were amplified by two pairs of primers (the sequences are shown in Table 44 below, and primer identification positions are simultaneously labeled in a figure (FIG. 90)) to identify the insertion of the sequence into the corresponding position.

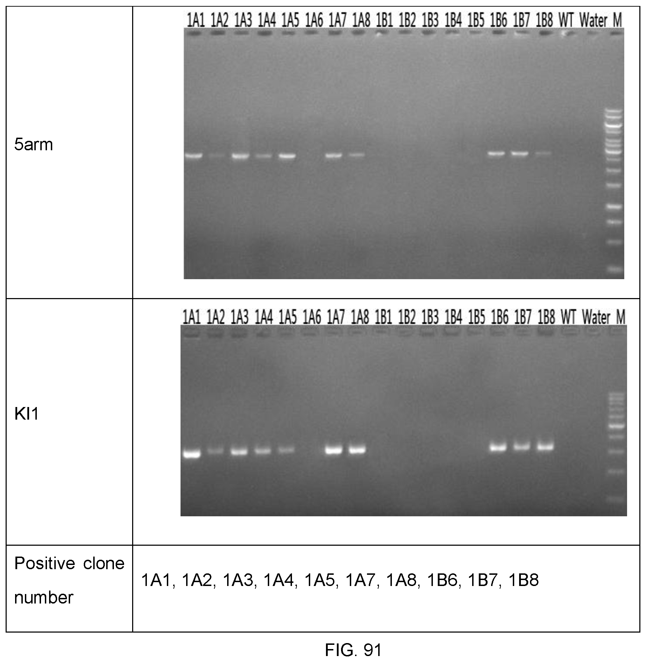

There were ten clones with positive bands simultaneously amplified by the two pairs of primers (FIG. 91): 1A1, 1A2, 1A3, 1A4, 1A5, 1A7, 1A8, 1B6, 1B7, and 1B8, and the ten clones were used as positive clones in the first step.

**Table 44**

| Aspect | Identification region | Product size | Primer name | Primer sequence | SEQ ID NO. |
|---|---|---|---|---|---|
| ES1-V1 | 5'arm | 2800 | L1-F1 | ggtccaaccaccagacagtgt | 207 |
| | | | L1-R1 | TAATGACCCCGTAATT GATTAC | 208 |
| | KI1 | 320 | L1-F2 | gaggaaattgcatcgcattg | 209 |
| | | | L1-R2 | AACTATAACGACCGC GTGAGT | 210 |

### 2. Insertion of about 360 kb of a human V, J, and C genome sequence (including IGLC7, IGLJ7, IGLC6, IGLJ6, IGLC5, IGLJ5, IGLC4, IGLJ4, IGLC3, IGLJ3, IGLC2, IGLJ2, IGLC1, IGLJ1, IGLV3-1, IGLV3-2, IGLV4-3, IGLV3-4, IGLV2-5, IGLV3-6, IGLV3-7, IGLV2-8, IGLV3-9, IGLV3-10, IGLV2-11, IGLV3-12, IGLV3-13, IGLV2-14, IGLV3-15, IGLV3-16, IGLV3-17, IGLV2-18, IGLV3-19, IGLV1-20, IGLV3-21, IGLV3-22, IGLV2-23, IGLV3-24, IGLV3-25, IGLV3-26, IGLV3-27, IGLV2-28, IGLV3-29, IGLV3-30, IGLV3-31, IGLV3-32, IGLV2-23, IGLV2-34 and all intergenic sequences) between loxP and lox5171 of a genome of the cells obtained in the first step to obtain positive clones

Constructed fused BAC and Cre were electroporated into the positive cell clone 1A1 in the first step: BAC has Puro resistance (both ends of a Puro antibody have inverted terminal repeats), and both ends of a human sequence on the BAC carry loxP and lox5171 elements which are in the same direction as those on the positive clones in the first step, and PB3' (3' ITR, 3' inverted terminal repeat) is present between the lox5171 element and a human genome sequence, and sequences between loxP and lox5171 on the BAC and the positive cell clones in the first step were replaced under the action of a Cre recombinase, and Neo resistance was replaced by the human genome sequence and Puro resistance on the BAC; and cells were subjected to G418 drug screening and related ES clones were selected for culture, amplified and identified by PCR typing.

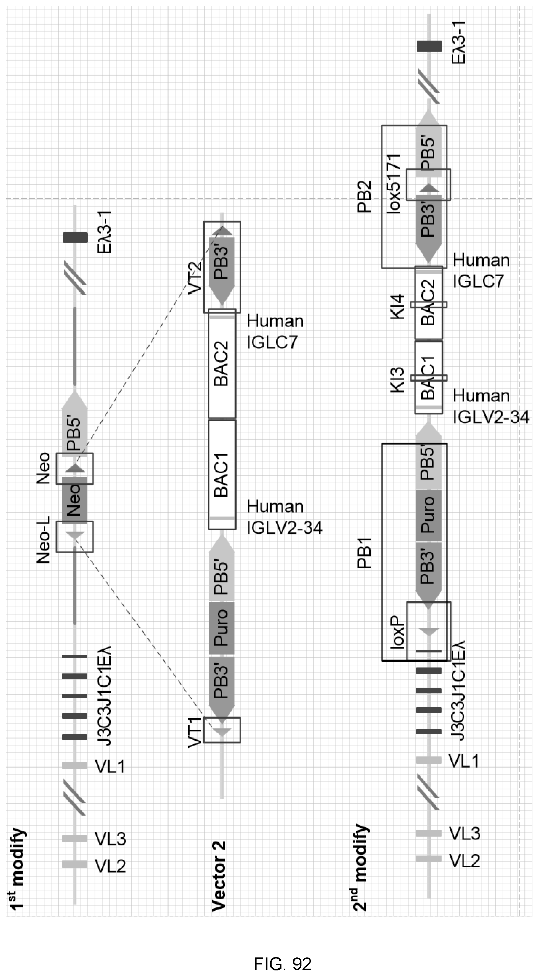

Replacement of the sequences was identified by 10 pairs of primers (the sequences shown in Table 45 below, and primer identification positions are simultaneously labeled in a figure (FIG. 92)), where 2 pairs of the primers, primers for VT1 and VT2, were used to identify whether the vector was randomly inserted into the genome, and the cells needed to be identified as negative before they were confirmed to be correctly cloned.

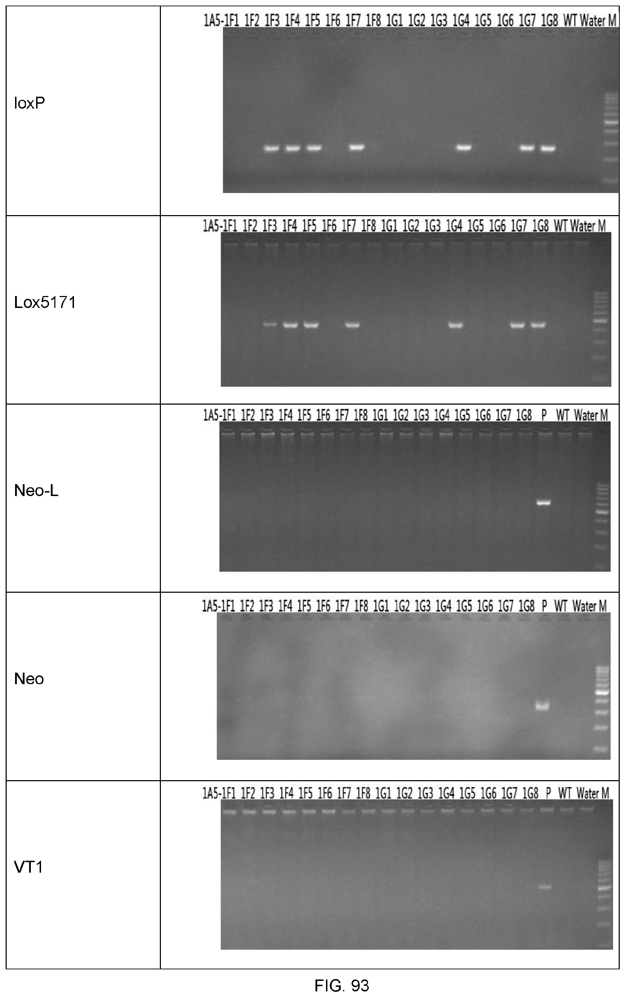

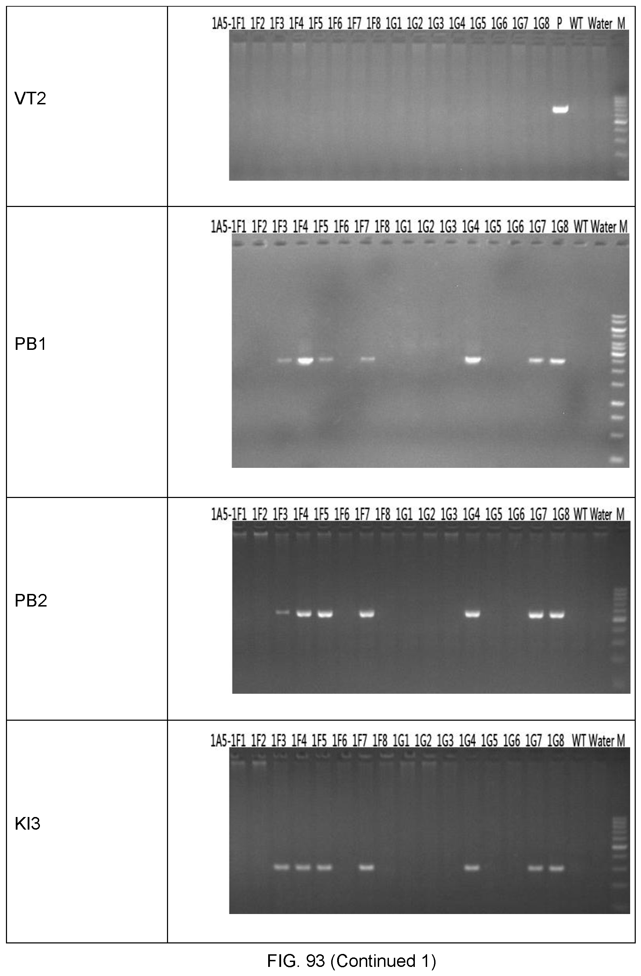

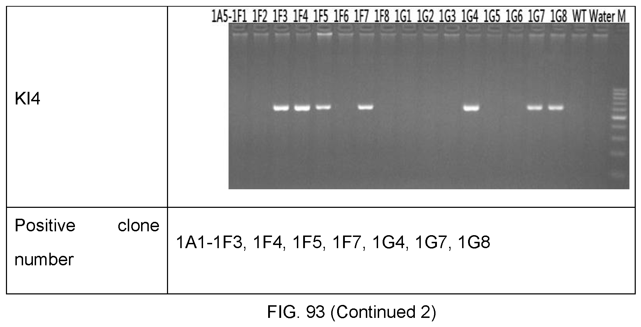

There were 7 clones with positive bands simultaneously amplified by the remaining 8 pairs of primers (FIG. 93): 1A1-1F3, 1A1-1F4, 1A1-1F5, 1A1-1F7, 1A1-1G4, 1A1-1G7, and 1A1-1G8, and the 7 clones were used as positive clones in the second step.

**Table 45**

| Aspect | Identification region | Product size | Primer name | Primer sequence | SEQ ID NO. |
|---|---|---|---|---|---|
| ES1-V1+BAC | IoxP | 250 | L1-F3 | atcatcttcaaggccccagcta | 211 |
| | | | L1-R3 | ACCTCGATATACAGACCGA TA | 212 |
| | Lox5171 | 450 | L1-F4 | ttacacttacatactaataa | 213 |
| | | | L1-R4 | TTAACCCTAGAAAGATAGT CTG | 214 |
| | Neo-L | 600 | L1-F5C | catgtaggtgtcatgtctccat | 215 |
| | | | L1-R5 | AATCCCCGTGAGTCAAAC CG | 216 |
| | Neo | 320 | L1-F6 | gaaattgcatcgcattgtctg | 217 |
| | | | L1-R6 | TGAACTATAACGACCGCGT GA | 218 |
| | VT1 | 500 | L1-F7 | cttcttcgcccccgttttcacc | 219 |
| | | | L1-R7 | CGCATGATTATCTTTAACG TAC | 220 |
| | VT2 | 690 | L1-F8 | ctcagtttctcacattggggta | 221 |
| | | | L1-R8 | GGCTGCATCCGATGCAAG TGTG | 222 |
| | PB1 | 2500 | L1-F9 | catgtaggtgtcatgtctcc | 223 |
| | | | L1-R9 | GACCAGGCCTCCTGGTTT AAC | 224 |
| | PB2 | 520 | L1-F10 | ttgtctgagccactcttttt | 225 |
| | | | L1-R10 | AGATAGGAGTTGGGTGGT GCA | 226 |
| | KI3 | 300 | L1-F11 | gatgccatggtgtggaagga | 227 |
| | | | L1-R11 | AAGCCCTTGGCATAGCTG GG | 228 |
| | KI4 | 600 | L1-F12 | tggacattggctaagtaaacat | 229 |
| | | | L1-R12 | GCTACGGAGGGAGAGGGT GCT | 230 |

### 3. Electroporation of pBase (a PiggyBac transposase) on the cell clones obtained in the second step

pBase (the PiggyBac transposase) was electroporated into the positive cell clone 1A1-1G4 in the second step: under the action of the PiggyBac transposase, two inverted terminal repeats will be cut from a genome, a Puro sequence and lox5171 will be lost, and the positive cell clone will have only the inserted human gene sequence and one loxP element; and ES clones were selected for culture, amplified and identified by PCR typing.

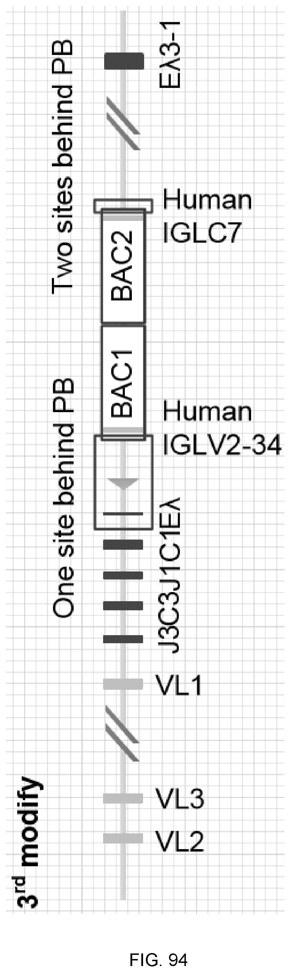

Deletions of sequences were identified by three pairs of primers (the sequences are shown in Table 46 below, and primer identification positions are simultaneously labeled in a figure (FIG. 94)).

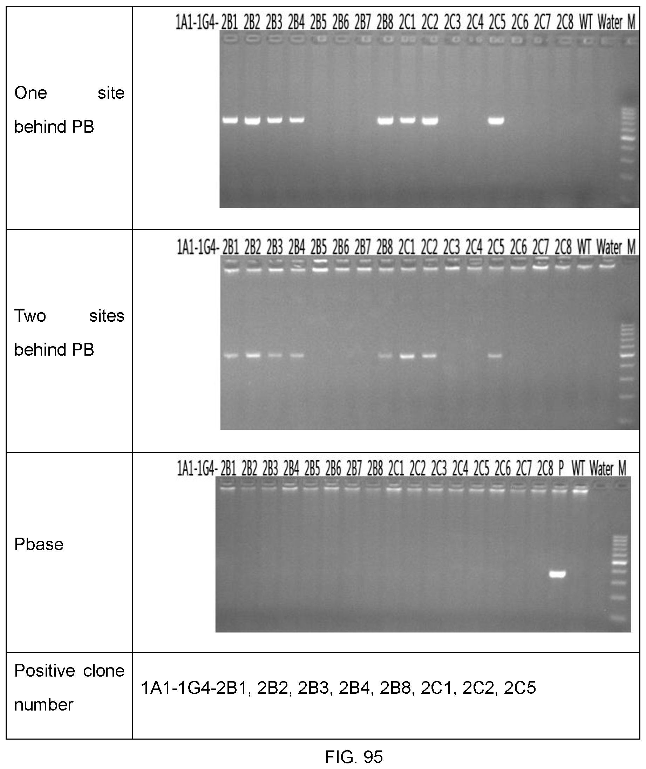

There were eight clones with positive bands simultaneously amplified by the three pairs of primers (FIG. 95): 1A1-1G4-2B1, 1A1-1G4-2B2, 1A1-1G4-2B3, 1A1-1G4-2B4, 1A1-1G4-2B8, 1A1-1G4-2C1, 1A1-1G4-2C2, and 1A1-1G4-2C5, and the eight clones were used as positive clones in the third step.

**Table 46**

| Aspect | Identification region | Product size | Primer name | Primer sequence | SEQ ID NO. |
|---|---|---|---|---|---|
| ES1-V1+BAC-PB | One site behind PB | 690 | L1-F13 | acatcttagctcttgaggtatg | 231 |
| | | | L1-R13 | AACGAACATATTGGGA CACAA | 232 |
| | Two sites behind PB | 500 | L1-F14 | acaacctaaaagaaatagcct | 233 |
| | | | L1-R14 | GGTTGATCATAGTACC TGTAT | 234 |
| | pbase | 352 | pbase-F1 | CTGGACGAGCAGAAC GTGATCG | 36 |
| | | | pbase-R1 | CGAAGAAGGCGTAGA TCTCGTCCTC | 37 |

### 4. F0 mice obtained from ES1

The positive cell clones obtained in the third step were injected into a blastocyst, and the blastocyst was then transplanted into surrogate female mice, and F0 mice were born by gestation of about 20 days; and paws of young mice of 5-7 d were cut, DNA was extracted, and genotypes of the mice were confirmed by PCR typing identification.

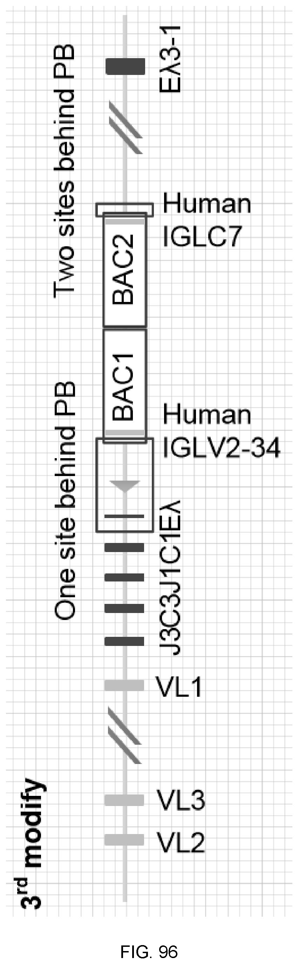

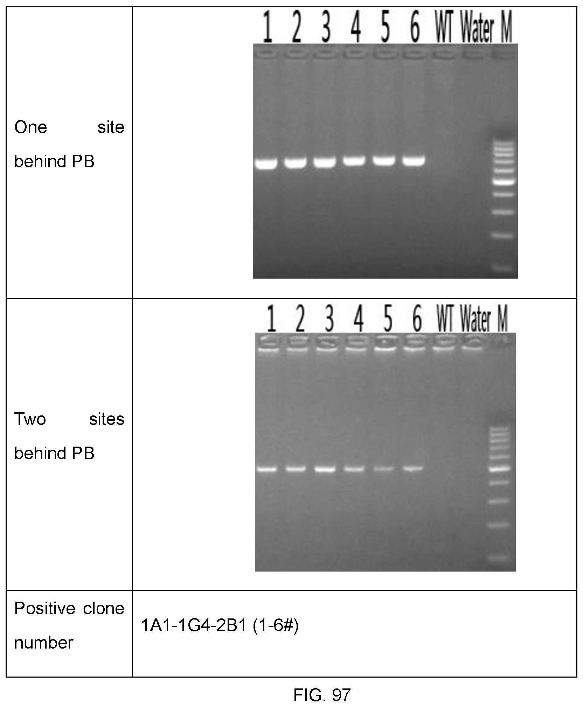

A total of 6 mice were born after injection of the clone 1A1-1G4-2B1 and all the 6 mice were co-identified as positive mice by 3 pairs of primers (Table 47). The schematic diagram and identification diagram of the F0 mouse genome are shown in FIGS. 96 and 97, respectively.

**Table 47**

| Aspect | Identification region | Product size | Primer name | Primer sequence | SEQ ID NO. |
|---|---|---|---|---|---|
| ES1-F0 | One site behind PB | 690 | L1-F13 | acatcttagctcttgaggtat g | 231 |
| | | | L1-R13 | AACGAACATATTGG GACACAA | 232 |
| | Two sites behind PB | 500 | L1-F14 | acaacctaaaagaaatag cct | 233 |
| | | | L1-R14 | GGTTGATCATAGTA CCTGTAT | 234 |

### 5. F1 mice obtained from ES1

The F0 mice that were identified as the positive mice were mated and bred with wild type mice to obtain F1 generation mice.

Genotypes of the mice were confirmed by PCR typing identification of F1 generation mouse tail genomic DNA.

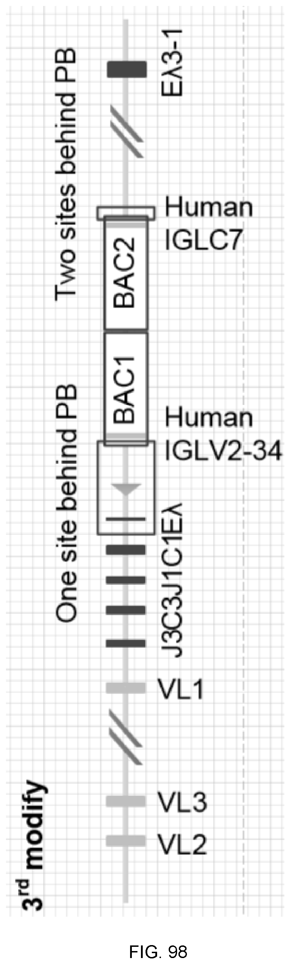

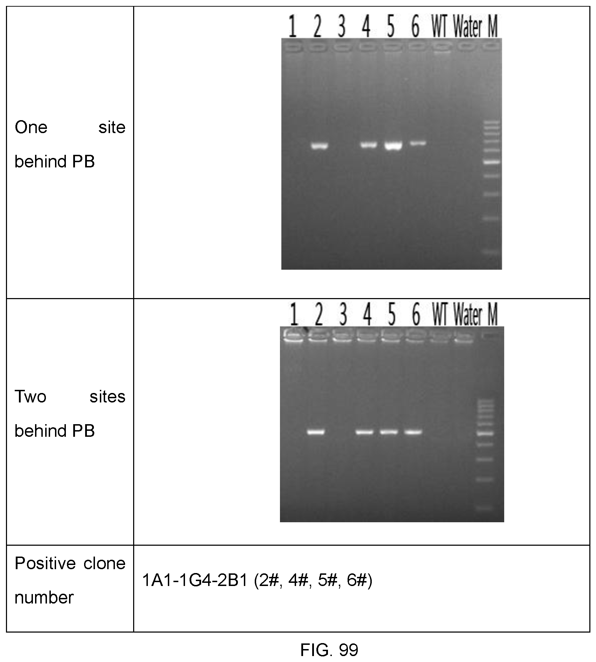

A total of 6 F1 mice were born, ES1 was co-identified with 2 pairs of primers (Table 48), and a total of 4 mice (2#, 4#, 5#, and 6#) were positive F1 mice. The schematic diagram and identification diagram of the F0 mouse genome are shown in FIGS. 98 and 99, respectively.

**Table 48**

| Aspect | Identification region | Product size | Primer name | Primer sequence | SEQ ID NO. |
|---|---|---|---|---|---|
| ES1-F1 | One site behind PB | 690 | L1-F13 | acatcttagctcttgaggtatg | 231 |
| | | | L1-R13 | AACGAACATATTGGG ACACAA | 232 |
| | Two sites behind PB | 500 | L1-F14 | acaacctaaaagaaatagcc t | 233 |
| | | | L1-R14 | GGTTGATCATAGTAC CTGTAT | 234 |

### II. ES cell line II

1. Insertion of about 20 kb of a human gene sequence (including IGLV4-69, IGLVI-68, IGLV10-67 and all intergenic sequences) into a mouse Iglc1 gene and downstream of Igl Enhancer.

A constructed vector was electroporated into wild-type ES cells. The vector was Neo-resistant. The cells were subjected to G418 drug screening. Related ES clones were selected for culture, amplified and identified by PCR typing.

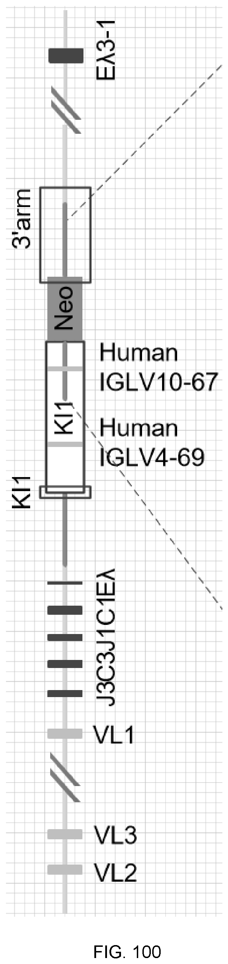

The insertion of the sequence into the corresponding position was identified by two pairs of primers (the sequences are shown in Table 49 below, and primer identification positions are simultaneously labeled in a figure (FIG. 100)).

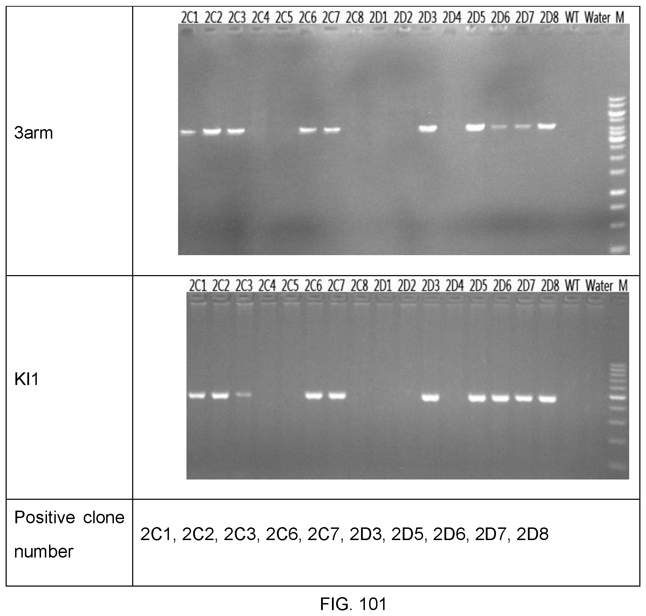

There were ten clones with positive bands simultaneously amplified by the two pairs of primers (FIG. 101): 2C1, 2C2, 2C3, 2C6, 2C7, 2D3, 2D5, 2D6, 2D7, and 2D8, and the ten clones were used as positive clones in the first step.

**Table 49**

| Aspect | Identification region | Product size | Primer name | Primer sequence | SEQ ID NO. |
|---|---|---|---|---|---|
| ES2-V1 | 3'arm | 4300 | L2-F1 | ggctacccgtgatattgctgaaga | 235 |
| | | | L2-R1 | AGACAGTGAGATGCAG CAGGCA | 236 |
| | KI1 | 450 | L2-F2 | tggaggaacttctgggtattta | 237 |
| | | | L2-R2 | AGCATAACTTCCAACGT TGTT | 238 |

2. Insertion of about 20 kb of a human V genome sequence (including IGLVIV-66-1, IGLVV-66, IGLVIV-65 and all intergenic sequences) while introducing two lox sites loxp-lox2272 to obtain positive clones.

A constructed vector was electroporated into the positive cell clone 2C1 in the first step: the vector carries Puro resistance and a homology arm on one end is placed on the human sequence with the positive clones in the first step, so Neo resistance will be deleted by homologous recombination in this step, and both ends of Puro resistance carry PB3' (3' ITR, 3' inverted terminal repeat)-lox2272 and loxP elements, respectively; cells were subjected to Puromycin drug screening and related ES clones were selected for culture, amplified and identified by PCR typing.

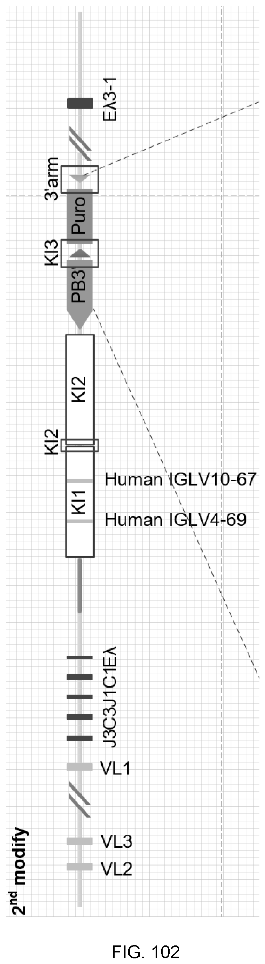

The insertion of the sequence into the corresponding position was identified by 3 pairs of primers (the sequences are shown in Table 50 below, and primer identification positions are simultaneously labeled in a figure (FIG. 102)).

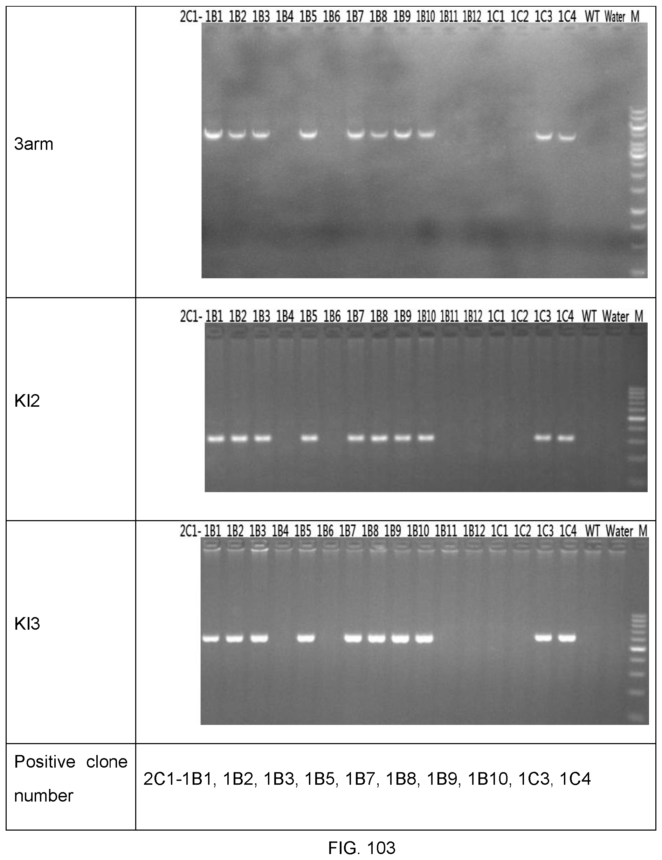

There were ten clones with positive bands simultaneously amplified by the three pairs of primers (FIG. 103): 2C1-1B1, 2C1-1B2, 2C1-1B3, 2C1-1B5, 2C1-1B7, 2C1-1B8, 2C1-1B9, 2C1-1B10, 2C1-1C3, and 2C1-1C4, and the ten clones were used as positive clones in the second step.

**Table 50**

| Aspect | Identification region | Product size | Primer name | Primer sequence | SEQ ID NO. |
|---|---|---|---|---|---|
| ES2-V1V2 | 3'arm | 4100 | L2-F3 | ctgctgcaacttacctccggg | 239 |
| | | | L2-R3 | GGGTGGGTTGGGATC TAGGGT | 240 |
| | KI2 | 310 | L2-F4 | atccaagcctagccagtactga | 241 |
| | | | L2-R4 | AGTCTACTAAATGTTC ACAGTAT | 242 |
| | KI3 | 600 | L2-F5 | aatcccagcactttgggaggct | 243 |
| | | | L2-R5 | CGTGCTACTTCCATTT GTCACGT | 244 |

3. Insertion of about 390 kb of a human V, J, and C genome sequence (including IGLV7-35, IGLV1-36, IGLV5-37, IGLV(I)-38, IGLV5-39, IGLV1-40, IGLV1-41, IGLVVII-41-1, IGLVI-42, IGLV7-43, IGLV1-44, IGLV5-45, IGLV7-46, IGLV1-47, IGLV5-48, IGLV9-49, IGLV1-50, IGLV1-51, IGLV5-52, IGLVIV-53, IGLV10-54, IGLV11-55, IGLVI-56, IGLV6-57, IGLVV-58, IGLVIV-59, IGLV4-60, IGLV8-61, IGLV1-62, IGLVI-63 and all intergenic sequences) between loxp and lox2272 of a genome of the cells obtained in the second step to obtain positive clones.

Constructed fused BAC and Cre were electroporated into the positive cell clone 2C1-1C3 in the second step: BAC has Neo resistance (both ends of Neo resistance have inverted terminal repeats), and both ends of a human sequence on the BAC carry loxP and lox2272 elements which are in the same direction as those on the positive clones in the second step, and PB5' (5' ITR, 5' inverted terminal repeat) is present between the lox2272 element and a human genome sequence, and sequences between loxP and lox2272 on the BAC and the positive cell clones in the second step were replaced under the action of a Cre recombinase, and Puro resistance was replaced by the human genome sequence and Neo resistance on the BAC; and cells were subjected to G418 drug screening and related ES clones were selected for culture, amplified and identified by PCR typing.

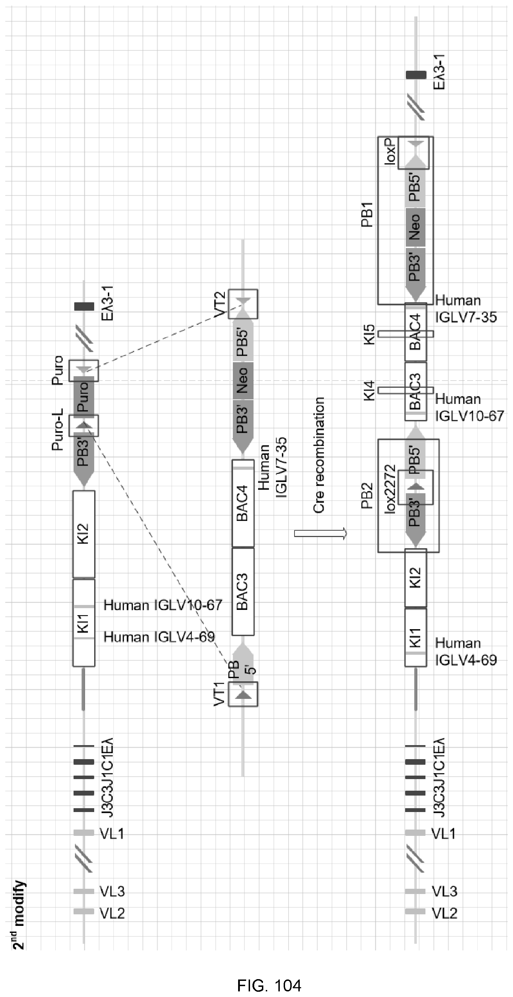

Replacement of the sequences was identified by 10 pairs of primers (the sequences are shown in Table 51 below, and primer identification positions are simultaneously labeled in a figure (FIG. 104)), where 2 pairs of the primers, primers for VT1 and VT2, were used to identify random insertions and the cells needed to be identified as negative before they were confirmed to be correctly cloned.

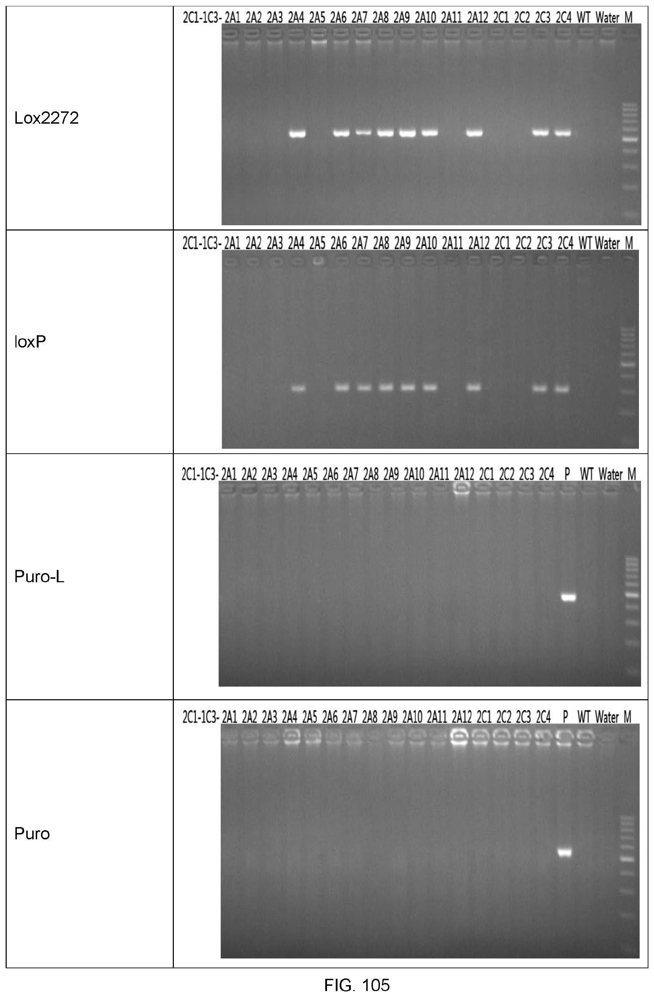

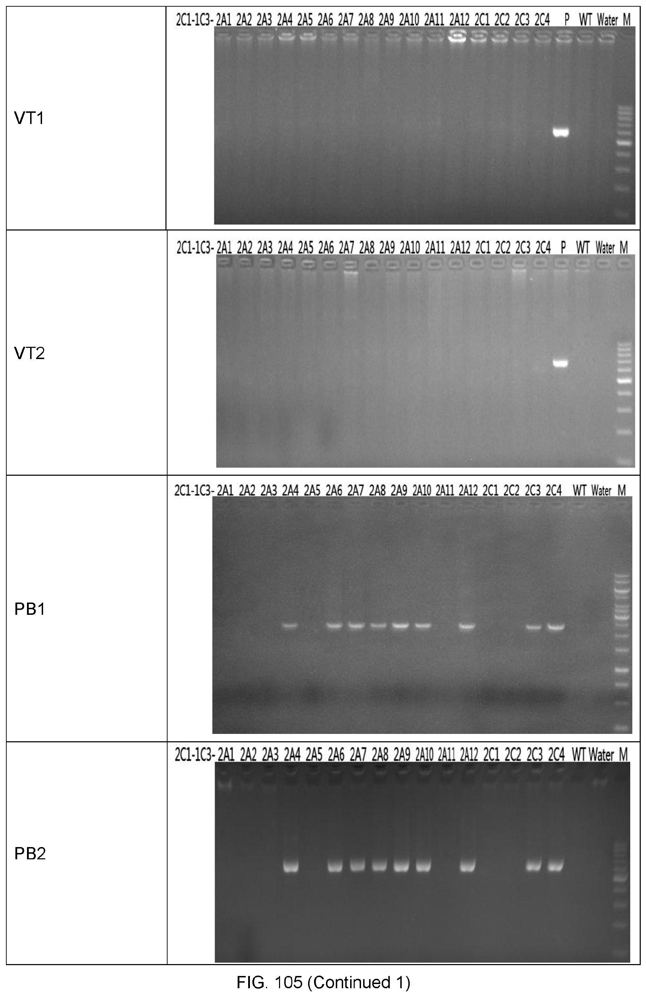

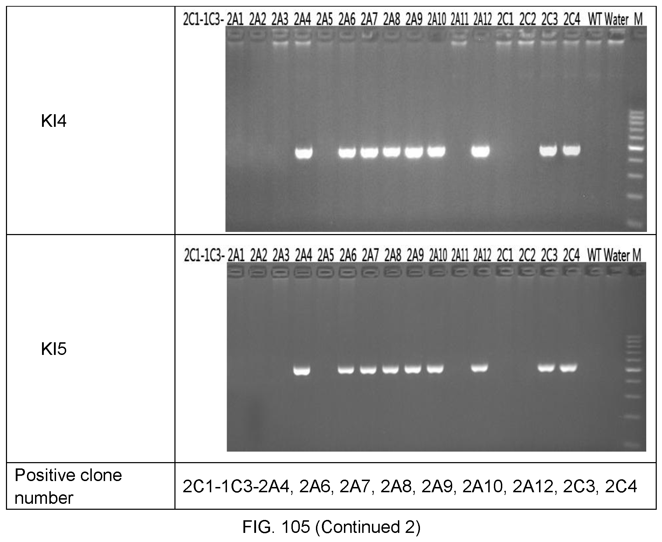

There were nine clones with positive bands simultaneously amplified by the remaining eight pairs of primers (FIG. 105): 2C1-1C3-2A4, 2C1-1C3-2A6, 2C1-1C3-2A7, 2C1-1C3-2A8, 2C1-1C3-2A9, 2C1-1C3-2A10, 2C1-1C3-2A12, 2C1-1C3-2C3, and 2C1-1C3-2C4, and the nine clones were used as positive clones in the third step.

**Table 51**

| Aspect | Identification region | Product size | Primer name | Primer sequence | SEQ ID NO. |
|---|---|---|---|---|---|
| ES2-FV+BAC | lox2272 | 520 | L2-F6 | gaaagataatcatattgtgacg | 245 |
| | | | L2-R6 | TGCTCTCTCTTTCTAA ATAGCG | 246 |
| | IoxP | 310 | L2-F7 | ttgacaagcacgcctcacggga | 247 |
| | | | L2-R7 | TGCCTGGAAGGAGGC AGCACTCC | 248 |
| | Puro-L | 450 | L2-F8 | tatcggtctgtatatcgaggt | 249 |
| | | | L2-R8 | CGTGCTACTTCCATTT GTCAC | 250 |
| | Puro | 520 | L2-F9 | ggaaggtgccactcccactgtc | 251 |
| | | | L2-R9 | TATAAAACACGTATCTA GGCTCT | 300 |
| | VT1 | 600 | L2-F10 | cgtggccaatatggacaactt | 252 |
| | | | L2-R10 | CGCGTGAGTCAAAAT GACGCAT | 253 |
| | VT2 | 690 | L2-F11 | cctcacgggagctccaagcg | 254 |
| | | | L2-R11 | CTGATTATTAGTCTGG GACCA | 255 |
| | PB1 | 2300 | L2-F12 | gagagaaattatacaggaatga | 256 |
| | | | L2-R12 | AATAGAATTGAAGATA GATAGG | 257 |
| | PB2 | 600 | L2-F13 | gaaactccattaaccctagaa | 258 |
| | | | L2-R13 | TAGTAAAGATTAACCC TAGAA | 259 |
| | KI4 | 480 | L2-F14 | acgctgcactaaggccccaccc | 260 |
| | | | L2-R14 | GGCCCAAATAATGAAT CATGG | 261 |
| | KI5 | 500 | L2-F15 | acagcctgggctacaaagcaa g | 262 |
| | | | L2-R15 | GTCTCACTGTCACCCA GGCTG | 263 |

4. Electroporation of pBase (a PiggyBac transposase) on the cell clones obtained in the third step.

pBase (the PiggyBac transposase) was electroporated into the positive cell clone 2C1-1C3-2C3 in the third step: under the action of the PiggyBac transposase, two inverted terminal repeats will be cut from a genome, a Neo sequence and lox2272 will be lost, and the positive cell clone will have only the inserted human gene sequence and one loxP element; and ES clones were selected for culture, amplified and identified by PCR typing.

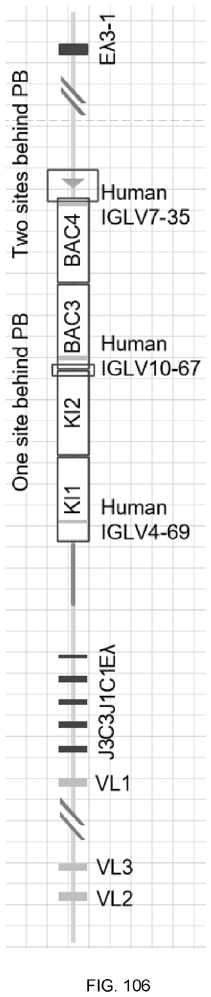

Deletions of sequences were identified by three pairs of primers (the sequences are shown in Table 52 below, and primer identification positions are simultaneously labeled in a figure (FIG. 106)).

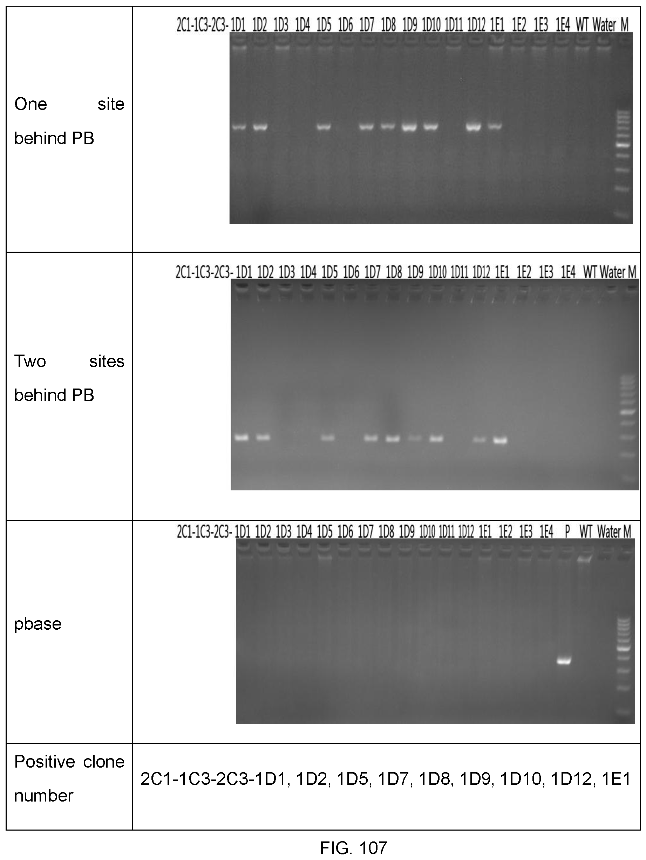

There were nine clones with positive bands simultaneously amplified by the three pairs of primers (FIG. 107): 2C1-1C3-2C3-1D1, 2C1-1C3-2C3-1D2, 2C1-1C3-2C3-1D5, 2C1-1C3-2C3-1D7, 2C1-1C3-2C3-1D8, 2C1-1C3-2C3-1D9, 2C1-1C3-2C3-1D10, 2C1-1C3-2C3-1D12, and 2C1-1C3-2C3-1E1, and the nine clones were used as positive clones in the third step.

**Table 52**

| Aspect | Identification region | Product size | Primer name | Primer sequence | SEQ ID NO. |
|---|---|---|---|---|---|
| ES2-FV+BAC-PB | One site behind PB | 690 | L2-F16 | tcattcctatgacttcattga | 264 |
| | | | L2-R16 | AAACACACCTCTCTTC ATTT | 265 |
| | Two sites behind PB | 280 | L2-F17 | tggaactatagctataaaatgt | 266 |
| | | | L2-R17 | GAGAGTGTCCATTGC CTGGAAG | 267 |
| | pbase | 352 | pbase-F1 | CTGGACGAGCAGAAC GTGATCG | 36 |
| | | | pbase-R1 | CGAAGAAGGCGTAGA TCTCGTCCTC | 37 |

### 5. F0 mice obtained from ES2

The positive cell clones obtained in the fourth step were injected into a blastocyst, and the blastocyst was then transplanted into surrogate female mice, and mice were born by gestation of about 20 days; and paws of young mice of 5-7 d were cut, DNA was extracted, and genotypes of the mice were confirmed by PCR typing identification.

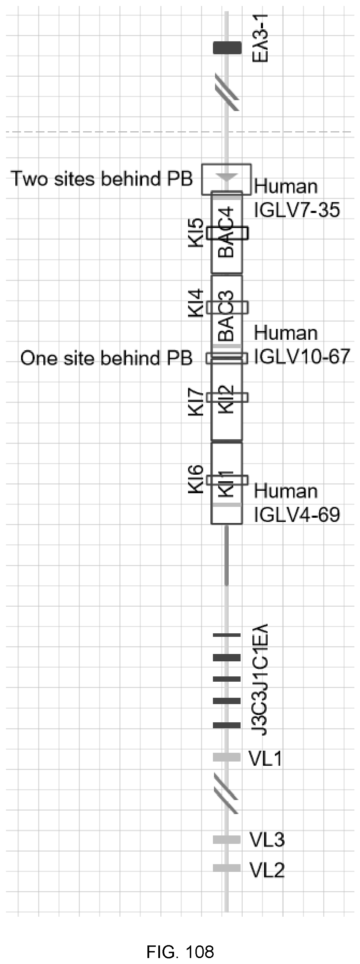

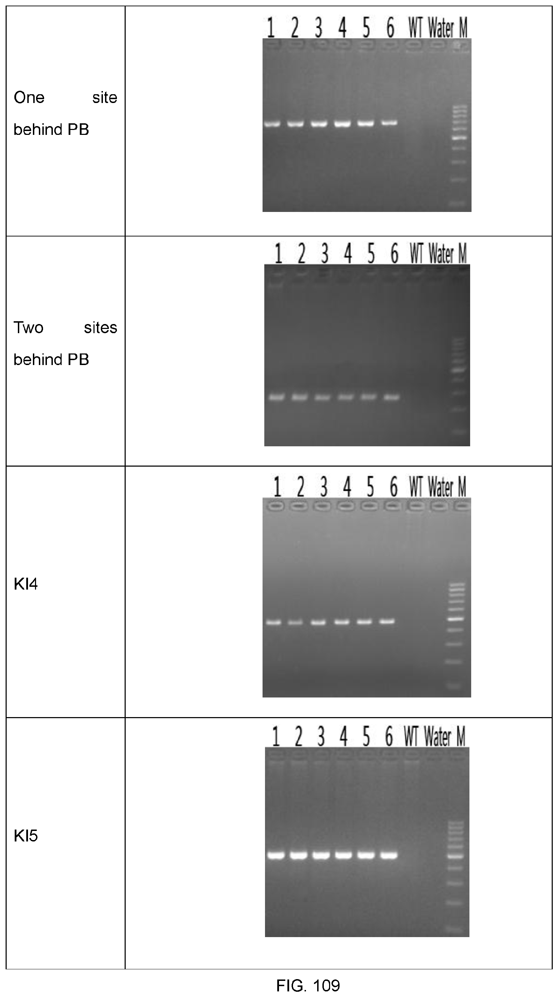

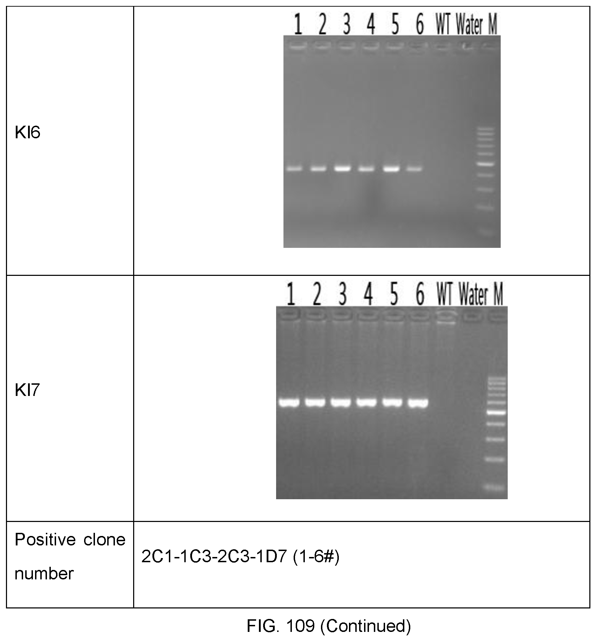

A total of 6 mice were born after injection of the clone 2C1-1C3-2C3-1D7 and all the 6 mice were co-identified as positive mice by 6 pairs of primers (Table 53). The schematic diagram and identification diagram of the F0 mouse genome are shown in FIGS. 108 and 109, respectively.

**Table 53**

| Aspect | Identification region | Product size | Primer name | Primer sequence | SEQ ID NO. |
|---|---|---|---|---|---|
| ES2-F0 | One site behind PB | 690 | L2-F16 | tcattcctatgacttcattga | 264 |
| | | | L2-R16 | AAACACACCTCTCTTCA TTT | 265 |
| | Two sites behind PB | 280 | L2-F17 | tggaactatagctataaaatgt | 266 |
| | | | L2-R17 | GAGAGTGTCCATTGCCT GGAAG | 267 |
| | KI4 | 480 | L2-F14 | acgctgcactaaggccccaccc | 260 |
| | | | L2-R14 | GGCCCAAATAATGAATC ATGG | 261 |
| | KI5 | 500 | L2-F15 | acagcctgggctacaaagcaag | 262 |
| | | | L2-R15 | GTCTCACTGTCACCCAG GCTG | 263 |
| | KI6 | 450 | L2-F16 | atgctctccatccatggctctc | 268 |
| | | | L2-R16 | ACCATTCCCATCTCTGA TTGA | 269 |
| | KI7 | 600 | L2-F17 | agggtcaccatctcctgcactg | 270 |
| | | | L2-R17 | TTGGTGAAGACTCCAGT CTGTG | 271 |

### 6. F1 mice obtained from ES2

The F0 mice that were identified as the positive mice were mated with CMV-Cre mice to obtain F1 generation mice.

Genotypes of the mice were confirmed by PCR typing identification of F1 generation mouse tail genomic DNA.

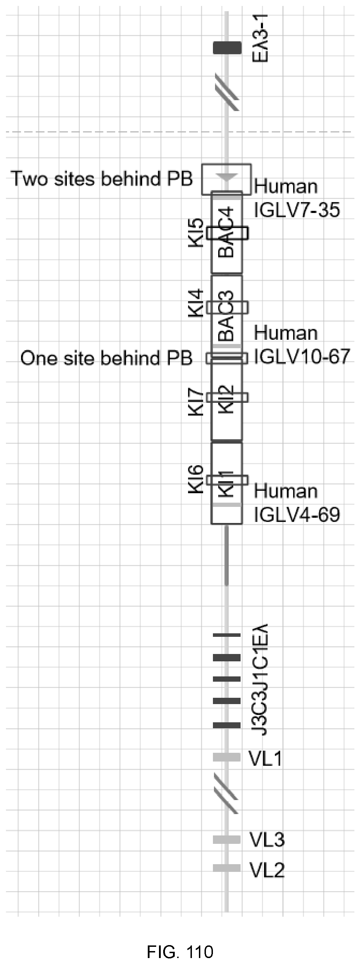

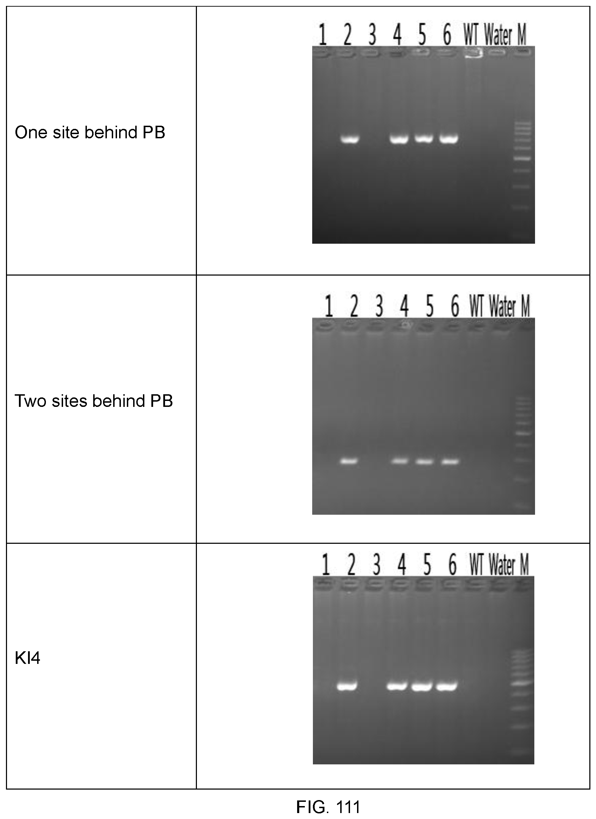

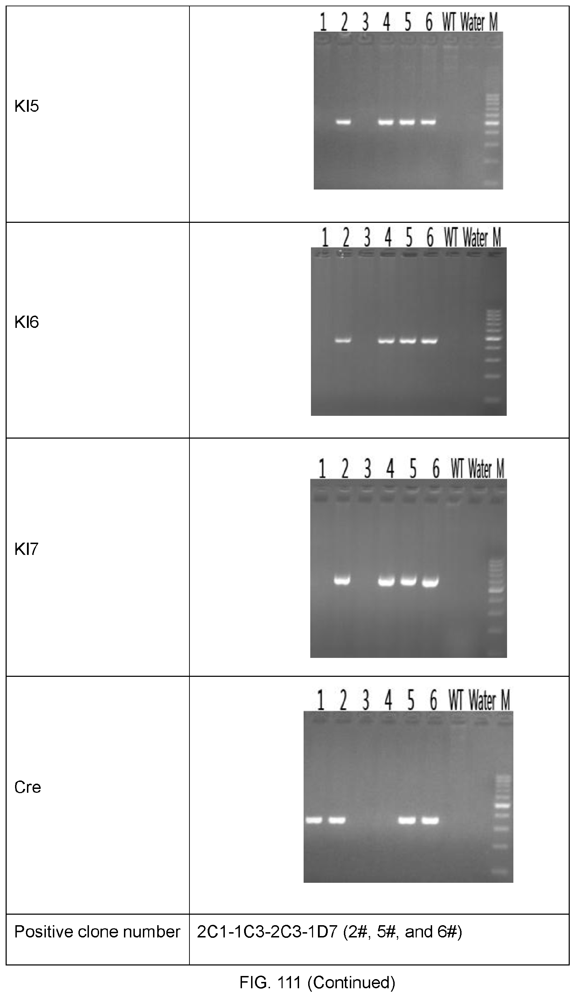

A total of 6 F1 mice were born, ES2 was co-identified with 6 pairs of primers (Table 54), CMV-Cre was identified with 1 pair of primers, and a total of 3 mice (2#, 5#, and 6#) were identified as double gene positive mice. The schematic diagram and identification diagram of the F1 mouse genome are shown in FIGS. 110 and 111, respectively.

**Table 54**

| Aspect | Identificatio n region | Produc t size | Primer name | Primer sequence | SEQ ID NO. |
|---|---|---|---|---|---|
| ES2-F1 | One site behind PB | 690 | L2-F16 | tcattcctatgacttcattga | 264 |
| | | | L2-R16 | AAACACACCTCTCTTCAT TT | 265 |
| | Two sites behind PB | 280 | L2-F17 | tggaactatagctataaaatgt | 266 |
| | | | L2-R17 | GAGAGTGTCCATTGCCT GGAAG | 267 |
| | KI4 | 480 | L2-F14 | acgctgcactaaggccccaccc | 260 |
| | | | L2-R14 | GGCCCAAATAATGAATCA TGG | 261 |
| | KI5 | 500 | L2-F15 | acagcctgggctacaaagcaag | 262 |
| | | | L2-R15 | GTCTCACTGTCACCCAG GCTG | 263 |
| | KI6 | 450 | L2-F16 | atgctctccatccatggctctc | 268 |
| | | | L2-R16 | ACCATTCCCATCTCTGAT TGA | 269 |
| | KI7 | 600 | L2-F17 | agggtcaccatctcctgcactg | 270 |
| | | | L2-R17 | TTGGTGAAGACTCCAGT CTGTG | 271 |
| | Cre | 349 | CMV-MF2 | GTAGGCGTGTACGGTGG GAGGT | 40 |
| | | | CMV-MR | TCCAGGTATGCTCAGAAA ACGCC | 41 |

### III. ES1 and ES2 mouse hybridization

1. Positive F1 mice of ES1 were hybridized with double gene F1 positive mice obtained from ES2 to obtain Cre-positive, and ES1-positive and ES2-positive F2 generation mice.

The gene was identified as ectopic by three pairs of primers (Table 55), and Cre-positive mice was identified by 1 pair of Cre primers.

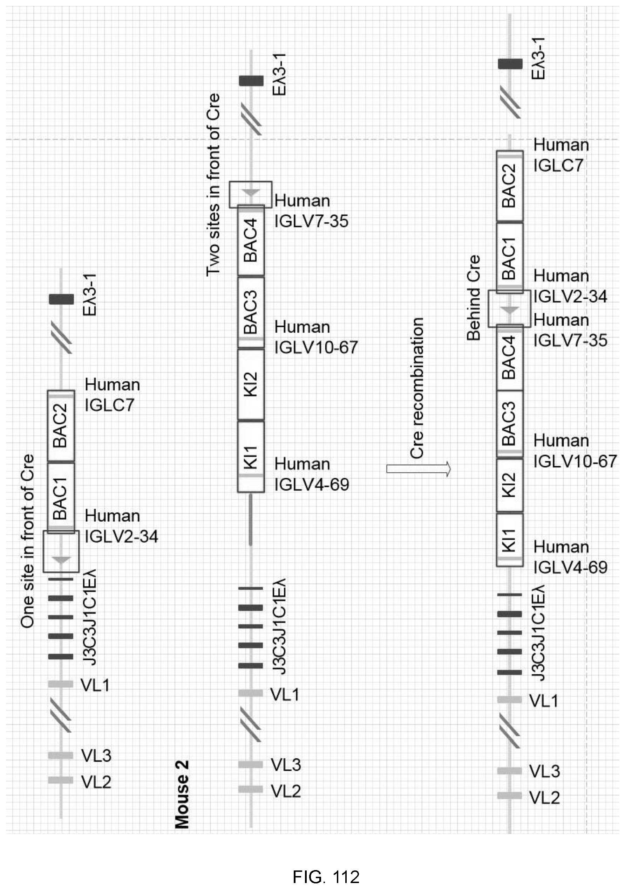

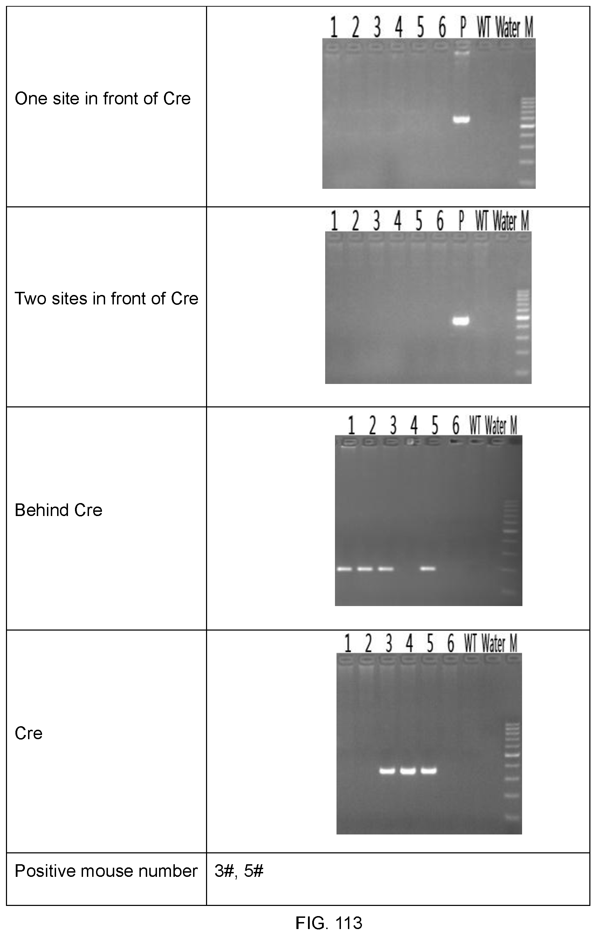

Two mice were identified as ectopic and carrying Cre: 3# and 5#. The schematic diagram and identification diagram of the F2 generation mouse genome are shown in FIGS. 112 and 113, respectively.

**Table 55**

| Aspect | Identification region | Produc t size | Primer name | Primer sequence | SEQ ID NO. |
|---|---|---|---|---|---|
| ES1 F1 generatio n X ES2+Cre F1 generatio n | One site in front of Cre | 600 | L3-F1 | tgagtataaggttgc agattt | 272 |
| | | | L3-R1 | GCTGAGTGGA TCTGGAAATC | 273 |
| | Two sites in front of Cre | 450 | L3-F2 | acatacacatttgatg ttct | 274 |
| | | | L3-R2 | AGCCACACCC TTAGCTTCTTG | 275 |
| | Behind Cre | 200 | L3-F3 | gatgttctgatgtttgc tat | 276 |
| | | | L3-R3 | CGTTCCTTCC CATCCCAGCT C | 277 |
| | Cre | 349 | CMV-MF2 | GTAGGCGTGT ACGGTGGGAG GT | 40 |
| | | | CMV-MR | TCCAGGTATG CTCAGAAAAC GCC | 41 |

2. The obtained F2 positive mice were mated with wild type mice to obtain a blastocyst of 3.5 d, ES cell lines were established, different ES cell lines were tested by PCR, and cells that do not carry Cre but have undergone ectopia encompassing the human genomic sequences of ES1 and ES2 and having a loxP sequence between IGLV2-34 and IGLV7-35 were selected.

The gene was identified as ectopic by three pairs of primers (Table 56), and Cre-positive mice was identified by 1 pair of Cre primers.

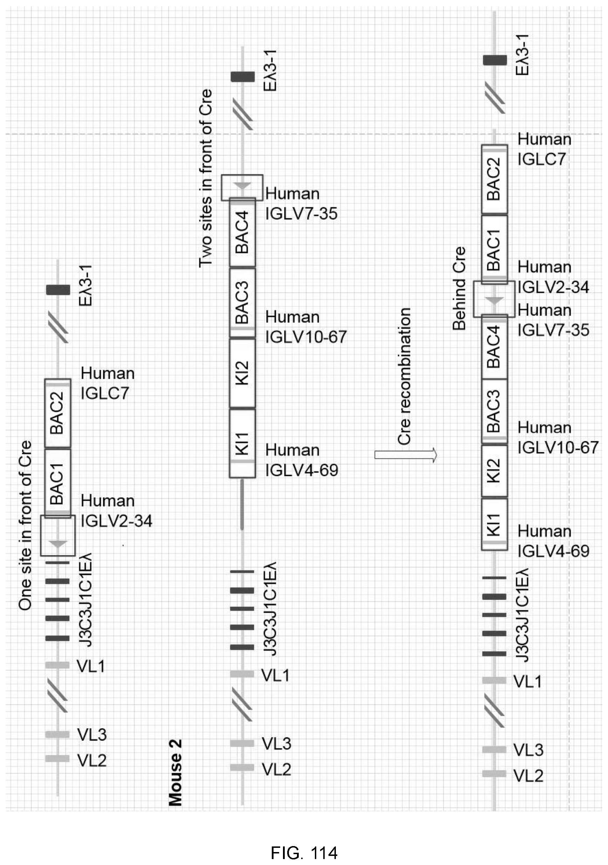

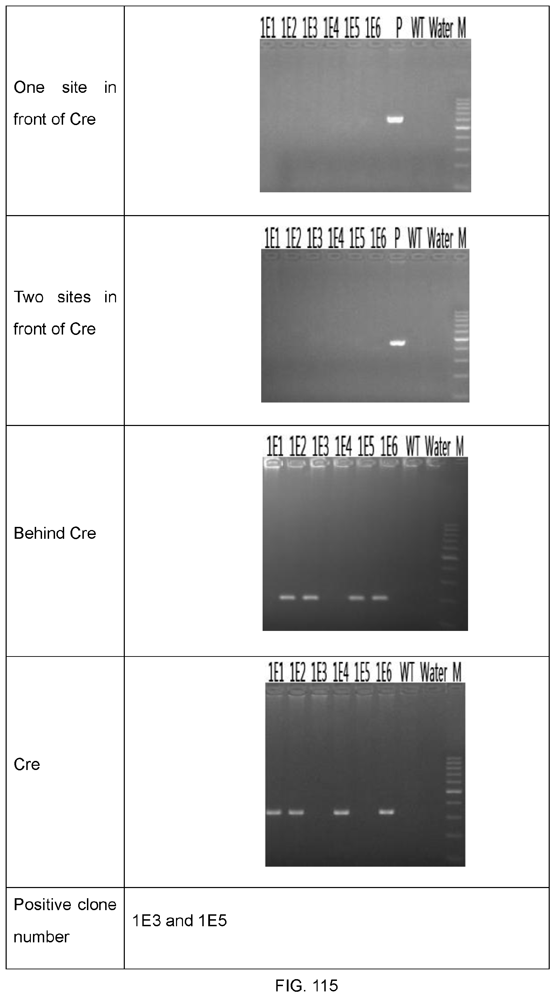

Two clones were identified as ectopic and not carrying Cre: 1E3 and 1E5, and the two clones were used as an ES cell line III. The schematic diagram and identification diagram of genomic recombination are shown in FIGS. 114 and 115, respectively.

**Table 56**

| Step | Aspect | Identificati on site | Size | Primer name | Primer sequence | SEQ ID NO. |
|---|---|---|---|---|---|---|
| Establishin g a cell line from F2 | ES cells | One site in front of Cre | 600 | L3-F1 | tgagtataaggttgcag attt | 272 |
| | | | | L3-R1 | GCTGAGTGGAT CTGGAAATC | 273 |
| | | Two sites in front of Cre | 450 | L3-F2 | acatacacatttgatgtt ct | 274 |
| | | | | L3-R2 | AGCCACACCCTT AGCTTCTTG | 275 |
| | | Behind Cre | 200 | L3-F3 | gatgttctgatgtttgctat | 276 |
| | | | | L3-R3 | CGTTCCTTCCCA TCCCAGCTC | 277 |
| | | Cre | 349 | CMV-MF2 | GTAGGCGTGTAC GGTGGGAGGT | 40 |
| | | | | CMV-MR | TCCAGGTATGCT CAGAAAACGCC | 41 |

### 3. Performing homologous recombination KO (knockout of all mouse gene sequences in V, J and C regions of a Lambda chain) on the ES cell line III

A constructed vector was electroporated into the positive cell clone 1E3 in the first step. The vector was Neo-resistant. The cells were subjected to G418 drug screening. Related ES clones were selected for culture, amplified and identified by PCR typing.

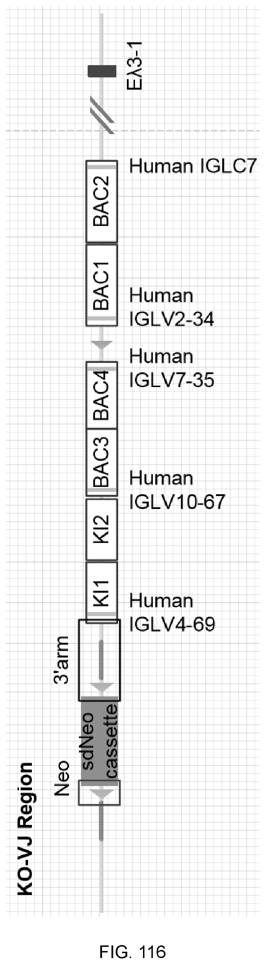

Genes between the V, J and C regions were identified to have been recombined by Neo resistance by two pairs of primers (the sequences are shown in Table 57 below, and primer identification positions are simultaneously labeled in a figure (FIG. 116)), representing that all genes in a mouse IgL chain had undergone recombinant deletion.

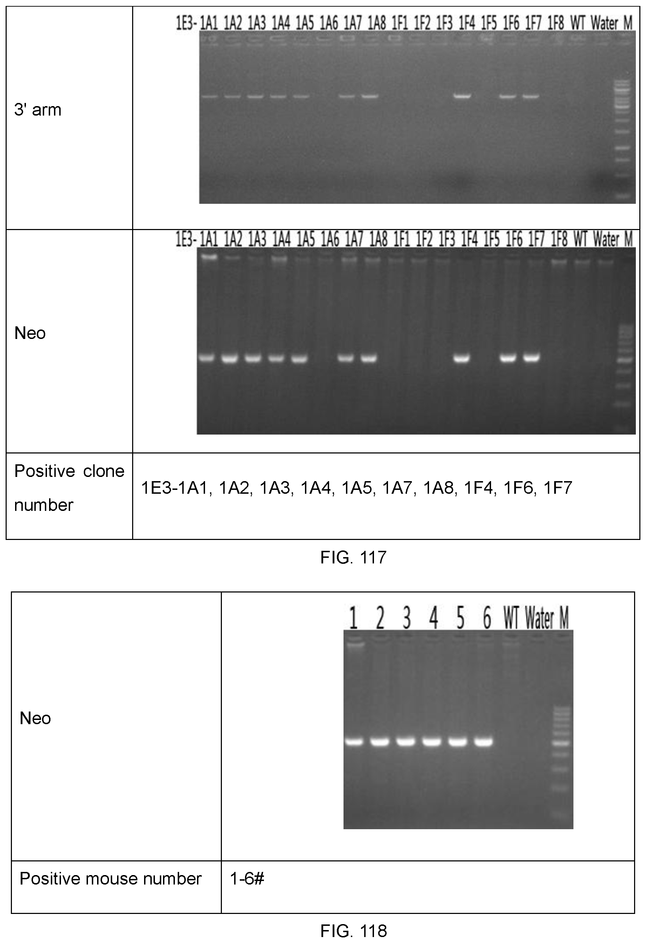

There were ten clones with positive bands simultaneously amplified by the two pairs of primers (FIG. 117): 1E3-1A1, 1E3-1A2, 1E3-1A3, 1E3-1A4, 1E3-1A5, 1E3-1A7, 1E3-1A8, 1E3-1F4, 1E3-1F6, and 1E3-1F7, and the ten clones were used as positive clones in the second step.

**Table 57**

| Step | Aspect | Identificatio n site | Size | Primer name | Primer sequence | SEQ ID NO. |
|---|---|---|---|---|---|---|
| V region KO | ES cells | 3'arm | 4300 | L3-F4 | ttctctggccagcctaggac t | 278 |
| | | | | L3-R4 | GTTGGTGTTACAT GCATGTATGT | 279 |
| | | Neo | 510 | L3-F5 | ggggaagtagaacaaga acac | 280 |
| | | | | L3-R5 | TCTGAGTAGGTGT CATTCTATT | 281 |

### Injection of the positive cell clones obtained in the third step to obtain positive F0 mice

The positive cell clones obtained in the third step were injected into a blastocyst, and the blastocyst was then transplanted into surrogate female mice, and mice were born by gestation of about 20 days; and paws of young mice of 5-7 d were cut, DNA was extracted, and genotypes of the mice were confirmed by PCR typing identification.

A total of 6 mice were born after injection of the clone 1E3-1A1 and all the 6 mice were identified as positive mice by 1 pair of primers (Table 58). The dentification results are shown in FIG. 118.

**Table 58**

| Aspect | Identificati on region | Identificatio n site | Size | Primer name | Primer sequence | SEQ ID NO. |
|---|---|---|---|---|---|---|
| F0 mice | V region KO | Neo | 510 | L3-F5 | ggggaagtagaacaa gaacac | 280 |
| | | | | L3-R5 | TCTGAGTAGGT GTCATTCTATT | 281 |

### Obtaining positive F1 mice

The F0 mice that were identified as the positive mice were mated with wild type mice to obtain F1 generation mice in which Neo underwent self-deletion.

Genotypes of the mice were confirmed by PCR typing identification of F1 generation mouse tail genomic DNA.

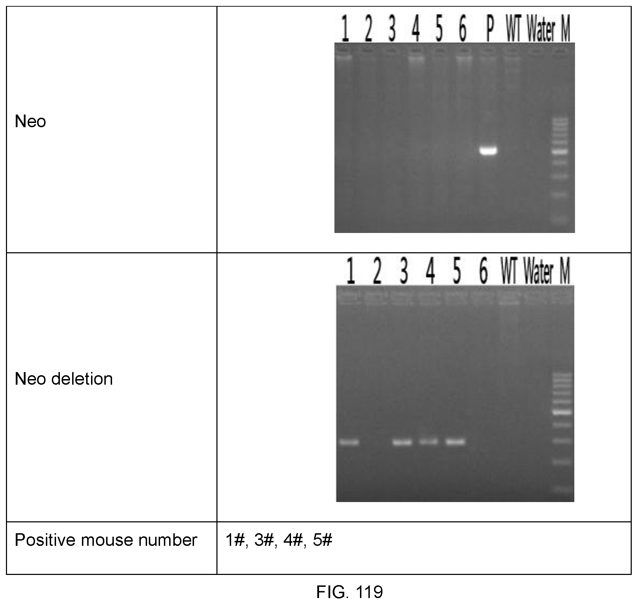

A total of 6 F1 mice were born, and co-identified by 2 pairs of primers (Table 59), and a total of 4 mice (1#, 3#, 4#, and 5#) were positive mice. The identification results are shown in FIG. 119.

**Table 59**

| Aspect | Identificati on region | Identificatio n site | Size | Primer name | Primer sequence | SEQ ID NO. |
|---|---|---|---|---|---|---|
| F1 mice | V region KO | Neo | 510 | L3-F5 | ggggaagtagaacaa gaacac | 280 |
| | | | | L3-R5 | TCTGAGTAGGTG TCATTCTATT | 281 |
| | | Neo deletion | 300 | L3-F6 | tatgccttgatgacaagc tt | 282 |
| | | | | L3-R6 | GTTGTTGCTGTT TCCAGTACAGG | 283 |

### Obtaining homozygous mice

The female and male F1 mice that were identified as the positive mice were mated with each other to obtain homozygous mice.

Genotypes of the mice were confirmed by PCR typing identification of F2 generation mouse tail genomic DNA.

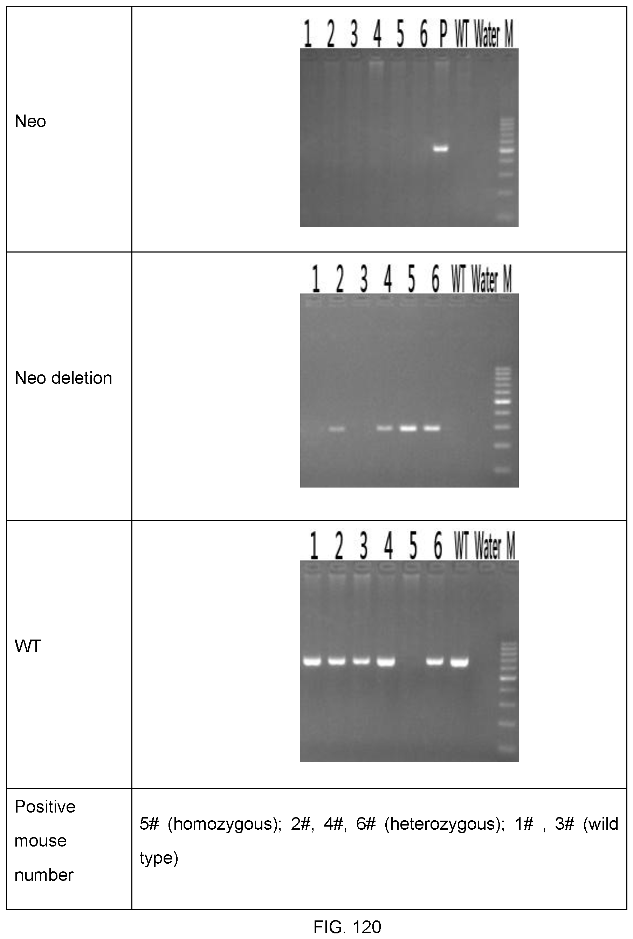

A total of 6 F2 mice were born, and co-identified with 3 pairs of primers (Table 60) to determine that the genotype of a mouse 5# was homozygous; the genotype of mice 2#, 4#, and 6# was heterozygous; and the genotype of mice 1# and 3# was wild-type. The identification results are shown in FIG. 120.

**Table 60**

| Aspect | Identification region | Identification site | Size | Primer name | Primer sequence | SEQ ID NO. |
|---|---|---|---|---|---|---|
| F2 mice | V region KO | Neo | 510 | L3-F5 | ggggaagtagaac aagaacac | 280 |
| | | | | L3-R5 | TCTGAGTAGG TGTCATTCTAT T | 281 |
| | | Neo deletion | 300 | L3-F6 | tatgccttgatgaca agctt | 282 |
| | | | | L3-R6 | GTTGTTGCTG TTTCCAGTAC AGG | 283 |
| | | WT | 690 | L3-F7 | gatgatggtgagtg gtcttgg | 284 |
| | | | | L3-R7 | CTACAAAACA TTCACTAGGA A | 285 |

### Breeding performance testing

The obtained homozygous mice continued to be bred in a manner of mating one male mouse with one female mouse, and the breeding performance of the mice was counted. A total of 24 breeding pairs were mated, and the number of mice born 1 month after each breeding pair was mated was counted. The results are shown in Table 61.

**Table 61**

| Breeding pair | Number of births | Breeding pair | Number of births |
|---|---|---|---|
| Breeding pair 1 | 7 | Breeding pair 13 | 4 |
| Breeding pair 2 | 6 | Breeding pair 14 | 5 |
| Breeding pair 3 | 7 | Breeding pair 15 | 6 |
| Breeding pair 4 | 5 | Breeding pair 16 | 8 |
| Breeding pair 5 | 4 | Breeding pair 17 | 6 |
| Breeding pair 6 | 5 | Breeding pair 18 | 1 |
| Breeding pair 7 | 0 | Breeding pair 19 | 6 |
| Breeding pair 8 | 3 | Breeding pair 20 | 5 |
| Breeding pair 9 | 5 | Breeding pair 21 | 0 |
| Breeding pair 10 | 7 | Breeding pair 22 | 6 |
| Breeding pair 11 | 8 | Breeding pair 23 | 4 |
| Breeding pair 12 | 6 | Breeding pair 24 | 5 |

There were a total of 24 breeding pairs, 22 breeding pairs gave birth to mice after 1 month of mating, and the effective breeding pairs reached 91.7%, while the mice had an average birth of 5.4, which was consistent with the number of births of wild type mice. It was confirmed that the breeding performance of mice of this genotype was normal.

### V. Lambda chain immune repertoire sequencing detection

The obtained homozygous female mice were euthanized, spleens of the mice were dissected, after RNA was extracted and totalRNA extracted from a sample was detected to be qualified, library construction was performed, followed by immune repertoire sequencing (Immuno-Seq uses B/T lymphocytes as a research objective to specifically amplify a variable region (a V region) that determines the diversity of a B cell receptor (BCR) or a T cell receptor (TCR) by 5' RACE or a multiplex PCR technology, so as to comprehensively evaluate the diversity of an immune system combined with a high-throughput sequencing technology); and sequences obtained by sequencing were subjected to quality control by using quality control software, and the sequencing background was filtered, then the sequences were aligned with V and J genes of an IMGT immune cell receptor library, the corresponding gene fragments were searched to find accurate V and J gene fragments and sequence loci, and information such as V and J gene frequencies, clone frequency distribution, and the number of polypeptide sequences were statistically analyzed.

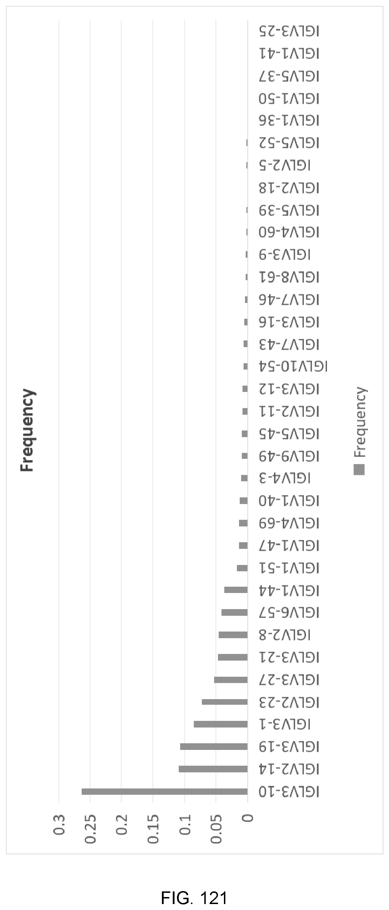

Based on the obtained gene usage frequency data, a distribution graph of a gene usage frequency at a reads level was plotted. Based on the sequencing results, it was confirmed that IGLV3-10 was used at the highest frequency at the reads level in spleen RNA of unimmunized mice, followed by IGLV2-14, IGLV3-19, and IGLV3-1, and the usage frequencies sequentially decreased as shown in FIG. 121.

2. The statistical analysis of CDR3 amino acid sequence characteristics is an important step in immunopanel analysis, by refining to each clone, different CDR3 sequences evolved from a sequence of each germline antibody are subjected to quantitative sequence abundance analysis, and finally, it is convenient to select sequences for further antibody expression.

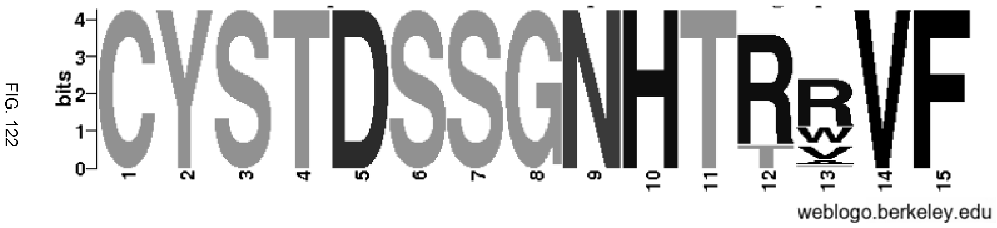

Weblogo profiling of a CDR3 amino acid sequence of each sample at the reads level is shown in FIG. 122. The results showed an amino acid sequence with the highest CDR3 abundance in unimmunized homozygous mice.

### Example 4. Preparation and identification of heavy-chain and Kappa light-chain genetically modified mice

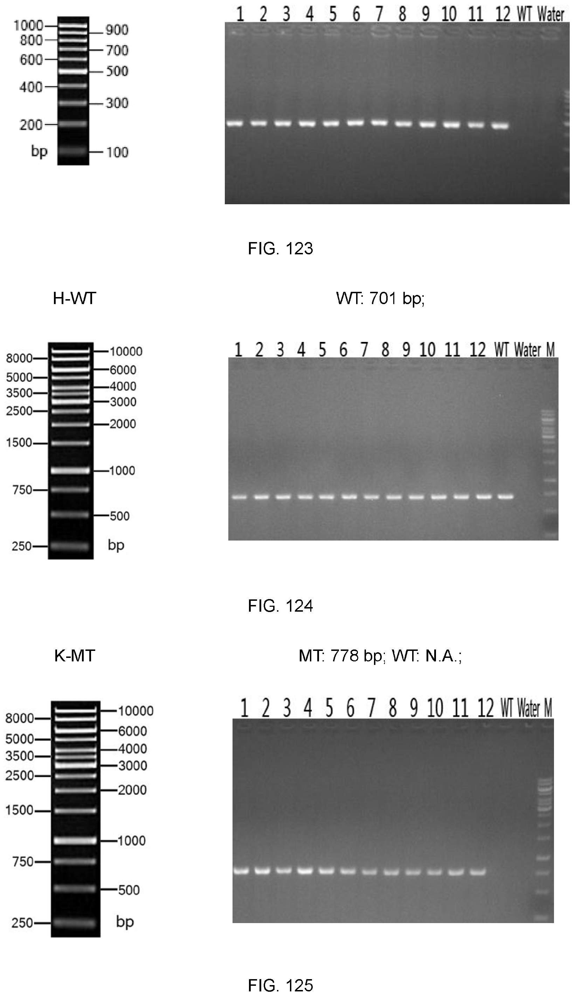

1. Heavy-chain homozygous mice and Kappa light-chain homozygous mice were mated in a ratio of 1:1, wherein the sex was not limited, and the Kappa light-chain homozygous mice were either female mice or male mice; and mice were gradually born after 1 month of mating, and all the mice born were double gene heterozygous mice. The identification results are shown in FIGS. 123 to 126.

Twelve mice were born, the heavy chain was identified by two pairs of primers H-MT and H-WT (Table 62), the mice were heterozygous mice, the Kappa light chain was identified by two pairs of primers K-MT and K-WT, and the mice were also heterozygous mice, indicating that the twelve mice were heavy-chain and Kappa light-chain double gene heterozygous mice.

**Table 62**

| Primer | Sequence (5'-3') | SEQ ID NO. |
|---|---|---|
| H-MT | VT496-F9: TCTGCTGACTCTCCTGTTACC | 140 |
| | VT496-R9: ATCTCTGTCCTTCTCAATGTGG | 141 |
| H-WT | H-WF: AGTGAGTGAATTGAGTGAATGGG | 191 |
| | H-WR: TTGGCTCCTCCATGTGTGAGC | 192 |
| K-MT | IGKN-10F: GCTGGACAACGTGAGAAAGGCTG | 286 |
| | IGKN-10R: TCAAACTGATTAGGAACTGACTACC | 287 |
| K-WT | IGK3-KOF3: AGGCTCGGGTGACTACTAGG | 288 |
| | IGK3-KOR3: CCTAGCCCCAACCTGACTTC | 289 |

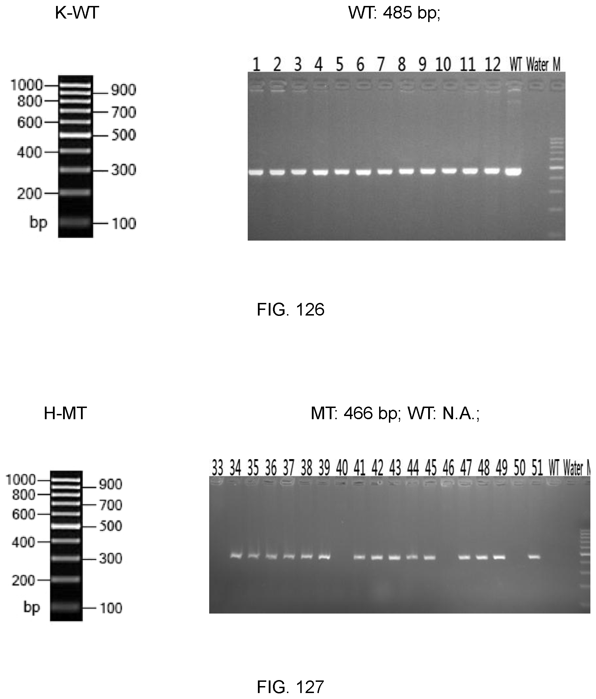

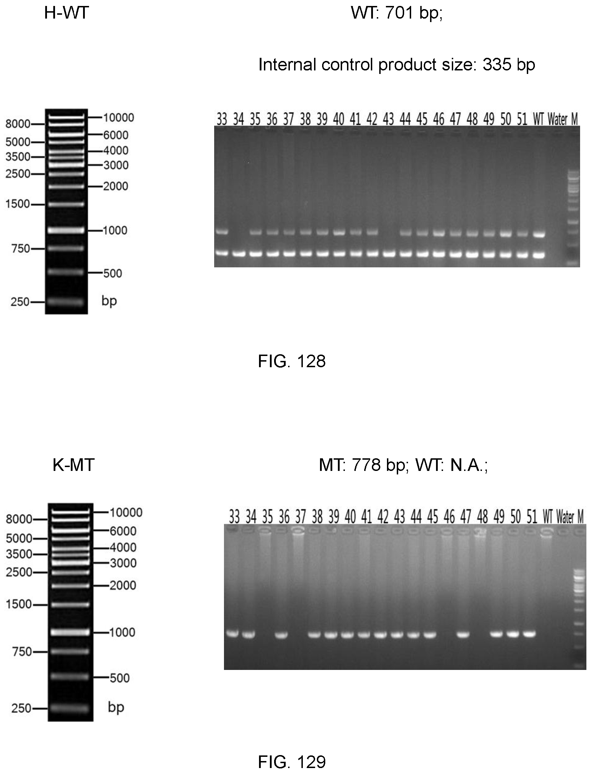

2. The heavy-chain and Kappa light-chain double gene heterozygote mice were mated in a manner of mating 1 male mouse with 2 female mice; mice were gradually born after 1 month of mating, and when offspring mice were 5-7 days old, paws of the offspring mice were cut for genotype identification, and the results are shown in FIGS. 127 to 130.

Nineteen mice were born, the heavy chain was identified by two pairs of primers H-MT and H-WT, and the Kappa light chain was identified by two pairs of primers K-MT and K-WT, while one pair of internal reference primers were respectively added during the identifications: F3-R3 and F6-R6. The primer pairs are shown in Table 63.

The results indicated that a mouse 34# was a double gene homozygous mouse.

**Table 63**

| Primer | Sequence (5'-3') | SEQ ID NO. |
|---|---|---|
| H-MT | VT496-F9: TCTGCTGACTCTCCTGTTACC | 140 |
| | VT496-R9: ATCTCTGTCCTTCTCAATGTGG | 141 |
| H-WT | H-WF: AGTGAGTGAATTGAGTGAATGGG | 191 |
| | H-WR: TTGGCTCCTCCATGTGTGAGC | 192 |
| | Internal reference primer F3: 5'-CATGCCAATGGTTCACTCTAAGGT-3' | 290 |
| | Internal reference primer R3: 5'-TCTCTATGTCCCAAAGTGCAGACAC-3' | 291 |
| K-MT | IGKN-10F: GCTGGACAACGTGAGAAAGGCTG | 286 |
| | IGKN-10R: TCAAACTGATTAGGAACTGACTACC | 287 |
| K-WT | IGK3-KOF3: AGGCTCGGGTGACTACTAGG | 288 |
| | IGK3-KOR3: CCTAGCCCCAACCTGACTTC | 289 |
| | Internal reference primer F6: 5'-GCAGAAGAGGACAGATACATTCAT-3' | 292 |
| | Internal reference primer R6: 5'-CCTACTGAAGAATCTATCCCACAG-3' | 293 |

3. The proportion of the double gene homozygous mice in breeding pairs was counted, and the results are shown in Table 64 below. The results showed that the double gene homozygotes were born at a ratio of 1/16, and the results were consistent with the Mendelian ratio.

**Table 64**

| Breeding pair | Number of births | Number of double gene homozygous mice |
|---|---|---|
| Breeding pair 1 | 10 | 1 |
| Breeding pair 2 | 15 | 2 |
| Breeding pair 3 | 13 | 0 |
| Breeding pair 4 | 14 | 1 |
| Breeding pair 5 | 12 | 1 |
| Breeding pair 6 | 10 | 0 |
| Breeding pair 7 | 8 | 0 |
| Breeding pair 8 | 7 | 1 |
| Breeding pair 9 | 14 | 1 |
| Breeding pair 10 | 14 | 2 |
| Breeding pair 11 | 15 | 1 |
| Breeding pair 12 | 16 | 1 |
| Breeding pair 13 | 8 | 1 |
| Breeding pair 14 | 9 | 0 |
| Breeding pair 15 | 14 | 2 |
| Breeding pair 16 | 13 | 1 |
| Breeding pair 17 | 16 | 1 |
| Breeding pair 18 | 12 | 0 |
| Breeding pair 19 | 11 | 1 |
| Breeding pair 20 | 15 | 0 |

### Example 5. Breeding of heavy-chain and Lambda light-chain double gene homozygotes

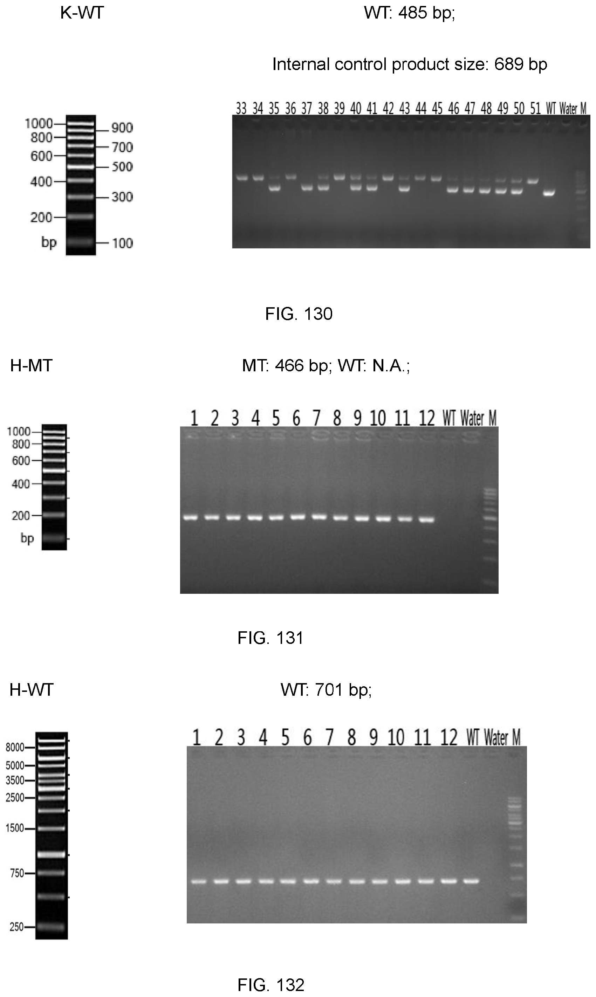

1. Heavy-chain homozygous mice and Lambda light-chain homozygous mice were mated in a ratio of 1:1, wherein the sex was not limited, and the Lambda light-chain homozygous mice were either female mice or male mice; and mice were gradually born after 1 month of mating, and all the mice born were double gene heterozygous mice. The identification results are shown in FIGS. 131 to 134.

A total of 12 mice were born, numbered from 1 to 12: where heavy chain genes of all the mice were confirmed to be heterozygous by identification with two pairs of primers H-MT and H-WT; and Lambda light chain genes of all the mice were also confirmed to be heterozygous by identification with two pairs of primers L-MT and L-WT, thereby confirming that the 12 mice were double gene heterozygote mice, where internal reference primers F5R5 were added during the Lambda identification process to confirm that there was no problem in the identification process. The primer pairs are shown in Table 65.

**Table 65.**

| Primer | Sequence (5'-3') | SEQ ID NO. |
|---|---|---|
| H-MT | VT496-F9: TCTGCTGACTCTCCTGTTACC | 140 |
| | VT496-R9: ATCTCTGTCCTTCTCAATGTGG | 141 |
| H-WT | H-WF: AGTGAGTGAATTGAGTGAATGGG | 191 |
| | H-WR: TTGGCTCCTCCATGTGTGAGC | 192 |
| L-MT | IGLN-PB4F: AGCTTCATTTCACTCCACTTCC | 294 |
| | IGLN-PB4R: ACACAACTCTGAGGTCACAAATGC | 295 |
| L-WT | VT462-KF2: TGTGGAGACCGTCATCTTCTCCTGC | 296 |
| | VT462-KR1: ACCCTTAGCTTCTTGTAGATGGTTG | 297 |
| | Inner reference primer F5: 5'-CTATCAGGGATACTCCTCTTTGCC-3' | 298 |
| | Internal reference primer R5: 5'-GATACAGGAATGACAAGCTCATGGT-3' | 299 |

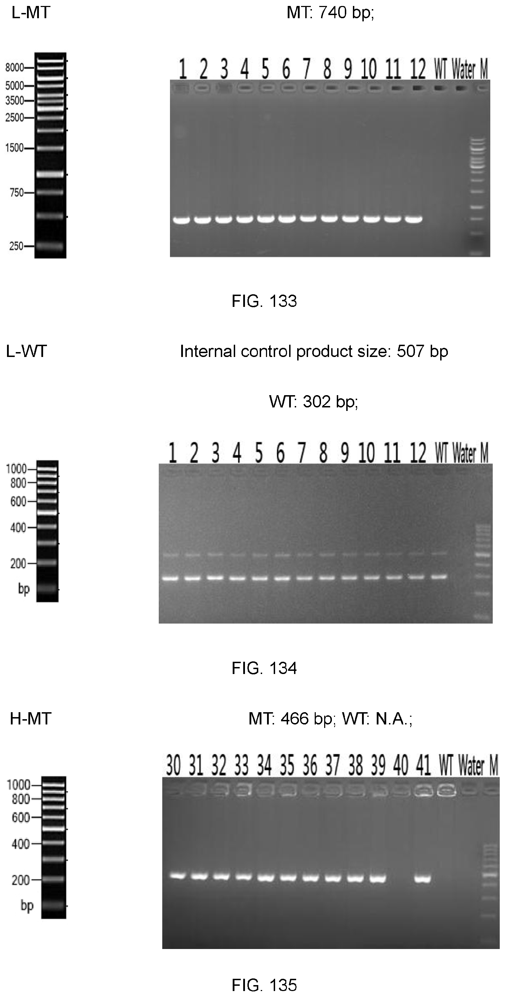

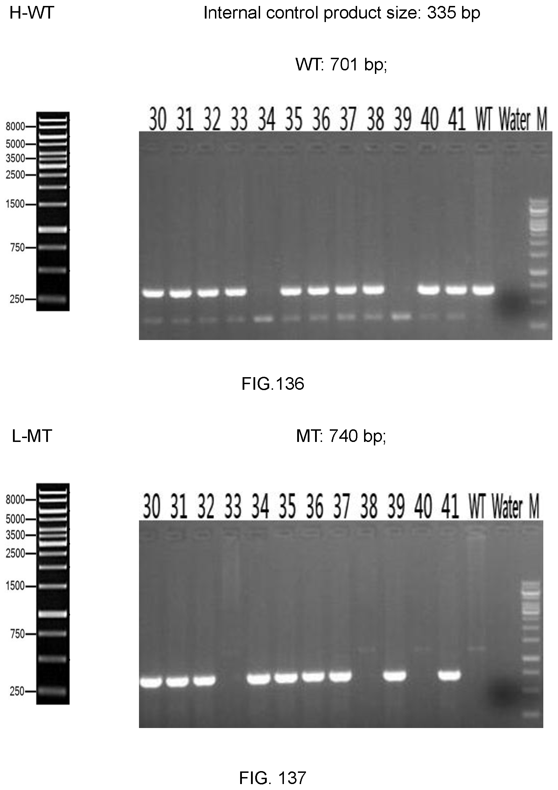

The heavy-chain and Lambda light-chain double gene heterozygote mice were mated in a manner of mating 1 male mouse with 2 female mice; mice were gradually born after 1 month of mating, and when offspring mice were 5-7 days old, paws of the offspring mice were cut for genotype identification, and the results are shown in FIGS. 135 to 138.

12 mice were born, numbered from 30 to 41: by identification with two pairs of primers H-MT and H-WT, it was confirmed that: heavy chain genes of mice 34 and 39 were homozygous; and by identification with two pairs of primers L-MT and L-WT, it was confirmed that: Lambda light chain genes of mice 31, 32, 36, 39 and 41 were homozygous, thereby confirming that a mouse 39 was a heavy-chain and Lambda light-chain double gene homozygous mouse. The primer pairs are shown in Table 66.

**Table 66**

| Primer | Sequence (5'-3') | SEQ ID NO. |
|---|---|---|
| H-MT | VT496-F9: TCTGCTGACTCTCCTGTTACC | 140 |
| | VT496-R9: ATCTCTGTCCTTCTCAATGTGG | 141 |
| H-WT | H-WF: AGTGAGTGAATTGAGTGAATGGG | 191 |
| | H-WR: TTGGCTCCTCCATGTGTGAGC | 192 |
| | Internal reference primer F5: 5'-CATGCCAATGGTTCACTCTAAGGT-3' | 290 |
| | Internal reference primer R5: 5'-TCTCTATGTCCCAAAGTGCAGACAC-3' | 291 |
| L-MT | IGLN-PB4F: AGCTTCATTTCACTCCACTTCC | 294 |
| | IGLN-PB4R: ACACAACTCTGAGGTCACAAATGC | 295 |
| L-WT | VT462-KF2: TGTGGAGACCGTCATCTTCTCCTGC | 296 |
| | VT462-KR1: ACCCTTAGCTTCTTGTAGATGGTTG | 297 |
| | Internal reference primer F5: 5'-CTATCAGGGATACTCCTCTTTGCC-3' | 298 |
| | Internal reference primer R5: 5'-GATACAGGAATGACAAGCTCATGGT-3' | 299 |

3. The proportion of the double gene homozygous mice in breeding pairs was counted, and the results are shown in Table 67. The results showed that the double gene homozygotes were born at a ratio of about 1/16, and the results were consistent with the Mendelian ratio.

**Table 67**

| Breeding pair | Number of births | Number of double gene homozygous mice |
|---|---|---|
| Breeding pair 1 | 10 | 0 |
| Breeding pair 2 | 12 | 1 |
| Breeding pair 3 | 8 | 1 |
| Breeding pair 4 | 12 | 2 |
| Breeding pair 5 | 6 | 1 |
| Breeding pair 6 | 9 | 0 |
| Breeding pair 7 | 14 | 0 |
| Breeding pair 8 | 15 | 1 |
| Breeding pair 9 | 11 | 2 |
| Breeding pair 10 | 8 | 0 |
| Breeding pair 11 | 16 | 1 |
| Breeding pair 12 | 16 | 2 |
| Breeding pair 13 | 8 | 0 |
| Breeding pair 14 | 7 | 0 |
| Breeding pair 15 | 12 | 1 |
| Breeding pair 16 | 13 | 0 |
| Breeding pair 17 | 15 | 0 |
| Breeding pair 18 | 12 | 1 |
| Breeding pair 19 | 11 | 0 |
| Breeding pair 20 | 7 | 1 |

### Example 6. Breeding of heavy-chain, Kappa light-chain and Lambda light-chain three gene homozygotes

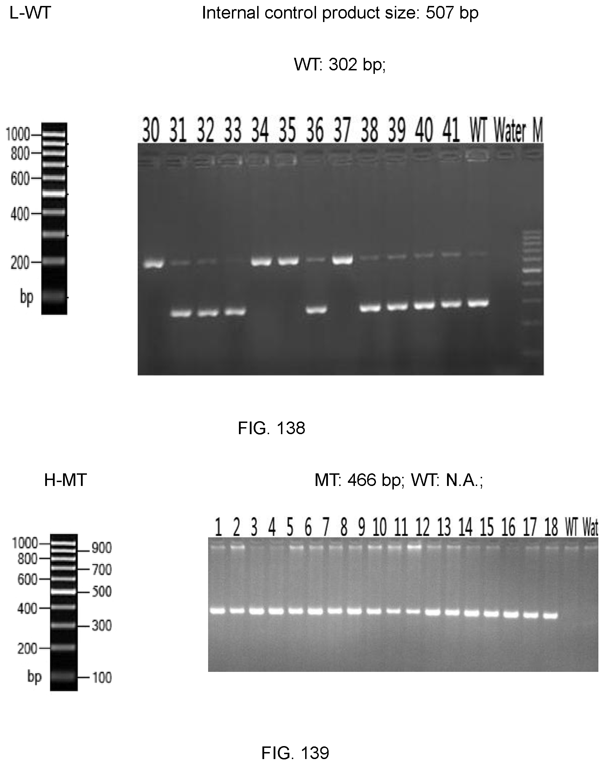

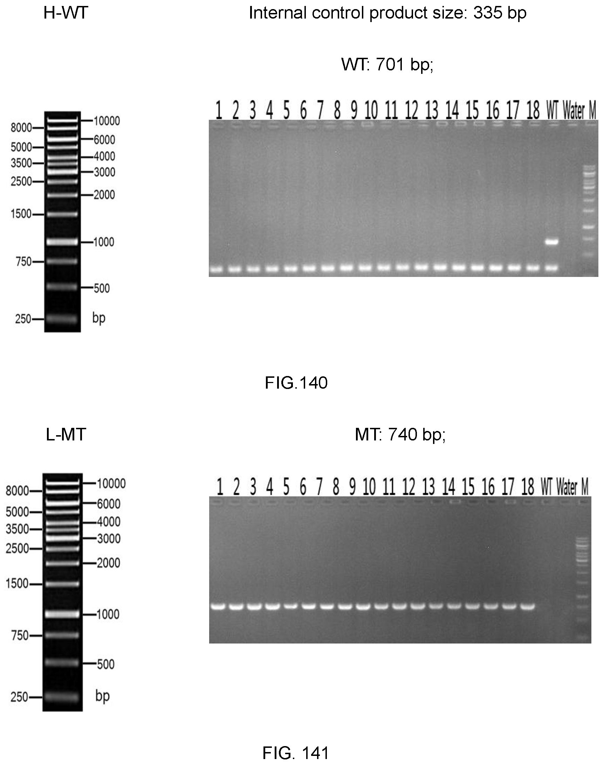

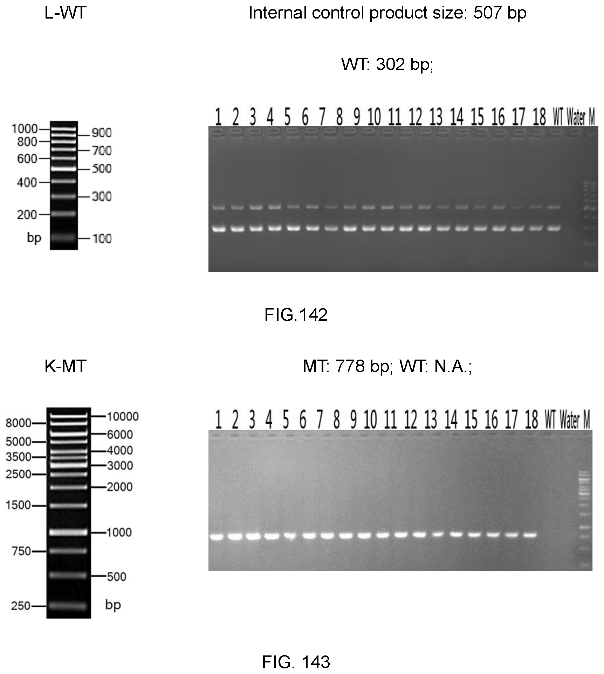

Heavy-chain and Kappa light-chain double gene homozygous mice and heavy-chain and Lambda light-chain homozygous mice were mated in a ratio of 1:1, wherein the sex was not limited; and mice were gradually born after 1 month of mating, all the mice born were heavy-chain homozygous, Kappa light-chain heterozygous, and Lambda chain heterozygous mice, and the results are shown in FIGS. 139-144.

A total of 18 mice were born, numbered from 1 to 18: where heavy chain genes of all the mice were confirmed to be homozygous by identification with two pairs of H-MT and H-WT; Kappa light chain genes of all the mice were confirmed to be heterozygous by identification with two pairs of primers K-MT and K-WT; Lambda light chain genes of all the mice were also confirmed to be heterozygous by identification with two pairs of primers L-MT and L-WT, thereby confirming that all the 18 mice were mice in which heavy chain genes were homozygous, Kappa light chain genes were heterozygous, and Lambda genes were heterozygous, where internal reference primers F3R3 were added during the heavy chain gene identification process, internal reference primers were added during the Kappa gene identification process, and internal reference primers F5R5 were added during the Lambda gene identification process to confirm that there was no problem in the identification processes. The primer pairs are shown in Table 68.

**Table 68**

| Primer | Sequence (5'-3') | SEQ ID NO. |
|---|---|---|
| H-MT | VT496-F9: TCTGCTGACTCTCCTGTTACC | 140 |
| | VT496-R9: ATCTCTGTCCTTCTCAATGTGG | 141 |
| H-WT | H-WF: AGTGAGTGAATTGAGTGAATGGG | 191 |
| | H-WR: TTGGCTCCTCCATGTGTGAGC | 192 |
| | Internal reference primer F3: 5'-CATGCCAATGGTTCACTCTAAGGT-3' | 290 |
| | Internal reference primer R3: 5'-TCTCTATGTCCCAAAGTGCAGACAC-3' | 291 |
| L-MT | IGLN-PB4F: AGCTTCATTTCACTCCACTTCC | 294 |
| | IGLN-PB4R: ACACAACTCTGAGGTCACAAATGC | 295 |
| L-WT | VT462-KF2: TGTGGAGACCGTCATCTTCTCCTGC | 296 |
| | VT462-KR1: ACCCTTAGCTTCTTGTAGATGGTTG | 297 |
| | Internal reference primer F5: 5'-CTATCAGGGATACTCCTCTTTGCC-3' | 298 |
| | Internal reference primer R5: 5'-GATACAGGAATGACAAGCTCATGGT-3' | 299 |
| K-MT | IGKN-10F: GCTGGACAACGTGAGAAAGGCTG | 286 |
| | IGKN-10R: TCAAACTGATTAGGAACTGACTACC | 287 |
| K-WT | IGK3-KOF3: AGGCTCGGGTGACTACTAGG | 288 |
| | IGK3-KOR3: CCTAGCCCCAACCTGACTTC | 289 |
| | Internal reference primer F6: 5'-GCAGAAGAGGACAGATACATTCAT-3' | 292 |
| | Internal reference primer R6: 5'-CCTACTGAAGAATCTATCCCACAG-3' | 293 |

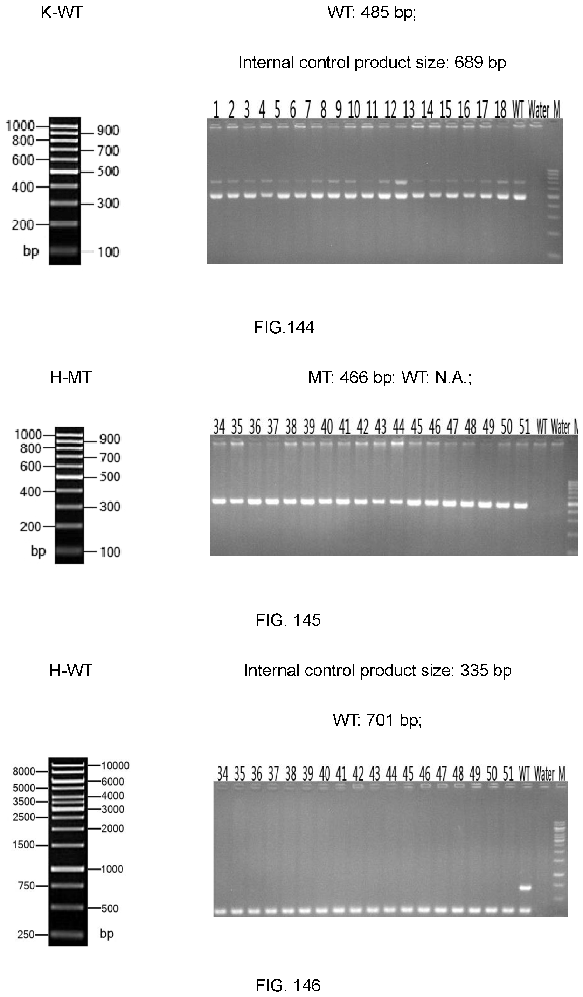

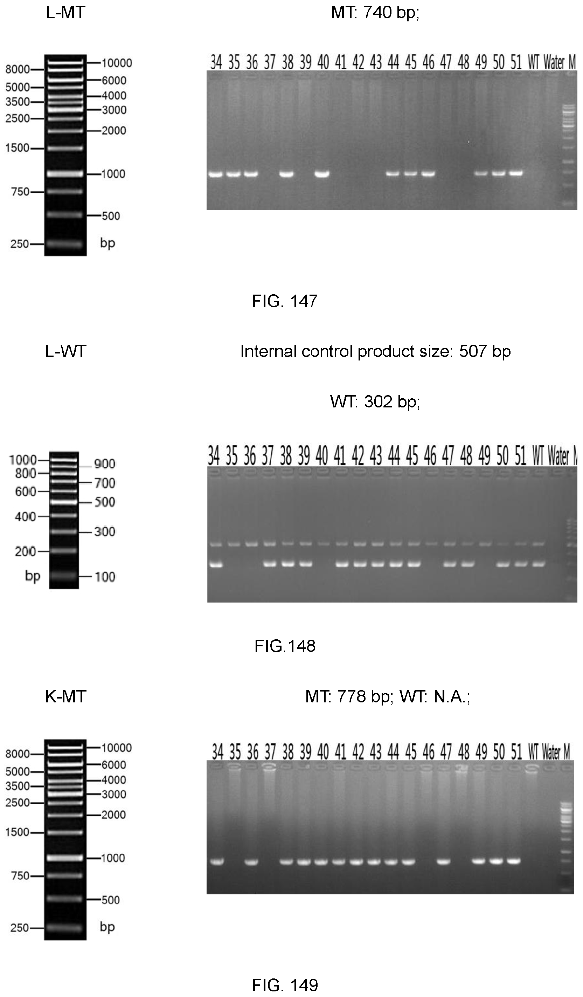

2. The heavy-chain homozygous, Kappa light-chain heterozygous and Lambda chain heterozygous mice were mated in a manner of mating 1 male mouse with 2 female mice; mice were gradually born after 1 month of mating, and when offspring mice were 5-7 days old, paws of the offspring mice were cut for genotype identification, and the results are shown in FIGS. 145 to 150, and heavy-chain, Kappa light-chain and Lambda light-chain three gene homozygotes were obtained.

A total of 18 mice were born, numbered from 34 to 51: where heavy chain genes of all the mice were confirmed to be homozygous by identification with two pairs of H-MT and H-WT; Kappa light chain genes of mice 34, 36, 39, 42, 44, 45 and 51 were confirmed to be homozygous by identification with two pairs of primers K-MT and K-WT; Lambda light chain genes of mice 35, 36, 40, 46, and 49 were confirmed to be homozygous by identification with two pairs of primers L-MT and L-WT, thereby confirming that a mouse 36 in the 18 mice was a mouse in which a heavy chain gene was homozygous, a Kappa light chain gene was homozygous, and a Lambda gene was homozygous; internal reference primers F5R5 were added during all the identification processes to confirm that there was no problem in the identification processes. The primer pairs are shown in Table 69.

**Table 69**

| Primer | Sequence (5'-3') | SEQ ID NO. |
|---|---|---|
| H-MT | VT496-F9: TCTGCTGACTCTCCTGTTACC | 140 |
| | VT496-R9: ATCTCTGTCCTTCTCAATGTGG | 141 |
| H-WT | H-WF: AGTGAGTGAATTGAGTGAATGGG | 191 |
| | H-WR: TTGGCTCCTCCATGTGTGAGC | 192 |
| | Internal reference primer F3: 5'-CATGCCAATGGTTCACTCTAAGGT-3' | 290 |
| | Internal reference primer R3: 5'-TCTCTATGTCCCAAAGTGCAGACAC-3' | 291 |
| L-MT | IGLN-PB4F: AGCTTCATTTCACTCCACTTCC | 294 |
| | IGLN-PB4R: ACACAACTCTGAGGTCACAAATGC | 295 |
| L-WT | VT462-KF2: TGTGGAGACCGTCATCTTCTCCTGC | 296 |
| | VT462-KR1: ACCCTTAGCTTCTTGTAGATGGTTG | 297 |
| | Internal reference primer F5: 5'-CTATCAGGGATACTCCTCTTTGCC-3' | 298 |
| | Internal reference primer R5: 5'-GATACAGGAATGACAAGCTCATGGT-3' | 299 |
| K-MT | IGKN-10F: GCTGGACAACGTGAGAAAGGCTG | 286 |
| | IGKN-10R: TCAAACTGATTAGGAACTGACTACC | 287 |
| K-WT | IGK3-KOF3: AGGCTCGGGTGACTACTAGG | 288 |
| | IGK3-KOR3: CCTAGCCCCAACCTGACTTC | 289 |
| | Internal reference primer F6: 5'-GCAGAAGAGGACAGATACATTCAT-3' | 292 |
| | Internal reference primer R6: 5'-CCTACTGAAGAATCTATCCCACAG-3' | 293 |

3. The proportion of the double gene homozygous mice in breeding pairs was counted, and the results are shown in Table 70 below. The results showed that the three gene homozygotes were born at a ratio of about 1/16, and the results were consistent with the Mendelian ratio.

**Table 70**

| Breeding pair | Number of births | Number of double gene homozygous mice |
|---|---|---|
| Breeding pair 1 | 5 | 2 |
| Breeding pair 2 | 16 | 1 |
| Breeding pair 3 | 17 | 1 |
| Breeding pair 4 | 15 | 0 |
| Breeding pair 5 | 12 | 0 |
| Breeding pair 6 | 13 | 0 |
| Breeding pair 7 | 10 | 1 |
| Breeding pair 8 | 15 | 1 |
| Breeding pair 9 | 12 | 1 |
| Breeding pair 10 | 7 | 1 |
| Breeding pair 11 | 9 | 2 |
| Breeding pair 12 | 12 | 0 |
| Breeding pair 13 | 11 | 1 |
| Breeding pair 14 | 17 | 0 |
| Breeding pair 15 | 10 | 1 |
| Breeding pair 16 | 13 | 1 |
| Breeding pair 17 | 12 | 0 |
| Breeding pair 18 | 9 | 0 |
| Breeding pair 19 | 12 | 0 |
| Breeding pair 20 | 16 | 2 |

3. Breeding performance statistics of heavy-chain, Kappa light-chain and Lambda light-chain three gene homozygous mice.

Three gene homozygous mice were mated in a ratio of mating 1 male mouse with 1 female mouse, and the birth of mice was counted one month after a cage was closed. The results are shown in Table 71, there were a total of 24 breeding pairs, 20 breeding pairs gave birth to mice after 1 month of mating, the effective breeding pairs reached 83.3%, while the mice had an average birth of 5.95, which was consistent with the number of births of wild type mice. It was confirmed that the breeding performance of mice of this genotype was normal.

**Table 71**

| Breeding pair | Number of births | Breeding pair | Number of births |
|---|---|---|---|
| Breeding pair 1 | 5 | Breeding pair 13 | 7 |
| Breeding pair 2 | 7 | Breeding pair 14 | 7 |
| Breeding pair 3 | 6 | Breeding pair 15 | 8 |
| Breeding pair 4 | 0 | Breeding pair 16 | 5 |
| Breeding pair 5 | 3 | Breeding pair 17 | 6 |
| Breeding pair 6 | 8 | Breeding pair 18 | 4 |
| Breeding pair 7 | 6 | Breeding pair 19 | 0 |
| Breeding pair 8 | 4 | Breeding pair 20 | 6 |
| Breeding pair 9 | 5 | Breeding pair 21 | 0 |
| Breeding pair 10 | 0 | Breeding pair 22 | 6 |
| Breeding pair 11 | 6 | Breeding pair 23 | 8 |
| Breeding pair 12 | 5 | Breeding pair 24 | 7 |

### Example 7. Immunization of mice capable of generating fully human antibodies

1. In order to generate a humoral immune response against PD-L1 in mice capable of generating fully human antibodies, three mice capable of generating fully human antibodies HKL-10, HKL-47, and HKL-49 were selected for immunization. Two C57BL/6N wild type mice were used as a control. The mice were immunized for the first time by subcutaneous injection with a complete Freund's adjuvant (CFA) uniformly mixed with 0.05 mg of PD-L1 His (Kactus Biosystems, PDL-HM110) protein, followed by subcutaneous injection with an incomplete Freund's adjuvant (IFA) uniformly mixed with 0.025 mg of PD-L1 His protein, immunization was performed once every other 2 weeks, and a total of four immunizations were performed, so that antigen-specific antibodies were generated in the the mice capable of generating fully human antibodies. The Immunization periodic table is shown in Table 72 below.

**Table 72. Immunization cycle schedule**

| | Immunizing dose | Immunization method |
|---|---|---|
| One week before immunization | blood collection&titer test | |
| First immunization | 50 µg/mounse PD-L1-His protein+CFA | Multi-point subcutaneous injection |
| Second immunization | 25 µg/mouse PD-L1-His protein+IFA | Multi-point subcutaneous injection |
| One week after immunization | blood collection&titer test | |
| Third immunization | 25 µg/mouse PD-L1-His protein+IFA | Multi-point subcutaneous injection |
| One week after immunization | blood collection&titer test | |
| Fourth immunization | 25 µg/mouse PD-L1-His protein+IFA | Multi-point subcutaneous injection |
| One week after immunization | blood collection&titer test | |
| Rush immunization | 20 µg/mouse PD-L1-His protein | Intraperitoneal injection |
| After three days | Take B cells | |

Sera from the mice capable of generating fully human antibodies after the second, third and fourth immunizations were respectively taken for serum titer monitoring.

2 µg/ml of antigen was coated overnight with CBS (a carbonate buffer solution).

After washing with PBST (phosphate buffered saline), blocking was performed with 2% BSA at 37°C for 2 h.

After washing with PBST, blocking was performed with serum dilutions (fold dilutions starting from 1:2000, 8 gradients) at 37°C for 2 h.

After washing with PBST, a horseradish peroxidase-labeled goat anti-mouse secondary antibody (diluted in 1:10000 in PBS) was added, and incubation was performed at 37°C for 1 h.

After washing with PBST, a TMB substrate solution (Beyotime, P0209-500ml) was added for color development at 37°C for 5-10 min, and the reaction was stopped by addition of a stop solution, and a serum titer was detected by measuring the optical density at 450 nm.

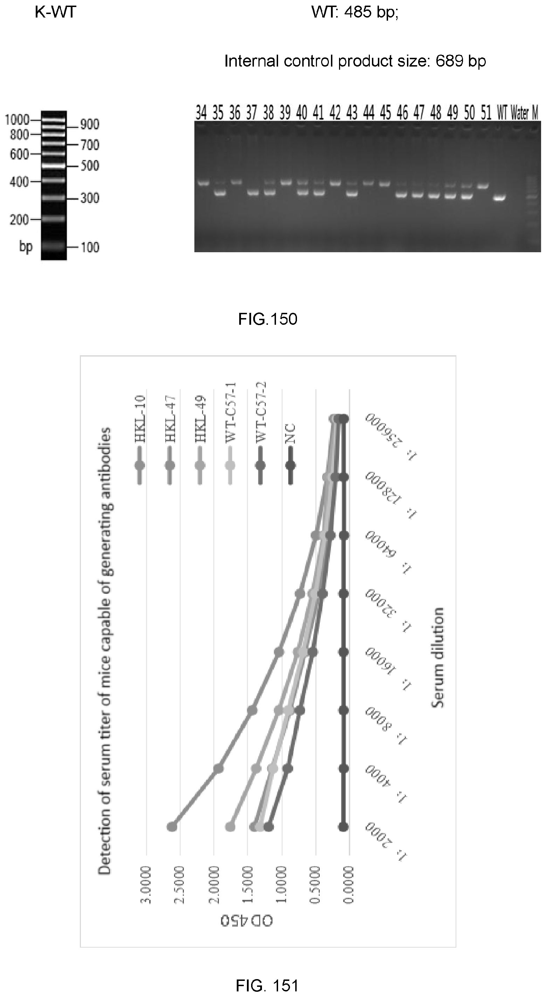

The results are shown in FIG. 151, HKL-47 mice with higher titers were selected for phage library construction.

### 2. Establishment of a phage library and sequence analysis

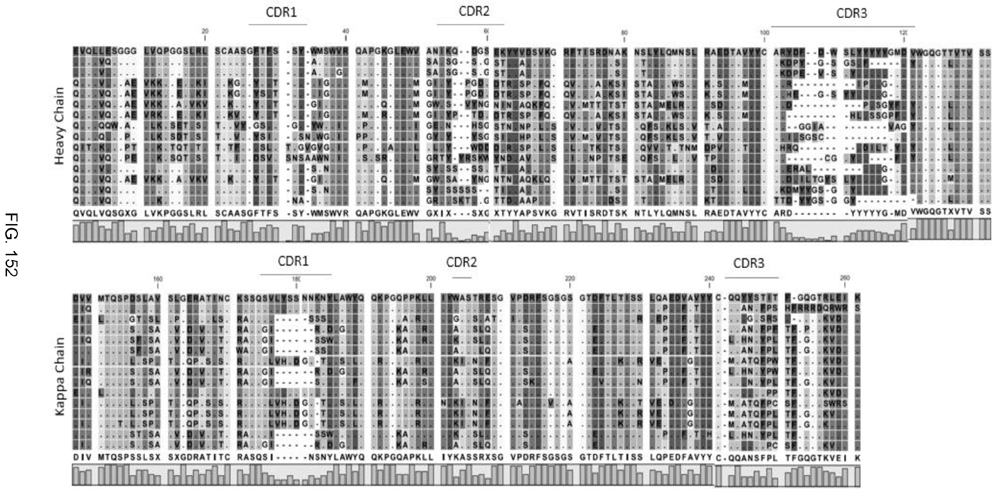

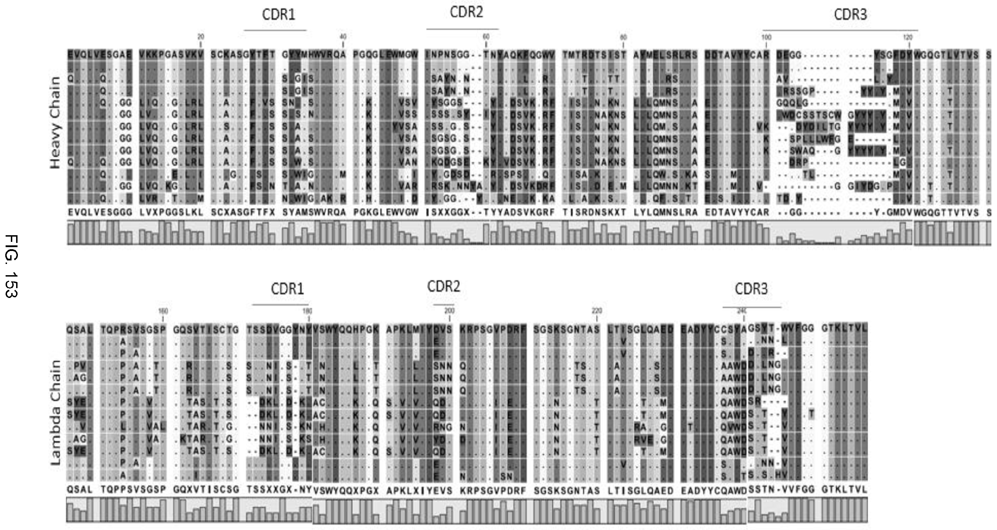

Spleens of mice with the highest serum titer after four immunizations were collected and ground to collect cells. Total RNA of spleen cells was extracted with an RNA extraction kit (Foregene, RE-03011). cDNA was synthesized and antibody VH and VL sequences were amplified by nested PCR, a vector pComb3XSS (EDITGENE, 63890) and a target fragment were separately recovered by Sfil digestion, a ligation product was electrotransformed into TG1 competent cells, a PD-L1 scFv (VH-V Kappa, VH-V Lambda) antibody library was constructed. Clones were randomly picked from a titer plate for determining the number of library transformants for sequencing identification and the results are shown in FIGS. 152 and 153, indicating that the antibody sequences were all human antibody sequences and the sequence diversity was rich.

## Claims

1. A method for preparing genetically modified mice, comprising:
(i) obtaining heavy chain locus modified mice, comprising
(ia) inserting a first partial segment of a human immunoglobulin heavy chain variable region locus between an mIgHJ region and an mIgHC region of an immunoglobulin heavy chain locus of a first mouse, wherein the first partial segment comprises a first partial hIgHV contiguous segment, a whole hIgHD segment and a whole hIgHJ segment, the first partial segment does not comprise a segment between downstream of an hIgHV1-2 gene and upstream of an hIgHV6-1 gene, and first recombination sites are comprised upstream of the first partial segment and downstream of the mIgHJ region;
(ib) inserting a second partial segment of the human immunoglobulin heavy chain variable region locus between an mIgHJ region and an mIgHC region of an immunoglobulin heavy chain locus of a second mouse, wherein the second partial segment is located upstream of the first partial segment, the second partial segment comprises a second partial hIgHV contiguous segment, and second recombination sites are comprised between downstream of the second partial hIgHV contiguous segment and upstream of the mIgHC region;
(ic) hybridizing the first mouse with the second mouse, screening a third mouse, and inserting the second partial segment of the human immunoglobulin heavy chain variable region locus and the first partial segment downstream of the second partial segment between an mIgHJ region and an mIgHC region of an immunoglobulin heavy chain locus of the third mouse, wherein third recombination sites are present between the second partial segment and the first partial segment; and
(id) respectively knocking out an mIgHV5-1 gene and a whole mIgHV segment upstream of the mIgHV5-1 gene, a contiguous segment between an mIgHD1-1 gene and an mIgHJ4 gene, and mIgHD3-1, mIgHD5-1, and mIgHD1-3 genes of the immunoglobulin heavy chain locus of the third mouse to obtain the heavy chain locus genetically modified mice;
(ii) obtaining Kappa light chain locus modified mice, wherein immunoglobulin Kappa light chain loci of the Kappa light chain locus modified mice sequentially comprise a whole hIgKV segment, a whole hIgKJ segment and an mIgKC segment, and a whole mIgKV segment and a whole mIgKJ segment are knocked out;
(iii) obtaining Lambda light chain locus modified mice, wherein immunoglobulin Lambda light chain loci of the Lambda light chain locus modified mice sequentially comprise a whole hIgLV segment and a whole hIgLJ-C segment, and a whole mIgLV segment, an mIgLJ segment, and a whole mIgLC segment are knocked out; and
(iv) mating the modified mice obtained in the steps (i), (ii) and (iii), screening triple positive mice, mating the obtained male and female triple positive mice with each other, and screening homozygous mice, wherein preferably, the modified mice obtained in the steps (i) and (ii) are mated and screening is performed to obtain first double positive homozygous mice, and the modified mice obtained in the steps (i) and (iii) are mated and screening is performed to obtain second double positive homozygous mice, and the first double positive homozygous mice and the second double positive homozygous mice are mated with each other and screening is performed to obtain triple positive homozygous mice.

2. The method according to claim 1, wherein the step (iii) comprises:
(iiia) inserting a first partial segment of a human immunoglobulin Lambda light chain locus between mIgLC1 and mIgL Enhancer 3-1 of an immunoglobulin Lambda light chain locus of a seventh mouse, wherein the first partial segment comprises a first partial hIgLV contiguous segment, a whole hIgLJ segment, and a whole hIgLC segment, and seventh recombination sites are comprised upstream of the first partial segment and downstream of mIgLC1;
(iiib) inserting a second partial segment of the human immunoglobulin Lambda light chain locus between mIgLC1 and mIgL Enhancer 3-1 of an immunoglobulin Lambda light chain locus of an eighth mouse, wherein the second partial segment is located upstream of the first partial segment on the human immunoglobulin Lambda light chain locus, the second partial segment comprises a second partial hIgLV contiguous segment, the first partial hIgLV contiguous segment and the second partial hIgLV contiguous segment together constitute the whole hIgLV segment, and eighth recombination sites are comprised between downstream of the second partial hIgLV contiguous segment and upstream of mIgL Enhancer 3-1;
(iiic) hybridizing the seventh mouse with the eighth mouse, screening a ninth mouse, and inserting the second partial segment of the human immunoglobulin Lambda light chain locus and the first partial segment downstream of the second partial segment between mIgLC1 and mIgL Enhancer 3-1 of an immunoglobulin Lambda light chain locus of the ninth mouse, wherein ninth recombination sites are present between the second partial segment and the first partial segment; and
(iiid) knocking out a whole mIgLV segment, a whole mIgLJ segment, and a whole mIgLC segment of the immunoglobulin Lambda light chain locus of the ninth mouse to obtain the Lambda light chain locus modified mice.

3. The method according to claim 2, having one or more of the following features:
(a) the first partial hIgLV contiguous segment comprises a contiguous segment between an hIgLV3-1 gene and an hIgLV2-34 gene; and the second partial hIgLV contiguous segment comprises a contiguous segment between an hIgLVI-70 gene and an hIgLV7-35 gene; and
(b) the seventh recombination sites, the eighth recombination sites, and the ninth recombination sites are loxP sites.

4. The method according to claim 2, wherein the step (iiia) comprises:
(iiia1) introducing loxP and lox5171-PB5' between mIgLC1 and mIgL Enhancer 3-1;
(iiia2) recombining a BAC vector comprising the first partial segment of the human immunoglobulin Lambda light chain locus, and an loxP site and an lox5171 site which are located at both ends of the first partial segment and in the same direction as that in the step (iiia1), and a Cre recombinase with a genome obtained in the step (iiia1), wherein PB3' is comprised between the lox5171 site and the first partial segment; and
(iiia3) contacting a recombinant genome obtained in the step (iiia2) with a PiggyBac transposase, and screening a genome comprising the first partial segment and the loxP site located upstream of the first partial segment.

5. The method according to claim 2, wherein the step (iiib) comprises:
(iiib1) inserting a contiguous segment comprised between an hIgLV4-69 gene and an hIgLV10-67 gene between the mIgLC1 and the mIgL Enhancer 3-1;
(iiib2) inserting a contiguous segment comprised between an hIGLVIV-66-1 and an hIGLVIV-65 gene, and PB3'-lox2272 and loxP sites downstream of the inserted segment in the step (iiib1);
(iiib3) recombining a BAC vector comprising a contiguous segment between an hIGLV7-35 and an hIGLVI-63 gene, and loxP and lox2272 which are located at both ends and in the same direction as that in the step (iiib2), and a Cre recombinase with a genome obtained in the step (iiib2), wherein PB5' is comprised between the lox2272 site and the contiguous segment described in this step; and
(iiib4) contacting a recombinant genome obtained in the step (iiib3) with a PiggyBac transposase, and screening a genome comprising the second partial segment and the loxP site located downstream of the second partial segment.

6. The method according to claim 2, wherein the step (iiic) comprises:
(iiic1) screening Cre-positive mice comprising the first partial segment and the second partial segment; and
(iiic2) mating the mice obtained in the step (iiic1) with wild type mice, and screening mice that do not carry Cre but comprise the first partial segment and the second partial segment, wherein loxP sites are present between the second partial segment and the first partial segment.

7. The method according to claim 2, wherein the step (iiid) comprises:
(iiid1) mating the ninth mouse with a wild type mouse, and screening positive mice; and
(iiid2) mating the male and female positive mice obtained in the step (iiid1) with each other, and screening homozygous mice.

8. The method according to claim 1, wherein the step (ii) comprises:
(iia) inserting a first partial segment of a human immunoglobulin Kappa light chain variable region locus between an mIgKJ region and an mIgKC region of an immunoglobulin Kappa light chain locus of a fourth mouse, wherein the first partial segment comprises a first partial hIgKV contiguous segment and a whole hIgKJ segment, and fourth recombination sites are comprised upstream of the first partial segment and downstream of the mIgKJ region;
(iib) inserting a second partial segment of the human immunoglobulin Kappa light chain variable region locus between an mIgKJ region and an mIgKC region of an immunoglobulin Kappa light chain locus of a fifth mouse, wherein the second partial segment is located upstream of the first partial segment, the second partial segment comprises a second partial hIgKV contiguous segment, the first partial hIgKV contiguous segment and the second partial hIgKV contiguous segment together constitute the whole hIgKV segment, and fifth recombination sites are comprised between downstream of the second partial hIgKV contiguous segment and upstream of the mIgKC region;
(iic) hybridizing the fourth mouse with the fifth mouse, screening a sixth mouse, and inserting the second partial segment of the human immunoglobulin Kappa light chain variable region locus and the first partial segment downstream of the second partial segment between an mIgKJ region and an mIgKC region of an immunoglobulin Kappa light chain locus of the sixth mouse, wherein sixth recombination sites are present between the second partial segment and the first partial segment; and
(iid) knocking out a whole mIgKV segment and a whole mIgKJ segment of the immunoglobulin Kappa light chain locus of the sixth mouse to obtain the Kappa light chain locus modified mice .

9. The method according to claim 8, having one or more of the following features:
(a) the first partial hIgKV contiguous segment of the first partial segment comprises a contiguous segment between an hIgKV4-1 gene and an hIgKV1-37 gene;
(b) the second partial hIgKV contiguous segment of the second partial segment comprises a contiguous segment between an hIgKV3D-7 gene and an hIgKV2D-40 gene; and
(c) the fourth recombination sites, the fifth recombination sites, and the sixth recombination sites are loxP sites.

10. The method according to claim 8, wherein in the step (iia), the first partial hIgKV contiguous segment and the whole hIgKJ segment of the first partial segment are inserted between the mIgKJ region and the mIgKC region by at least two steps comprising:
(iia1) inserting a first contiguous segment comprised between an hIgKV1-5 gene and an hIgKJ5 gene between the mIgKJ region and the mIgKC region; and
(iia2) inserting a second contiguous segment comprised between an hIgKV1-37 and an hIgKV1-6 gene upstream of the first contiguous segment.

11. The method according to claim 10, wherein the step (iia1) specifically comprises:
(iia11) inserting a contiguous segment comprised between an hIgKJ1 gene and the hIgKJ5 gene between the mIgKJ region and the mIgKC region;
(iia12) inserting a contiguous segment comprised between an hIgKV4-1 gene and an hIgKV5-2 gene upstream of the inserted segment in the step (iia11);
(iia13) inserting genes in a contiguous segment comprised between upstream of hIgKV5-2 and downstream of hIgHKV2-4 upstream of the inserted segment in the step (iia12); and
(iia14) inserting a contiguous segment comprised between an hIgHKV2-4 gene and an hIgKV1-5 gene, and loxP and lox2272-PB5' sites upstream of the inserted segment in the step (iia13).

12. The method according to claim 10, wherein the step (iia2) specifically comprises:
(iia21) recombining a BAC vector comprising the second contiguous segment between the hIgKV1-6 gene and the hIgKV1-37 gene, and an loxP site and an lox2272 site which are located at both ends of the second contiguous segment and in the same direction as that in the step (iia14), and a Cre recombinase with a genome obtained in step (iia1), wherein PB3' is comprised between the lox2272 site and the second contiguous segment; and
(iia22) contacting a recombinant genome obtained in the step (iia21) with a PiggyBac transposase, and screening a genome comprising the first partial segment and the loxP site located upstream of the first partial segment.

13. The method according to any one of claims 8 to 12, wherein in the step (iib), the second partial hIgKV contiguous segment of the second partial segment is inserted upstream of the first partial segment by at least the following steps comprising:
(iib1) inserting a contiguous segment comprised between the hIgKV2D-40 gene and an hIgKV2D-38 gene between the mIgKJ region and the mIgKC region;
(iib2) inserting a contiguous segment comprised between an hIgKV1D-37 and an hIgKV1D-35 gene, and PB3'-lox5171 and loxP sites downstream of the inserted segment in the step (iib1);
(iib3) recombining a BAC vector comprising a contiguous segment between an hIgKV3D-34 gene and the hIgKV3D-7 gene, and loxP and lox5171 which are located at both ends and in the same direction as that in the step (iib2), and a Cre recombinase with a genome obtained in the step (iib2), wherein PB5' is comprised between the lox5171 site and the contiguous segment described in this step; and
(iib4) contacting a recombinant genome obtained in the step (iib3) with a PiggyBac transposase, and screening a genome comprising the second partial segment and the loxP site located downstream of the second partial segment.

14. The method according to claim 13, wherein the step (iic) comprises:
(iic1) screening Cre-positive mice comprising the first partial segment and the second partial segment; and
(iic2) mating the mice obtained in the step (iic1) with wild type mice, and screening mice that do not carry Cre but comprise the first partial segment and the second partial segment, wherein loxP sites are present between the second partial segment and the first partial segment.

15. The method according to claim 8, wherein the step (iid) comprises:
(iid1) mating the sixth mouse with a wild type mouse, and screening positive mice; and
(iid2) mating the male and female positive mice obtained in the step (iid1) with each other, and screening homozygous mice.

16. The method according to claim 1, having one or more of the following features:
(a) the first partial hIgHV contiguous segment of the first partial segment comprises a contiguous segment between an hIgHV4-28 gene and the hIgHV1-2 gene;
(b) the second partial hIgHV contiguous segment of the second partial segment comprises a contiguous segment between an hIgHV3-74 gene and an hIgHV3-30 gene; and
(c) the first recombination sites, the second recombination sites, and the third recombination sites are loxP sites.

17. The method according to claim 1, wherein immunoglobulin heavy chain loci of the genetically modified mice sequentially comprise: (i) an Adam6a gene of the mice; (ii) an Adam6b gene of the mice; (iii) a contiguous segment between an hIgHV3-74 gene and an hIgHV3-30 gene; (iv) a contiguous segment between an hIgHV4-28 gene and the hIgHV1-2 gene; (v) a contiguous segment between the hIgHV6-1 gene and an hIgHJ6 gene; and (vi) an mIgHC region.

18. The method according to claim 1, wherein in the step (ia), the first partial hIgHV contiguous segment, the whole hIgHD segment and the whole hIgHJ segment of the first partial segment are inserted between the mIgHJ region and the mIgHC region by at least two steps comprising:
(ia1) inserting a first contiguous segment comprised between the hIgHV6-1 gene and an hIgHJ6 gene between the mIgHJ region and the mIgHC region; and
(ia2) inserting a second contiguous segment comprised between an hIgHV4-28 gene and the hIgHV1-2 gene upstream of the first contiguous segment.

19. The method according to claim 18, wherein the step (ia1) specifically comprises:
(ia11) inserting a contiguous segment comprised between an hIgHD6-25 gene and the hIgHJ6 gene between the mIgHJ region and the mIgHC region;
(ia12) inserting a contiguous segment comprised between an hIgHD6-13 gene and an hIgHD5-24 gene upstream of the inserted segment in the step (ia11);
(ia13) inserting a contiguous segment comprised between an hIgHD1-1 gene and an hIgHD5-12 gene upstream of the inserted segment in the step (ia12); and
(ia14) inserting a contiguous segment comprised between the hIgHV6-1 gene and upstream of the hIgHD1-1 gene, and loxP and lox2272-PB5' sites upstream of the inserted segment in the step (ia13).

20. The method according to claim 18, wherein the step (ia2) specifically comprises:
(ia21) recombining a BAC vector comprising the second contiguous segment between the hIgHV4-28 gene and the hIgHV1-2 gene, and an loxP site and an lox2272 site which are located at both ends of the second contiguous segment and in the same direction as that in the step (ia14), and a Cre recombinase with a genome obtained in the step (ia1), wherein PB3' is comprised between the lox2272 site and the second contiguous segment; and
(ia22) contacting a recombinant genome obtained in the step (ia21) with a PiggyBac transposase, and screening a genome comprising the first partial segment and the loxP site located upstream of the first partial segment.

21. The method according to any one of claims 16 to 20, wherein in the step (ib), the second partial hIgHV contiguous segment of the second partial segment is inserted upstream of the first partial segment by at least the following steps comprising:
(ib1) inserting a contiguous segment comprised between the hIgHV3-74 gene and an hIgHV3-72 gene between the mIgHJ region and the mIgHC region;
(ib2) inserting a contiguous segment comprised between an hIgHV2-70 gene and an hIgHV1-69D gene, and PB3'-lox5171 and loxP sites downstream of the inserted segment in the step (ib1);
(ib3) recombining a BAC vector comprising a contiguous segment between an hIgHV1-69-2 gene and the hIgHV3-30 gene, and loxP and lox5171 which are located at both ends and in the same direction as that in the step (ib2), and a Cre recombinase with a genome obtained in the step (ib2), wherein PB5' is comprised between the lox5171 site and the contiguous segment described in this step; and
(ib4) contacting a recombinant genome obtained in the step (ib3) with a PiggyBac transposase, and screening a genome comprising the second partial segment and the loxP site located downstream of the second partial segment.

22. The method according to claim 21, wherein the step (ic) comprises:
(ic1) screening Cre-positive mice comprising the first partial segment and the second partial segment; and
(ic2) mating the mice obtained in the step (ic1) with wild type mice, and screening mice that do not carry Cre but comprise the first partial segment and the second partial segment, wherein loxP sites are present between the second partial segment and the first partial segment.

23. The method according to claim 1, wherein the step (id) comprises:
(id1) mating the third mouse with a wild type mouse, and screening positive mice; and
(id2) mating the male and female positive mice obtained in the step (id1) with each other, and screening homozygous mice.

24. A genetically modified mouse genome, wherein
(a) immunoglobulin heavy chain loci of genetically-modified mice sequentially comprise: (i) an Adam6a gene of the mice; (ii) an Adam6b gene of the mice; (iii) a second partial segment of a human immunoglobulin heavy chain variable region locus, comprising a second partial hIgHV contiguous segment; (iv) a first partial segment of the human immunoglobulin heavy chain variable region locus, located downstream of the second partial segment and comprising a first partial hIgHV contiguous segment, a whole hIgHD segment and a whole hIgHJ segment, the first partial segment not comprising a segment between downstream of an hIgHV1-2 gene and upstream of an hIgHV6-1 gene; and (v) an mIgHC region;
(b) immunoglobulin Kappa light chain loci of the genetically modified mice sequentially comprise: (i) a second partial segment of a human immunoglobulin Kappa light chain variable region locus, comprising a second partial hIgKV contiguous segment; (ii) a first partial segment of the human immunoglobulin Kappa light chain variable region locus, located downstream of the second partial segment and comprising a first partial hIgKV contiguous segment and a wholel hIgHJ segment, the first partial segment and the second partial segment together constituting a whole hIgKV segment; and (iii) an mIgKC region; and
(c) immunoglobulin Lambda light chain loci of the genetically modified mice sequentially comprise: (i) a second partial segment of a human immunoglobulin Lambda light chain locus, comprising a second partial hIgLV contiguous segment; and (ii) a first partial segment of the human immunoglobulin Lambda light chain locus, located downstream of the second partial segment and comprising a first partial hIgLV contiguous segment and a whole hIgHJ-C segment, the first partial segment and the second partial segment together constituting a whole hIgLV segment.

25. The mouse genome according to claim 24, having one or more of the following features:
(i) the second partial hIgHV contiguous segment of the human immunoglobulin heavy chain variable region locus comprises or is a contiguous segment between an hIgHV3-74 gene and an hIgHV3-30 gene;
(ii) the first partial segment of the human immunoglobulin heavy chain variable region locus comprises or is a contiguous segment between an hIgHV4-28 gene and the hIgHV1-2 gene and a contiguous segment between the hIgHV6-1 gene and an hIgHJ6 gene located downstream;
(iii) the immunoglobulin heavy chain loci of the mice do not comprise an mIgHV5-1 gene and a whole mIgHV segment upstream of the mIgHV5-1 gene, a contiguous segment between an mIgHD1-1 gene and an mIgHJ4 gene, and mIgHD3-1, mIgHD5-1, and mIgHD1-3 genes;
(iv) the second partial hIgKV contiguous segment comprises or is a contiguous segment between an hIgKV3D-7 gene and an hIgKV2D-40 gene;
(v) the first partial hIgKV contiguous segment comprises or is a contiguous segment between an hIgK1-37 gene and an hIgKJ5 gene;
(vi) the second partial hIgLV contiguous segment comprises or is a contiguous segment between an hIgLVI-70 gene and an hIgLV7-35 gene;
(vii) the first partial hIgLV contiguous segment comprises or is a contiguous segment between an hIgLV3-1 gene and an hIgLV2-34 gene;
(viii) the fertility of the mice is not decreased compared with non-genetically modified wild type mice;
(ix) the first partial segment and the second partial segment of a heavy chain, a Kappa light chain and a Lambda light chain are not rearranged; and
(x) the mice can produce a human-mouse chimeric antibody comprising a human heavy chain variable region and a mouse heavy chain constant region and a human Kappa light chain variable region and a mouse Kappa light chain constant region, or comprising a human heavy chain variable region and a mouse heavy chain constant region and a human Lambda light chain variable region and a human Lambda light chain constant region.

26. A cell, tissue, organ, or mouse comprising the mouse genome according to claim 24 or 25, wherein preferably, the cell is an embryonic cell, a B cell or a hybridoma cell; preferably, the tissue is a white pulp of a spleen or lymphoid nodules thereof; and preferably, the organ is a spleen.

27. A method for preparing a monoclonal antibody, comprising:
(a) immunizing mice having the genome according to claim 24 or 25 with an antigen;
(b) isolating cells producing and comprising a monoclonal antibody against the antigen from the mice; and
(c) culturing the cells to obtain the monoclonal antibody;
wherein preferably, the cells are splenocytes, B cells or hybridoma cells; preferably, the monoclonal antibody has a human heavy chain variable region, a human Kappa light chain variable region, a mouse heavy chain constant region, and a mouse Kappa light chain constant region, or the monoclonal antibody has a human heavy chain variable region, a human Lambda light chain variable region, a mouse heavy chain constant region, and a human Lambda light chain constant region, and does not have a mouse heavy chain variable region, a mouse Kappa light chain variable region, and a mouse Lambda light chain constant region.

28. Use of the cell, tissue, organ or mouse according to claim 27 in the preparation of a monoclonal antibody, wherein preferably, the monoclonal antibody:
(i) has a human heavy chain variable region, and a human Kappa light chain variable region, and does not have a mouse heavy chain variable region, and a mouse Kappa light chain variable region;
(ii) has a human heavy chain variable region, and a human Lambda light chain variable region, and does not have a mouse heavy chain variable region, and a mouse Lambda light chain variable region;
(iii) has a human heavy chain variable region, and a human Kappa light chain variable region, a mouse heavy chain constant region and a mouse Kappa light chain constant region; or
(iii) has a human heavy chain variable region, a human Lambda light chain variable region, a mouse heavy chain constant region and a human Lambda light chain constant region.
